# EUROPEAN PATENT SPECIFICATION

(11) **EP 1 472 349 B1**
(45) Date of publication and mention of the grant of the patent: **29.10.2008**
(21) Application number: 03731662.7
(22) Date of filing: 24.01.2003
(51) Int. Cl.: C12N 15/10, C12Q 1/68

(54) **METHODS FOR MULTIPLE PARAMETER SCREENING AND EVOLUTION OF CELLS TO PRODUCE SMALL MOLECULES WITH MULTIPLE FUNCTIONALITIES**
SCREENINGMETHODEN NACH MEHREREN PARAMETERN UND ENTWICKLUNG VON ZELLEN, UM KLEINE MOLEKÜLE MIT MEHREREN FUNKTIONALITÄTEN ZU PRODUZIEREN
METHODES PERMETTANT DE FAIRE EVOLUER ET DE CRIBLER DES PARAMETRES MULTIPLES DE CELLULES AFIN DE PRODUIRE DES PETITES MOLECULES A FONCTIONNALITES MULTIPLES

(30) Priority: 25.01.2002 WO PCT/DK02/00057; 01.08.2002 DK 200201174
(43) Date of publication of application: 03.11.2004
(73) Proprietor: Evolva AG, 4123 Allschwil (CH)
(72) Inventor: GOLDSMITH, Neil, Oxford Oxfordshire OX4 1NX (GB); NIELSEN, Soeren, V., S., DK-3450 Alleroed (DK); SOERENSEN, Alexandra, M., P., Santana, DK-2840 Holte (DK); NIELSEN, Curt, Aimé, Friis, DK-5250 Odense SV (DK)
(74) Representative: HOEIBERG A/S
(86) International application number: PCT/DK2003/000044
(87) International publication number: WO 2003/062419

(56) References cited:
- WO-A-01/73000
- WO-A-96/34112
- WO-A-98/17811
- WO-A-98/58085
- WO-A-02/059290
- US-A- 6 025 155
- GIFT ELIZABETH ET AL: "FACS-based isolation of slowly growing cells: Double encapsulation of yeast in gel microdrops." NATURE BIOTECHNOLOGY, vol. 14, no. 7, 1996, pages 884-887, XP001147516 ISSN: 1087-0156 cited in the application

### SEQUENCE LISTING

<110> Evolva Biotech AS
   Goldsmith, Neil
   Nielsen, Curt
   Sørensen, Alexandra M. P.
   Nielsen, Søren V.S.
<120> Methods for multiple parameter screening and evolution of cells to produce small molecules with multiple functionalities
<130> P 670 DK00
   <150> PCT/DK02/00057
   <151> 2002-01-25
<150> DK-PA 2002 01174
   <151> 2002-08-01
<160> 5
   <170> PatentIn version 3.1
<210> 1
   <211> 3417
   <212> DNA
   <213> Artificial sequence
<220>
   <223> Synthetic
   <220>
   <221> misc_feature
   <222> (1902)..(2759)
   <223> Ampicillin resistance gene
<220>
   <221> rep_origin
   <222> (959)..(1899)
   <223> ColE1
<220>
   <221> misc_feature
   <222> (2891)..(3347)
   <223> f1-phage origin of replication
<220>
   <221> terminator
   <222> (495)..(823)
   <223> ADH1
<220>
   <221> promoter
   <222> (49)..(437)
   <223> Met25 promoter
<400> 1
   ctgatttgcc cgggcagttc aggctcatca ggcgcgccat gcagggattc ttcggatgca     60
   agggttcgaa tcccttagct ctcattattt tttgcttttt ctcttgaggt cacatgatcg    120
   caaaatggca aatggcacgt gaagctgtcg atattgggga actgtggtgg ttggcaaatg    180
   actaattaag ttagtcaagg cgccatcctc atgaaaactg tgtaacataa taaccgaagt    240
   gtcgaaaagg tggcaccttg tccaattgaa cacgctcgat gaaaaaaata agatatatat    300
   aaggttaagt aaagcgtctg ttagaaagga agtttttcct ttttcttgct ctcttgtctt    360
   ttcatctact atttccttcg tgtaatacag ggtcgtcaga tacatagata caattctatt    420
   acccccatcc atacaagctt ggcgccgaat tcgtcgaccc ggggatccgc ggccgcaggc    480
   ctaaattgat ctagagcttt ggacttcttc gccagaggtt tggtcaagtc tccaatcaag    540
   gttgtcggct tgtctacctt gccagaaatt tacgaaaaga tggaaaaggg tcaaatcgtt    600
   ggtagatacg ttgttgacac ttctaaataa gcgaatttct tatgatttat gatttttatt    660
   attaaataag ttataaaaaa aataagtgta tacaaatttt aaagtgactc ttaggtttta 720
   aaacgaaaat tcttgttctt gagtaactct ttcctgtagg tcaggttgct ttctcaggta 780
   tagcatgagg tcgctcttat tgaccacacc tctaccggca tgccgatggg ttaactgatc 840
   aatgcatcct gcatggcgcg cctgatgagc ctgaactgcc cgggcaaatc agctggacgt 900
   ctgcctgcat taatgaatcg gccaacgcgc ggggagaggc ggtttgcgta ttgggcgctc 960
   ttccgcttcc tcgctcactg actcgctgcg ctcggtcgtt cggctgcggc gagcggtatc 1020
   agctcactca aaggcggtaa tacggttatc cacagaatca ggggataacg caggaaagaa 1080
   catgtgagca aaaggccagc aaaaggccag gaaccgtaaa aaggccgcgt tgctggcgtt 1140
   tttccatagg ctccgccccc ctgacgagca tcacaaaaat cgacgctcaa gtcagaggtg 1200
   gcgaaacccg acaggactat aaagatacca ggcgtttccc cctggaagct ccctcgtgcg 1260
   ctctcctgtt ccgaccctgc cgcttaccgg atacctgtcc gcctttctcc cttcgggaag 1320
   cgtggcgctt tctcatagct cacgctgtag gtatctcagt tcggtgtagg tcgttcgctc 1380
   caagctgggc tgtgtgcacg aaccccccgt tcagcccgac cgctgcgcct tatccggtaa 1440
   ctatcgtctt gagtccaacc cggtaagaca cgacttatcg ccactggcag cagccactgg 1500
   taacaggatt agcagagcga ggtatgtagg cggtgctaca gagttcttga agtggtggcc 1560
   taactacggc tacactagaa ggacagtatt tggtatctgc gctctgctga agccagttac 1620
   cttcggaaaa agagttggta gctcttgatc cggcaaacaa accaccgctg gtagcggtgg 1680
   tttttttgtt tgcaagcagc agattacgcg cagaaaaaaa ggatctcaag aagatccttt 1740
   gatcttttct acggggtctg acgctcagtg gaacgaaaac tcacgttaag ggattttggt 1800
   catgagatta tcaaaaagga tcttcaccta gatcctttta aattaaaaat gaagttttaa 1860
   atcaatctaa agtatatatg agtaaacttg gtctgacagt taccaatgct taatcagtga 1920
   ggcacctatc tcagcgatct gtctatttcg ttcatccata gttgcctgac tccccgtcgt 1980
   gtagataact acgatacggg agggcttacc atctggcccc agtgctgcaa tgataccgcg 2040
   agacccacgc tcaccggctc cagatttatc agcaataaac cagccagccg gaagggccga 2100
   gcgcagaagt ggtcctgcaa ctttatccgc ctccatccag tctattaatt gttgccggga 2160
   agctagagta agtagttcgc cagttaatag tttgcgcaac gttgttgcca ttgctacagg 2220
   catcgtggtg tcacgctcgt cgtttggtat ggcttcattc agctccggtt cccaacgatc 2280
   aaggcgagtt acatgatccc ccatgttgtg caaaaaagcg gttagctcct tcggtcctcc 2340
   gatcgttgtc agaagtaagt tggccgcagt gttatcactc atggttatgg cagcactgca 2400
   taattctctt actgtcatgc catccgtaag atgcttttct gtgactggtg agtactcaac 2460
   caagtcattc tgagaatagt gtatgcggcg accgagttgc tcttgcccgg cgtcaatacg 2520
   ggataatacc gcgccacata gcagaacttt aaaagtgctc atcattggaa aacgttcttc 2580
   ggggcgaaaa ctctcaagga tcttaccgct gttgagatcc agttcgatgt aacccactcg 2640
   tgcacccaac tgatcttcag catcttttac tttcaccagc gtttctgggt gagcaaaaac 2700
   aggaaggcaa aatgccgcaa aaaagggaat aagggcgaca cggaaatgtt gaatactcat 2760
   actcttcctt tttcaatatt attgaagcat ttatcagggt tattgtctca tgagcggata 2820
   catatttgaa tgtatttaga aaaataaaca aataggggtt ccgcgcacat ttccccgaaa 2880
   agtgccacct gacgcgccct gtagcggcgc attaagcgcg gcgggtgtgg tggttacgcg 2940
   cagcgtgacc gctacacttg ccagcgccct agcgcccgct cctttcgctt tcttcccttc 3000
   ctttctcgcc acgttcgccg gctttccccg tcaagctcta aatcgggggc tccctttagg 3060
   gttccgattt agtgctttac ggcacctcga ccccaaaaaa cttgattagg gtgatggttc 3120
   acgtagtggg ccatcgccct gatagacggt ttttcgccct ttgacgttgg agtccacgtt 3180
   ctttaatagt ggactcttgt tccaaactgg aacaacactc aaccctatct cggtctattc 3240
   ttttgattta taagggattt tgccgatttc ggcctattgg ttaaaaaatg agctgattta 3300
   acaaaaattt aacgcgaatt ttaacaaaat attaacgctt acaatttcca ttcgccattc 3360
   aggctgcgca actgttggga agggcgatcg gtgcgggcct cttcgctatt acgccag 3417
<210> 2
   <211> 3501
   <212> DNA
   <213> Artificial sequence
<220>
   <223> Synthetic
   <220>
   <221> misc_feature
   <222> (1986)..(2843)
   <223> Ampicillin resistance gene
<220>
   <221> rep_origin
   <222> (1043)..(1983)
   <223> ColE1
<220>
   <221> misc_feature
   <222> (2975)..(3431)
   <223> f1-phage origin of replication
<220>
   <221> terminator
   <222> (579)..(907)
   <223> ADH1
<220>
   <221> promoter
   <222> (49)..(519)
   <223> Cup1 promoter
<400> 2
   ctgatttgcc cgggcagttc aggctcatca ggcgcgccat gcagggataa gccgatccca      60
   ttaccgacat ttgggcgcta tacgtgcata tgttcatgta tgtatctgta tttaaaacac     120
   ttttgtatta tttttcctca tatatgtgta taggtttata cggatgattt aattattact     180
   tcaccaccct ttatttcagg ctgatatctt agccttgtta ctagttagaa aaagacattt     240
   ttgctgtcag tcactgtcaa gagattcttt tgctggcatt tcttctagaa gcaaaaagag     300
   cgatgcgtct tttccgctga accgttccag caaaaaagac taccaacgca atatggattg      360
   tcagaatcat ataaaagaga agcaaataac tccttgtctt gtatcaattg cattataata     420
   tcttcttgtt agtgcaatat catatagaag tcatcgaaat agatattaag aaaaacaaac     480
   tgtacaatca atcaatcaat catcacataa aatgttcaaa gcttggcgcc gaattcgtcg     540
   acccggggat ccgcggccgc aggcctaaat tgatctagag ctttggactt cttcgccaga 600
   ggtttggtca agtctccaat caaggttgtc ggcttgtcta ccttgccaga aatttacgaa 660
   aagatggaaa agggtcaaat cgttggtaga tacgttgttg acacttctaa ataagcgaat 720
   ttcttatgat ttatgatttt tattattaaa taagttataa aaaaaataag tgtatacaaa 780
   ttttaaagtg actcttaggt tttaaaacga aaattcttgt tcttgagtaa ctctttcctg 840
   taggtcaggt tgctttctca ggtatagcat gaggtcgctc ttattgacca cacctctacc 900
   ggcatgccca tgggttaact gatcaatgca tcctgcatgg cgcgcctgat gagcctgaac 960
   tgcccgggca aatcagctgg acgtctgcct gcattaatga atcggccaac gcgcggggag 1020
   aggcggtttg cgtattgggc gctcttccgc ttcctcgctc actgactcgc tgcgctcggt 1080
   cgttcggctg cggcgagcgg tatcagctca ctcaaaggcg gtaatacggt tatccacaga 1140
   atcaggggat aacgcaggaa agaacatgtg agcaaaaggc cagcaaaagg ccaggaaccg 1200
   taaaaaggcc gcgttgctgg cgtttttcca taggctccgc ccccctgacg agcatcacaa 1260
   aaatcgacgc tcaagtcaga ggtggcgaaa cccgacagga ctataaagat accaggcgtt 1320
   tccccctgga agctccctcg tgcgctctcc tgttccgacc ctgccgctta ccggatacct 1380
   gtccgccttt ctcccttcgg gaagcgtggc gctttctcat agctcacgct gtaggtatct 1440
   cagttcggtg taggtcgttc gctccaagct gggctgtgtg cacgaacccc ccgttcagcc 1500
   cgaccgctgc gccttatccg gtaactatcg tcttgagtcc aacccggtaa gacacgactt 1560
   atcgccactg gcagcagcca ctggtaacag gattagcaga gcgaggtatg taggcggtgc 1620
   tacagagttc ttgaagtggt ggcctaacta cggctacact agaaggacag tatttggtat 1680
   ctgcgctctg ctgaagccag ttaccttcgg aaaaagagtt ggtagctctt gatccggcaa 1740
   acaaaccacc gctggtagcg gtggtttttt tgtttgcaag cagcagatta cgcgcagaaa 1800
   aaaaggatct caagaagatc ctttgatctt ttctacgggg tctgacgctc agtggaacga 1860
   aaactcacgt taagggattt tggtcatgag attatcaaaa aggatcttca cctagatcct 1920
   tttaaattaa aaatgaagtt ttaaatcaat ctaaagtata tatgagtaaa cttggtctga 1980
   cagttaccaa tgcttaatca gtgaggcacc tatctcagcg atctgtctat ttcgttcatc 2040
   catagttgcc tgactccccg tcgtgtagat aactacgata cgggagggct taccatctgg 2100
   ccccagtgct gcaatgatac cgcgagaccc acgctcaccg gctccagatt tatcagcaat 2160
   aaaccagcca gccggaaggg ccgagcgcag aagtggtcct gcaactttat ccgcctccat 2220
   ccagtctatt aattgttgcc gggaagctag agtaagtagt tcgccagtta atagtttgcg 2280
   caacgttgtt gccattgcta caggcatcgt ggtgtcacgc tcgtcgtttg gtatggcttc 2340
   attcagctcc ggttcccaac gatcaaggcg agttacatga tcccccatgt tgtgcaaaaa   2400
   agcggttagc tccttcggtc ctccgatcgt tgtcagaagt aagttggccg cagtgttatc   2460
   actcatggtt atggcagcac tgcataattc tcttactgtc atgccatccg taagatgctt   2520
   ttctgtgact ggtgagtact caaccaagtc attctgagaa tagtgtatgc ggcgaccgag   2580
   ttgctcttgc ccggcgtcaa tacgggataa taccgcgcca catagcagaa ctttaaaagt   2640
   gctcatcatt ggaaaacgtt cttcggggcg aaaactctca aggatcttac cgctgttgag   2700
   atccagttcg atgtaaccca ctcgtgcacc caactgatct tcagcatctt ttactttcac   2760
   cagcgtttct gggtgagcaa aaacaggaag gcaaaatgcc gcaaaaaagg gaataagggc   2820
   gacacggaaa tgttgaatac tcatactctt cctttttcaa tattattgaa gcatttatca   2880
   gggttattgt ctcatgagcg gatacatatt tgaatgtatt tagaaaaata aacaaatagg   2940
   ggttccgcgc acatttcccc gaaaagtgcc acctgacgcg ccctgtagcg gcgcattaag   3000
   cgcggcgggt gtggtggtta cgcgcagcgt gaccgctaca cttgccagcg ccctagcgcc   3060
   cgctcctttc gctttcttcc cttcctttct cgccacgttc gccggctttc cccgtcaagc   3120
   tctaaatcgg gggctccctt tagggttccg atttagtgct ttacggcacc tcgaccccaa   3180
   aaaacttgat tagggtgatg gttcacgtag tgggccatcg ccctgataga cggtttttcg   3240
   ccctttgacg ttggagtcca cgttctttaa tagtggactc ttgttccaaa ctggaacaac   3300
   actcaaccct atctcggtct attcttttga tttataaggg attttgccga tttcggccta   3360
   ttggttaaaa aatgagctga tttaacaaaa atttaacgcg aattttaaca aaatattaac   3420
   gcttacaatt tccattcgcc attcaggctg cgcaactgtt gggaagggcg atcggtgcgg   3480
   gcctcttcgc tattacgcca g                                              3501
<210> 3
   <211> 4188
   <212> DNA
   <213> Artificial sequence
<220>
   <223> Synthetic
   <220>
   <221> misc_feature
   <222> (2673)..(3530)
   <223> Ampicillin resistance gene
<220>
   <221> rep_origin
   <222> (1730)..(2670)
   <223> ColEl
<220>
   <221> misc_feature
   <222> (3662)..(4118)
   <223> f1-phage origin of replication
<220>
   <221> terminator
   <222> (1027)..(1355)
   <223> ADH1
<220>
   <221> promoter
   <222> (582)..(969)
   <223> Met25 promoter
<220>
   <221> misc_feature
   <222> (1365)..(1603)
   <223> ARS1 (autonomous replicating sequence) for Yeast replication
<220>
   <221> misc_feature
   <222> (49)..(574)
   <223> lambda spacer DNA (22428-22923)
<400> 3
   ctgatttgcc cgggcagttc aggctcatca ggcgcgccat gcagggattc tggaaattgc 60
   aacgaaggaa gaaacctcgt tgctggaagc ctggaagaag tatcgggtgt tgctgaaccg 120
   tgttgataca tcaactgcac ctgatattga gtggcctgct gtccctgtta tggagtaatc 180
   gttttgtgat atgccgcaga aacgttgtat gaaataacgt tctgcggtta gttagtatat 240
   tgtaaagctg agtattggtt tatttggcga ttattatctt caggagaata atggaagttc 300
   tatgactcaa ttgttcatag tgtttacatc accgccaatt gcttttaaga ctgaacgcat 360
   gaaatatggt ttttcgtcat gttttgagtc tgctgttgat atttctaaag tcggtttttt 420
   ttcttcgttt tctctaacta ttttccatga aatacatttt tgattattat ttgaatcaat 480
   tccaattacc tgaagtcttt catctataat tggcattgta tgtattggtt tattggagta 540
   gatgcttgct tttctgagcc atagctctga tatcagatct tcttcggatg caagggttcg 600
   aatcccttag ctctcattat tttttgcttt ttctcttgag gtcacatgat cgcaaaatgg 660
   caaatggcac gtgaagctgt cgatattggg gaactgtggt ggttggcaaa tgactaatta 720
   agttagtcaa ggcgccatcc tcatgaaaac tgtgtaacat aataaccgaa gtgtcgaaaa 780
   ggtggcacct tgtccaattg aacacgctcg atgaaaaaaa taagatatat ataaggttaa 840
   gtaaagcgtc tgttagaaag gaagtttttc ctttttcttg ctctcttgtc ttttcatcta 900
   ctatttcctt cgtgtaatac agggtcgtca gatacataga tacaattcta ttacccccat 960
   ccatacaagc ttggcgccga attcgtcgac ccggggatcc gcggccgcag gcctaaattg 1020
   atctagagct ttggacttct tcgccagagg tttggtcaag tctccaatca aggttgtcgg 1080
   cttgtctacc ttgccagaaa tttacgaaaa gatggaaaag ggtcaaatcg ttggtagata 1140
   cgttgttgac acttctaaat aagcgaattt cttatgattt atgattttta ttattaaata 1200
   agttataaaa aaaataagtg tatacaaatt ttaaagtgac tcttaggttt taaaacgaaa 1260
   attcttgttc ttgagtaact ctttcctgta ggtcaggttg ctttctcagg tatagcatga 1320
   ggtcgctctt attgaccaca cctctaccgg catgcccatg ggttcttttg aaaagcaagc 1380
   ataaaagatc taaacataaa atctgtaaaa taacaagatg taaagataat gctaaatcat 1440
   ttggcttttt gattgattgt acaggaaaat atacatcgca gggggttgac ttttaccatt 1500
   tcaccgcaat ggaatcaaac ttgttgaaga gaatgttcac aggcgcatac gctacaatga 1560
   cccgattctt gctagccttt tctcggtctt gcaaacaacc gccaactgat caatgcatcc 1620
   tgcatggcgc gcctgatgag cctgaactgc ccgggcaaat cagctggacg tctgcctgca 1680
   ttaatgaatc ggccaacgcg cggggagagg cggtttgcgt attgggcgct cttccgcttc 1740
   ctcgctcact gactcgctgc gctcggtcgt tcggctgcgg cgagcggtat cagctcactc 1800
   aaaggcggta atacggttat ccacagaatc aggggataac gcaggaaaga acatgtgagc 1860
   aaaaggccag caaaaggcca ggaaccgtaa aaaggccgcg ttgctggcgt ttttccatag 1920
   gctccgcccc cctgacgagc atcacaaaaa tcgacgctca agtcagaggt ggcgaaaccc 1980
   gacaggacta taaagatacc aggcgtttcc ccctggaagc tccctcgtgc gctctcctgt 2040
   tccgaccctg ccgcttaccg gatacctgtc cgcctttctc ccttcgggaa gcgtggcgct 2100
   ttctcatagc tcacgctgta ggtatctcag ttcggtgtag gtcgttcgct ccaagctggg 2160
   ctgtgtgcac gaaccccccg ttcagcccga ccgctgcgcc ttatccggta actatcgtct 2220
   tgagtccaac ccggtaagac acgacttatc gccactggca gcagccactg gtaacaggat 2280
   tagcagagcg aggtatgtag gcggtgctac agagttcttg aagtggtggc ctaactacgg 2340
   ctacactaga aggacagtat ttggtatctg cgctctgctg aagccagtta ccttcggaaa 2400
   aagagttggt agctcttgat ccggcaaaca aaccaccgct ggtagcggtg gtttttttgt 2460
   ttgcaagcag cagattacgc gcagaaaaaa aggatctcaa gaagatcctt tgatcttttc 2520
   tacggggtct gacgctcagt ggaacgaaaa ctcacgttaa gggattttgg tcatgagatt 2580
   atcaaaaagg atcttcacct agatcctttt aaattaaaaa tgaagtttta aatcaatcta 2640
   aagtatatat gagtaaactt ggtctgacag ttaccaatgc ttaatcagtg aggcacctat 2700
   ctcagcgatc tgtctatttc gttcatccat agttgcctga ctccccgtcg tgtagataac 2760
   tacgatacgg gagggcttac catctggccc cagtgctgca atgataccgc gagacccacg 2820
   ctcaccggct ccagatttat cagcaataaa ccagccagcc ggaagggccg agcgcagaag 2880
   tggtcctgca actttatccg cctccatcca gtctattaat tgttgccggg aagctagagt 2940
   aagtagttcg ccagttaata gtttgcgcaa cgttgttgcc attgctacag gcatcgtggt 3000
   gtcacgctcg tcgtttggta tggcttcatt cagctccggt tcccaacgat caaggcgagt 3060
   tacatgatcc cccatgttgt gcaaaaaagc ggttagctcc ttcggtcctc cgatcgttgt 3120
   cagaagtaag ttggccgcag tgttatcact catggttatg gcagcactgc ataattctct 3180
   tactgtcatg ccatccgtaa gatgcttttc tgtgactggt gagtactcaa ccaagtcatt 3240
   ctgagaatag tgtatgcggc gaccgagttg ctcttgcccg gcgtcaatac gggataatac 3300
   cgcgccacat agcagaactt taaaagtgct catcattgga aaacgttctt cggggcgaaa 3360
   actctcaagg atcttaccgc tgttgagatc cagttcgatg taacccactc gtgcacccaa 3420
   ctgatcttca gcatctttta ctttcaccag cgtttctggg tgagcaaaaa caggaaggca 3480
   aaatgccgca aaaaagggaa taagggcgac acggaaatgt tgaatactca tactcttcct 3540
   ttttcaatat tattgaagca tttatcaggg ttattgtctc atgagcggat acatatttga 3600
   atgtatttag aaaaataaac aaataggggt tccgcgcaca tttccccgaa aagtgccacc 3660
   tgacgcgccc tgtagcggcg cattaagcgc ggcgggtgtg gtggttacgc gcagcgtgac 3720
   cgctacactt gccagcgccc tagcgcccgc tcctttcgct ttcttccctt cctttctcgc 3780
   cacgttcgcc ggctttcccc gtcaagctct aaatcggggg ctccctttag ggttccgatt 3840
   tagtgcttta cggcacctcg accccaaaaa acttgattag ggtgatggtt cacgtagtgg 3900
   gccatcgccc tgatagacgg tttttcgccc tttgacgttg gagtccacgt tctttaatag 3960
   tggactcttg ttccaaactg gaacaacact caaccctatc tcggtctatt cttttgattt 4020
   ataagggatt ttgccgattt cggcctattg gttaaaaaat gagctgattt aacaaaaatt 4080
   taacgcgaat tttaacaaaa tattaacgct tacaatttcc attcgccatt caggctgcgc 4140
   aactgttggg aagggcgatc ggtgcgggcc tcttcgctat tacgccag 4188
<210> 4
   <211> 11466
   <212> DNA
   <213> Artificial sequence
<220>
   <223> Synthetic
   <220>
   <221> misc_feature
   <222> (3560)..(4247)
   <223> Tetrahymena thermophila macronuclear telomere
<220>
   <221> misc_feature
   <222> (6024)..(6711)
   <223> Tetrahymena thermophila macronuclear telomere
<220>
   <221> misc_feature
   <222> (9644)..(10388)
   <223> Autonomous replicating sequence
<220>
   <221> misc_feature
   <222> (10488)..(11465)
   <223> Centromere IV
<220>
   <221> rep_origin
   <222> (7198)..(7198)
   <223> Origin of replication, PMB1
<220>
   <221> misc_feature
   <222> (1962)..(2765)
   <223> URA3, orotidine-5'-phosphate decarboxylase coding sequence
<220>
   <221> misc_feature
   <222> (4893)..(5552)
   <223> HIS3, imidazoleglycerolphosphate dehydratase, coding sequence
<220>
   <221> misc_feature
   <222> (7956)..(8816)
   <223> AP(R), beta-lactamase, ampR ampicillin resistance, coding sequence
<220>
   <221> misc_feature
   <222> (9129)..(9803)
   <223> TRP1, phosphoribosylanthranilate isomerase, coding sequence
<400> 4
   ttctcatgtt tgacagctta tcatcgataa gctttaatgc ggtagtttat cacagttaaa     60
   ttgctaacgc agtcaggcac cgtgtatgaa atctaacaat gcgctcatcg tcatcctcgg    120
   caccgtcacc ctggatgctg taggcatagg cttggttatg ccggtactgc cgggcctctt    180
   gcgggatatc gtccattccg acagcatcgc cagtcactat ggcgtgctgc tagcgctata    240
   tgcgttgatg caatttctat gcgcacccgt tctcggagca ctgtccgacc gctttggccg    300
   ccgcccagtc ctgctcgctt cgctacttgg agccactatc gactacgcga tcatggcgac    360
   cacacccgtc ctgtggatca attcccttta gtataaattt cactctgaac catcttggaa    420
   ggaccggtaa ttatttcaaa tctctttttc aattgtatat gtgttatgtt atgtagtata    480
   ctctttcttc aacaattaaa tactctcggt agccaagttg gtttaaggcg caagacttta    540
   atttatcact acggaattgg cgcgccaatt ccgtaatctt gagatcgggc gttcgatcgc    600
   cccgggagat ttttttgttt tttatgtctt ccattcactt cccagacttg caagttgaaa    660
   tatttctttc aagggaattg atcctctacg ccggacgcat cgtggccggc atcaccggcg    720
   ccacaggtgc ggttgctggc gcctatatcg ccgacatcac cgatggggaa gatcgggctc    780
   gccacttcgg gctcatgagc gcttgtttcg gcgtgggtat ggtggcaggc cccgtggccg    840
   ggggactgtt gggcgccatc tccttgcatg caccattcct tgcggcggcg gtgctcaacg    900
   gcctcaacct actactgggc tgcttcctaa tgcaggagtc gcataaggga gagcgtcgac    960
   cgatgccctt gagagccttc aacccagtca gctccttccg gtgggcgcgg ggcatgacta   1020
   tcgtcgccgc acttatgact gtcttcttta tcatgcaact cgtaggacag gtgccggcag   1080
   cgctctgggt cattttcggc gaggaccgct ttcgctggag cgcgacgatg atcggcctgt 1140
   cgcttgcggt attcggaatc ttgcacgccc tcgctcaagc cttcgtcact ggtcccgcca 1200
   ccaaacgttt cggcgagaag caggccatta tcgccggcat ggcggccgac gcgctgggct 1260
   acgtcttgct ggcgttcgcg acgcgaggct ggatggcctt ccccattatg attcttctcg 1320
   cttccggcgg catcgggatg cccgcgttgc aggccatgct gtccaggcag gtagatgacg 1380
   accatcaggg acagcttcaa ggatcgctcg cggctcttac cagcctaact tcgatcactg 1440
   gaccgctgat cgtcacggcg atttatgccg cctcggcgag cacatggaac gggttggcat 1500
   ggattgtagg cgccgcccta taccttgtct gcctccccgc gttgcgtcgc ggtgcatgga 1560
   gccgggccac ctcgacctga atggaagccg gcggcacctc gctaacggat tcaccactcc 1620
   aagaattgga gccaatcaat tcttgcggag aactgtgaat gcgcaaacca acccttggca 1680
   gaacatatcc atcgcgtccg ccatctccag cagccgcacg cggcgcatcc ccccccccct 1740
   ttcaattcaa ttcatcattt tttttttatt cttttttttg atttcggttt ctttgaaatt 1800
   tttttgattc ggtaatctcc gaacagaagg aagaacgaag gaaggagcac agacttagat 1860
   tggtatatat acgcatatgt agtgttgaag aaacatgaaa ttgcccagta ttcttaaccc 1920
   aactgcacag aacaaaaacc tgcaggaaac gaagataaat catgtcgaaa gctacatata 1980
   aggaacgtgc tgctactcat cctagtcctg ttgctgccaa gctatttaat atcatgcacg 2040
   aaaagcaaac aaacttgtgt gcttcattgg atgttcgtac caccaaggaa ttactggagt 2100
   tagttgaagc attaggtccc aaaatttgtt tactaaaaac acatgtggat atcttgactg 2160
   atttttccat ggagggcaca gttaagccgc taaaggcatt atccgccaag tacaattttt 2220
   tactcttcga agacagaaaa tttgctgaca ttggtaatac agtcaaattg cagtactctg 2280
   cgggtgtata cagaatagca gaatgggcag acattacgaa tgcacacggt gtggtgggcc 2340
   caggtattgt tagcggtttg aagcaggcgg cagaagaagt aacaaaggaa cctagaggcc 2400
   ttttgatgtt agcagaattg tcatgcaagg gctccctatc tactggagaa tatactaagg 2460
   gtactgttga cattgcgaag agcgacaaag attttgttat cggctttatt gctcaaagag 2520
   acatgggtgg aagagatgaa ggttacgatt ggttgattat gacacccggt gtgggtttag 2580
   atgacaaggg agacgcattg ggtcaacagt atagaaccgt ggatgatgtg gtctctacag 2640
   gatctgacat tattattgtt ggaagaggac tatttgcaaa gggaagggat gctaaggtag 2700
   agggtgaacg ttacagaaaa gcaggctggg aagcatattt gagaagatgc ggccagcaaa 2760
   actaaaaaac tgtattataa gtaaatgcat gtatactaaa ctcacaaatt agagcttcaa 2820
   tttaattata tcagttatta ctcgggcgta atgattttta taatgacgaa aaaaaaaaaa 2880
   ttggaaagaa aagggggggg gggcagcgtt gggtcctggc cacgggtgcg catgatcgtg 2940
   ctcctgtcgt tgaggacccg gctaggctgg cggggttgcc ttactggtta gcagaatgaa 3000
   tcaccgatac gcgagcgaac gtgaagcgac tgctgctgca aaacgtctgc gacctgagca 3060
   acaacatgaa tggtcttcgg tttccgtgtt tcgtaaagtc tggaaacgcg gaagtcagcg 3120
   ccctgcacca ttatgttccg gatctgcatc gcaggatgct gctggctacc ctgtggaaca 3180
   cctacatctg tattaacgaa gcgctggcat tgaccctgag tgatttttct ctggtcccgc 3240
   cgcatccata ccgccagttg tttaccctca caacgttcca gtaaccgggc atgttcatca 3300
   tcagtaaccc gtatcgtgag catcctctct cgtttcatcg gtatcattac ccccatgaac 3360
   agaaattccc ccttacacgg aggcatcaag tgaccaaaca ggaaaaaacc gcccttaaca 3420
   tggcccgctt tatcagaagc cagacattaa cgcttctgga gaaactcaac gagctggacg 3480
   cggatgaaca ggcagacatc tgtgaatcgc ttcacgacca cgctgatgag ctttaccgca 3540
   gccctcgagg gataagcttc atttttagat aaaatttatt aatcatcatt aatttcttga 3600
   aaaacatttt atttattgat cttttataac aaaaaaccct tctaaaagtt tatttttgaa 3660
   tgaaaaactt ataaaaattt atgaaaacta caaaaaataa aatttttaat taaaataatt 3720
   ttgataagaa cttcaatctt tgactagcta gcttagtcat tttgagattt taattaatat 3780
   tttatgttta ttcatatata aactattcaa aatattatag aatttaaaca ttttaacatc 3840
   ttaatcattc ataaataact aaaaatcaaa gtattacatc aataaataac ttttactcaa 3900
   tgtcaaagaa ttattggggt tggggttggg gttggggttg gggttggggt tggggttggg 3960
   gttggggttg gggttggggt tggggttggg gttggggttg gggttggggt tggggttggg 4020
   gttggggttg gggttggggt tggggttggg gttggggttg gggttggggt tggggttggg 4080
   gttggggttg gggttggggt tggggttggg gttggggttg gggttggggt tggggttggg 4140
   gttggggttg gggttggggt tggggttggg gttggggttg gggtgggaaa acagcattca 4200
   ggtattagaa gaatatcctg attcaggtga aaatattgtt gatgcgcggg atcctcgggg 4260
   acaccaaata tggcgatctc ggccttttcg tttcttggag ctgggacatg tttgccatcg 4320
   atccatctac caccagaacg gccgttagat ctgctgccac cgttgtttcc accgaagaaa 4380
   ccaccgttgc cgtaaccacc acgacggttg ttgctaaaga agctgccacc gccacggcca 4440
   ccgttgtagc cgccgttgtt gttattgtag ttgctcatgt tatttctggc acttcttggt 4500
   tttcctctta agtgaggagg aacataacca ttctcgttgt tgtcgttgat gcttaaattt 4560
   tgcacttgtt cgctcagttc agccataata tgaaatgctt ttcttgttgt tcttacggaa 4620
   taccacttgc cacctatcac cacaactaac tttttcccgt tcctccatct cttttatatt 4680
   ttttttctcg atcgagttca agagaaaaaa aaagaaaaag caaaaagaaa aaaggaaagc 4740
   gcgcctcgtt cagaatgaca cgtatagaat gatgcattac cttgtcatct tcagtatcat 4800
   actgttcgta tacatactta ctgacattca taggtataca tatatacaca tgtatatata 4860
   tcgtatgctg cagctttaaa taatcggtgt cactacataa gaacaccttt ggtggaggga 4920
   acatcgttgg taccattggg cgaggtggct tctcttatgg caaccgcaag agccttgaac 4980
   gcactctcac tacggtgatg atcattcttg cctcgcagac aatcaacgtg gagggtaatt 5040
   ctgctagcct ctgcaaagct ttcaagaaaa tgcgggatca tctcgcaaga gagatctcct 5100
   actttctccc tttgcaaacc aagttcgaca actgcgtacg gcctgttcga aagatctacc 5160
   accgctctgg aaagtgcctc atccaaaggc gcaaatcctg atccaaacct ttttactcca 5220
   cgcgccagta gggcctcttt aaaagcttga ccgagagcaa tcccgcagtc ttcagtggtg 5280
   tgatggtcgt ctatgtgtaa gtcaccaatg cactcaacga ttagcgacca gccggaatgc 5340
   ttggccagag catgtatcat atggtccaga aaccctatac ctgtgtggac gttaatcact 5400
   tgcgattgtg tggcctgttc tgctactgct tctgcctctt tttctgggaa gatcgagtgc 5460
   tctatcgcta ggggaccacc ctttaaagag atcgcaatct gaatcttggt ttcatttgta 5520
   atacgcttta ctagggcttt ctgctctgtc atctttgcct tcgtttatct tgcctgctca 5580
   ttttttagta tattcttcga agaaatcaca ttactttata taatgtataa ttcattatgt 5640
   gataatgcca atcgctaaga aaaaaaaaga gtcatccgct aggtggaaaa aaaaaaatga 5700
   aaatcattac cgaggcataa aaaaatatag agtgtactag aggaggccaa gagtaataga 5760
   aaaagaaaat tgcgggaaag gactgtgtta tgacttccct gactaatgcc gtgttcaaac 5820
   gatacctggc agtgactcct agcgctcacc aagctcttaa aacgagaatt aagaaaaagt 5880
   cgtcatcttt cgataagttt ttcccacagc aaagcaatag tagaaaaaaa caatgggaaa 5940
   cgttgaatga agacaaagcg tcgtggttta aaaggaaata cgctcacgta catgctaggg 6000
   aacaggaccg tgcagcggat cccgcgcatc aacaatattt tcacctgaat caggatattc 6060
   ttctaatacc tgaatgctgt tttcccaccc caaccccaac cccaacccca accccaaccc 6120
   caaccccaac cccaacccca accccaaccc caaccccaac cccaacccca accccaaccc 6180
   caaccccaac cccaacccca accccaaccc caaccccaac cccaacccca accccaaccc 6240
   caaccccaac cccaacccca accccaaccc caaccccaac cccaacccca accccaaccc 6300
   caaccccaac cccaacccca accccaaccc caaccccaac cccaacccca accccaataa 6360
   ttctttgaca ttgagtaaaa gttatttatt gatgtaatac tttgattttt agttatttat 6420
   gaatgattaa gatgttaaaa tgtttaaatt ctataatatt ttgaatagtt tatatatgaa 6480
   taaacataaa atattaatta aatctcaaaa atgactaagc tagctagtca aagattgaag 6540
   ttcttatcaa aattatttta attaaaaatt ttattttttg tagttttcat aaatttttat 6600
   aagtttttca ttcaaaaata aacttttaga agggtttttt gttataaaag atcaataaat 6660
   aaaatgtttt tcaagaaatt aatgatgatt aataaatttt atctaaaaat gaagcttatc 6720
   cctcgagggc tgcctcgcgc gtttcggtga tgacggtgaa aacctctgac acatgcagct 6780
   cccggagacg gtcacagctt gtctgtaagc ggatgccggg agcagacaag cccgtcaggg 6840
   cgcgtcagcg ggtgttggcg ggtgtcgggg cgcagccatg acccagtcac gtagcgatag 6900
   cggagtgtat actggcttaa ctatgcggca tcagagcaga ttgtactgag agtgcaccat 6960
   atgcggtgtg aaataccgca cagatgcgta aggagaaaat accgcatcag gcgctcttcc 7020
   gcttcctcgc tcactgactc gctgcgctcg gtcgttcggc tgcggcgagc ggtatcagct 7080
   cactcaaagg cggtaatacg gttatccaca gaatcagggg ataacgcagg aaagaacatg 7140
   tgagcaaaag gccagcaaaa ggccaggaac cgtaaaaagg ccgcgttgct ggcgtttttc 7200
   cataggctcc gcccccctga cgagcatcac aaaaatcgac gctcaagtca gaggtggcga 7260
   aacccgacag gactataaag ataccaggcg tttccccctg gaagctccct cgtgcgctct 7320
   cctgttccga ccctgccgct taccggatac ctgtccgcct ttctcccttc gggaagcgtg 7380
   gcgctttctc atagctcacg ctgtaggtat ctcagttcgg tgtaggtcgt tcgctccaag 7440
   ctgggctgtg tgcacgaacc ccccgttcag cccgaccgct gcgccttatc cggtaactat 7500
   cgtcttgagt ccaacccggt aagacacgac ttatcgccac tggcagcagc cactggtaac 7560
   aggattagca gagcgaggta tgtaggcggt gctacagagt tcttgaagtg gtggcctaac 7620
   tacggctaca ctagaaggac agtatttggt atctgcgctc tgctgaagcc agttaccttc 7680
   ggaaaaagag ttggtagctc ttgatccggc aaacaaacca ccgctggtag cggtggtttt 7740
   tttgtttgca agcagcagat tacgcgcaga aaaaaaggat ctcaagaaga tcctttgatc 7800
   ttttctacgg ggtctgacgc tcagtggaac gaaaactcac gttaagggat tttggtcatg 7860
   agattatcaa aaaggatctt cacctagatc cttttaaatt aaaaatgaag ttttaaatca 7920
   atctaaagta tatatgagta aacttggtct gacagttacc aatgcttaat cagtgaggca 7980
   cctatctcag cgatctgtct atttcgttca tccatagttg cctgactccc cgtcgtgtag 8040
   ataactacga tacgggaggg cttaccatct ggccccagtg ctgcaatgat accgcgagac 8100
   ccacgctcac cggctccaga tttatcagca ataaaccagc cagccggaag ggccgagcgc 8160
   agaagtggtc ctgcaacttt atccgcctcc atccagtcta ttaattgttg ccgggaagct 8220
   agagtaagta gttcgccagt taatagtttg cgcaacgttg ttgccattgc tgcaggcatc 8280
   gtggtgtcac gctcgtcgtt tggtatggct tcattcagct ccggttccca acgatcaagg 8340
   cgagttacat gatcccccat gttgtgcaaa aaagcggtta gctccttcgg tcctccgatc 8400
   gttgtcagaa gtaagttggc cgcagtgtta tcactcatgg ttatggcagc actgcataat 8460
   tctcttactg tcatgccatc cgtaagatgc ttttctgtga ctggtgagta ctcaaccaag 8520
   tcattctgag aatagtgtat gcggcgaccg agttgctctt gcccggcgtc aacacgggat 8580
   aataccgcgc cacatagcag aactttaaaa gtgctcatca ttggaaaacg ttcttcgggg 8640
   cgaaaactct caaggatctt accgctgttg agatccagtt cgatgtaacc cactcgtgca 8700
   cccaactgat cttcagcatc ttttactttc accagcgttt ctgggtgagc aaaaacagga 8760
   aggcaaaatg ccgcaaaaaa gggaataagg gcgacacgga aatgttgaat actcatactc 8820
   ttcctttttc aatattattg aagcatttat cagggttatt gtctcatgag cggatacata 8880
   tttgaatgta tttagaaaaa taaacaaata ggggttccgc gcacatttcc ccgaaaagtg 8940
   ccacctgacg tctaagaaac cattattatc atgacattaa cctataaaaa taggcgtatc 9000
   acgaggccct ttcgtcttca agaattaatt cggtcgaaaa aagaaaagga gagggccaag 9060
   agggagggca ttggtgacta ttgagcacgt gagtatacgt gattaagcac acaaaggcag 9120
   cttggagtat gtctgttatt aatttcacag gtagttctgg tccattggtg aaagtttgcg 9180
   gcttgcagag cacagaggcc gcagaatgtg ctctagattc cgatgctgac ttgctgggta 9240
   ttatatgtgt gcccaataga aagagaacaa ttgacccggt tattgcaagg aaaatttcaa 9300
   gtcttgtaaa agcatataaa aatagttcag gcactccgaa atacttggtt ggcgtgtttc 9360
   gtaatcaacc taaggaggat gttttggctc tggtcaatga ttacggcatt gatatcgtcc 9420
   aactgcatgg agatgagtcg tggcaagaat accaagagtt cctcggtttg ccagttatta 9480
   aaagactcgt atttccaaaa gactgcaaca tactactcag tgcagcttca cagaaacctc 9540
   attcgtttat tcccttgttt gattcagaag caggtgggac aggtgaactt ttggattgga 9600
   actcgatttc tgactgggtt ggaaggcaag agagccccga aagcttacat tttatgttag 9660
   ctggtggact gacgccagaa aatgttggtg atgcgcttag attaaatggc gttattggtg 9720
   ttgatgtaag cggaggtgtg gagacaaatg gtgtaaaaga ctctaacaaa atagcaaatt 9780
   tcgtcaaaaa tgctaagaaa taggttatta ctgagtagta tttatttaag tattgtttgt 9840
   gcacttgcct gcaggccttt tgaaaagcaa gcataaaaga tctaaacata aaatctgtaa 9900
   aataacaaga tgtaaagata atgctaaatc atttggcttt ttgattgatt gtacaggaaa 9960
   atatacatcg cagggggttg acttttacca tttcaccgca atggaatcaa acttgttgaa 10020
   gagaatgttc acaggcgcat acgctacaat gacccgattc ttgctagcct tttctcggtc 10080
   ttgcaaacaa ccgccggcag cttagtatat aaatacacat gtacatacct ctctccgtat 10140
   cctcgtaatc attttcttgt atttatcgtc ttttcgctgt aaaaacttta tcacacttat 10200
   ctcaaataca cttattaacc gcttttacta ttatcttcta cgctgacagt aatatcaaac 10260
   agtgacacat attaaacaca gtggtttctt tgcataaaca ccatcagcct caagtcgtca 10320
   agtaaagatt tcgtgttcat gcagatagat aacaatctat atgttgataa ttagcgttgc 10380
   ctcatcaatg cgagatccgt ttaaccggac cctagtgcac ttaccccacg ttcggtccac 10440
   tgtgtgccga acatgctcct tcactatttt aacatgtgga attaattcta aatcctcttt 10500
   atatgatctg ccgatagata gttctaagtc attgaggttc atcaacaatt ggattttctg 10560
   tttactcgac ttcaggtaaa tgaaatgaga tgatacttgc ttatctcata gttaactcta 10620
   agaggtgata cttatttact gtaaaactgt gacgataaaa ccggaaggaa gaataagaaa 10680
   actcgaactg atctataatg cctattttct gtaaagagtt taagctatga aagcctcggc 10740
   attttggccg ctcctaggta gtgctttttt tccaaggaca aaacagtttc tttttcttga 10800
   gcaggtttta tgtttcggta atcataaaca ataaataaat tatttcattt atgtttaaaa 10860
   ataaaaaata aaaaagtatt ttaaattttt aaaaaagttg attataagca tgtgaccttt 10920
   tgcaagcaat taaattttgc aatttgtgat tttaggcaaa agttacaatt tctggctcgt 10980
   gtaatatatg tatgctaaag tgaactttta caaagtcgat atggacttag tcaaaagaaa 11040
   ttttcttaaa aatatatagc actagccaat ttagcacttc tttatgagat atattataga 11100
   ctttattaag ccagatttgt gtattatatg tatttacccg gcgaatcatg gacatacatt 11160
   ctgaaatagg taatattctc tatggtgaga cagcatagat aacctaggat acaagttaaa 11220
   agctagtact gttttgcagt aatttttttc ttttttataa gaatgttacc acctaaataa 11280
   gttataaagt caatagttaa gtttgatatt tgattgtaaa ataccgtaat atatttgcat 11340
   gatcaaaagg ctcaatgttg actagccagc atgtcaacca ctatattgat caccgatata 11400
   tggacttcca caccaactag taatatgaca ataaattcaa gatattcttc atgagaatgg 11460
   cccaga 11466
<210> 5
   <211> 4313
   <212> DNA
   <213> Artificial sequence
<220>
   <223> Synthetic
   <220>
   <221> misc_feature
   <222> (3787)..(4243)
   <223> f1-phage origin of replication
<220>
   <221> misc_feature
   <222> (2798).. (3655)
   <223> Ampicillin resistance gene
<220>
   <221> terminator
   <222> (1100)..(1428)
   <223>
<220>
   <221> promoter
   <222> (655)..(1042)
   <223> Met25 promotor
<220>
   <221> rep_origin
   <222> (1855) . . (2795)
   <223> ColE1
<400> 5
   ctgatttgcc cgggcagttc aggctcatca ggcgcgccat gcagggatcg gcgttttccg 60
   gaactggaaa accgacatgt tgatttcctg aaacgggata tcatcaaagc catgaacaaa 120
   gcagccgcgc tggatgaact gataccgggg ttgctgagtg aatatatcga acagtcaggt 180
   taacaggctg cggcattttg tccgcgccgg gcttcgctca ctgttcaggc cggagccaca 240
   gaccgccgtt gaatgggcgg atgctaatta ctatctcccg aaagaatccg cataccagga 300
   agggcgctgg gaaacactgc cctttcagcg ggccatcatg aatgcgatgg gcagcgacta 360
   catccgtgag gtgaatgtgg tgaagtctgc ccgtgtcggt tattccaaaa tgctgctggg 420
   tgtttatgcc tactttatag agcataagca gcgcaacacc cttatctggt tgccgacgga 480
   tggtgatgcc gagaacttta tgaaaaccca cgttgagccg actattcgtg atattccgtc 540
   gctgctggcg ctggccccgt ggtatggcaa aaagcaccgg gataacacgc tcaccatgaa 600
   gcgtttcact aatgggcgtg gcttctggtg cctgggcggt aaagcggaga tcttcttcgg 660
   atgcaagggt tcgaatccct tagctctcat tattttttgc tttttctctt gaggtcacat 720
   gatcgcaaaa tggcaaatgg cacgtgaagc tgtcgatatt ggggaactgt ggtggttggc 780
   aaatgactaa ttaagttagt caaggcgcca tcctcatgaa aactgtgtaa cataataacc 840
   gaagtgtcga aaaggtggca ccttgtccaa ttgaacacgc tcgatgaaaa aaataagata 900
   tatataaggt taagtaaagc gtctgttaga aaggaagttt ttcctttttc ttgctctctt 960
   gtcttttcat ctactatttc cttcgtgtaa tacagggtcg tcagatacat agatacaatt 1020
   ctattacccc catccataca agcttggcgc cgaattcgtc gacccgggga tccgcggccg 1080
   caggcctaaa ttgatctaga gctttggact tcttcgccag aggtttggtc aagtctccaa 1140
   tcaaggttgt cggcttgtct accttgccag aaatttacga aaagatggaa aagggtcaaa 1200
   tcgttggtag atacgttgtt gacacttcta aataagcgaa tttcttatga tttatgattt 1260
   ttattattaa ataagttata aaaaaaataa gtgtatacaa attttaaagt gactcttagg 1320
   ttttaaaacg aaaattcttg ttcttgagta actctttcct gtaggtcagg ttgctttctc 1380
   aggtatagca tgaggtcgct cttattgacc acacctctac cggcatgccc atggatgacc 1440
   cctccagcgt gttttatctc tgcgagcata atgcctgcgt catccgccag caggagctgg 1500
   actttactga tgcccgttat atctgcgaaa agaccgggat ctggacccgt gatggcattc 1560
   tctggttttc gtcatccggt gaagagattg agccacctga cagtgtgacc tttcacatct 1620
   ggacagcgta cagcccgttc accacctggg tgcagattgt caaagactgg atgaaaacga 1680
   aaggggatac gggaaaacgt aaaaccttcg taaacaccac gctcggtgag atgatcaatg 1740
   catcctgcat ggcgcgcctg atgagcctga actgcccggg caaatcagct ggacgtctgc 1800
   ctgcattaat gaatcggcca acgcgcgggg agaggcggtt tgcgtattgg gcgctcttcc 1860
   gcttcctcgc tcactgactc gctgcgctcg gtcgttcggc tgcggcgagc ggtatcagct 1920
   cactcaaagg cggtaatacg gttatccaca gaatcagggg ataacgcagg aaagaacatg 1980
   tgagcaaaag gccagcaaaa ggccaggaac cgtaaaaagg ccgcgttgct ggcgtttttc 2040
   cataggctcc gcccccctga cgagcatcac aaaaatcgac gctcaagtca gaggtggcga 2100
   aacccgacag gactataaag ataccaggcg tttccccctg gaagctccct cgtgcgctct 2160
   cctgttccga ccctgccgct taccggatac ctgtccgcct ttctcccttc gggaagcgtg 2220
   gcgctttctc atagctcacg ctgtaggtat ctcagttcgg tgtaggtcgt tcgctccaag 2280
   ctgggctgtg tgcacgaacc ccccgttcag cccgaccgct gcgccttatc cggtaactat 2340
   cgtcttgagt ccaacccggt aagacacgac ttatcgccac tggcagcagc cactggtaac 2400
   aggattagca gagcgaggta tgtaggcggt gctacagagt tcttgaagtg gtggcctaac 2460
   tacggctaca ctagaaggac agtatttggt atctgcgctc tgctgaagcc agttaccttc 2520
   ggaaaaagag ttggtagctc ttgatccggc aaacaaacca ccgctggtag cggtggtttt 2580
   tttgtttgca agcagcagat tacgcgcaga aaaaaaggat ctcaagaaga tcctttgatc 2640
   ttttctacgg ggtctgacgc tcagtggaac gaaaactcac gttaagggat tttggtcatg 2700
   agattatcaa aaaggatctt cacctagatc cttttaaatt aaaaatgaag ttttaaatca 2760
   atctaaagta tatatgagta aacttggtct gacagttacc aatgcttaat cagtgaggca 2820
   cctatctcag cgatctgtct atttcgttca tccatagttg cctgactccc cgtcgtgtag 2880
   ataactacga tacgggaggg cttaccatct ggccccagtg ctgcaatgat accgcgagac 2940
   ccacgctcac cggctccaga tttatcagca ataaaccagc cagccggaag ggccgagcgc 3000
   agaagtggtc ctgcaacttt atccgcctcc atccagtcta ttaattgttg ccgggaagct 3060
   agagtaagta gttcgccagt taatagtttg cgcaacgttg ttgccattgc tacaggcatc 3120
   gtggtgtcac gctcgtcgtt tggtatggct tcattcagct ccggttccca acgatcaagg 3180
   cgagttacat gatcccccat gttgtgcaaa aaagcggtta gctccttcgg tcctccgatc 3240
   gttgtcagaa gtaagttggc cgcagtgtta tcactcatgg ttatggcagc actgcataat 3300
   tctcttactg tcatgccatc cgtaagatgc ttttctgtga ctggtgagta ctcaaccaag 3360
   tcattctgag aatagtgtat gcggcgaccg agttgctctt gcccggcgtc aatacgggat 3420
   aataccgcgc cacatagcag aactttaaaa gtgctcatca ttggaaaacg ttcttcgggg 3480
   cgaaaactct caaggatctt accgctgttg agatccagtt cgatgtaacc cactcgtgca   3540
   cccaactgat cttcagcatc ttttactttc accagcgttt ctgggtgagc aaaaacagga   3600
   aggcaaaatg ccgcaaaaaa gggaataagg gcgacacgga aatgttgaat actcatactc   3660
   ttcctttttc aatattattg aagcatttat cagggttatt gtctcatgag cggatacata   3720
   tttgaatgta tttagaaaaa taaacaaata ggggttccgc gcacatttcc ccgaaaagtg   3780
   ccacctgacg cgccctgtag cggcgcatta agcgcggcgg gtgtggtggt tacgcgcagc   3840
   gtgaccgcta cacttgccag cgccctagcg cccgctcctt tcgctttctt cccttccttt   3900
   ctcgccacgt tcgccggctt tccccgtcaa gctctaaatc gggggctccc tttagggttc   3960
   cgatttagtg ctttacggca cctcgacccc aaaaaacttg attagggtga tggttcacgt   4020
   agtgggccat cgccctgata gacggttttt cgccctttga cgttggagtc cacgttcttt   4080
   aatagtggac tcttgttcca aactggaaca acactcaacc ctatctcggt ctattctttt   4140
   gatttataag ggattttgcc gatttcggcc tattggttaa aaaatgagct gatttaacaa   4200
   aaatttaacg cgaattttaa caaaatatta acgcttacaa tttccattcg ccattcaggc   4260
   tgcgcaactg ttgggaaggg cgatcggtgc gggcctcttc gctattacgc cag          4313

## Description

### Field of invention

The present invention relates to the field of selecting cells producing compounds that fulfil at least 2 predetermined functionalities. The invention primarily focuses on methods for screening of cells for two or more functionalities related to small molecules generated by host cells with novel gene assemblies. The methods in the aspect of the invention are combined with methods for evolution of cells to acquire at least two predetermined functionalities related to small molecules. Furthermore, the invention relates to methods for generation of lead compounds that are selected for a primary function and at least one further secondary function. The methods according to the invention may in particular be used in high troughput screening.

### Background of invention

### Screening and selection

Methods for screening of cells for a given functionality using automated screening methods are known from the prior art. Examples of such methods include:

US 20010041333 A1 (Short & Keller) discloses among other thing a method for screening a polynucleotide library transformed into a suitable organism in a FACS (Fluorescence activated cell sorter). The library may be screened to determine whether the mixture has one or more specified activities. Another possibility is to screen scvf (single-chain fragment variable) libraries against multiple binding targets (multiple epitope species, multiple receptor species) to obtain a multiplicity of scfv which have different binding specifities.

US 6,174,673 (Diversa) discloses the use of co-encapsulation of a library and an assay together with FACS. It is speculated that one could employ mixtures of substrates to simultaneously detect multiple enzyme activities of interest simultaneously or sequentially. FACS instruments can detect molecules that fluoresce at different wavelengths, hence substrates, which fluoresce at different wavelengths and indicate different enzyme activities can be employed.

WO 98/58085 (Diversa) discloses screening methods very similar to those of US 6,174,673. According to the description clones can be expressed to yield backbone structures, which can then be decorated in metabolically rich hosts, and finally screened for an activity of interest. Alternatively, clones can be expressed to yield small molecules directly, which can be screened for an activity of interest. Furthermore, multiple probes can be designed and utilised to allow "multiplex" screening and/or enrichment. "multiplex" screening and/or enrichment means that one is screening and/or enriching for more than one desirable outcome in multiple parallel and simultaneous screens.

US 5,837,458 (Maxygen) relates to methods for recursive sequence recombination. Different ways of screening and selection are described, among these the use of gel microdrops and facs with a reporter cell incorporated into the geldrop.

WO 00/08212 (Cellay) relates to forming a population of gel microdroplets encapsulating a population of biological entities (such as cells, vira, chromosomes), whereby at least some microdrops each encapsulate a single entity, contacting the population of microdrops with a probe which hybridises to a complementary sequence in an entity in at least one microdrop, and isolating the at least one microdrop.

WO 98/58085 (Diversa) relates to the use of FACS for screening expression libraries, which may be co-encapsulated with the "screen".

WO 01/32829 (Novo Nordisk) discloses a method for screening host cells from a DNA library. The host cells are put into "sample" and the secretion of product into the sample is detected using fluorescence. A further feature is the establishment of a "means for correlation" between the host cell secretion and the fluorescence. The means for correlation are a physical link such as a chemical bond between the sample and the fluorescent label. The advantage is that the connection between cell and secreted product is not lost.

As can be seen from the above, most of the prior art focuses on development of modified enzymes and on modified antibodies. Common for all the references is that they focus on screening the cells for one functionality at a time.

Other references focussed on the development of reporter systems mention the possibility of having different reporter systems wherein the signals can be distinguished.

US 6,020,192 (University of Florida) relates to humanised green fluorescent protein genes. It is mentioned that GFPs that produce different colours permit simultaneous use of multiple reporter genes. As an example it is stated that differently coloured GFPs can be used to identify multiple cell populations in a mixed cell culture. Other options include tracking and determining the location of multiple proteins within a single cell, tissue or organism; differential promoter analysis in which gene expression from two different promoters is determined in the same cell, tissue or organism; and FACS sorting of mixed cell populations. The concept of using distinguishable reporter genes for the simultaneous screening for different traits harboured in the same cell is not disclosed.

Metcalf et al 1993 (Gene 129:17-25) discloses E. coli multiple deletional mutants that allow the simultaneous use of multiple reporter genes and Wood et al 1989, (Science 244:700-702) discloses several luciferase genes which produce luciferase which cause different colours when bioluminescent. It is stated that these could be useful in experiments in which multiple reporter genes are needed.

These multiple reporter systems may be useful for screening for the presence of more than one enzyme in a cell, but they do not focus on screening for properties related to small molecules produced by the host cells.

WO 98/41869 (Chromaxome) discloses an approach to drug screening that is designed to couple a screening assay both temporally and spatially to natural product synthesis in a microorganism. The reference provides a screening unit which is a gel droplet comprising a producing species that produces natural products for the drug screen, and an assay system that detects or measures a desired biological activity. A producing species is coencapsulated with an assay system in a screening unit when the producing species is at a phase in its life cycle that is optimal for producing natural products, such as secondary metabolites. The producing species is spatially positioned relative to the assay system in the same unit such that compounds produced by the producing species can come into contact with the assay system. If a compound possesses the desired activity, the assay system will generate a signal that enables the identification and/or isolation of the screening unit. The reference does not address other functionalities of the compounds than pharmacological ones. Thus in order to optimise a pharmaceutical compound many rounds of successive screens will have to be carried out and most of the compounds found in the first rounds will not meet all the criteria as seen in standard drug discovery. Furthermore, the system is dependent on the cell cycle, since there is no control over gene expression in the producing species.

US 20010047029 (Handelsman et al) discloses triaryl cationic compounds that exhibit broad spectrum antibiotic and antifungal activity, pharmaceutical compositions containing the compounds, and methods of treating bacterial and fungal infections using the compounds. The compounds were initially isolated by screening a 25,000-member bacterial artificial chromosome (BAC) library of environmental (eDNA) from soil. At least one clone produced a dark brown melanin-like compound that was found to have antibiotic activity. The compounds were isolated and synthesized de novo. From within the positive clone, a single open reading frame that shares extensive sequence similarity with members of the 4-hydroxyphenylpyruvate family of enzymes was found to be necessary and sufficient to confer the production of at least one of the subject compounds on E. coli.

WO 89/10566 (MASSACHUSETTS INSTITTUTE OF TECHNOLOGY) discloses a process for capturing molecules at binding sites within gel microdroplets. The process also allows measurement or isolation of cells based on the captured molecules. The process involves creation of gel microdroplets (gel microdroplets) with binding sites for molecules secreted or released from cells. Gel microdroplets can optionally be incubated, and then measured for the presence of molecules captured at the gel microdroplet binding sites. The process allows measurement, and isolation of cells based on measurement, or allows isolation without measurement. This invention also comprises gel microdroplets containing marker entities which enhance the measurement of gel microdroplets, as are processes for forming and using such gel microdroplets.

WO 00/17643 (Cellomics Inc) and WO 98/38490 (Biodx Inc) disclose systems, methods and screens for monitoring the physiological response of cells to the addition of biologically active compounds. Methods of combining high throughput with high content spatial information at the cellular and subcellular level as well as temporal information about changes in physiological, biochemical and molecular activities are disclosed. These methods allow multiple types of cellular reactions to be studied simultaneously. The system is therefore suitable for further characterisation of lead compounds or for monitoring environmental samples for the presence of bio-active substances. However, these methods depend on fixation of the cells and are thus destructive and do not allow the recovery of live cells, therefore making the system useless for screening with the purpose of recovering the cells that produce the desired compounds. Furthermore the systems are based on multiwell formats of 96 or 386 wells and are thus not suited for the extremely high throughput needed when screening very large populations (10⁶-10¹²). The systems are also only able to give information on multiple aspects of known compounds. They do not provide any means for optimising small molecules with respect to multiple parameters.

### Drug development

Drug development begins with the identification of a lead compound, based on the ability of the compound to exhibit a desired biological effect, such as the ability to inhibit bacterial growth, inhibit the activity of a target enzyme, increase or modulate the uptake of neurotransmitters, and the like. Biological activity is typically determined on the basis of in vitro experimentation or assays designed to rapidly identify candidate drugs. Typically, the selection criteria have be very harsh to select only a small percentage of the compounds tested, since further development is low throughput and very expensive as it depends on medicinal chemists optimising very few (1-5) compounds per week.

Once a candidate or lead compound has been identified and selected for further development, its ADME/PK characteristics are determined. ADME/PK concerns the absorption, distribution, metabolism, excretion and pharmacokinetics of drugs in the body. The ADME/PK properties of a drug are critical, and often serve to distinguish pharmaceutical products from mere lead compounds. For example, a drug that is poorly absorbed orally may require intravenous (or other parenteral) administration to be effective, which may be unacceptable for the condition to be treated. A compound effective as an antibiotic may be ineffective to treat bacterial meningitis if its distribution does not carry it to the central nervous system. A compound that is rapidly metabolised and/or excreted may not reside in the body long enough to serve its intended purpose.

Thus, for a compound to be useable as a drug it must fulfil multiple functional requirements. It must interact with the target(s) and affect the function of the target in the desired manner. At the same time it should not interact with many other (often similar) targets or have major non-specific effects. And then it must further have the right physical-chemical parameters and be metabolised by the body in an acceptable manner.

Because of this intrinsic difficulty and complexity, the process of discovering and developing drugs has a very poor success rate and is thus extremely expensive ($600mn per successful compound) and very time consuming (c. 8-12 years from discovery to clinic). Only c. 1 in 15 primary screens produce a compound that makes it into pre-clinical development and only 1 in 10 of these compounds then make it to market. The average pharmaceutical company spends 250 man-years of research and development effort for every compound that enters the clinic. Most pharmaceutical companies are, in consequence, failing to launch new drugs at the rate they require to satisfy their investors.

There is thus a need in the art to develop methods for producing lead compounds. By definition, optimised compounds have to fulfil multiple requirements. Thus, the current invention addresses one of the major bottlenecks in drug development.

### Summary of invention

It is an aim of the present invention to screen cells or compositions of cells for two or more desired properties or functionalities. The principle behind the screening of cells according to the invention is to produce a great diversity of genes in each cell and a great diversity of genes among cells in a composition of cells, subject the composition to screening, and optionally evolution. The genes may be exchanged among the cells to evolve cells producing one or more compounds with the two or more desired properties or functionalities.

In a first aspect the invention relates to a method for screening a cell producing at least one compound, said screening being for two or more functionalities of said compound, said method comprising the steps of
a) providing a composition of cells, said composition of cells comprising cells each with at least two heterologous expressible nucleotide sequences, at least one of said heterologous sequences being located on an artificial chromosome in said cell, at least 2 cells of the composition contain different heterologous expressible nucleotide sequences, said cells being denoted producer cells,
b) performing one screening of said population of cells for at least 2 parameters related to the functionalities, and determining a selection criterion for each parameter,
c) selecting cells meeting at least one predetermined selection criterion.

The invention provides a method for generation of novel gene combinations through insertion of multiple heterologous genes into host cells with screening of these cells for two or more functionalities caused by small molecules synthesised by the host cells. By combining two or more functionalities in one screening round, the screening process is speeded up and the likelihood of identifying compounds fulfilling all criteria is increased. The invention also permits the identifcation of cells capable of synthesising compounds that are not only active against e.g. a pharmacological target, but at the same time fulfil another criterion, e.g. related to ADMET parameters or pharmacokinetic parameters. The compounds synthesised by the cells identified by the methods of the present invention may thus have a higher degree of likelihood to make it into drugs, because they are not only identified for their activity against a pharmacological target. The cells as defined in step a) can be referred to as producer cells because they produce the compounds screened and selected for. This is to distinguish them from reporter cells which can be used in connection with the screening and selection.

According to one preferred embodiment, the promoters of the expression cassettes are controllable, so that gene expression can be controlled. In such a system, gene expression is not dependent on the cell cycle and the chance of discovering a novel compound are increased.

The design and assembly of expression cassettes contained within the artificial chromosomes as well as the many possible sources are described in detailed description part of the present invention. Other vectors apart from artificial chromosomes can be used for harbouring the heterologous expressible nucleotide sequences but preferably substantially all of these are placed on one or more artificial chromosomes inserted into the host cell, because artificial chromosomes can harbour large genetic inserts and replicate stably in the cells especially when selectable genetic markers are inserted into the artificial chromosomes.

The compound for which the screening and selection is performed can either be contained within the cell or be excreted from said cell. According to a preferred embodiment of the invention the compound selected for is a product of enzymatic activity, i.e. a non-native primary or a secondary metabolite synthesised or converted by at least one enzyme present in the selected cells. More preferably, a compound according to the invention is produced by the combined activities of at least 2 enzymes, at least one of those enzymes being encoded by a heterologous expressible nucleotide sequence, which is preferably located on an artificial chromosome. According to this embodiment the definition of a compound does not encompass polynucleotides or translation products. On the other hand the invention may also result in the generation of novel proteins and enzymes through combination of different protein sub-units from different sources but these are not directly selected for.

One of the many applications of the present invention is the development of drugs or drug candidates. Therefore often at least one parameter is a parameter related to activity against a pharmacological target. This could be in the embodiment of screening for more than one aspect of the same pharmacological activity, e.g. screening for the activation/deactivation of a transcription factor and for the expression of one or more genes regulated by the transcription factor. In another embodiment it could be screening for a desired pharmacological activity and the absense of an undesired phamarcological activity. In another embodiment of this aspect, at least one parameter is a parameter related to activity against a pharmacological target, and at least one further parameter is an ADME parameter. The invention also provides screening methods which utilise any combination of the above parameters, e.g. in any order. By optimising compounds on multiple criteria, drug candidates can be identified that have an improved chance of getting through the costly clinical trials.

The parameters related to activity against a pharmacological target may be one or more of
- interaction or non-interaction with a ligand pharmacological target.
- interaction or absence of interaction with an enzyme pharmacological target.
- interaction or non-interaction with a receptor pharmacological target.
- inhibition or enhancement of expression of a gene or set of genes encoding a pharmacological target.
- the inhibition or enhancement of expression may be caused by the ability of a compound to bind to the promoter sequence of the target gene, or the ability to bind to or not bind to transcription factors.
- inhibition or stimulation of growth of a reporter cell such as a reporter cell at a particular physiological stage. This reporter cell may be selected from the group consisting of bacteria, fungi, protozoa, helminth, algae, plants, invertebrates, vertebrates, mammalian cells, somatic human cells, pathogenic microorganisms, agricultural pests, cells infected by an intracellular pathogen, virus-infected cells, tumor cells. The reporter cell may also be a whole live organism, e.g., bacteria, fungi, protozoa, algae, plants, invertebrates, insects, tardigrada, parasites, agricultural pests.

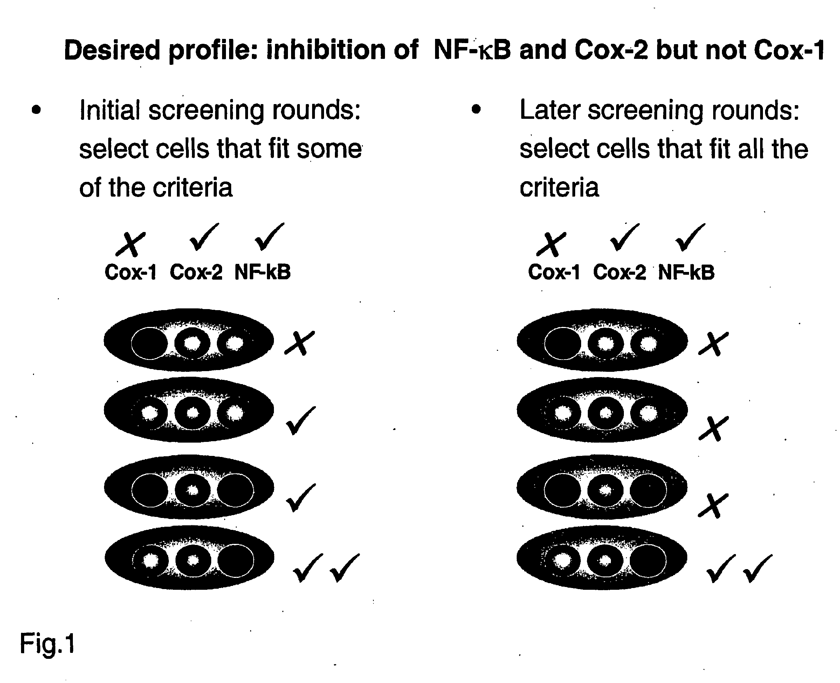

Preferably the invention comprises selection of cells meeting a predetermined value of more than one parameter, such as selection of cells meeting a predetermined value of each parameter. In early rounds of screening it may be chosen to select cells meeting one or a few of several selection criteria and in later rounds of screening cells meeting all selection criteria are often selected as illustrated in Figure 1.

Any number of screens may be performed on the cells simultaneously. For example one may screen for at least 3 parameters related to the two or more functionalities. One may also screen for at least 4 parameters related to the two or more functionalities. Higher numbers are also possible, so that the method comprises performing at least one screening for at least 5 parameters related to the two or more functionalities, such as at least 6 parameters, for example at least 7 parameters, such as at least 8 parameters, for example at least 9 parameters, such as at least 10 parameters, for example at least 15 parameters, such as at least 20 parameters, for example at least 25 parameters, such as at least 50 parameters.

The type and strength of the selection criteria/criterion may be changed and/or increased for at least some of the repeats as described further below.

Another aspect of the invention relates to optimising compounds using a method for evolving a cell producing at least one compound, said compound having two or more predetermined functionalities, said method comprising the steps of
a) providing a composition of cells, said composition of cells comprising cells with at least two heterologous expressible nucleotide sequences, at least one of said heterologous sequences being located on an artificial chromosome in said cell, at least 2 cells of the composition containing different heterologous expressible nucleotide sequences, said cells being denoted producer cells,
b) performing one screening of said population of cells for at least 2 parameters related to the functionalities, and determining a selection criterion for each parameter,
c) selecting cells meeting at least one predetermined selection criterion,
d) combining expressible nucleotide sequences of the selected cells with expressible nucleotide sequences from another composition of cells, thereby obtaining at least one new composition of cells, said new composition of cells comprising cells with at least two heterologous expressible nucleotide sequences, at least one of said heterologous sequences being located on an artificial chromosome in said cell, at least 2 cells of the composition containing different heterologous expressible nucleotide sequences,
e) optionally repeating steps b) to d) until at least one cell has acquired a compound having the at least two predetermined functionalities.

The evolution methods according to the present invention combine the potential for evolution of cells based on assembly or insertion of a large collection of heterologous genes into artificial chromosomes vectors that can be inserted into host cells for co-ordinated expression of the genes located on the artificial chromosomes. The expressed genes interact with each other and with the genes of the host cells and create novel or modified synthetic pathways. The heterologous genes are combined with other heterologous genes form other host cells (and as the case may be from other sources) in a stepwise evolution of the cells to acquire the ability to produce a compound fulfilling the selection criteria.

The term "expressible sequence" is used with its normal meaning, i.e. a sequence capable of being expressed in the host cells in question.

In step d) the combination of expressible sequences may be combined in a one-step process, or by a process of several steps of mixing or combining the expressible sequences, independent of whether the combination relates to combination of expressible sequences as such or combination of expression cassettes or combination of chromosomes.

Step e) may be repeated until cells having the desired functionalities are obtained. Thereby step e) may be repeated from 0 to at least 200 times, preferably from 0 to 150 times, such as from 0 to 100 times, such as from 0 to 80 times, such as from 0 to 60 times, such as from 0 to 20 times.

The screening functionality is the functionality during the screening rounds. The screening functionality is normally different from the desired final functionality, but in some embodiments the screening functionality is identical with the desired functionality. The screening functionality is also referred to as the predetermined functionality in the present context.

The said another composition of cells may comprise cells that contain expressible nucleotide sequences likely to confer at least one of the functionalities to the cells in order to increase the likelihood of reaching the evolution target in as few rounds as possible. The another composition of cells may also previously have been screened for a third functionality, or said another composition of cells may comprise cells capable of expressing at least one predetermined protein/enzyme or synthesising at least one predetermined compound or substance. Further, said another composition of cells may be chosen at random or may be chosen to contain genes homologous to the genes contributing to the desired functionality or it may be chosen from expression states known to produce compounds with at least one of the desired functionalities. Said another composition may also be the first composition, resulting in mixing of heterologouos expressible nucleotide sequences within the same population leading to cells with novel combinations of heterologous expressible nucleotide sequences.

In a further aspect the invention relates to a screening system comprising a producer cell and at least two reporter systems, wherein each of the reporter systems is directed to a parameter related to a functionality of one compound produced by the cell.
The screening system can be used for multiple parameter screening methods and evolution methods according to the invention

The physical layout of the screening system may be in the form of a gel droplet together with the at least two reporter systems. It may also be a cell in a semi-solid or a liquid environment together with the at least two reporter systems. Further examples of the physical layout of the screening system are disclosed in the detailed description part of the present invention.

In a further aspect the invention relates to a method for generation of optimised lead compounds, said method comprising screening a composition of producer cells for at least two parameters related to at least two predetermined functionalities, said composition of cells comprising cells each with at least two heterologous expressible nucleotide sequences, at least one of said heterologous sequences being located on an artificial chromosome in said cell, at least 2 cells of the composition containing different heterologous expressible nucleotide sequences.

By optimised lead compounds is meant that the compounds have more than one predetermined functionality. Traditionally, lead compounds are defined as such if they just have activity against a pharmacological target. By this method lead compounds are identified together with the cells capable of synthesising the lead compounds and as the lead compounds are screened for two or more functionalities they have a higher chance of becoming e.g. a drug. As explained above, this method also speeds up the process of developing lead compounds.

Preferably the lead compounds are drug lead compounds and at least one parameter is related to absorption, distribution, metabolism, excretion or toxicity and at least one further parameter is related to activity against a pharmacological target.

Throughout the present invention it is intended that screening for two or more functionalities also includes the possibility of screening the cells for two closely related functionalities at the same time, such as using two assays for the same enzyme, it being understood that every assay is specific and the activity measured in two closely related assays are diferent. However, preferably the two or more functionalities relate to more different functions.

### Definitions

One screening: In the present context the term "one screening" means one simultanous screening, exemplified by having at least two different assay systems, one for each parameter, simultaneously together with the cell, as opposed to first screening for one parameter and then screening for another parameter. Therefore, one screening means that one cell is subjected to two different screening assays at the same time.

Reporter system: In the present context the term "reporter system" is used to describe the output of an assay for one functionality. A reporter system produces a detectable readout, preferably a fluorescent readout. If different reporter systems are used, each produces a different (preferably fluorescent) readout. Each cell also contains a number of heterologous controllable expressible nucleotide sequences. These expressible sequences enable the cell to produce multiple new compounds. The compounds can then interact with the different reporter systems and lead to the production of fluorescent readouts.

Screening unit: In the present context the term "screening unit" is used to describe a microenvironment wherein the compound and the reporter systems may be brought in contact with each other for facilitating the interactions necessary for the method of the invention.

Expressible nucleotide sequence: a nucleotide sequence capable of being transcribed and optionally translated in the relevant host cell species.

### Oligonucleotides

Any fragment of nucleic acids having approximately from 2 to 10000 nucleic acids.

### Restriction site

For the purposes of the present invention the abbreviation RSn (n=1,2,3, etc) is used to designate a nucleotide sequence comprising a restriction site. A restriction site is defined by a recognition sequence and a cleavage site. The cleavage site may be located within or outside the recognition sequence. The abbreviation "rs₁" or "rs₂" is used to designate the two ends of a restriction site after cleavage. The sequence "rs₁-rs₂" together designate a complete restriction site.

The cleavage site of a restriction site may leave a double stranded polynucleotide sequence with either blunt or sticky ends. Thus, "rs₁" or "rs₂" may designate either a blunt or a sticky end.

In the notation used throughout the present invention, formulae like:
RS1-RS2-SP-PR-X-TR-SP-RS2-RS1
should be interpreted to mean that the individual sequences follow in the order specified. This does not exclude that part of the recognition sequence of e.g. RS2 overlap with the spacer sequence, but it is a strict requirement that all the items except RS1 and RS1' are functional and remain functional after cleavage and re-assemblage. Furthermore, the formulae do not exclude the possibility of having additional sequences inserted between the listed items. For example introns can be inserted as described in the invention below and further spacer sequences can be inserted between RS1 and RS2 and between TR and RS2. Important is that the sequences remain functional.

Furthermore, when reference is made to the size of the restriction site and/or to specific bases within it, only the bases in the recognition sequence are referred to.

### Expression state

An expression state is a state in any specific tissue of any individual organism at any one time. Any change in conditions leading to changes in gene expression leads to another expression state. Different expression states are found in different individuals, in different species but they may also be found in different organs in the same species or individual, and in different tissue types in the same species or individual. Different expression states may also be obtained in the same organ or tissue in any one species or individual by exposing the tissues or organs to different environmental conditions comprising but not limited to changes in age, disease, infection, drought, humidity, salinity, exposure to xenobiotics, physiological effectors, temperature, pressure, pH, light, gaseous environment, chemicals such as toxins.

### Artificial chromosome

As used herein, an artificial chromosome (AC) is a piece of DNA that can stably replicate and segregate alongside endogenous chromosomes. For eukaryotes the artificial chromosome may also be described as a nucleotide sequence of substantial length comprising a functional centromer, functional telomeres, and at least one autonomous replicating sequence. It has the capacity to accommodate and express heterologous genes inserted therein. It is referred to as a mammalian artificial chromosome (MAC) when it contains an active mammalian centromere. Plant artificial chromosome and insect artificial chromosome (BUGAC) refer to chromosomes that include plant and insect centromers, respectively. A human artificial chromosome (HAC) refers to a chromosome that includes human centromeres, AVACs refer to avian artificial chromosomes. A yeast artificial chromosome (YAC) refers to chromosomes are functional in yeast, such as chromosomes that include a yeast centromere. The artificial chromosomes may be linear or circular.

As used herein, stable maintenance of chromosomes occurs when at least about 85%, preferably 90%, more preferably 95%, more preferably 99% of the cells retain the chromosome. Stability is measured in the presence of a selective agent. Preferably these chromosomes are also maintained in the absence of a selective agent. Stable chromosomes also retain their structure during cell culturing, suffering neither intrachromosomal nor interchromosomal rearrangements.

Producer species: a species of cell with two or more heterologous expressible nucleotide sequences contained with expression cassettes.

### Detailed description of the drawings

Fig. 1 shows an example of multiple parameter screening for compounds synthesised by cells. In the example each cell was engineered with 3 different reporter systems. Each reporter system produces a different fluorescent readout Each cell also comprises a number of heterologous expressible nucleotide sequences. These heterologous nucleotide sequences enable the cell to produce multiple new compounds. The compounds can interact with the reporter systems and lead to fluorescent readout(s). In the specific example, compounds that inhibit Cox-2 and NF-κB and do not ihibit Cox1 are desired. In the specific example, it is shown that in early rounds, cells that meet one, two or all three of the criteria are selected. In later rounds, only cells that fit all the selection criteria are selected.

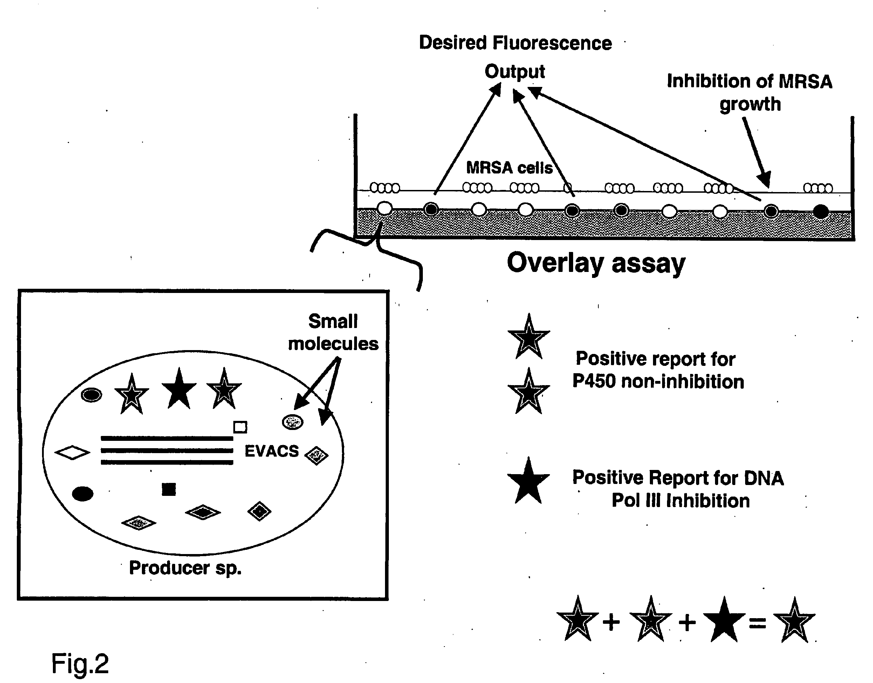

Fig. 2 shows an example of a multiple parameter screen setup for novel antibacterials. It includes S. aureus growth inhibition, DNA Polymerase III inhibition and P450 non-inhibition assays. The screen is assembled by, for example, transforming a library of producer strains with GFP reporter systems for the non-inhibition of a few selected human P450s and for inhibition of recombinant Bacillus subtilis DNA Pol III. The library is then plated and overlayed with an MRSA (Methicillin-resistant *Staphylococcus aureus* )strain. The compounds have to cross the producer's cell wall and reach the MRSA strain thus the screen will also select for compounds that have a reasonable solubility profile. Producer cells in zones cleared of MRSA cells and which produce the desired combination of fluorescent colours are selected.

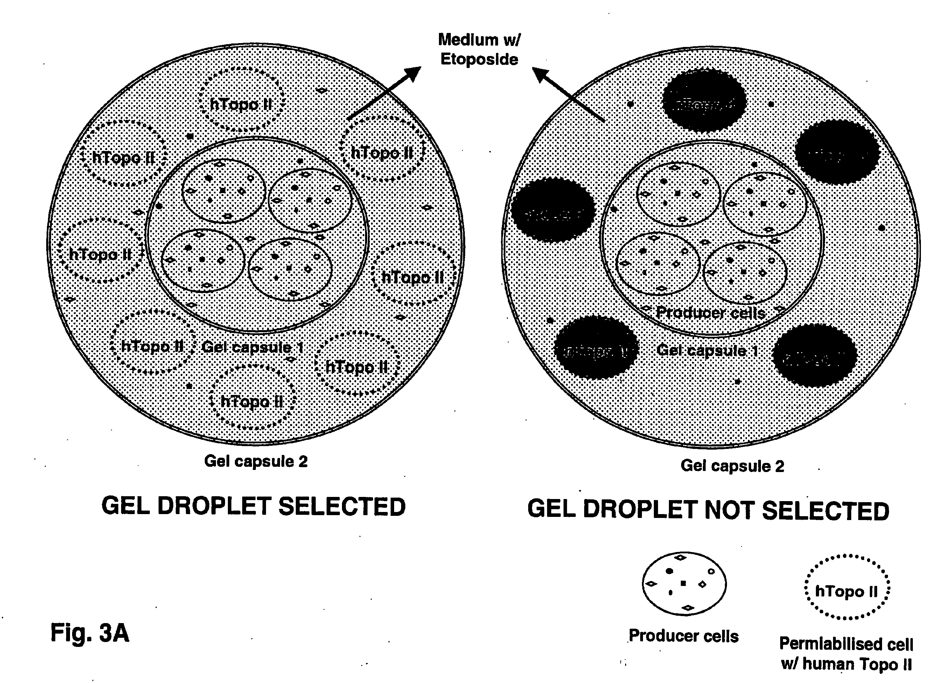

Fig. 3A Illustrates a multiple parameter screen setup for cancer chemoprotectants. In the assay, a producer species library is gel encapsulated so that on average each capsule has 1 producer cell. The cells in the droplets are the allowed to grow for a few generations in order to have multiple copies of the producer species and thus more compound being produced. These clonal cell lines are the double gel encapsluated with a permeabilised yeast expressing human DNA topoisomerase II other than its native gene. The gel droplet environment contains a topo II poison and a marker for the specific double strand breaks produced by this enzyme. Gel droplets where yeasts in both inner and outer droplet have survived and that are not stained are selected.

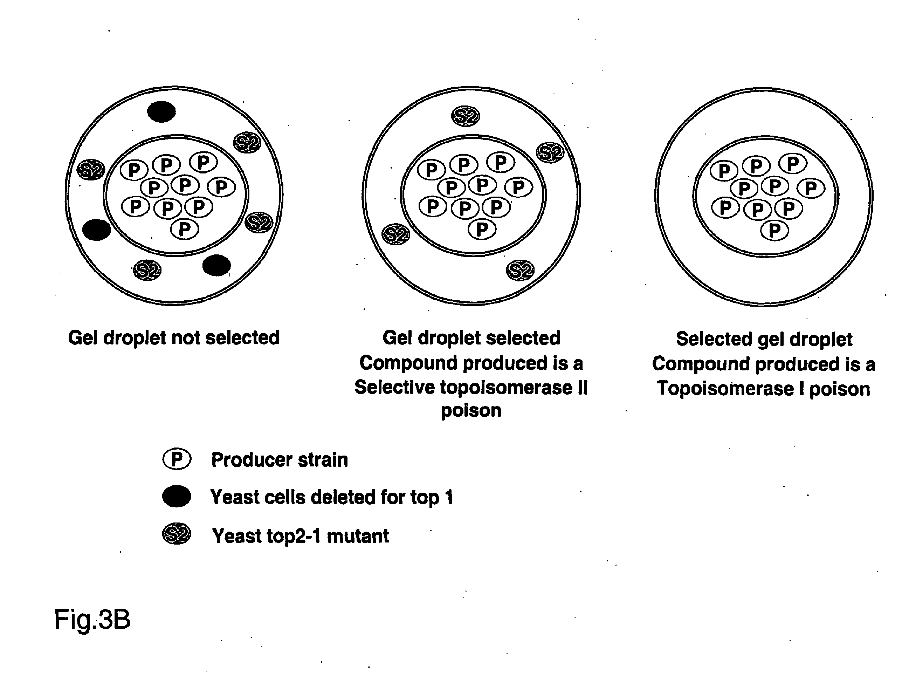

Fig. 3B shows a multiple parameter screen set-up for novel chemotherapy agents that are selective DNA topoisomerase II poisons.

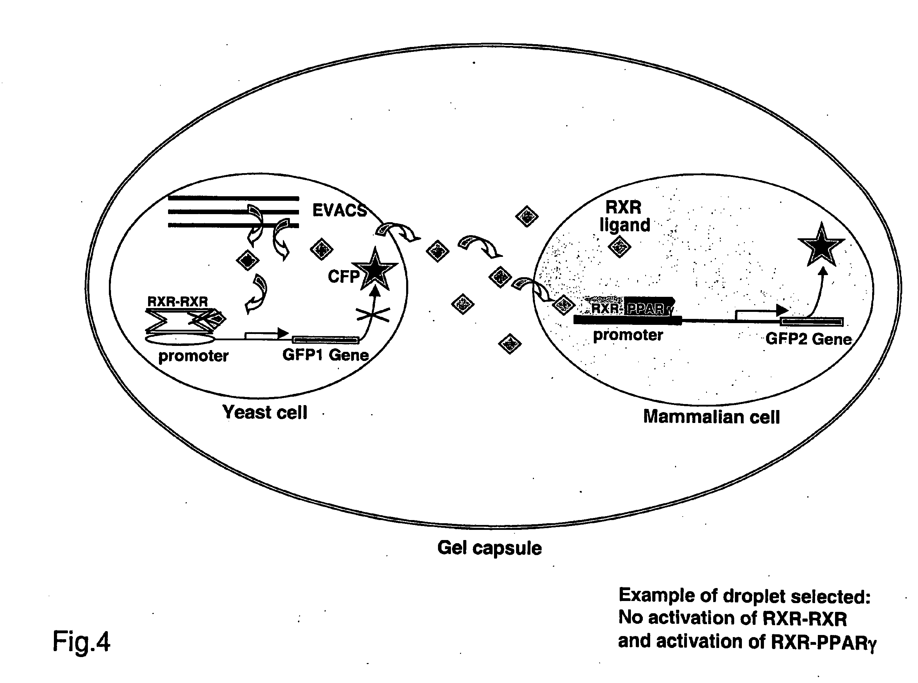

Fig. 4 shows a multiple parameter screen set up where the gel encapsulation of a producer species library reporting RXR-RXR activation with a mammalian cell line reporting RXRα-PPARγ activation. Gel droplets that indicate RXRα-PPARγ activation but not RXR-RXR are selected.

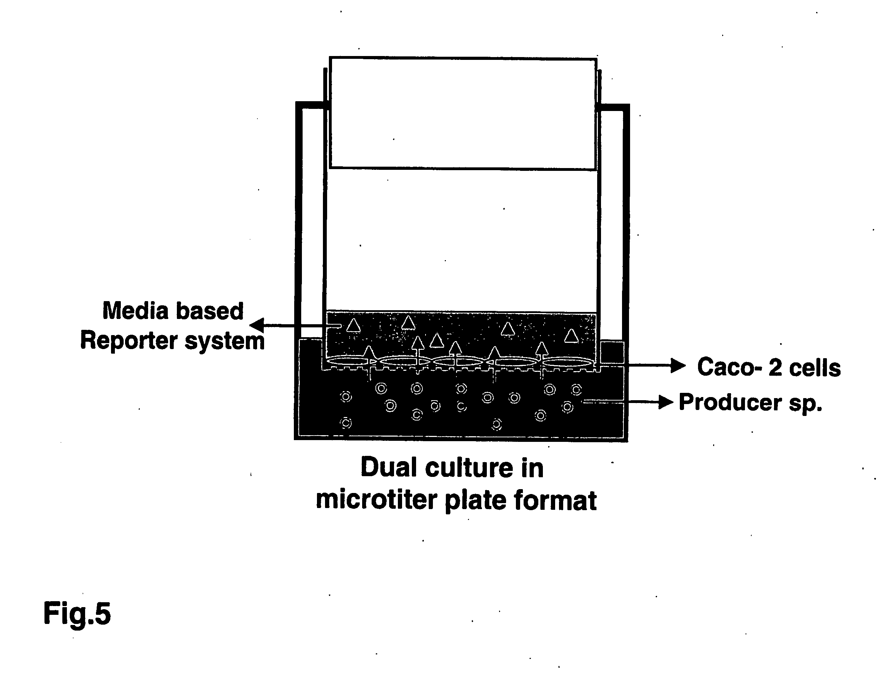

Fig. 5 shows an example of a multiple parameter screen for absorption and a pharmacological activity. By using a dual culture system and timing the time of cell selection, it is possible to select producer cells that have the desired pharmacological activity and a good absorption profile.

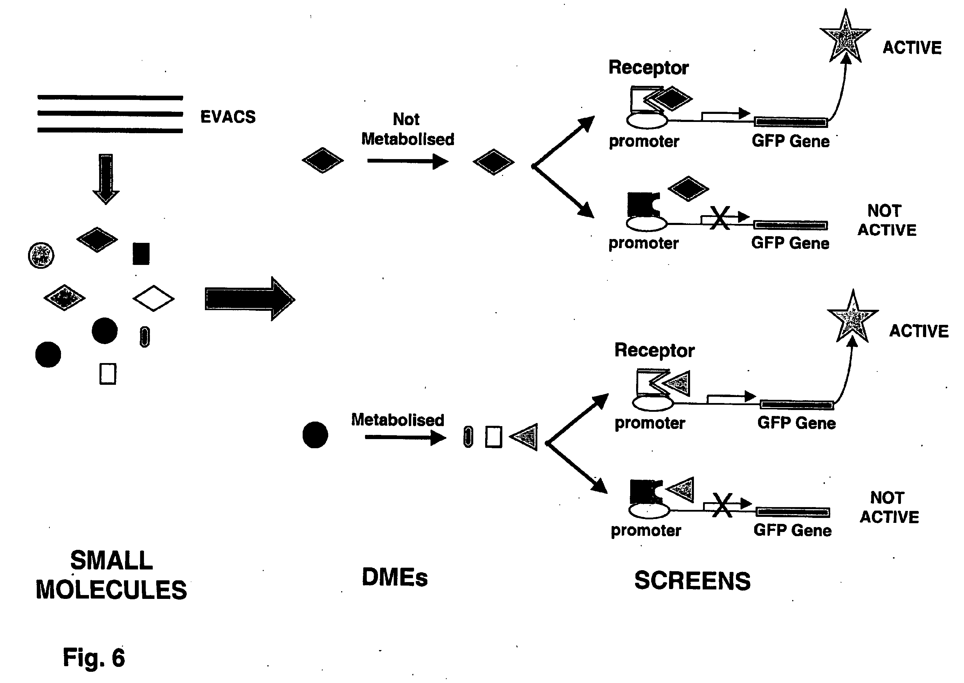

Fig. 6 shows an example of a screening system which minimises the number of false positives generated by compounds that are rapidly metabolised by the human drug metabolising enzymes (DMEs) and also leads to the discovery of compounds that are active after being metabolised and which would otherwise remain undiscovered.

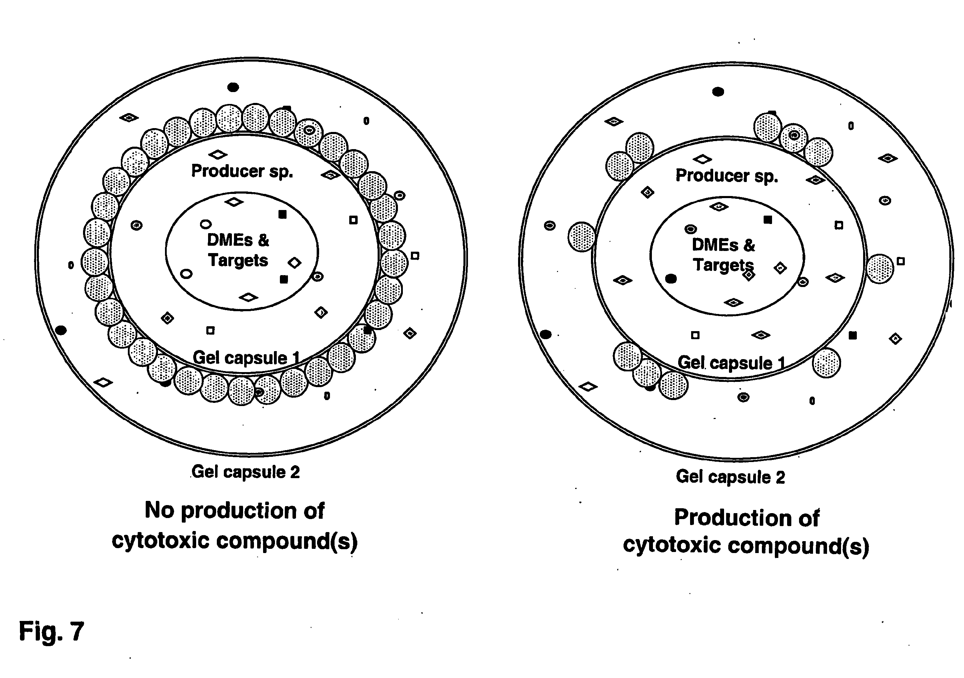

Fig. 7 shows a schematic representation of a screening system of the present invention to evaluate target activity, metabolism by DMEs and cytotoxicity: Using a double gel encapsulation system where in the first droplet are clonal lines of the producer species transformed with the pharmacological target and DMEs, and in the second droplet are hepatocytes, it is possible to screen for target activity, DME metabolism and hapatotoxicity simultaneously.

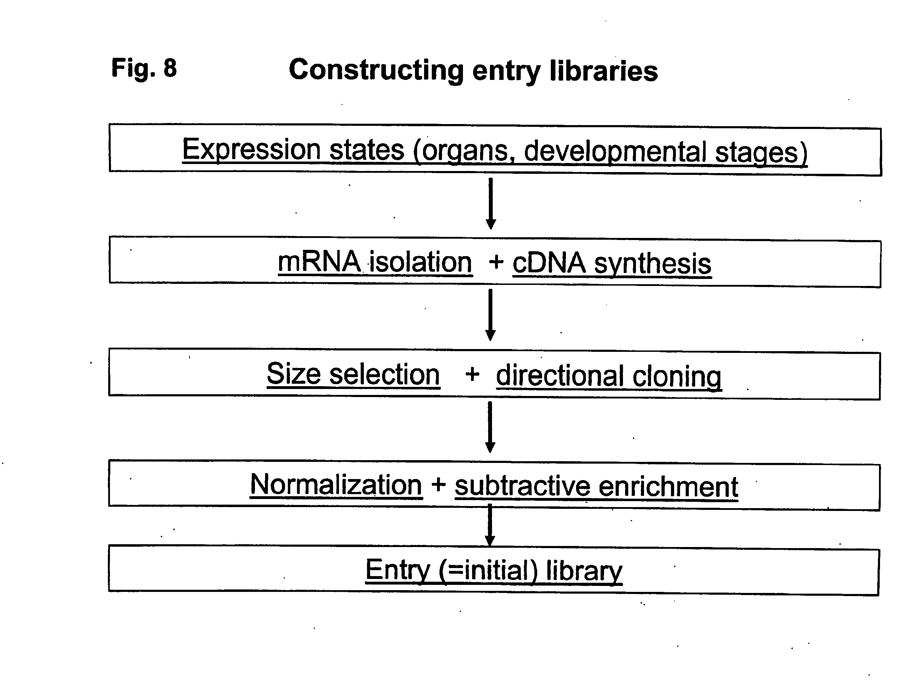

Fig. 8 shows a flow chart of the steps leading from an expression state to incorporation of the expressible nucleotide sequences in an entry library (a nucleotide library according to the invention).

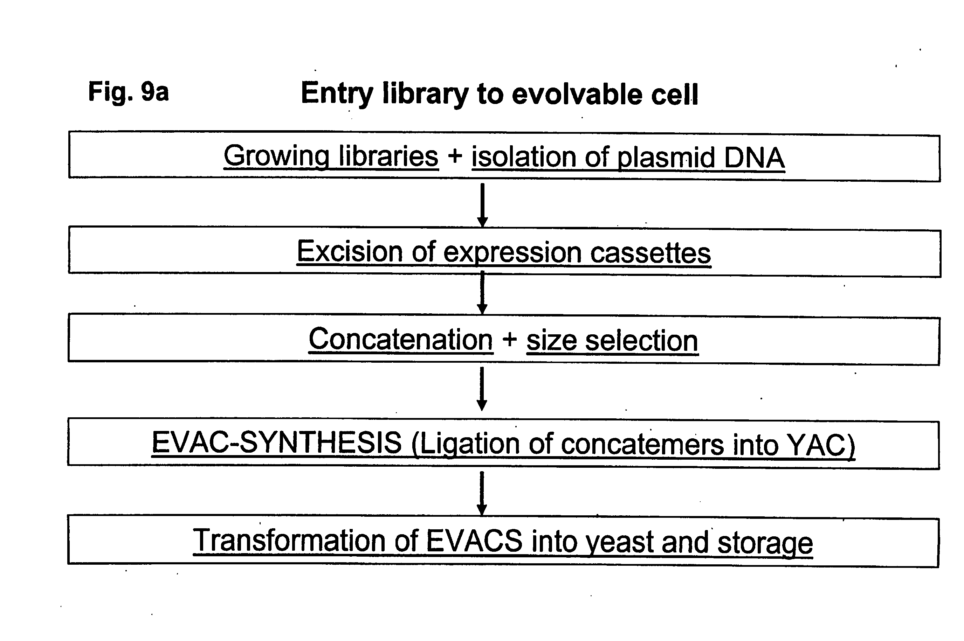

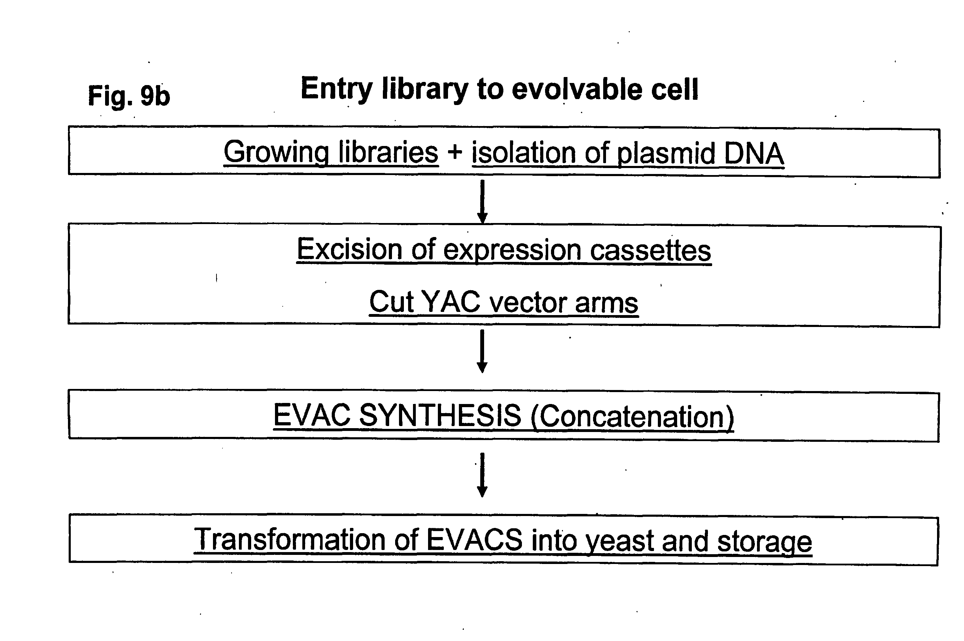

Fig. 9 shows a flow chart of the steps leading from an entry library comprising expressible nucleotide sequences to evolvable artificial chromosomes (EVAC) transformed into an appropriate host cell. Fig. 9a shows one way of producing the EVACs which includes concatenation, size selection and insertion into an artificial chromosome vector. Fig. 9b shows a one step procedure for concatenation and ligation of vector arms to obtain EVACs.

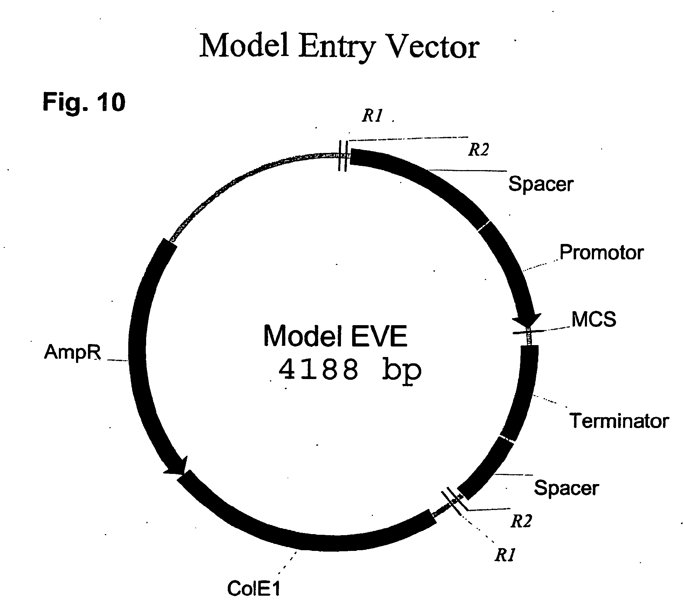

Fig. 10 shows a model entry vector. MCS is a multi cloning site for inserting expressible nucleotide sequences. Amp R is the gene for ampicillin resistance. Col E is the origin of replication in E. coli. R1 and R2 are restriction enzyme recognition sites.

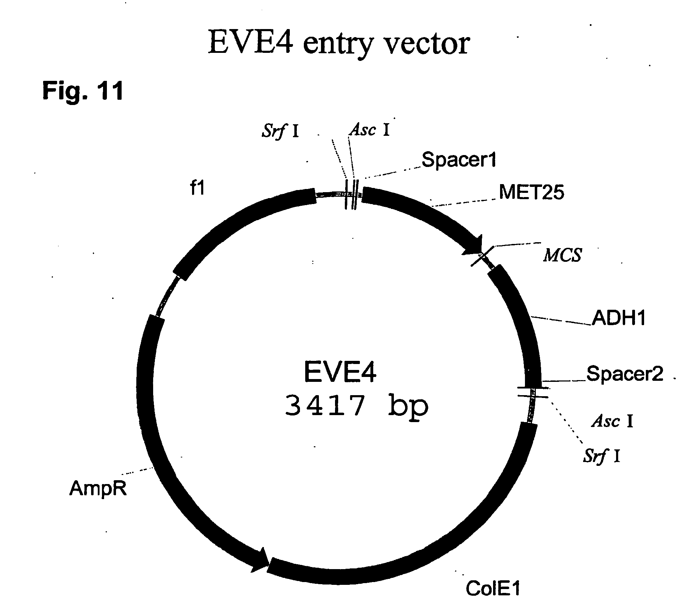

Fig. 11 shows an example of an entry vector according to the invention, EVE4. MET25 is a promoter, ADH1 is a terminator, f1 is an origin of replication for filamentous phages, e.g. M13. Spacer 1 and spacer 2 are constituted by a few nucleotides deriving from the multiple cloning site, MCS, Scfl and Ascl are restriction enzyme recognition sites. Other abbreviations, see Fig. 10. The sequence of the vector is set forth in SEQ ID NO 1.

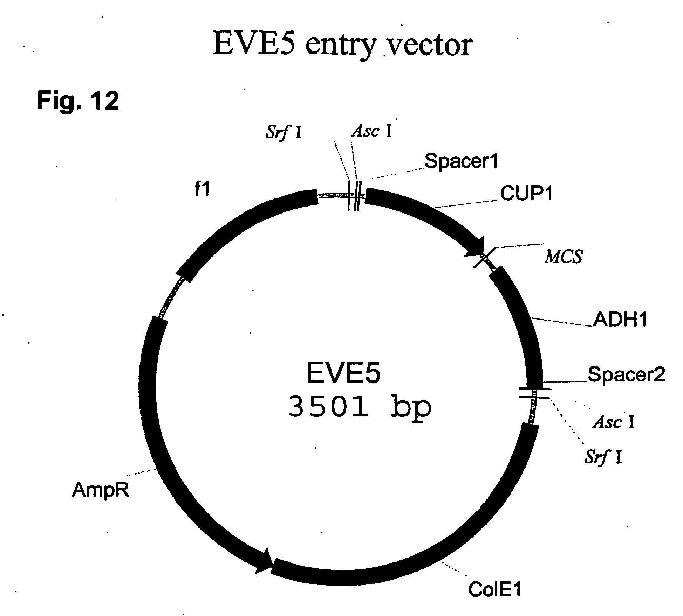

Fig 12 shows an example of an entry vector according to the invention, EVE5. CUP1 is a promoter, ADH1 is a terminator, f1 is an origin of replication for filamentous phages, e.g. M13. Spacer 1 and spacer 2 are constituted by a few nucleotides deriving from the multiple cloning site, MCS, Scfl and AscI are restriction enzyme recognition sites. Other abbreviations, see Fig. 10. The sequence of the vector is set forth in SEQ ID NO 2.

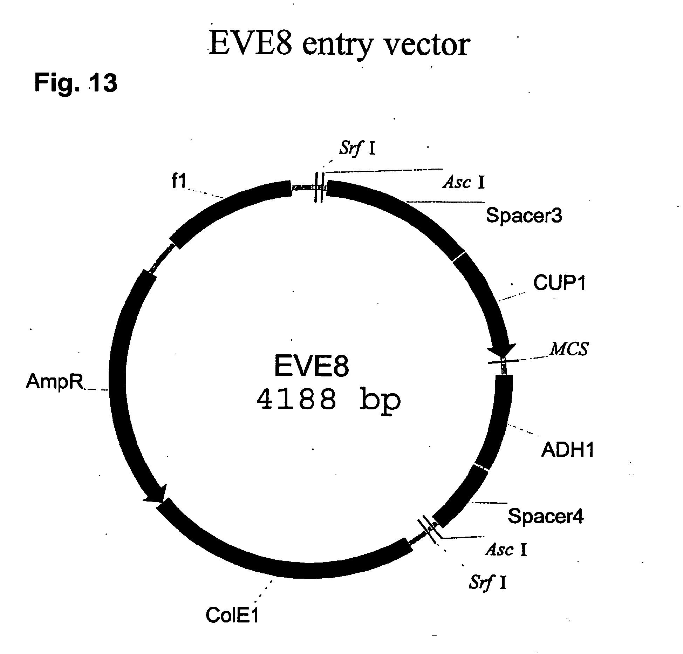

Fig 13 shows an example of an entry vector according to the invention, EVE8. CUP1 is a promoter, ADH1 is a terminator, f1 is an origin of replication for filamentous phages, e.g. M13. Spacer3 is a 550 bp fragment of lambda phage DNA fragment. Spacer4 is a ARS1 sequence from yeast. Scfl and Ascl are restriction enzyme recognition sites. Other abbreviations, see Fig. 10. The sequence of the vector is set forth in SEQ ID NO 3.

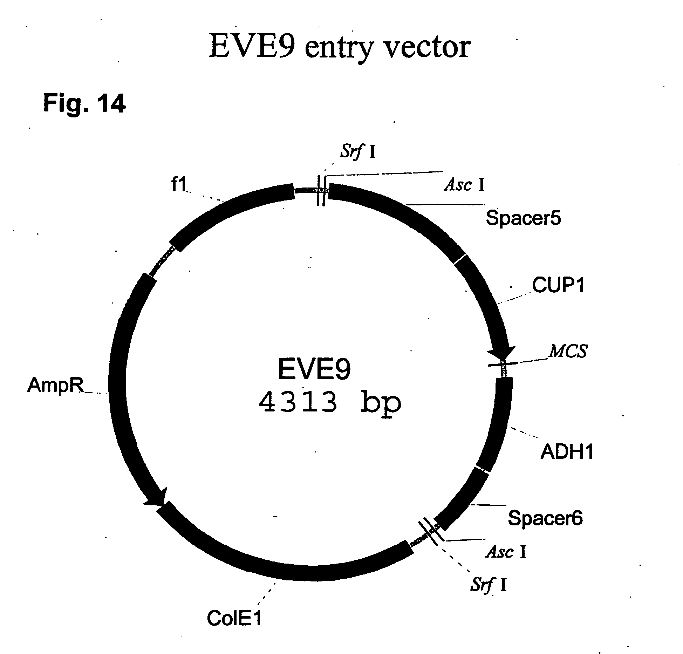

Fig. 14 shows an example of an entry vector according to the invention, EVE9. Met25 is a promoter, ADH1 is a terminator. Spacer 5 and 6 are lambda phage DNA. The nucleotide sequence of the vector is set forth in SEQ ID NO 5.

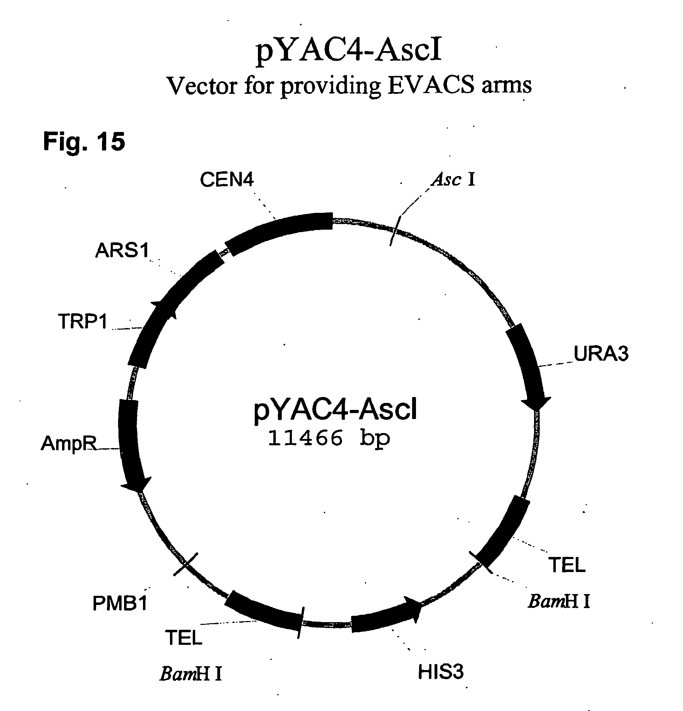

Fig. 15 shows a vector (pYAC4-Ascl) for providing arms for an evolvable artificial chromosome (EVAC) into which a concatemer according to the invention can be cloned. TRP1, URA3, and HIS3 are yeast auxotrophic marker genes, and AmpR is an E. coli antibiotic marker gene. CEN4 is a centromere and TEL are telomeres. ARS1 and PMB1 allow replication in yeast and E. coli respectively. BamH I and Asc I are restriction enzyme recognition sites. The nucleotide sequence of the vector is set forth in SEQ ID NO 4.

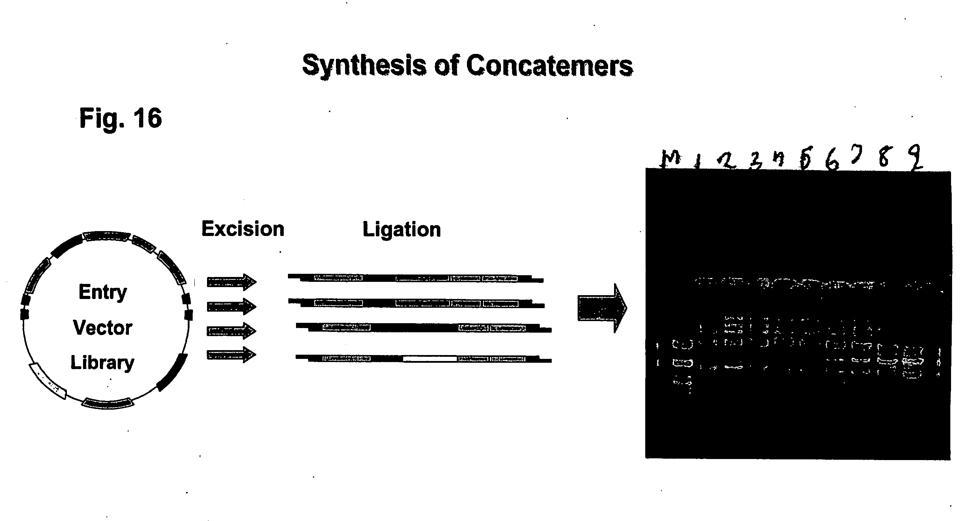

Fig. 16. shows the general concatenation strategy. On the left is shown a circular entry vector with restriction sites, spacers, promoter, expressible nucleotide sequence and terminator. These are excised and ligated randomly.

| Lane | F/Y |
|---|---|
| 1 | 100/1 |
| 2 | 50/1 |
| 3 | 20/1 |
| 4 | 10/1 |
| 5 | 5/1 |
| 6 | 2/1 |
| 7 | 1/1 |
| 8 | 1/2 |
| 9 | 1/5 |

| | |
|---|---|
| Legend: Lane M: molecular weight marker, λ-phage DNA digested w. Pst1. Lanes 1-9, concatenation reactions. Ratio of fragments to yac-arms(F/Y) as in table. | |

Fig 17a and 17b. illustrates the integration of concatenation with synthesis of evolvable artificial chromosomes and how concatemer size can be controlled by controlling the ratio of vector arms to expression cassettes, as described in example 18.

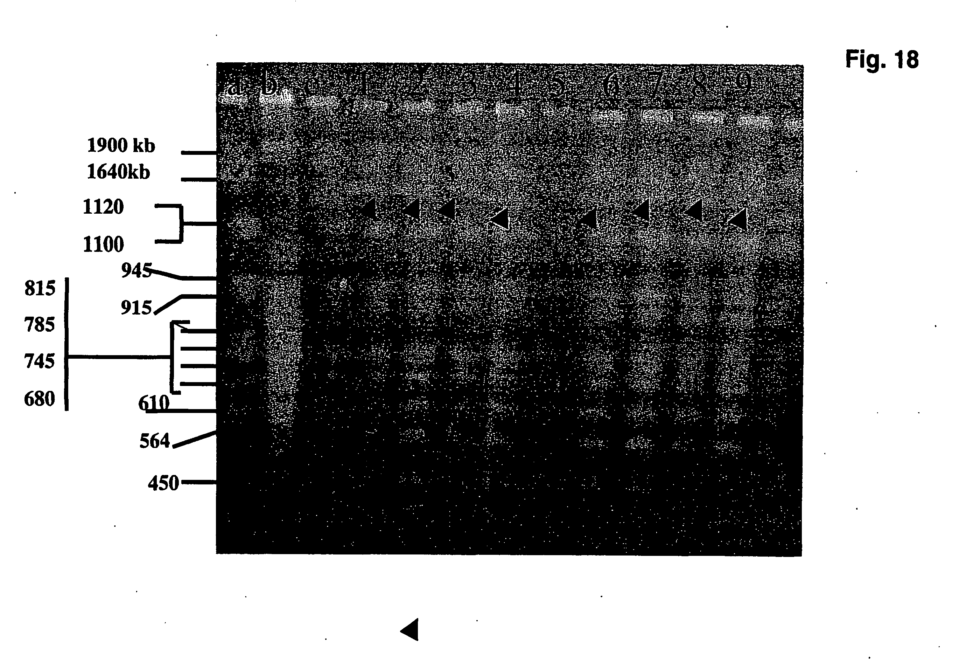

Fig 18. EVAC gel Legend: PFGE of EVAC containing clones :
Lanes. a: Yeast DNA PFGE markers(strain YNN295), b: lambda ladder, c: non-transformed host yeast, 1 - 9 : EVAC containing clones. EVACs in size range 1400-1600 kb. Lane 2 shows a clone containing 2 EVACs sized -1500 kb and -550 kb respectively. The 550kb EVAC is comigrating with the 564kb yeast chromosome and is resulting in an increased intensity of the band at 564 kb relative to the other bands in the lane. Arrows point up to EVAC bands.

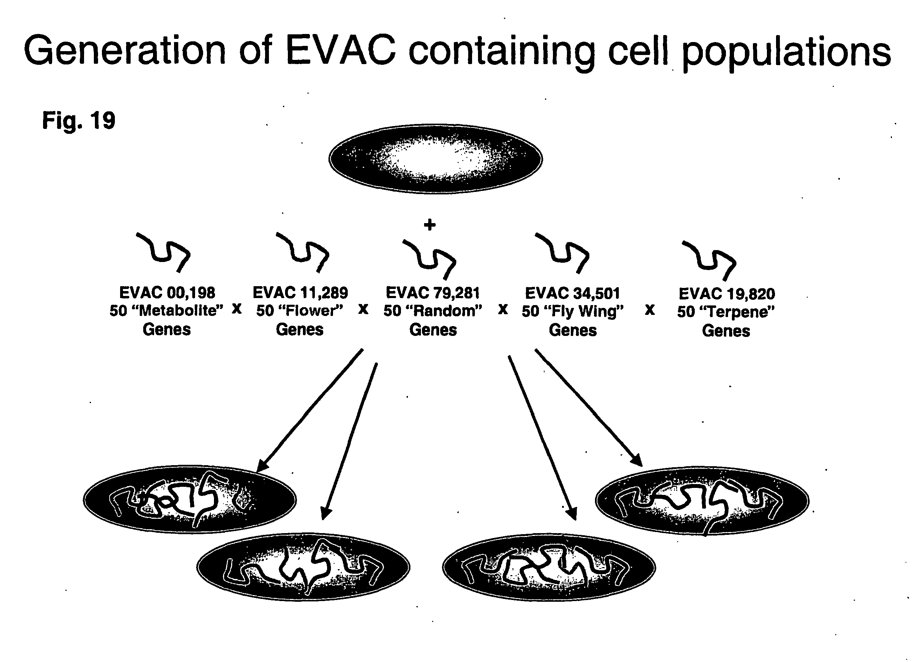

Fig. 19 shows an example of generation of an EVAC containing cell population. EVACs (Evolvable Artificial Chromosome) are artificial chromosomes composed of concatemers of expression cassettes containing heterologous DNA, so that each gene is under the control of an externally controllable promoter. Large numbers of heterologous genes from multiple sources can thus be combined in a single host cell.

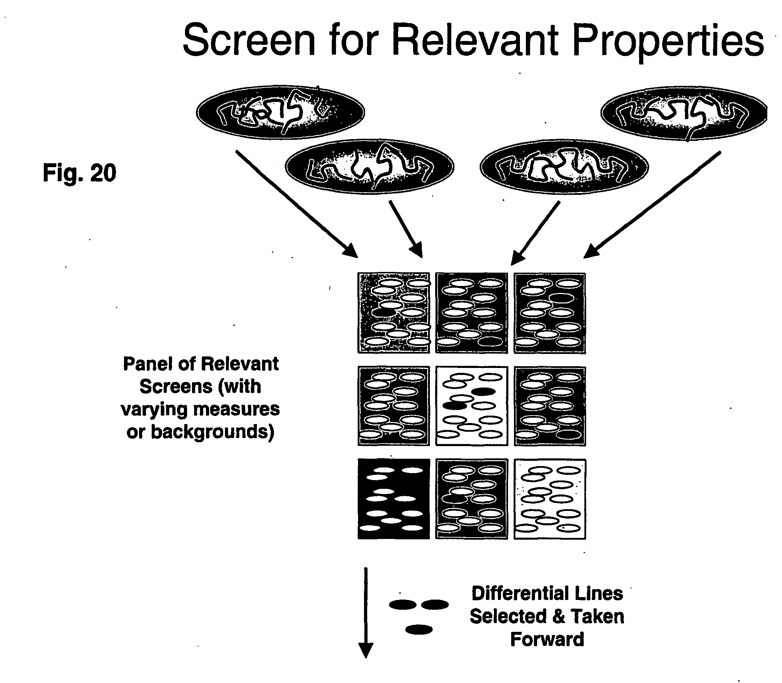

Fig. 20 shows the general principle for screening EVAC containing cell populations. The cell population is amplified and subjected to a panel of screens that are relevant to a desired functionality. Positive subpopulations are selected.

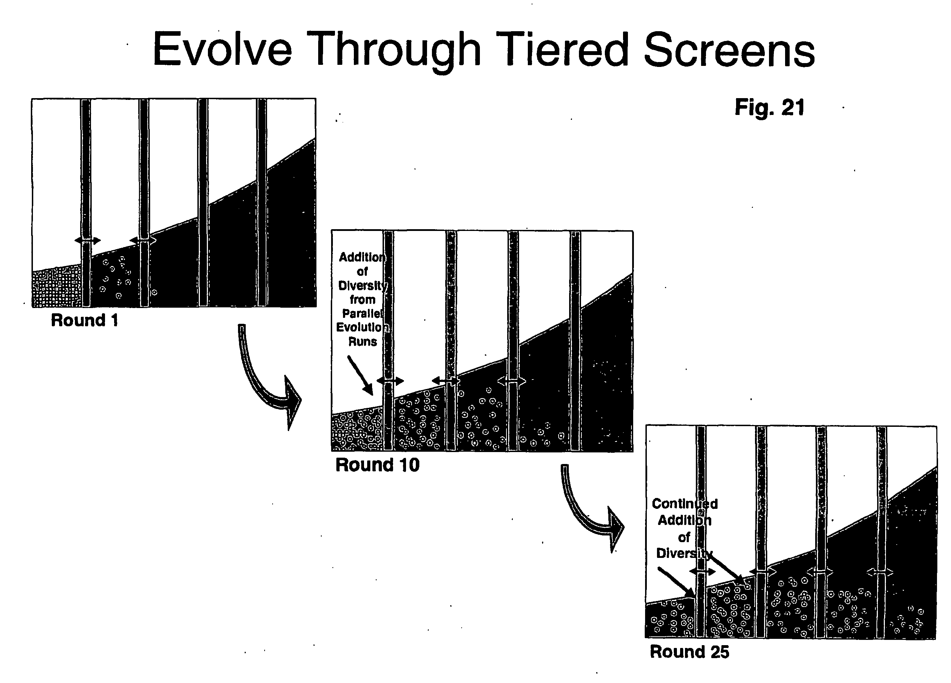

Fig. 21 shows how cell populations evolve through a tiered set of selection conditions, always taking the best performing cell populations further in the process until an optimal functionality/property is evolved.

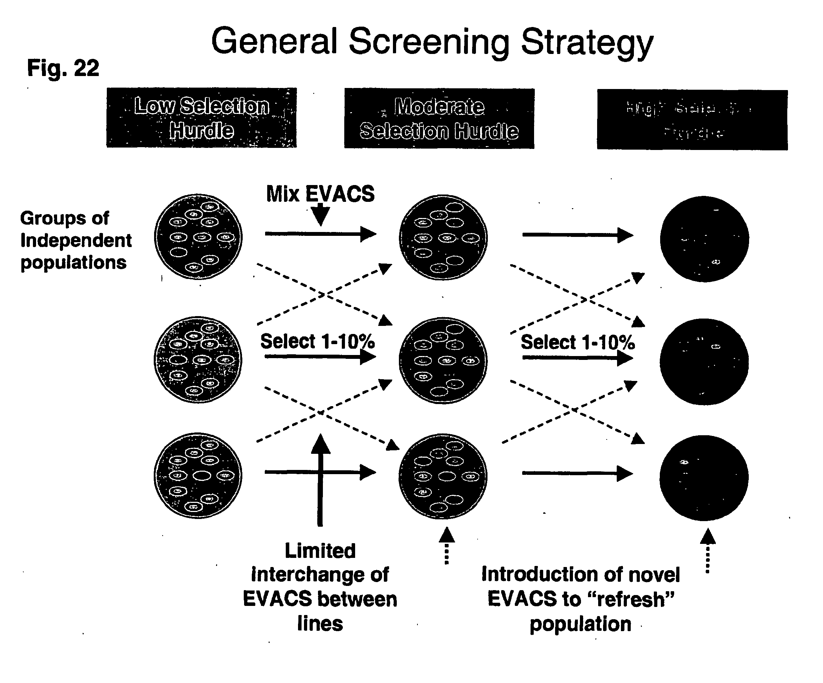

Fig. 22 shows a general screening strategy. Independent populations are subjected to the same set of screens, and genetic material from the different selected sub-populations is combined together with novel genetic diversity introduced between selection rounds.

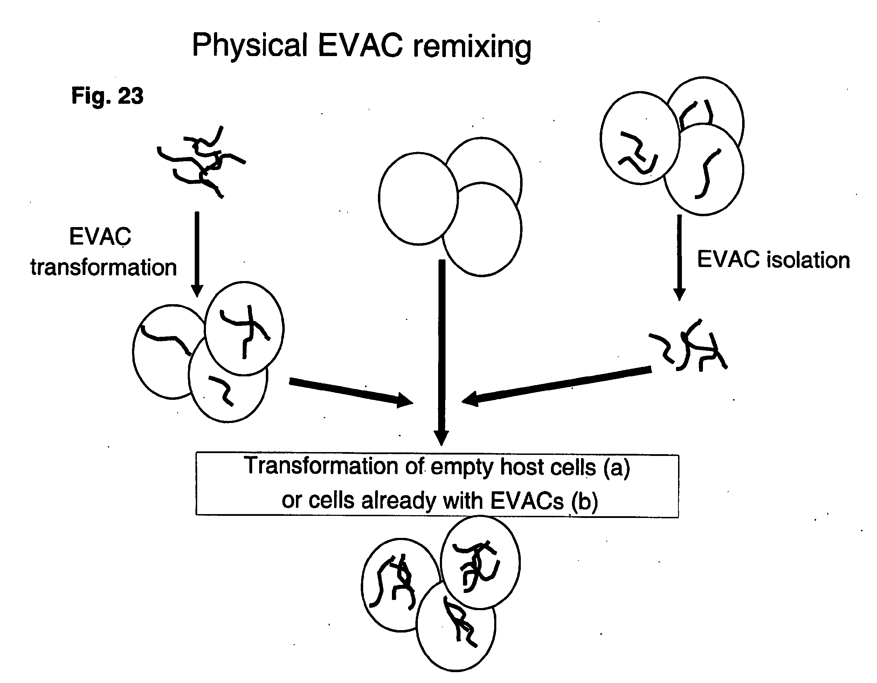

Fig. 23 shows physical remixing of EVACs. EVACs are isolated from the host and used for transformation of either empty host cells or for transformation of host cells already containing EVACS to obtain new combinations of EVACs in each host cell.

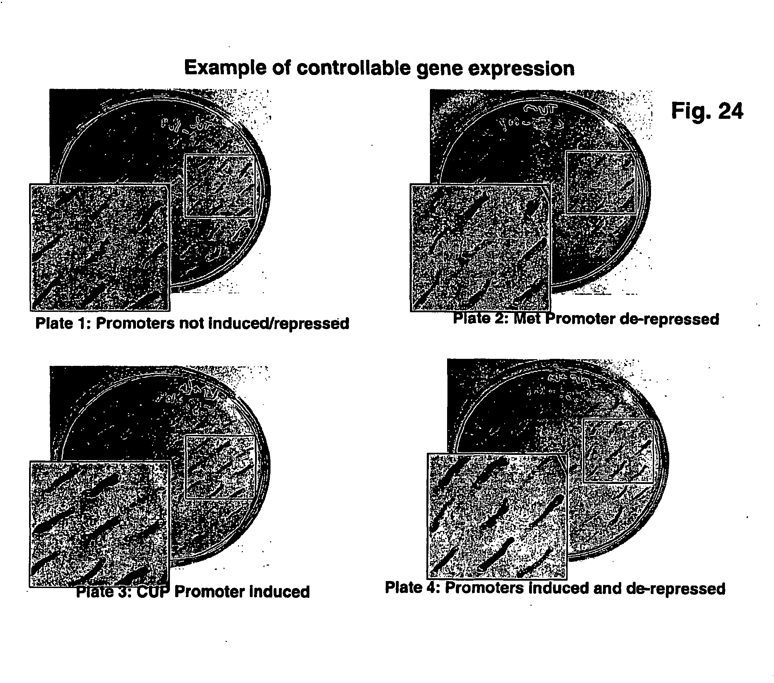

Fig. 24 shows an example of controllable gene expression in a cell population containing EVACs enriched in genes that code for carotenoid synthetic enzymes. The expression cassettes contain either a Met 25 or a CUP I promoter. Orange and red colonies ar obtained as a function of the promoter activation. Intensity of colour and number of coloured colonies increases in the following order: CUP + Met > CUP > Met. Uninduced colonies are white.

### Detailed Description of the Invention

The following provides a background description on how to apply the screening methods according to the invention to evolve cells that produce compounds with multiple functionalities.

The screening and/or evolution may lead to the production of novel optimised molecules as well as production at various scales of compounds of commercial value.

Thus, by "Evolution of a cell" is meant change of a cell's phenotype towards a novel phenotype due to expression of a novel combination of genes. By "evolution of a composition" is meant change of the properties of a composition due to a novel combination of cells expressing a novel combination of genes. The novel combinations are selected using the selection methods of the current invention.

In seeking to evolve molecules with defined pharmaceutical, industrial, nutritional or other properties one must have a method of selecting for those genetic patterns that encode for phenotypes that are consistent with these properties.

Each cell in a cell population, given that it is genetically different from other cells, has an intrinsic variability that can potentially express itself in one or more ways. For the purposes of the current invention the term Output shall be taken to mean a property of the cell that is consequent to the expression of one or more expression cassettes. Optionally the property may be consequent to both the expression of one or more expression cassettes and the expression of a certain set of host genes.

Outputs can be measured according to various different criteria. These criteria may be directly or indirectly linked to the functional or structural properties that are being optimised. Alternatively they may be inversely linked to functional or structural properties that are not desired.

Outputs can be measured either directly or by means of a reporter construct. For the purposes of this document the term Reporter Construct shall be taken to mean a genetic or molecular device for measuring whether a given cell or subset of cells in a cell population vary in respect of a given output from other cells or subsets of cells in the cell population. Example reporter constructs include a genetic construct that produces a fluorescent protein in response to the activation of a transcription factor. Another example of a reporter construct is a coloured/flurorescnt enzyme substrate, to which an enzyme is added that converts the substrate to another molecule with a different colour/fluorescence. Similarly the reporter system could be an enzyme converting a substrate into a couloured/fluorescent product. Should the cell produce an output that inhibits the enzyme, the colour change will not occur.

Other reporter systems could include without limitation the survival of cells subjected to the selection criteria, cells able to metabolise a predetermined substance, cells able to produce a substance that preferentially absorbs electromagnetic radiation at one or more frequencies, cells having enzymatic efficacy in the media etc.

The term Proximal shall be taken to mean a location that is either within the same cell as the expression construct or which is sufficently close to said cell such that the concentration of a molecule or molecules diffusing from an intact or lysed cell, or being actively pumped from the cell, is at least one picomole in the vicinity of the location

Reporter constructs can be placed proximal to a cell either before or after the expression construct have been engineered into the cell. Methods of incorporating the reporter construct into a proximal location include but are not limited to standard transformation techniques, the mating of two different yeast mating types, or systems providing physical proximity between cell and reporter construct, for example gel microdroplet co-encapsulation of cell and reporter construct.

Outputs of cells that may be measured either by proximal reporter contructs or by other means include, but are not limited to:
- Novel spectral properties
- Induced cytochrome oxidase activity
- Changed size, morphology, stickiness or adhesive properties or lack thereof
- Superior growth
- Ability to grow on substrates they cannot normally grow on
- Ability to grow in the presence of a toxin
- Ability to grow on sublethal substrates
- Ability to grow in the absence of normal essential requirements
- Ability to grow on media comprising one or more inhibitors
- Ability to grow under changed physical conditions, such as temperature, osmolarity, electromagnetic radiation including light of certain wavelengths.
- Ability to grow under magnetic field of certain force.
- Secretion or the lack of it from the cell
- The inhibition or prevention of inhibition of an enzyme
- The activation of a receptor.
- The prevention of an activating molecule binding to a receptor.
- The inhibition or promotion of binding of small molecules or proteins to nucleic acid or peptide sequences.
- The inhibition or promotion of transcription or translation of post translational processing.
- Changes in the transport or localisation of molecules within the cell or within organelles.
- Changes in the DNA content or morphology of the cell.
- The production of small molecules with certain properties that allow their selective isolation (e.g. all the chromatography principles available to the skilled practitioner).
- The production of small molecules with certain spectroscopic properties (defined broadly to include visible light, microwaves, IR, UV, X-ray, etc.).
- Changes in the morphology of the cell, including the prevention or promotion of cell differentiation.
- The induction of apoptotic pathways.
- Chemical indicator.

### MULTIPLE PARAMETER SCREENING

For a compound to be useable as a drug it must fulfil multiple functional requirements. It must interact with the target(s) and affect the function of the target in the desired manner. At the same time it should not interact with any other (often similar) targets or have major non-specific effects. Further, it must have the right physical-chemical parameters and be metabolised by the body in an acceptable manner.

Because of this intrinsic difficulty and complexity, the process of discovering and developing drugs has a very poor success rate and is extremely expensive ($600mn per successful compound) and very time consuming (c. 8-12 years from discovery to clinic). Only c. 1 in 15 primary screens produce a compound that makes it into pre-clinical development and only 1 in 10 of these compounds then make it to market. The average pharmaceutical company spends 250 man-years of research and development effort for every compound that enters the clinic. Most pharmaceutical companies are, in consequence, failing to launch new drugs at the rate they require to satisfy their investors.

An alternative to the current process is the evolution of small molecules compounds towards multiple properties simultaneously, with these properties being related, either directly or indirectly to the therapeutic target(s) the small molecule has to interact with, the targets it should not interfere with, the ADMET properties it should fulfil, etc.

### Multiple Pharmacological Activities

Due to the vast number of known targets and relationships between those targets that are currently known, it is not in the scope of the present invention to describe all know targets and their correlations. Table 1 discloses a list of relevant pharmacological targets. The list is included merely to illustrate examples of targets and is not to be interpreted as limiting the scope of the invention.

**Table 1: Drug targets**

| |
|---|
| 3β hydroxysteroid dehydrogenase 3-hydroxy-3-methylglutaryl coenzyme A 5-adenosyl homocysteine hydrolase 5-HT₃ receptor 5-HT₄ receptor 23S rRNA of the 50S ribosomal unit 30S rRNA from 50S ribosomal unit 50S ribosomal unit binding site |
| |
| α2 antiplasmin α-adrenergic receptor α-subunit of Na⁺/K⁺ATPase (3 isoforms) α-amylase α-glucosidase ACTH receptor Adenosine deaminase Adrenocortical steroid synthesis Adrenocorticosteroid receptor Adrenergic receptor β₁, β₂ Adrenocorticotropic hormone Androgen receptor Angiotensin-converting enzyme (ACE) Angiotensin II formation Angiotensin II receptor Antiplatelet/antithrombotic agent Arginine vasopressin receptor Angiotensin receptors, AT1, AT2 ATP-sensitive K⁺ channel Antigcoagulant protein C Antigcoagulant protein S Androgen receptor Apoptosis Aminoacyl tRNA site on 30S ribosomal unit (tetracycline) Acetylcholinesterase Adrenergic receptors α1, α2, β1, β2, β3 Aromatase ATP sensitive K⁺ channels Ascorbic acid |
| |

| |
|---|
| β-amyloid β-adrenergic receptor β-lactamase β-subunit of DNA-dependent RNA polymerase β-adrenergic receptors, b1 β-tubulin subunit of microtubules Benzodiazepine receptor Butyrylcholinesterase Bradykinin receptors, B₁, and B₂ |
| |

| |
|---|
| Carbonic anhydrase, type IV, II Ca²⁺ channel Ca²⁺ channel, Voltage-activated T-type Catechol-O-methyltranferase Calcitonin Cell surface receptors for sulfonylureas on pancreatic β cells *Cell surface receptors for glitinides on pancreatic* β *cells* Cholecystokinin (CCK_{A}, CCK_{B}) Choline acetyltransferase Cholinesterase Carnitine Calcineurin Corticosteroid nuclear receptor Cyclophilin, cyclosporin binding protein CD₃ glycoprotein on T lymphocytes *CD33 receptor* *CD20 receptor* CG-rich DNA (actinomycin) Coagulation factor II, VII, IX, X Corticosteroid adrenocorticotropin receptors Cyclooxygenase 1, 2 (COX-1, COX-2) Cyclic nucleotide phosphodiesterase Cyclooxygenase Cytochrome P450 reductase Cytochrome P450 11β (11β hydroxylase) Cytochrome P450 17α C17-20 lyase Cytochrome P450 aldo, aldosterone synthase Cytochrome P450 side chain cleavage (scc) enzyme Cytochrome P450-dependent sterol 14 α-demethylase |
| |

| |
|---|
| _{D}-alanyl _{D}-alanine synthetase Dihydropteroate synthetase Deoxycytidine kinase Dihydroorotate dehydrogenase Dihydrofolate reductase Dopamine D1-D5 receptors DNA chain elongation factor DNA cross-linking DNA-dependent RNA polymerase DNA gyrase, subunit a DNA methylation DNA polymerases I+III |
| |
| DNA primase DNA topoisomerase DNA alkylation DNA topoisomerase IV DNA alkylation (oxamniquine) |
| |

| |
|---|
| Erythropoietin Endo-β-d-glucuronidase Estrogen receptor |
| |

| |
|---|
| Factors VII; VIII Fusion protein (respiratory syncytial virus) FKBP, tacrolimus binding protein, FK506 binding protein Folic acid Follicle-stimulating hormone (FSH) FSH receptor |
| |

| |
|---|
| Glycerol phosphate oxidase GABA_{A} receptor (6α variants, 3β, 2δ, 3γ variants GABA transaminase GABA_{A}-associated ion channel Glutamic acid decarboxylase Glutamate/aspartate receptors, AMPA, GLU 1-4, KA, GLU 5-7, NMDA 1,2_{A-D}, mGLU 1-7 Glycinamide ribonucleotide transformylase Granulocyte colony-stimulating factor receptor GHRH receptor *Glucagon receptor* Glucoamylase Glucocorticoid receptor (GR) GnRH receptor Gonadotropin releasing hormone (GnHR) Guanylyl kinase G-protein coupled adenosine receptor Ganglionic adrenergic neurons/norepinephrine transporter Guanylate cyclase (nitroprusside) Guanylyl cyclase (NO) Granulocyte colony-stimulating factor Granulocyte-macrophage colony-stimulating factor Growth hormone receptor Growth hormone-releasing hormone (GHRH) Glycine receptor α, β |
| |

| |
|---|
| H⁺, K⁺ ATPase, proton pump H₁ histamine receptor H₂ histamine receptor HCl secretion by gastric cells Helicase HIV Protease HSV thymidine kinase Hemoglobulin protease |

| |
|---|
| Heparin antagonist Hypoxanthine-guanine phosphoribosyl transferase *Her-2* receptor Histamine receptors H₁, H₂, H₃ Hepatic sulfotransferase as a catalyst |
| |

| |
|---|
| Intercellular adhesion molecule 1 Interleukin 1 receptor Interleukin (IL-1, -2, -3, -4, -5, -6, -7, -8, -9, -10, -11, -12 Interleukin-2 receptor IGF-1 receptor, IGF-2 receptor Iodothyrinine-59-deiodinase, type 1, type 2 Influenza A virus M₂ protein Inosine 5' phosphatedehydrogenase Insulin-like growth factor 1 Interleukin-2 receptor Inosinate dehydrogenase Interferon a Interferon a receptor Inosine monophosphate dehydrogenase Integrase Interferon α Interferon α receptor Interferon γ Insulin Insulin-like growth factor (IGF-1, IGF-2) Insulin receptor, α and β subunits Insulin transporter |
| |

| |
|---|
| Kallikrein, aprotinin, C-esterase, α2 macroglobulin Kinin |
| |

| |
|---|
| _{L}-alanyl racemase L-aromatic amino acid decarboxylase L-type voltage-sensitive Ca²⁺ channel Leukocyte integrins Leukotriene A hydrolase Leukotriene B₄ receptor Leukotriene C₄ receptor Leukotriene C synthase Leukotriene D₄/E₄ receptor Lipocortin (protein), inhibits phospholiphase A₂ Lipoxygenases (12-lipoxygenase (platelets), 5-lipoxygenase (leukocytes) LH/choriogonadotropin (CG) receptor Luteinizing hormone (LH) Lactamase Lipoprotein lipase |
| |

| |
|---|
| M₁ receptor, muscarinic cholinergic µ and δ receptor in gastrointestinal tract Macrophage colony-stimulating factor |

| |
|---|
| Microbial dihydrofolate reductase Microtubular protein Mineralocorticoid receptor Mineralocorticoid receptor (MR) Monoamine oxidase (MAO)-A Monoamine oxidase (MAO)-B Muscarinic receptor, M₁, 3 subunits Muscarinic receptor, M₂ , 3 subunits Muscarinic receptor, M₃ , 3 subunits Muscarinic receptor, M₄ , 3 subunits Mycobacterial RNA polymerase |
| |

| |
|---|
| N-acyl hydrolase Na⁺ channel, α1, β1, β3 Na⁺ channel α, β, γ Na⁺/Cl-symporter Na⁺/K⁺/2Cl-symporter Niacin receptor Nicotinic acid Nicotinic receptor Nicotinic cholinergic receptors, muscle N_{M} α, β, δ, γ, ε Nicotinic cholinergic receptors, neuronal, N_{N} α2, α3, α4, α5, α6, α7, α8, α9, β2, β3, β4 Neuramidase Neuropeptide Y, Y1, Y2 receptors Noradrenaline transporter |
| |

| |
|---|
| Opioid receptors µ₁₋₂, δ₁₋₂, κ₁₋₃ Oxytocin & receptor |
| |

| |
|---|
| Platelet-derived growth factor Parathyroid hormone (PTH) Peroxidase Progesterone receptor Prolactin Prolactin receptor Parasite β-tubulin Parasite dihydrofolate reductase Parasite glutamate gated Cl⁻ channel Penicillin-binding protein 1a (PBP 1a, 1b), transpeptidase PBP 2a, 2b PBP 3, 4, 5, 6, 7 Platelet glycoprotein IIb/IIIa (fibrinogen receptor) Plasma protein transferrin (β1 glycprotein) Pyridoxine receptor Penicilloyl enzyme Peptidyl site of the 50S ribosomal unit Primase Phosphodiesterase (type IV, cyclic nucleotide phosphodiesterase) Phospholiphase A₂, C Platelet-activating factor Prostacyclin synthase |
| |
| Plasmodial heme polymerase Progesterone receptor Pyridoxine Phospholipase Cβ Purine receptors, P1 (A_{1,2a,2b,3}), P_{2X}, P_{2Y} Peroxisome proliferator-activated receptor Pancrelipase Potassium channel Prostaglandin 15-OH dehydrogenase Prostaglandin D-DP receptor Prostaglandin E1, E2, E3-EP receptor Prostaglandin F-FP receptor Prostaglandin I2-IP receptor Prostaglandin I₂ (PGI₂) receptor Prostaglandin F₂ receptor Prostaglandin synthetase Prostaglandin I₂ receptor |
| |
| Reverse transcriptase Ribosomal protein from 50S ribosomal unit (streptomycin) Rh 0 Riboflavin receptor Retinoic acid α, X receptors Ribonucleoside diphosphate reductase Ribonucelotide reductase |
| |
| Somatostatin Somatostatin receptors, several Steroid 5 α reductase 1, 2 Sucrase Squalene epoxidase Stem cell factor, c-kit ligand Serotonin receptors (5-HT) 5-HT_{1A-F}, 5-HT_{2A-C}, 5-HT₃, 5-HT₄₋₇ Succinic semialdehyde dehydrogenase Spindle formation Scission of DNA Secretion of vasopressin K receptor |
| |
| Topoisomerase I, II, III, IV Tubulin Thrombopoietin Thrombin Tissue plasminogen activtor Thymidylate synthetase Tachykinins, NK1, NK2, NK3 Tryptaminergic receptor Thromboxane A₂ TP receptor, platelet and non-platelet Thromboxane synthase Thyroid-stimulating hormone (TSH) receptor, TRα 1,2, TRβ 1,2 Tumor necrosis factor receptor Trypanothione reductase |
| Type I cyclic nucleotide phosphodiesterase Type III cyclic AMP phosphodiesterase Type V cyclic nucleotide phosphodiesterase Transpeptidase Thymic lymphocyte antibodies Tumour necrosis factor alpha |
| Thiamine |
| |
| Uridine monophosphate pyrophosphorylase |
| |
| Vascular cellular adhesion molecule 1 receptor Vasopressin receptors V₁ₐ, V_{1b}, V₂, Viral DNA polymerase Vitamin A nuclear receptor Vitamin E Vitamin K & receptor Vitamin B₁₂ receptor Vitamin D nuclear receptor |
| Voltage-activated Ca²⁺ channel, L-type |

Below are examples of diseases and the different targets involved in these diseases and strategies of how to develop optimised compounds. It is also presented in outline examples of how new potential drugs for these targets would be screened and/or evolved using the present invention.

### 1) Disease Target: Bacterial infections (inhibition of DNA Polymerase III, P450 inhibition and Multi-drug resistance S. aureus growth inhibition)

The widespread emergence of resistance has significantly limited the efficacy of classical antibiotic therapy for bacterial disease. Fuelled largely by the excessive and often unnecessary use of antibiotics in humans and animals, antibiotic resistance has resulted in increased patient morbidity, mortality and overall cost of health care. Methicillin-resistant *Staphylococcus aureus* (MRSA) is now the most prevelant nosocomial pathogen in the United States, and the enterococci, as opportunistic pathogens, are among the top four causes of nosocomial infection. Indeed, the percentage of enterococcal isolates resistant to essentially every antibiotic, including vancomycin, continues to increase. Thus a premium is placed upon the discovery of inhibitors that function by a novel or at least different mechanism than currently approved antibiotics, as these would be expected to circumvent current bacterial resistance mechanisms. *S*. *aureus* is a very important human pathogen and has favorable growth characteristics for use in high-throughput screening. Use of an antibiotic-resistant strain will a *priori* select for hits that have activity against a multi-drug resistant strain.

DNA Polymerase III is a DNA polymerase-exonuclease (Pol-Exo) that is essential for the replicative DNA synthesis of Gram positive organisms. Since DNA Pol III is essential for the replication of Gram positive bacteria, the inhibition of DNA Pol III offers a specific and alternative way to treat antibiotic resistant gram positive bacteria.

Many patients with severe disease may be administered multiple anti-infectives as well as other drugs to treat (non-infectious) underlying disease. In this case, drug classes that are not metabolized via the major P450 liver enzymes are preferable.

### Desired therapeutic profile:

- *Gram positive-specific:* Systemic administration of agents with a very broad spectrum may have the undesired effect of creating resistance in the normal host Gastrointestinal flora. Therefore, more disease-specific antibiotics might have an advantage in gaining hospital formulary approval and overall wider acceptance.
- *Orally-active:* The ideal drug candidate would be orally-active with additional formulations for intravenous use. Multiple dosing is acceptable however anything approaching continuous infusion requires very careful consideration. Improvement or equivalence with dosing regimens of competitive therapies is important.
- *Safety*: The ideal drug candidate would be microorganism-specific and devoid of significant side-effects and drug interactions within at least 10-fold of Cₘₐₓ in the therapeutic dosing range.

### Multiple parameter screens:

A multiple parameter screen would thus include S. aureus growth inhibition, DNA Polymerase III inhibition and P450 inhibition. A screen could be assembled by, for example, transforming a library of producer strains with recombinant Bacillus subtilis DNA Pol III combined with the inclusion of P450 enzymes and reporter substrates in the surrounding medium. The library would then be plated and overlayed with an MRSA strain. An assay where the compounds have to cross the producer's cell wall and reach the reporter strain will also select for compounds that have a reasonable solubility profile.

Figure 2 exemplifies such a multiple parameter screen where producer cells in zones cleared of MRSA cells and which produce the desired combination of fluorescent colours would be selected.

### 2) Disease target: Cancer - Inhibition of solid tumour growth and prevention of metastasis (inhibition of NF-κB, inhibition Cox-2, no inhibition Cox-1)

Cancer is the second leading cause of death in the US, causing one in every four deaths. Existing treatments for surgically inoperable cancers include chemotherapy and radiation treatments. These are highly toxic because they are non selective or at best only partially selective. There exists a critical need for new therapeutics to inhibit tumor growth and prevent metastasis. A premium is placed upon molecules that prevent metastasis and which work through a selective mechanism so as to avoid or minimize side effects.

Nuclear Factor κB (NF-κB) is a transcription factor that, by regulating the expression of multiple inflammatory and immune genes, plays a critical role in host defense and several pathogenic processes. Its most common inducible form is composed of the proteins p65 and p50, and usually exists as a molecular complex with one of several inhibitory molecules, the IκBs, in the cytoplasm. Proteins that are regulated by NF-κB include TNFα, IL-1β, IL-2, IL-6, IL-8, iNOS, COX-2, intercellular adhesion molecule-1 (ICAM-1), vascular-cell adhesion molecule-1 (VCAM-1) and E-selectin (Cancer J., 1998, 4, S92; Int J. Biochem. Cell. Biol., 1997, 29, (6), 867).

Activation of NF-κB can lead to the synthesis of the inducible form of cyclooxygenase (COX-2). This enzyme has a critical role in the response of tissues to injury or infectious agents and are essential components of the inflammatory response, the ultimate repair of injury, and carcinogenesis. Several population-based studies have detected a 40-50% decrease in relative risk for colorectal cancer in persons who regularly use Aspirin and other NSAIDs. Attempts to determine the molecular basis for these observations found that both human and animal colorectal tumors express high levels of COX-2, whereas the normal intestinal mucosa has low to undetectable COX-2 expression. These findings led to the hypothesis that COX-2 plays a role in colon cancer growth and progression (Faseb, 1998, 12, 1063). Since Aspirin also inhibits NF-κB these findings also suggest that inhibiting NF-κB may prevent tumour growth and progression. Another way in which COX-2 seems to be involved in cancerinogenesis is by protecting cells from apoptosis (J. Nat. Cancer Inst., 1998, 90, (11), 802). Therefore, inhibition of NF-κB can help control tumor growth by one further process since it leads to less COX-2 induced protection from apoptosis. Inhibition of NF-κB also leads to an increase in Tumor Necrosis Factor (TNF) which in turn leads to an increase in apoptosis.

Immense effort is being devoted to developing new molecules that are direct inhibitors of the enzymatic activity of COX-2. However, an alternative approach is to find new agents that can prevent expression of the respective genes coding for the activities since there are already examples that inhibition of a single mediator does not eliminate all symptoms of a disease (Inflamm. Res., 1997, 46, 282).

### Desired therapeutic profile:

- *NF-κB inhibitor with increased selectivity*: There are several drugs that act by partial inhibition of NF-κB but they all produce side effects due to interactions with other targets. Any new NF-κB inhibitor would have to be selective.
- *Selective COX-2 inhibitor with a Cox-2*/*Cox-1 differential inhibitory activity as low as possible:* Prostanoids that are derived from the COX-1 pathway regulate platelet aggregation via thromboxane A2, the function and integrity of gut mucosa, and kidney function via prostaglandin E2 and prostacyclin. Cox-2 is expressed in various cell types, including monocytes, fibroblasts and synovial cells, in response to inflammatory stimuli. Consequently, COX-1 inhibition by NSAIDs is associated with gastrointestinal and renal toxicity, whereas, COX-2 inhibition limits the formation of pro-inflammatory cytokines at the site of the inflammatory response and has anticancer effects.
- *Orally-active:* Given the severity of the medical problem, an orally-active drug would be desired but not essential.
- *Safety*: The ideal candidate would be selective and devoid of significant side-effects and drug interactions. However again, given the severity of many cancers and lack of therapeutic options, there is significant history of compounds that are less-than-ideal in these aspects.

### Multiple Parameter Screen:

A multiple parameter screen set up could for example be the double gel encapsulation of a producer species library with 2 different mammalian cell lines. The first gel capsule would contain the producer cell and a mammalian cell reporting NF-κB and Cox-1 inhibition while the second capsule would contain a second mammalian cell line reporting Cox-2 inhibition. Gel droplets producing the desired fluorescence output would be selected.

### 3) Disease target: Cancer (survival in the presence of DNA Topoisomerase II α poisons, no production of DNA double strand breaks and inhibition of human DNA topoisomerase II activity)

Chemotherapy is one of the most common approaches to the treatment of cancer. All chemotherapy drugs interfere with cell growth, and they all have some form of side effects. These vary from the highly undesirable to side effects so severe as to prevent further chemotherapy.

An underlying problem of chemotherapy is that cancer cells are not that different from normal undifferentiated or fast growing tissues and therefore, killing a cancer cell tends to kill such cells as well. This side effect effectively limits the dose at which the chemotherapeutic can be applied, and hence limits the efficacy that can be achieved. Consequently, there is a need for the development of novel chemotherapeutic agents to overcome these central problems of cancer chemotherapy.

The most common way to address the above problems of cancer chemotherapy is to look for compounds or delivery systems that increase the specificity for cancer cells. However an alternative approach is to use compounds that protect vulnerable normal tissue against the proposed chemotherapeutic agent. Such protectants should of course not be harmful to the normal cells and either not reach, or not be functional in the cancer cells. A number of protectant approaches are in clinical use today.

Many chemotherapeutic agents, e.g., Doxorubicin and Etoposide have a large part of their toxicity (and hence clinical utility) due to the specific way in which they "poison" the enzyme Topoisomerase II, an enzyme with a crucial role in the elongation and termination stages of DNA replication. These drugs stabilise an intermediate DNA/enzyme/drug complex, creating double-stranded breaks in the DNA of treated cells. A second class of structures act by blocking the Topoisomerase II catalytic cycle at other points in the cycle and do not create double-stranded DNA breaks. These two types of compounds are antagonists to each other since they stabilise different points in the cycle. If one binds, the other cannot. Therefore, inhibitors of Topo II can be used to offset the effects of Topo II poisons.

Two highly homologous isoforms of mammalian topoisomerase II have been identified in tumor cells, topoisomerase II α (170 kDa) and topoisomerase II β (180 kDa) (Malonne, H. and Atassi, G., Anti-Cancer Drugs, 1997, 8, 811-822). The two isoforms differ in several biochemical and pharmacological properties, such as optimal salt concentration for *in vitro* catalytic activity, thermal stability and sensitivity to teniposide (a non-intercalative DNA topoisomerase II poison). Topoisomerase II α is the major drug target isoform in mammalian cells (Sehested et al, Cancer Research, 1998, 58, 1460-1468).

The discovery of new inhibitors of DNA topoisomerase II would enable the protection of certain vulnerable tissues against Topo II poisons and hence expand the efficacy of existing chemotherapy drugs and reduce side effects.

### Desired therapeutic profile:

- *DNA topoisomerase II* α *inhibitor:* Any new compound would have to be an inhibitor and not a poison of the enzyme.
- *Reversible inhibition of normal cell growth:* The effects of the drug should only last long enough to off set the effects of the chemotherapeutic agent
- *Orally-active:* Given the severity of the medical problem, an orally-active drug would be desired but probably not essential.
- *Safety:* The ideal candidate would have modest toxicity such that it does not by itself place an additional toxicity burden on the patient.

### Multiple Parameter Screen:

A multiple parameter screen set-up for DNA topoisomerase II catalytic inhibitors is illustrated in Fig. 3A. In the assay, a producer species library is gel encapsulated so that on average each capsule has 1 cell. Each encapsulated cell is allowed to grow for a few generations in order to establish a clonal cell line. These cell lines are then double gel encapsulated with a permeabilised yeast that relies on a human DNA topoisomerase II gene to survive. The gel droplet environment contains a DNA topoisomerase II poison and a stain specific for DNA double strand breaks. Gel droplets where cells have survived in both compartments and that are not stained are selected.

An improved multiple parameter screen set-up for cancer chemoprotectant is illustrated in Fig. 3B. In the assay, a producer species library is encapsulated so that on average each capsule has 1 cell and is allowed to grow for a few generations. These clonal lines are then double encapsulated with a permeabilised yeast that relies on a human DNA Topo II α to survive. The gel droplet environment contains a poison and a DNA double strand break stain. Gel droplets where the yeast cells in the outer layer have survived and that do not fluoresce or are stained are selected.

### 4) Disease target: Diabetes (ligand activation of RXRα, ligand specific activation of RXR-PPARγ, adipocyte differentiation).

Type 2 diabetes is one of the most common chronic diseases and is associated with co-morbidities, such as obesity, hypertension, hyperlipidemia and cardiovascular disease.

Peroxisome proliferator-activated receptors (PPARs) and retinoid X receptors (RXR) are transcription factors belonging to the family of ligand-inducible nuclear receptors. There are three related but distinct PPARs called PPAR-alpha, PPAR-beta/delta and PPAR-gamma that form heterodimmers with RXR. These receptors regulate expression of genes involved in fat and carbohydrate metabolism. RXR is unique among retinoid receptors as it can form homodimers and it can form heterodimers with multiple nuclear receptors including PPARs, retinoic acid receptors (RARs), vitamin D receptor, and thyroid hormone receptor.

PPARγ/RXR heterodimer regulates adipogenesis and insulin sensitivity both when activated by PPARγ ligands and/or RXR ligands. For example, insulin sensitizers, such as the drugs from the thiazolidinedione class (TZDs), exert their antidiabetic effects through a mechanism that involves activation of the gamma isoform of the nuclear receptor (PPARγ).

Activation of RXRα increases activation of PPARγ and insulin sensitivity. Clinical studies show that co-administration of retinoids (LG100268) +TZDs increases insulin sensitivity and glucose uptake by 60%

The retinoid receptors mediate the biological effects of natural and synthetic vitamin A derivatives, such as retinoic acid. RXR ligands interact with many different proteins, including members of the following protein families: RXR, RAR, retinoic acid receptor-related orphan receptor (RZR), cytoplasmic retinoic acid-binding proteins, retinal-binding protein, P-glycoprotein and cytochrome P450. The expression level of each of these proteins is likely to affect the potency and efficacy of retinoids in various cell types.

Rexinoids may have undesirable effects mediated by RXR homodimers or heterodimers partners other than PPARγ. Rexinoids for treating type 2 diabetes should thus be selective for the PPARγ/RXR heterodimer.

### Desired therapeutic profile:

- *Selectivity:* RXR agonists should be selective for the PPARγ/RXR heterodimer.
- *Orally-active:* The ideal drug candidate would be orally-active.
- *Safety:* The ideal drug candidate would be devoid of significant side-effects and drug interactions.

### Multiple Parameter Screen:

A multiple parameter screen set up could be the gel encapsulation of a producer species library reporting RXR-RXR activation with a mammalian cell line reporting PPARγ-RXR activation as well as P450 inhibition. Gel droplets that indicate PPARγ-RXR activation but not RXR-RXR are selected. Figure 4 examplifies such a system.

### Absorption, Distribution, Metabolism, Excretion & Toxicity (ADMET)

Major reasons for the failure of lead compounds in development often involve inappropriate kinetics or toxicity, thus there is a strong need to obtain the relevant information as early as possible in the discovery process in order to spend as little as possible on inadequate compounds. The pharmaceutical and biotech industries are thus currently focusing on transforming the traditionally very low throughput processes of physicochemical, pharmacokinetic and toxicity optimization studies into high throughput selection methods in order to obtain the relevant information as early in the discovery process as possible.

Through the use of evolutionary strategies and cell based systems, the present invention enables the inclusion of ADMET requirements in the lead generation process and thus reduces significantly the production and screening of thousands of compounds that are not drug like.

### Solubility

For drugs to be effective they must be able to reach their targets in effective amounts. In cell free assays the only limitation that exists in this regard is the compound's solubility in the assay buffer. In cell-based assays with intracellular targets, the ability of compounds to diffuse across cell membranes is dependent on their ability to partition into and out of lipid-rich membranes. This process is more efficient when compounds have a certain degree of lipophilicity in addition to being sufficiently water-soluble. If the cell culture medium contains proteins (such as from the presence of fetal calf serum) the degree of binding of the compound to serum proteins influences the freely diffusible fraction of compound and hence the amount available for interaction with the target. The extent of drug binding to serum proteins has a number of important implications in the living organism including transport and distribution.

The present invention uses a host species to produce the compounds. In a preferred embodiment it uses assays external to the producer species. Thus it is an inherent part of the process to evaluate the ability of compounds to diffuse across cell membranes. The presence of medium proteins is also an inherent part of the system.

Another aspect of the invention is the control of the expression or activity of the host's drug resistance pumps. This can be done by placing an externally controllable promoter in front of a sequence coding for a drug resistance pump. This control allows significant secretion of the compounds produced in the first rounds of screening, when the solubility of compounds produced is not a key selection criteria. In later rounds of screening the expression of the pumps can be progressively turned off in order for the compounds that reach the disease targets to have to cross the host's cell membrane and thus have a reasonable solubility profile.

### Absorption

The preferred route of drug delivery is oral administration. The intestinal membrane permeability is a critical characteristic that determines the extent and rate of drug absorption and ultimately the bioavailability. Other cells which are relevant for drug uptake include epithelial, epidermis, nasal, blood-brain and blood-testis barriers, as well as the kidney, liver, intestinal epithelial and lung cells, which are also routes for uptake of drugs.

Most models of absorption involve the use of cultured, immortalised cells, which are generally intestinal in nature and which give a good correlation with absorption in vivo. Most notable among them are CaCo-2 cells that derive from a human colon carcinoma cell line or a subclone of the CaCo-2 cell line, TC7. Other usefull cell lines for absorption studies are dog kidney cell line, the Madin-Derby Canine kidney cell line (MDCK) and everted intestinal rings and brush-border membrane vesicles (BBMV). These cell lines are grown in a confluent monolayer and used for permeability measurements which are based on the rate of appearance of test compound in the receiver compartment. The apical surface of the monolayer contains microvilli and thus retains many characteristics of the intestinal brush border. Furthermore, the apically located efflux pump, P-glycoprotein, the monocarboxylic acid transporter, the dipeptide transporter, the transporter for large neutral amino acids (LNAA) [Inui K-I, Yamamoto M, Saito H. T, J Pharmacol Exp Ther, 1992; 261: 195-201; Lu S, Guttendorf RJ, Stewart BH, Pharm Res, 1994; 11: S-258.] and metabolic enzymes [Bjorge S, Halelehle KL, Homan R, Rose SE, Turluck DA, Wright DS., Pharm Res, 1991; 8: 1441-1443] are all functionally expressed.

Figure 5 shows an example of a multiple parameter screen for absorption and a pharmacological activity. Using a dual culture system and timing the time of cell selection, it is possible to select producer cells that have produced compounds with the desired pharmacological activity and a good absorption profile.

More specifically, one functionality may be screened by culturing the cells in connection with immortalised mammalian cells and detecting the effect of the compound or a metabolite of the compound in a receiver compartment. The cultured immortalised cells can be grown in a confluent monolayer and compounds with desirable permeabilities be selected.

Drugs that inhibit P-glycoprotein can alter the absorption, disposition and elimination of co-administered drugs and can enhance bioavailability or cause unwanted drug-drug interactions. Thus another important aspect of absorption studies is to determine if a compound is a PGP inhibitor by a direct measure of inhibition of PGP-mediated digoxin transport across polarized human PGP cDNA -expressing LLC-PK1 cell monolayers.

In mammals, the ABC transporters, like MDR1 and MRP1, have a key role in the functioning of the blood-brain and blood-testis barriers, as well as in the kidney, liver, lung, and intestinal epithelial cells. MDR1 is expressed normally on apical membranes of cells derived from excretory tissues, as well as on the luminal surface of cerebral capillary cells *(Gottesman et al*., *1993; Cordon-Cardo et al*., *1989)*. MDR1 and MRP1 are present in the epithelia of the choroid plexus (CP) and both transporters participate in the blood-CSF permeation barrier *(Rao et a*/., *1999).* MDR1-Pgp contributes to the drug-permeation barrier in cerebral capillary endothelial cells and takes part in elimination of organic cations and xenobiotics from the central nervous system (CNS) *(Rao et al*., *1999; Schinkel et al., 1997).* MRP1 contributes to the basolateral broad-specifity drug-permeation barrier in CP, protects this epithelium from xenobiotics and extrudes organic anions and probably also some hydrophobic compounds from the CSF *(Wijnholds et al*., *2000)*. Some ABC transporters form and regulate specific membrane channels, while others are involved in the elimination of detoxified drug-conjugates, transport of phospholipids or bile acids, and even the initiation of antiviral immune-reaction or specific self-destruction in various cell types. Moreover, members of the ABC transporter family were shown to provide multidrug resistance in pathogenic bacteria and parasites (e.g. Plasmodium and Leishmania species), while also allowing multixenobiotic resistance (MXR) in a large variety of organisms living in a chemically polluted environment *(Kurelec et al*., *1989; 1992)*.

In order to predict the penetration of a compound through different pharmacological barriers, a wide range of ABC transporters-compound interactions are also being tested, e.g., Pgp/MDR1, MRP1, MRP2, MDR3, MRP3, MRP5, MRP6, MXR (BCRP, ABCG2).

### Metabolism

A drug, once it enters an organism, can experience a variety of biological fates. Drug metabolizing enzymes, including cytochromes P450, (present at high levels in liver, kidney, gut and other organs), can catalyze the chemical conversion of a particular drug to entities (metabolites) which are more aqueous-soluble and more readily excreted than the parent drug from which they were derived. If a parent drug is inherently metabolically unstable, undesirable pharmacokinetic behavior, such as an inappropriately short duration of action or poor oral bioavailability, can be observed. It is therefore, common practice in the industry to gain knowledge about the metabolic stability of lead candidates in order to identify compounds that may turn out to have poor pharmacokinetic profiles.

In addition, studies in drug metabolism can address the issue of possible drug-drug interactions, which are closely linked to the safe use of drugs in polytherapies. Most undesirable drug-drug interactions occur when two or more compounds compete for the same drug-metabolizing enzyme. The result is usually altered pharmacokinetics for one or more of the compounds involved, sometimes accounting for compound blood levels which are outside of the therapeutic window. These types of interactions can be foreseen with the assistance of studies of the inhibitory effects of test compounds with specific drug metabolizing enzymes.

Various *in vitro* methods are available which are being increasingly incorporated into drug discovery strategies. Among the most popular and widely utilized systems in use today are hepatic microsomes. These preparations retain activity of those enzymes that reside in the smooth endoplasmic reticulum, such as cytochromes P450 (CYP), flavin monooxygenases (FMOs), sulfotransferases, UDP-glycosyl transferases, glutathione transferases and N-acetyl transferases. Isolated hepatocytes appear to retain a broader spectrum of enzymatic activities, including not only reticular systems, but cytosolic and mitochondrial enzymes as well. Liver slices, which like hepatocytes retain a wide array of enzyme activities, are also increasingly used. Furthermore, both hepatocytes and liver slices are capable of assessing of enzyme induction *in vitro.* Isolated heterologous human CYP enzymes have been available for several years, being expressed from cDNA in yeast (Saccharomyces cerevisiae), bacterial (*Escherichia coli*), and mammalian (B-lymphoblastoid) cell lines [Ohgiya S, Komori M, Fujitani T, Miura T, Shinriki N, Kamataki T., Biochem Int, 1989; 18: 429-438; Winters DK, Cederbaum Al, Biochim Biophys Acta 1992; 1156: 43-49; Crespi CL, Gonzalez FJ, Steimel DT, Turner TR, Gelboin HV, Penman BW, Langenbach R, Chem Res Toxicol, 1991; 4: 566-572]. These systems have been used to ascertain whether a compound is a substrate for a particular CYP isozyme and, if so, what metabolite is generated by that enzyme.

Assays using recombinant human cytochromes P450, (including CYP2D6 &CYP2C19 that are polymorphically-encoded) as well as assays using isozyme-specific substrate and metabolite combinations in liver microsomal preparations can provide valuable information regarding a test compound's drug-drug interaction potential.

In the present invention the generation of small molecules is carried out by host cells that can themselves be transformed with a range of enzymes involved in human metabolism. These minimises the number of false positives generated by compounds that are rapidly metabolised by the human DMEs and also leads to the discovery of compounds that are active after being metabolised and which would otherwise remain undiscovered. (see figure 6).

In another aspect of the invention the drug metabolising enzymes are included extracellular to the small molecule producer cell in either cell free or cell based assays. In still another embodiment, some drug metabolising enzyme are included intracelularly and some extracellularly to the small molecule producer cell.

The invention may employ the simultaneous use of 2, 3, 4, 5, 6, 7, 8, 9, 10 or more different drug metabolising enzymes.

When using cell based assays, one preferred approach is the use of hepatocytes.

In yet another aspect of the invention reporter systems for activity of the drug metabolising enzyme(s) are included in order to gain information on enzyme inhibition. In yet another aspect of the invention, competition assays for drug-drug interactions can be carried out.

In specific cases, some of the drug metabolising enzymes are themselves the disease targets since several of these enzymes are known to be associated with several diseases.

Conceptually, the drug metabolizing enzymes are divided into two groups. Oxidative drug metabolizing enzymes, which include CYP450s and FMOs, catalyze the introduction of an oxygen atom into substrate molecules, generally resulting in hydroxylation or demethylation. The conjugative enzyme families include the UDP-glycosyltransferases (UGTs), glutathione transferases (GSTs), sulfotransferases (SULTs), and N-acetyltransferases (NATs). The conjugative drug metabolizing enzymes catalyze the coupling of endogenous small molecules to xenobiotics that usually results in the formation of soluble compounds that are more readily excreted.

### Cytochrome P450s

Cytochrome P450 proteins in humans are drug metabolizing enzymes and enzymes that are used to make cholesterol, steroids and other important lipids such as prostacyclins and thromboxane A2. These last two are metabolites of arachidonic acid. Mutations in cytochrome P450 genes or deficiencies of the enzymes are responsible for several human diseases. Induction of some P450s is a risk factor in several cancers since these enzymes can convert procarcinogens to carcinogens.

CYP450 enzymes in the liver catalyze the initial step in the biotransformation of xenobiotic compounds, including most drugs. These enzymes are members of a large family of mixed-function oxidases that catalyze the introduction of an oxygen atom into substrate molecules, often resulting in hydroxylated or dealkylated metabolites. The metabolism takes place in two phases. Phase I is chemical modification to add a functional group that can be used to attach a conjugate. The conjugate makes the modified compound more water soluble so it can be excreted in the urine. Many P450s add a hydroxyl group in a Phase I step of drug metabolism. The hydroxyl then serves as the site for further modifications in Phase 2 drug metabolism.

More than fifty CYP450 isozymes are known to exist in humans and they have been classified into 18 families and 43 subfamilies based on amino acid sequence similarities. Proteins from the same family are greater than 40% identical at the amino acid level, while those in the same subfamily are greater than 55% identical (Nelson, D.R. (1999) Arch. Biochem. Biophys. 369:1-10). In the standard nomenclature, the family is designated by a number followed by a letter designation for the subfamily, and a second number that identifies the individual member of that subfamily.
CYP1 drug metabolism (3 subfamilies, 3 genes, 1 pseudogene)
CYP2 drug and steroid metabolism (13 subfamilies, 16 genes, 16 pseudogenes)
CYP3 drug metabolism (1 subfamily, 4 genes, 2 pseudogenes)
CYP4 arachidonic acid or fatty acid metabolism (5 subfamilies, 11 genes, 10 pseudogenes)
CYP5 Thromboxane A2 synthase (1 subfamily, 1 gene)
CYP7A bile acid biosynthesis 7-alpha hydroxylase of steroid nucleus (1 subfamily member)
CYP7B brain specific form of 7-alpha hydroxylase (1 subfamily member)
CYP8A prostacyclin synthase (1 subfamily member)
CYP8B bile acid biosynthesis (1 subfamily member)
CYP11 steroid biosynthesis (2 subfamilies, 3 genes)
CYP17 steroid biosynthesis (1 subfamily, 1 gene) 17-alpha hydroxylase
CYP19 steroid biosynthesis (1 subfamily, 1 gene) aromatase forms estrogen
CYP20 Unknown function (1 subfamily, 1 gene)
CYP21 steroid biosynthesis (1 subfamily, 1 gene, 1 pseudogene)
CYP24 vitamin D degradation (1 subfamily, 1 gene)
CYP26A retinoic acid hydroxylase important in development (1 subfamily member)
CYP26B probable retinoic acid hydroxylase (1 subfamily member)
CYP26C probabvle retinoic acid hydroxylase (1 subfamily member)
CYP27A bile acid biosynthesis (1 subfamily member)
CYP27B Vitamin D3 1-alpha hydroxylase activates vitamin D3 (1 subfamily member)
CYP27C Unknown function (1 subfamily member)
CYP39 unknown function (1 subfamily member)
CYP46 cholesterol 24-hydroxylase (1 subfamily member)
CYP51 cholesterol biosynthesis (1 subfamily, 1 gene, 3 pseudogenes) lanosterol 14-alpha demethylase

The bulk of drugs are metabolised by a few members of the CYP1, 2, and 3 families and the metabolism occurs primarily in the liver, which contains the highest concentration of CYP450 in the body. However, the importance of extrahepatic metabolism in tissues such as the intestine and lung is also recognized.

The xenobiotic metabolizing P450s are approximately 50 kDa proteins anchored in the endoplasmic reticulum (ER) by a single transmembrane helix in the N-terminus. Cell fractionation using differential centrifugation results in particulate preparations enriched in endoplasmic reticulum, commonly referred to as microsomes. Detailed examination of microsomal fractions from many different individuals has demonstrated significant variability in expression patterns of individual isozymes, however some generalizations are possible (Guengerich, F.P. (1995) Cytochrome P450: Structure, Mechanism, and Biochemistry (Second Edition), Chapter 14, edited by Paul R. Ortiz de Montellano, Plenum Press, New York, Shimada, T., et al. (1994) J. Pharmacol. Exp. Ther. 270:414-23). On average, 70% of the P450s expressed in adult human liver consist of the following isozymes: 1 A2, 2A6, 2B6, the 2C subfamily (2C8, 2C9, 2C18, and 2C19), 2D6, 2E1, and the 3A subfamily (3A4 and 3A5).

Another very important aspect of the P450s is that polymorphisms cause significant differences in drug metabolism from population to population and *individuo* to *individuo*. A polymorphism is a difference in DNA sequence found at 1% or higher in a population. These differences in DNA sequence can lead to differences in drug metabolism, so they are important features of P450 genes in humans. CYP2C19 has a polymorphism that changes the enzyme's ability to metabolize mephenytoin (a marker drug). In Caucasians, the polymorphism for the poor metabolizer phenotype is only seen in 3% of the population. However, it is seen in 20% of the asian population. Because of this difference, it is important to be aware of a person's race when drugs are given that are metabolized differently by different populations. Some drugs that have a narrow range of effective dose before they become toxic might be overdosed in a poor metabolizer. A cytochrome P450 allele website is available from Sweden at http://www.imm.ki.se/CYPalleles/

Another aspect of the current invention is the ability to evolve drugs designed for specific populations or even individuos since the drug metabolic aspects can be addressed during the drug generation process.

Oxidation of organic molecules by P450s is quite complex (Ortiz de Montellano, P.R. (1995) Cytochrome P450: Structure, Mechanism, and Biochemistry (Second Edition), Chapter 8, edited by Paul R. Ortiz de Montellano, Plenum Press, New York), but the overall reaction can be represented simply by **Equation 1:**

**Equation 1:** **RH + O2 + NADPH + H+ →ROH + H2O + NADP+**

An electron from NADPH is transferred via the flavin domain of NADPH-P450 reductase to the heme domain of the CYP450 where the activation of molecular oxygen occurs. Substrates react with one of the oxygen atoms and the other is reduced to water. In some cases, the second electron can come from NADPH via cytochrome b5 reductase and cytochrome *b*5. During *in vitro* reconstitution experiments, cytochrome *b*5 can stimulate metabolism of various substrates by some CYP450 isozymes, notably 3A4, 2E1, and 2C9. However, the mechanism of this stimulation is not clearly understood. Apocytochrome b5 was shown to be as effective as the holoenzyme in stimulating reconstituted CYP3A4 reactions, so at least in this instance, it does not appear to be playing a direct role in electron transfer (Yamazaki, H., et al. (1996) J. Biol. Chem. 271:27438-44). The most widely held hypothesis is that cytochrome *b*5 acts allosterically to enhance the interaction between CYP450 and NADPH-P450 reductase, or it improves substrate binding.

### Flavin Monooxygenases (FMOs)

Flavin monooxygenases, like the CYP450 enzymes, are associated with the endoplasmic reticulum and catalyze the oxidation of organic compounds using molecular oxygen and NADPH as the source of electrons for the reduction of one of the oxygen atoms **(Equation 1).** However, they are mechanistically distinct from the CYP450s in that they react with oxygen and NADPH in the absence of substrate to form a 4α-hydroperoxy flavin enzyme intermediate. Thus, the FMOs exist in an activated form in the cell, and their interaction with a nucleophilic group such as an amine, thiol, or phosphate, is all that is required for completion of the catalytic cycle (Rettie, A.E. and Fisher, M.B. (1999) in Handbook of Drug Metabolism, pp131-147, edited by Thomas F. Woolf, Marcel Dekker, Inc, New York). The capacity to remain stable while poised in an activated state is a possible explanation for the extremely broad substrate specificity of the FMO isozymes. It has been proposed that essentially all of the energy required for catalysis is captured in the oxygen-activated intermediate, and that alignment or distortion of the substrate molecules is not required (Ziegler, D.M. (1993) Annu. Rev. Pharmacol. Toxicol. 33:179). It follows that the active site of FMOs is much less sterically defined than for other enzymes. FMO3 is the most abundant form in human liver and is believed to be the dominant member of this enzyme family in terms of overall drug metabolism (Rettie, A.E. and Fisher, M.B. (1999) in Handbook of Drug Metabolism, pp131-147, edited by Thomas F. Woolf, Marcel Dekker, Inc, New York).

### UDP glycosyltransferases (UGTs)

UDP glycosyltransferases catalyze the glucuronidation of xenobiotics at hydroxyl, carboxyl, amino, imino, and sulfyhydryl groups using UDP-glucuronic acid as a donor molecule (**Equation 2**). In general, this generates products that are more hydrophilic and thus more readily excreted in bile or urine.

**Equation 2:** **UDP-glucuronic acid + R →UDP + R-glucuronide**

Although glucuronidation generally is classified as Phase II metabolism - the phase occurring after CYP450 dependent oxidative metabolism - many compounds do not require prior oxidation because they already possess functional groups that can be glucuronidated. Examples of first-pass metabolism catalyzed by UGTs include the UGT2B7- dependent glucuronidation of morphine (Coffman, B., et al. (1996) Drug Metab. Dispos. 25:1-4) and the glucuronidation of 5-lipoxygenase inhibitors (antiinflammatories) (Coffman, B., et al. (1997) Drug Metab. Dispos. 25:1032-8); in the latter case, glucuronidation was demonstrated to be the rate-limiting step for *in vivo* plasma clearance. UGTs are 50-60 kDa integral membrane proteins with the major portion of the protein, including the catalytic domain, located in the lumen of the endoplasmic reticulum and a C-terminal anchoring region of 15-20 amino acids spanning the ER membrane (Radominska-Pandya, A., et al. (1999) Drug Metab. Rev. 31:817-99.11. Radominska- Pandya, A., et al. (1999) Drug Metab. Rev. 31:817-99). The aglycone-binding site is believed to be in the N-terminal portion the UGT polypeptide, which is the region of the protein that shows the greatest variability in sequence among UGT isozymes. The UDPGA binding domain is in the highly conserved C-terminal half of the protein. Although not a certainty, it has been hypothesized that association with lipid is required for UGT activity and may influence the access of aglycones to the active site. Two UGT families - UGT1 and UGT2 - have been identified in humans. Although members of these families are less than 50% identical in primary amino acid sequence, they exhibit significant overlap in substrate specificity (Radominska- Pandya, A., et al. (1999) Drug Metab. Rev. 31:817-99). The members of the UGT1 family that are expressed in human liver, where the majority of xenobiotic metabolism takes place, includes UGT1A1, 1 A3, 1 A4, 1 A6, and 1 A9. Although the UGT2 family has not been as extensively studied, it is known that UGT2B4, 2B7, 2B10, 2B11 and 2B15 are expressed in the liver (Radominska-Pandya, A., et al. (1999) Drug Metab. Rev. 31:817-99.11. Radominska- Pandya, A., et al. (1999) Drug Metab. Rev. 31:817-99). As is the case for other drug metabolizing enzymes such as CYP450s, inter-individual differences in UGT expression levels have been observed and linked to differences in drug responses (Weber, W. (1997) Pharmacogenetics, Oxford University Press, New York).

The human UGT1 family includes the major bilirubin metabolizing isoform (UGT1A1) and the isoform that preferentially conjugates planar phenols (UGT1A6). Isoforms in the UGT2 family metabolize a variety of endogenous steroid compounds, as well as xenobiotics. As with the CYP450s, classification of the UGTs based on substrate specificity is somewhat limited since there is a great deal of overlap in the biotransformation capacity for most of the human UGTs.

### Glutathione transferases (GSTs)

Glutathione transferases catalyze the formation of thioether conjugates between glutathione (GSH) and reactive xenobiotics by direct addition (**Equation 3**) or displacement of an electron-withdrawing group (**Equation 4**)**.**

**Equation 3:** **GSH + R →GS-R**

**Equation 4:** **GSH + R-X →GS-R + HX**

The major biological function of GSTs is believed to provide defense against electrophilic chemical species. The majority of GSTs are cytosolic homodimers composed of approximately 25 kDa subunits from one of four structural classes: Alpha (α), Mu (µ), Pi (π), and Theta (θ). The α isoform (GST A1-1) is restricted to a few tissues in mammals, including kidney, intestine, lung and liver. The p isoform (GST M1-1) is found in the liver, but relatively few other tissues. In contrast, the π isoform (GST P1-1) is widely distributed throughout the body, although it is notably absent in the liver. Additionally, GST P1-1 is abundant in most types of tumor cells.

### Sulfotransferases (SULTs)

Sulfotransferase enzymes catalyze the conjugation of sulfate groups onto a variety of xenobiotic and endogenous substrates that possess acceptor moieties such as hydroxyl and amine groups (**Equation 5**).

**Equation 5: R-XH + PAPS →R-SO4 + phosphoadenosine + H+**

The cofactor 3'-phosphoadenosine 5'-phosphosulfate (PAPS) is required for sulfonation by these enzymes. Although sulfonation generally causes molecules to lose their biological activity, several documented examples indicate that the addition of sulfate can lead to formation of highly reactive metabolic intermediates, such as minoxidil, and reactive electrophilic cations, such as sulfated N-hydroxy 2-acetylaminofluorene (McCall, J., et al. (1983) J. Med. Chem. 26:1791-3; Miller, J.A. (1994) Chem. Bio. Interact 92:329-41). Several sulfotransferase enzymes with different biochemical properties have been characterized in animal and human tissue. Two general classes exist in tissue fractions: the cytosolic enzymes, which are considered important in drug metabolism; and the membrane bound enzymes, which are involved in the sulfonation of glycosaminoglycans and glycoproteins (Weinshilboum, R.M., et al.(1997) FASEB J. 11:3-14). The human cytosolic sulfotransferase isozymes function as homodimers of 32-35 kDa subunits. There are currently 10 known sulfotransferases in humans, five of which are known to be expressed in adult liver (SULT1A1, SULT1 A2, SULT1 A3, SULT1 E and SULT2A1). It is expected that other new genes encoding sulfotransferases will be identified. The nomenclature of the different genes, their mRNA and protein products has recently been revised so that "SULT" is the accepted superfamily abbreviation (Raftogianis, R.B., et al. (1997) BBRC 239:298- 304). Allelic variants of sulfotransferase enzymes do exist and studying their frequency and functional role in drug disposition is a very active area of research.

### N-acetyl Transferases

N-acetyltransferases (NATs) catalyze the biotransformation of aromatic amines or hydrazines to the respective amides and hydrazides (**Equation 6**) using acetyl coenzyme A as a donor. They also will catalyze the O-acetylation of N-hydroxyaromatic amines to acetoxy esters (Equation 7).

**Equation 6: R-NH2 + CoA-S-COCH3 →R-NCOCH3 + CoA-SH**

**Equation 7: R-NHOH + CoA-S-COCH3 →R-NHOCOCH3 + CoA-SH**

There are two known NAT isoforms in humans called NAT1 and NAT2; both are 33 kDa cytosolic proteins found in the liver. NAT1 is also expressed in many other tissues, whereas NAT2 is expressed only in the liver and gut. The two isoforms have different, but overlapping substrate specificities, with no single substrate appearing to be exclusively acetylated by one isoform or the other. Genetic polymorphisms for N-acetylation are well documented, and may play a role in the susceptibility of certain individuals to bladder and colon cancer, as the NATs are involved in both the activation and detoxification of heterocyclic aromatic amine carcinogens (Weber, W. (1997) Pharmacogenetics, Oxford University Press, New York).

### Toxicity

One of the main forms of toxicity is hepatotoxycity. Freshly isolated human hepatocytes represent the best in vitro biological system in which to evaluate toxicity. Some human liver cell lines have been developed that reflect normal human liver metabolism (e.g., ACTIVTox from Amphioxus Inc, and Hep G2 from Cerep). These cell lines can be used in cell proliferation assays that give very good correlation with in vivo results.

In the present invention toxicity assessment is an inherent parameter of the screens since the compounds are produced in a host organism. Any compound that is very toxic will not be selected or detected since it will kill the host organism. Broadly speaking, toxicity can be screened for using a cell proliferation assay. A more accurate human toxicity assay can be incorporated in the multiple parameter screening procedure by for example encapsulating hepatocytes with the producer species and disease target(s) and select screening units that activated the disease target(s) in the desired way and have not inhibit hepatocyte growth.

Figure 7 shows a schematic representation of a screening system of the present invention to evaluate target activity, metabolism by DMEs and cytotoxicity: Using a double gel encapsulation system where in the first droplet are clonal lines of the producer species transformed with the pharmacological target and DMEs, and in the second droplet are hepatocytes, it is possible to screen for target activity, DME metabolism and hapatotoxicity.

### Mutagenicity

The mutagenic ability of a compound is another aspect that has to be addressed in a drug discovery programme. The mutagenicity of a compound can be evaluated by measuring the reverse-mutation rate in an organism. The organism can be animals or more preferably microorganisms. For example, there are several different strains with differing and complementary sensitivities to potential mutagens of the bacterium *Salmonella typhimurium.*

### MULTIPLE PARAMETER SCREENING FOR OTHER PURPOSES

### Screening for herbicides.

The effect of a compound as a herbicide can be screened with in vitro assays. Primary screens that test the effect as a herbicide include: toxicity, inhibition of photosynthesis, inhibition of central metabolic enzymes.

Examples of further screens that can be assayed simultaneously with the first group include: uptake (using hairy root cultures, organ cultures (including shoot cultures), metabolism, lack of toxicity towards other plants (in particular crops) or towards other organisms (animals, humans, insects, fungi).

### Screening for fungicides (agricultural)

Primary screens are much like the ones used in screening for or evolving herbicides except that fungal cells are used as reporter cells and for uptake.

Secondary screens are also more or less of the same type. One particular screen to perform is lack of toxicity towards plants, in particular crop plants.

### Screening for insecticides

Primary screens include the assays for the function of the compounds as insecticides, i.e. cell based assay for toxicity towards a specific species or group of species of insects, and/or assays for inhibition of specific enzymes in key metabolic functions of insects, or inhibition of reproduction.

Secondary screens may include uptake in specific insect organs using e.g. a confluent monolayer of insect cells from the organ in which the insecticide is to be taken up. A further screen includes metabolism by insect metabolic enzymes to test whether the compounds are metabolised or activated by these. Furthermore, it is relevant to screen for lack of toxicity or mutagenicity or teratogenicity towards animals and/or human beings. Another example of a secondary screen is lack of toxicity towards other species of insects.

### Screening for cosmetics

Primary screens are directed to the function of the compounds as cosmetics.

Secondary screens include the same as for screening for or evolution of pharamaceuticals, i.e. absorption (if relevant), distribution (if relevant), metabolism, excretion (if relevant) and toxicity, mutagenicity and teratogenicity.

### Screening for flavours

Primary screens may include automatic assaying for the desired flavour. "Artificial noses" have been developed that can assay for particular flavours or tastes. Artificial noses, or olfactory or vapor-selective detectors can detect low levels of odorants. Examples of such noses are disclosed in e.g. US 6,368,558 and references cited therein. The technique is also know as artificial olfactometry.

Secondary screens typically include toxicity, mutagenicity, teratogenicity, metabolism (by e.g. saliva enzymes).

Fine chemicals: other examples of multiple parameter screening and evolution include the evolution and screening for fine chemicals, food and feed additives, and catalysts.

### SCREENING TECHNIQUES

The selection of the positive cells can be achieved by establishing screens where only positive cells survive or by physically selecting positive cells. Survival of positive clones can e.g. be achieved by using assays based on
a. Survival in the presence of toxic substances
b. Survival in the presence of other organisms
c. Use of nutritional reporter genes, e.g., His, or of reporter genes that when giving desired response produce a vital protein, e.g., CDC25

Physical selection of positive cells can be done by the use of:
a. FACS & intracellular reporter assays (native or engineered)
b. FACS & gel encapsulation (single, double or more) & extracellular reporter systems [cell based (native or engineered) or cell free]
c. Overlay assay & extracellular reporter systems [cell based (native or engineered) or cell free] & picking (manual or automatic)
d. Single clonal cell line confinement to microtiter plate & extracellular reporter systems [cell based (native or engineered) or cell free] & picking (manual or automatic)
e. Plating & picking (manual or automatic)

### Flow cytometry

In traditional flow cytometry, it is common to analyze very large numbers of cells in a short period of time. Newly developed flow cytometers can analyze and sort up to 100,000 cells per second. In a typical flow cytometer, individual particles pass through an illumination zone and appropriate detectors, gated electronically, measure the magnitude of a pulse representing the extent of light scattered. The magnitude of these pulses are sorted electronically into "bins" or "channels", permitting the display of histograms of the number of cells possessing a certain quantitative property versus the channel number. It was recognized early on that the data accruing from flow cytometric measurements could be analyzed (electronically) rapidly enough that electronic cell-sorting procedures could be used to sort cells with desired properties into separate "buckets", a procedure usually known as fluorescence-activated cell sorting (FACS).

Fluorescence-activated cell sorting has been primarily used in studies of human and animal cell lines and the control of cell culture processes. Fluorophore labeling of cells and measurement of the fluorescence can give quantitative data about specific target molecules or subcellular components and their distribution in the cell population. Flow cytometry can quantitate virtually any cell-associated property or cell organelle for which there is a fluorescent probe (or natural fluorescence).

Cell sorters can handle cell sorting at rates of at least 10,000 cells per second, more preferably at least 50,000 per second, more preferably at least 100,000 per second.

### Gel microdroplet encapsulation

The gel microdroplet technology has had significance in amplifying the signals available in flow cytometric analysis, and in permitting the screening of microbial strains in strain improvement programs for biotechnology. Wittrup et al., (Biotechnolo. Bioeng. (1993) 42:351-356) developed a microencapsulation selection method which allows the rapid and quantitative screening of >10⁶ yeast cells for enhanced secretion of Aspergillus awamori glucoamylase. The method provides a 400-fold single-pass enrichment for high-secretion mutants.

Gel microdroplet or other related technologies can be used in the present invention to localize as well as amplify signals in the high throughput screening of cells. Preferably the screening methods of the present invention are laid out to ensure survival of the producer cell, so that these can be used for further rounds of evolution. However, it is also possible to isolate the expression cassettes and possibly even the artificial chromosomes from the cells and re-insert these into other host cells should this be necessary if the cells are killed by the screen.

Different types of encapsulation strategies and compounds or polymers can be used with the present invention. An encapsulation of particular relevance is the encapsulation in calcium alginate due to its broad applicability. Furthermore, calcium alginate beads can be made at room temperature and be dissolved by gentle procedures leaving the encapsulated cells alive.

A further feature of particular interest is the possibility of coating the beads (or gel microdroplets) with a lipid layer in order to make them impermeable to small molecules. This ensures that small molecules do not leak to the surroundings and that the connection between producer cell and small molecule is not lost during screening and sorting of gel microdroplets.

Encapsulation techniques may be employed to localize signal, even in cases where cells are no longer viable.

Gel microdrops (GMDs) are small (25 to 200 µm in diameter) particles made with a biocompatible matrix. In cases of viable cells, these microdrops serve as miniaturized petri dishes because cell progeny are retained next to each other, allowing isolation of cells based on clonal growth. The basic method has a significant degree of automation and high throughput. Cells are encapsulated together with substrates and particles containing a positive clones are sorted. Fluorescent substrate labeled glass beads can also be loaded inside the GMDs. In cases of non-viable cells, GMDs can be employed to ensure localization of signal.

Encapsulation can be in beads, low or high temperature agaroses, gel microdroplets made from agarose, polysacchoride, carbohydrate, alginate, carrageenan, chitosan, cellulose, pectin, dextran, or polyacrylamide, cells, such as ghost red blood cells or macrophages, liposomes, or any other means of encapsulating and localizing molecules.

Gel encapsulated cells may further be enclosed in a layer essentially non-penetrable by the compounds being screened. Thereby it is ensured that the compounds remain within the vicinity of the cell and that the physical connection between cell and compound is not lost. Furthermore leakage from gel droplet to gel droplet is prevented. The non-penetrable material may be a lipid material.

The cells and the reporter system(s) may be encapsulated into one layer of gel droplets. The cell may also be encapsulated in one layer of the gel droplet and at least one reporter system is encapsulated in another layer of the same gel droplet. In anoter embodiment, one layer comprises the cells and one or more reporter system(s), and a second layer comprises one or more different reporter system(s), and optionally a third or fourth or further layer comprises one or more reporter system(s). Furthermore, the cell may be encapsulated in one layer of the gel droplet and a first reporter system is encapsulated in another layer of the same gel droplet and at least a second reporter system is encapsulated into yet another layer of the same gel droplet.

For example, methods of preparing liposomes have been described (i.e., U.S. Pat. Nos. 5,653,996, 5,393,530 and 5,651,981), as well as the use of liposomes to encapsulate a variety of molecules U.S. Pat. Nos. 5,595,756, 5,605,703, 5,627,159, 5,652,225, 5,567,433, 4,235,871, 5,227,170). Entrapment of proteins, viruses, bacteria and DNA in erythrocytes during endocytosis has been described, as well (Journal of Applied Biochemistry 4, 418-435 (1982)). Erythrocytes employed as carriers in vitro or in vivo for substances entrapped during hypo-osmotic lysis or dielectric breakdown of the membrane have also been described (reviewed in Ihler, G. M. (1983) J. Pharm. Ther). These techniques are useful in the present invention to encapsulate samples for screening.

An environment suitable for facilitating molecular interactions include, for example, liposomes. Liposomes can be prepared from a variety of lipids including phospholipids, glycolipids, steroids, long-chain alkyl esters; e.g., alkyl phosphates, fatty acid esters; e.g., lecithin, fatty amines and the like. A mixture of fatty material may be employed such as combination of neutral steroid, a charge amphiphile and a phospholipid. Illustrative examples of phospholipids include lecithin, sphingomyelin and dipalmitoylphos-phatidylcholine. Representative steroids include cholesterol, cholestanol and lanosterol. Representative charged amphiphilic compounds generally contain from 12-30 carbon atoms. Mono- or dialkyl phosphate esters, or alkyl amines; e.g., dicetyl phosphate, stearyl amine, hexadecyl amine, dilauryl phosphate, and the like.

### Other screening systems

As an alternative to gel droplet screening the selection of positive cells meeting the at least one selection criterion may be performed by means of an overlay assay, said overlay assay comprising reporter system(s), and manual or automatic picking of positive cells.

Other systems for screening include the selection of positive cells meeting the at least one selection criterion and is performed by means of placing a single clonal cell line in one well of a microtiterplate, said well comprising reporter system(s), and manual or automatic picking of positive cells. This system takes advantage of the many systems developed for automatic handling and analysis of microtiterplates.

Cells may also simply be plated on medium and positive cells can be picked either automatically of manually.

Cells may also be engineered so that only positive cells are able to survive. These cells may be grown in liquid media or be plated.

### Evolution towards mutliple parameters

Evolution at its most general is a process, whereby a set of replicating and varying patterns are subjected to a selection process that favours the replication of certain of the variant patterns. The selection process acts on an emergent property (phenotype) that is encoded by the pattern and that varies as a consequence of the underlying variation in the pattern. Over the course of a series of replication events those patterns whose replication is most favoured come to dominate the population.

Variation in the patterns occurs as the result of changes in individual patterns or as the result of mixing of individual patterns. Which patterns come to dominate the population is partly a consequence of the selection criteria used and partly a function of the starting population.

In living organisms and cells the predominant replicating pattern consists of nucleotide sequences (DNA or - in some vira - RNA) and the criteria on which selection acts it typically mediated through other molecules such as (but not limited to) proteins, metabolites, and structural macromolecules that are encoded by the nucleotide sequence either directly or indirectly.

In genetic algorithms the replicating pattern consists of software defined magnetic states and the variation on which selection acts is typically (but not limited to) the solution of a mathematical algorithm encoded by the magnetic states either directly or indirectly.

The ability of a pattern to replicate in a given set of environmental parameters is often referred to as the "fitness" of the pattern. Fitness can be regarded as a mathematical property that replicating patterns "attempt to" optimise. The higher the fitness of any given pattern, the greater the chance it will produce one or more copies of itself, the higher the number of copies it will on average produce, and the lower the chance it will be destroyed prior to replication. As with any mathematical function the property that is optimised may itself be a complex function of otherwise independent properties. Thus evolution can optimise across more than one criteria. For instance the mating calls of many male insects are optimised to attract females of the same species whilst not attracting predators.

Cells containing genetic material are thus in principle able to evolve by virtue of the variations in the genetic sequence that occur within each cell and the consquences of this variation upon the fitness of the cell in a given set of environmental parameters and the ability of the cell to pass these genetic sequences on to descendant cells

For the purposes of this invention the term "Fitness Function" shall be taken to mean a mathemetical or algebraic equation that calculates a score and where the variable elements in the equation are output variables that vary between different cells within a *cell population*.

For the purposes of this invention the term "Fitness Score" shall be the score generated by the *fitness function* equation.

It shall be understood that any selection process conducted on cells may therefore be conducted according to the following general procedure:
- The fitness function (F') is defined so that it comprises the desired phenotype of the cell and mathematically relates this to measurable parameters
- Each cell or group of cells is measured on one or more parameters
- F' for the cell is calculated according to the measured parameters
- Those cells with the highest F' scores are removed from the screening locality and allowed to grow. Cells with lower F' scores are discarded. By the highest F' score is meant a predetermined percentage of the cells with the highest score, such as the best 1%, 5%, 10 % or 50%, or for very high selection pressures the best 1‰, the best 0.1 ‰, the best 0.01 ‰, the best 0.001 ‰, or the best 0.0001‰.

It is an important teaching of evolution that the criteria on which certain patterns are selected over other patterns is essentially arbitrary - in principle any criterion can be used. That arbitrary, human imposed criteria can be used to generate an evolutionary process in a whole organism is exemplified by the evolution of melanism in moths as a result of industrialisation, the evolution of pedigree dogs with various properties and the evolution of plants with e.g. enhanced levels of commercially valuable oils or more even fruiting times or more attractive scents and colours. The term "breeding" is often used to describe human imposed evolution. Such organisms have increased their fitness according to a given set of human imposed criteria. It shall be obvious from the these examples that it is not necessary for the fitness function equation to be explicitly described for the evolution to take place.

It is a further teaching that fitness functions and consequent selection pressures can lead to the organism expressing phenotypes that impose high costs on (and even in some cases kill) the organism. All that is required for this to be the case is that they confer a countervailing benefit that allows the underlying pattern that produces the phenotype to spread. One example is the evolution of the peacock's tail, which whilst making it highly visible and vulnerable to competitors and predators, improves its ability to attract mates and hence replicate. In organisms with diploid or higher ploidy and with sexual reproduction it is even possible for patterns that have a net cost to be maintained in the population at reasonable levels. One example of this is the maintenance of the sickle cell anaemia mutation in west african human populations. The heterozygote form of the mutation confers a benefit (by making the carrier more resistant to malaria) whilst the homozygote is costly (causing severe anaemia). The positive benefit of the heterozygote results in the underlying pattern being maintained in the population at a relatively high frequency.

It is a further teaching that multiple selection pressures, acting on a population at different locations and times help develop and maintain the variability of replicating patterns in the population.

It is a further teaching that if two identical selection pressures are applied to two independent but apparently identical populations then although such populations will each evolve similar phenotypes the genetic patterns that come to dominate the population (and that confer the evolved phenotype) may differ between the populations. An example of different genetic patterns conferring the same phenotype is streptomycin resistance in bacteria.

From the above it should be clear that organisms are capable of complex evolutionary responses to a wide range of environmental pressures.

The evolution according to the present invention is based on a series or cycle of steps of subjecting a composition of cells to screening and selecting cells exhibiting a predetermined functionality, as shown in Fig. 21. The cycles are repeated until the desired functionality, for example a target specificity and activity is obtained. Another example of general screening strategies is illustrated in Figure 22.

In other words, the method of evolution according to the present invention is based on the provision of
1. a suitable set of diverse genetic patterns and also
2. a way of selecting for those genetic patterns within this set that encode for phenotypes that are consistent with these properties and also
3. a way of generating novel genetic patterns from those patterns that were selected in step 2.

These steps may then be combined sequentially or in parallel or in some other essentially iterative basis. The present invention lays out how to achieve these requirements.

In another aspect of the invention, the methods may be applied to the generation of a pathway derived from sources from multiple natural kingdoms, phyla or orders in the host cell. An example of this would be the generation of a pathway to produce retinoids or other molecules by means of introduction of genes encoding for the production of carotenoid pathways (obtained from fungi, algae and/or plants) as well as genes encoding for the synthesis of Vitamin A (obtained from mammals) or genes encoding for the production of visual pigments (obtained from insects). By such targeted selection and combination of elements of biochemical pathways across kingdoms or phyla the likelihood of obtaining novel metabolites may be further increased.

As previously described a fitness function (F') can be defined that encapsulates the desired phenotype of the cell and mathematically relates this to one or more measured outputs. For example the fitness function may be defined as the multiplum of a cell's absorption at two different wavelengths or alternatively it may be defined as the level of inhibition of one enzyme, divided by the inhibition of another enzyme, or it may be defined as the level of cytotoxic poison that a cell can survive, multiplied by the rate or reproduction of the cell in the absence of the cytotoxin or it may be defined in numerous other ways

In each screening round cells are selected that have outputs that correspond to one or more elements of the fitness function. In a preferred embodiment early screening rounds only measure one output whilst later screening rounds measure multiple outputs.

Those cells with the highest *fitness scores* in the population are removed from the screening environment for later use and/or analysis. Cells with lower F' scores may be discarded. By the highest F' score can be meant a predetermined percentage with the highest score, such as the best 1%, 5%, 10 % or 50%, or for very intense selection or very large cell populations the best 1‰, the best 0.1 ‰, the best 0.01 ‰, the best 0.001 ‰, or the best 0.0001%. Alternatively an absolute fitness score can be defined and only those cells that exceed this score are selected. By this approach the percentage of cells that are selected may vary.

In a preferred embodiment of this invention the screening and selection processes should be conducted on a repetitive or iterative basis, with each iteration being conducted on a daughter population.

For each iteration of the screening step, the fitness score that the cells are categorised upon is defined and the cell population subjected to screening. Over a series of iterations the fitness score is elaborated such that it progressively approaches the desired target value. The fitness score may be elaborated either by being increased or by having additional factors added into the equation that derives the fitness score.

The selection criteria are hence progressively optimised towards the desired functionality through the necessary rounds or cycles of screening and selection. The steps are repeated until at least one cell having the desired functionality has been evolved, such as repeated at least twice, such as at least three times, such as at least four times, such as at least five times, such as at least ten times, such as at least twenty times, such as least fifty times, such as at least one hundred times, such as at least two hundred times

In another embodiment the steps are repeated until at least two cell lines, or at least five cell lines, or at least 10 cell lines, having the desired functionality have been evolved. In a preferred embodiment at least a part of the cell lines evolved have different genetic patterns or genotypes, in a more preferred embodiment all the cell lines evolved have different genetic patterns or genotypes. By the term cell lines is meant cells originating from cells having met the selection criteria related to the determined screening functionality.

The selection criteria (or threshold) for one or more outputs may be increased for each repetition. Increasing criteria may for example be increasing concentration of a chemical, such as a toxin, in growth media for each repeat, or decreasing concentration of one or more nutrition components in the growth media or decreasing sensitivity or proximity of a reporter construct. Other examples of increasing criteria may be repetitive changes of temperature, either increasing or decreasing depending on the cell type chosen.

The selection criteria may also change character per repeat, such as starting with a concentration of a chemical substance in the growth media, and adding a physical parameter, such as light, in the next repeat, or starting with measuring the activity against one enzyme and adding activity against another enzyme in the next repeat.

It is also within the scope of the present invention that selection criteria may be a mixture of the criteria discussed above, ie. increased concentration of a chemical combined with changes of physical parameters, and/or increased concentration of one chemical combined with changed concentration of another.

Through this approach and in accordance with the general principles of evolution, over a series of screening and selection cycles host lines that most demonstrate the required characteristics are selected for and come to dominate the population. Over a series of screens the required fitness score is raised or elaborated, favouring those combinations that have led to an improvement in the expression of the desired characteristics.

In one embodiment the host cell lines that are a priori believed to be interesting for a given target are selected and the selected lines evolved through a series of screens as set out in Figure 21.

In another embodiment the approach is one of an escalator of selection pressure using screens that move from the general / low activity to the Specific / high activity with the generation of new genetic patterns between each step.

In another embodiment the fitness score is deliberately raised only marginally between selection cycles, such as by no more than 50% or by no more than 25% or by no more than 10% or by no more than 5% or by no more than 1%. Such gradualist selection pressures allow low level responses to be built upon over a series of selection cycles. By selecting marginal improvements in the fitness score such an approach maximises the genetic diversity at each stage in the selection process.

### Generation of Novel Genetic Compositions

It is a requirement of evolutionary processes that new patterns are generated either in parallel to or sequential to selection steps. In systems where the patterns are based on genetic elements this requires that either new genetic elements are introduced or new combinations of existing genetic elements are created or both.

In the present invention new patterns can be achieved through one or more of the following processes. The term combining or remixing shall be taken to mean a process of generating new combinations of expression constructs using one or more of these approaches. The combination or remixing may be conducted at any step of the selection process and a preferred timing is when cells having elements of the predetermined functionality have been found in at least one of the compositions, and preferably in at least 0.1%, such as at least 1%, such as at least 2%, such as at least 5%, such as at least 10% or at least 50% of compositions. The term Daughter Population shall be taken to mean a cell population that is predominately genetically descendant from those cells in one or more cell populations that had a fitness score above a certain threshold and that is further characterised by most of the cells in the daughter population having been generated through a remixing step.

In principle the combination or remixing may be conducted by at least the following approaches: physical isolation and remixing of expression cassettes, physical isolation and remixing of artificial chromosomes containing expression cassettes, sexual crosses, cell- or protoplast fusion (vide Hugerat Y, Spencer F, Zenwirth D, Simchen G (1994). Genomics 22(1), p. 108-117), and YAC-duction (vide Curran BP, Bugeja VC (1996), Methods Mol. Biol. 53, p 45-49. One example of physical remixing is illustrated in Figure 23.

One advantage of physical isolattion is that any accumulating host mutations are removed by the remixing of genes into new host lines. Reporter genes can also be introduced as part of this process, allowing for the introduction of intracellular reporter assays. The remixing is preferably carried out in vitro by isolating the expressible sequences from at least two different cells, combining the individual expressible sequences in vitro into novel combinations, and introducing the combined expressible sequences into cells to obtain at least two cells with different combinations.

Due to the common structure of the expression cassettes according to a preferred embodiment of the invention (see the section starting page 98 "Concatamers"), these may easily be excised from the host cells again using a restriction enzyme specific for the rs₁-rs₂ restriction site According to the present invention the enzyme specific for the rs₁-rs₂ restriction site is preferably a rare cutter therefore the likelihood of cutting host genomic DNA fragments with a size similar to the size of the expression cassettes is very limited. After excision the expression cassettes may be mixed with other expression cassettes of similar structure and be re-concatenated to produce novel combinations and re-inserted into another host cell in another combination creating a greater diversity during the evolution steps.

The combination of expressible sequences may of course also be a combination of full length chromosomes in the cells, such as combination of artificial chromosomes. Combination of the artificial chromosomes may be achieved in at least 4 ways depending on the host cells. These are physical isolation, crosses, protoplast fusion and YAC-duction as described herein.

An alternative way of physically remixing expression cassettes is to isolate the artificial chromosomes from one or more cell populations and re-transform new host cells. The host cells may or may not already contain artificial chromosomes containing expression cassettes.

Thus, new genetic compositions may be achieved by induction of different mating types in the two (or more) populations followed by sexual crosses yielding cells that are diploid for the normal complement of chromosomes and contains the artificial chromosomes of both partners in the cross.

### Addition of new genetic material.

The remixing is preferably conducted with addition of new genetic material from another cell composition. The other composition may be chosen from compositions capable of expressing at least one predetermined phenotype, such as a protein or a metabolite, a given metabolic pathway or part thereof or it may be chosen at random.

In one embodiment it is desirable to conduct selection in a series of isolated populations that are then brought together once they have independently evolved useful traits. In this manner the use of independent selections for same phenotype provides different genetic backgrounds (a form of parallel evolution) that can then ideally act synergistically with each other.

In another embodiment the result of selection on two or more compositions is mixed at a certain step of evolution to create further modified compositions when aiming for at least one cell having the desired functionality.

Recombination of the expressible sequences, i.e. changes of the genetic material by for example cross-over, may be optionally avoided, through the construction of the genetic inserts, in particular spacer sequences, as well as due to a general attempt to suppress recombination in the cells. Thereby combination of intact genes or cDNA material is favoured, with lesser risk of destroying the function of the genetic material due to recombination.

Having obtained daughter populations exhibiting the desired functionality, the daughter population may then be subjected to further steps of screening and selection in order to optimise the cells.

### Evolved Cells

In another aspect the invention relates to the cells evolved having the desired functionality. In a preferred embodiment the cells evolved have a genetic construction as defined above in relation to the starting material, however often having another combination of heterologous genetic material than the starting population.

The evolved cells may be subjected to analysis with respect to the gene(s) responsible for the desired functionality in order to possibly optimise the genes leading to the desired phenotype.

However, the cells may also be used as such as production cells capable of producing a novel metabolite or a novel pathway. In this respect it is preferred that the cells evolved are cells suitable for production in for example fermentation tanks.

### Novel Molecules and Pathways

The aim of the evolution method according to the present invention is to evolve cells capable of producing new substances, such as new metabolites, new proteins, and/or having new pathways.

Thus, in a further aspect the present invention relates to a substance produced by the cells evolved according to the present invention, said substance being metabolites, proteins, carbohydrates, poly- and oligosaccharides, and nucleic acids. Since some of the interactions that produce the novel phenotypes are mediated by enzymes it is likely that the result will include novel compounds with chiral centres, which are especially difficult to produce via chemical synthesis.

Creation of novel pathways, may lead to the capability of creating cells capable of metabolising, i.e. converting, a compound, which is not metabolisable by the native, un-evolved cell.

### Specific strategies for pathway generation

In another embodiment the approach is to walk down a specific multi-step metabolite pathway in a manner analogous to playing a slot machine. Once the first step of the pathway is obtained the genetic material for that step is put on "hold" by increasing its relative abundance such that most cells in the cell population contain said genetic material and the other genetic materials are then varied (spun or permed) until the second step is achieved, which is then also put on "hold". This process is repeated until the entire pathway is obtained.

In another embodiment the approach is to go backward in the construction of a specific multi-step pathway. Once the last step of the pathway is obtained the genetic material for that step is put on "hold" by increasing its relative abundance such that most cells in the cell population contain said genetic material and the other genetic materials are then varied (spun or permed) until the next but last step is achieved, which is then also put on "hold". This process is repeated until the entire pathway is obtained.

Also, a combination of both embodiments may be conducted, so that the pathway is built up from "both ends".

In one embodiment of the invention the cells are subjected to the selection criteria under conditions that maximise the number of genes expressed by the cells, including the genes being heterologous to the cells. Alternatively the cells are subjected to the selection criteria under conditions that ensure that only a certain percentage of or a subset of the genes being heterologous to the cells are expressed

It should be understood that the above approaches are general in concept and lend themselves to the construction of many variants, depending on the desired goal.

Furthermore, it should be understood that by using a cell-based system an advantage is that the compounds may be selected also on parameters not being included in the fitness function, in that the system inherently promotes evolution of compounds exhibiting properties such as not being toxic to the cell, as well as compounds that diffuse rapidly within the cell.

Examples of the approaches to build known or structural class focused pathways are as follows:

For small to medium sized pathways, i.e. pathways of up to 6-7 steps from metabolites of the host cell, the screening strategy relies on enriching the founder population with relevant genes and on the reasonably high probability of assembling over a series of selection rounds pathways that produce a low level of the desired property.

For large pathways (i.e. more than 6-7 steps) the screening strategy may involve dividing the pathway into subsets and a) defining screening parameters for each subset in order to build a pathway forwards or b) identifying intermediate metabolites that are fed to the cell population in order to assemble the pathway backwards.

For example in the case of retinoid like compounds it is well known that carotenoids are metabolised by specific tissues in specific classes of organisms to produce retinoids. It is thus possible to first evolve a population of cells that produce carotenoids and then mix the genes of this population(s) with those of a population(s) enriched for retinoid genes and in this manner evolve a population that produce retinoid like compounds.

Another example is the case of Taxol like compounds, for which the exact biosynthetic pathway is not known but is predicted to be somewhere between 12 and 20 enzymatic steps from yeast metabolites and several of the intermediate compounds have been isolated. It is thus possible to start by feeding a metabolite that is a few steps from Taxol in order to identify a population of cells able to produce Taxol like compounds from this precursor. Once this is achieved, the genes responsible for that small pathway are locked, e.g., integrated in the host's genome, or incorporated in artificial chromosomes at such high levels that statistically they occur in most cells and a second evolution process is started. This time the precursor being fed to the cell population is an earlier metabolite from the Taxol biosynthesis. By repeating these partial evolutions a number of times, it is possible to evolve a population of cells that produce Taxol like compounds starting with host metabolites.

Finally it should be said it is also possible to produce a class of compounds using a combination of both approaches described, i.e., by starting simultaneous evolution processes that cover the pathway backwards and forwards.

### Diverse Genetic Patterns

Given that evolution is a statistical process it is necessary to provide sufficient genetic variation on which selection processes can act. In the present invention, this comprises two elements
- Providing a sufficiently large and diverse population
- Controlling the genetic basis of the diversity and how it expresses

Selection requires genetic diversity on which to operate. Thus the first requirement of the current invention is to provide a population of cells that embodies a genetic diversity. The term *"genetic diversity*" means that substantially all cells are different, in that they comprise different genes, and/or identical genes under control of different control system, such as different promoters, such that almost each cell initially represents a genotype not represented in any of the other cells. Of course due to cell division a few cells may be substantially identical.

The term "Cell Population" shall be taken to mean a population of cells where at least 10⁴ cells, such as at least 10⁵ cells, such as at least 10⁶ cells, such as at least 10⁷ cells, such as at least 10⁸ cells, such as at least 10⁹ cells, such as at least 10¹⁰ cells, such as at least 10¹¹ cells, such as at least 10¹² cells, such as at least 10¹³, such as at least 10¹⁴, such as at least 10¹⁶, for example at least 10¹⁸, such as at least 10²⁰ in the population represent a genotype not represented in any of the other cells.

Thus, the principle of the evolution method according to the invention is to obtain a population of cells having a very high genetic diversity.

One particular embodiment of this principle is to produce cells with combinations of concatemers comprising cassettes with expressible nucleotide sequences from a number of different expression states, which may be from any number of unrelated or distantly or closely related species, or from species from different kingdoms or phylae such that novel and random combinations of gene products are produced in one single cell.

By inserting novel genes into the host cell, and especially by inserting a high number of novel genes from different expression states, such as from a wide variety of species into a host cell, the gene products from this array of novel genes will interact with the pool of metabolites of the host cell and with each other and modify known metabolites and/or intermediates in novel ways to create novel compounds. Due to the high number of substantially different cells that can be generated using the methods according to the present invention, for example at least 10⁴ cells, such as at least 10⁵ cells, such as at least 10⁶ cells, such as at least 10⁷ cells, such as at least 10⁸, such as at least 10⁹, for example at least 10¹⁰, such as at least 10¹², it is more or less inevitable or at least likely that such large populations will lead to a sub-population having such an interaction. The sub-population having such interaction may comprise at most 10¹⁰ cells, such as at most 10⁹ cells, such as at most 10⁸, such as at most 10⁷ cells, such as at most 10⁶ cells, such as at most 10⁵ cells, such as at most 10⁴ cells, such as at most 10³ cells, such as at most 10² cells or just 10 cells.

### HOST CELLS

The host cells selected for this purpose are preferably cultivable under standard laboratory conditions using standard culture conditions, such as standard media and protocols. Preferably the host cells comprise a substantially stable cell line, in which the concatemers can be maintained for generations of cell division in a suitable manner. It is also of great advantage that standard techniques for transformation of the host cells are available, especially that methods are known for insertion of artificial chromosomes into the host cells. Host cells are also denoted producer cells.

It is also of advantage if the host cells are capable of undergoing meiosis to perform sexual recombination. It is also advantageous that meiosis is controllable through external manipulations of the cell culture. One especially advantageous host cell type is one where the cells can be manipulated through external manipulations into different mating types.

The host cell should preferably be conditionally deficient in the abilities to undergo homologous recombination. The host cell should preferably have a codon usage similar to that of the donor organisms. Furthermore, in the case of heterologous genomic DNA, if eukaryotic donor organisms are used, it is preferable that the host cell has the ability to process the donor messenger RNA properly, e.g., splice out introns.

The cells can be bacterial, archaebacteria, or eukaryotic and can constitute a homogeneous cell line or mixed culture. Suitable cells include the bacterial and eukaryotic cell lines commonly used in genetic engineering and protein expression. Suitable mammalian cells include those from, e.g., mouse, rat, hamster, primate, and human, both cell lines and primary cultures.

Preferred prokaryotic host organisms may include but are not limited to Escherichia coli, Bacillus subtilis, B licehniformis, B. cereus, Streptomyces lividans, Streptomyces coelicolor, Pseudomonas aeruginosa, Myxococcus xanthus. Rhodococcus, Streptomycetes, Actinomycetes, Corynebacteria, Bacillus, Pseudomonas, Salmonella, and Erwinia. The complete genome sequences of E. coli and Bacillus subtilis are described by Blattner et al., Science 277, 1454-1462 (1997); Kunst et al., Nature 390, 249-256 (1997)).

Preferred eukaryotic host organisms are mammals, fish, insects, plants, algae and fungi.

Examples of mammalian cells include those from, e.g., monkey, mouse, rat, hamster, primate, and human, both cell lines and primary cultures. Preferred mammalian host cells include but are not limited to those derived from humans, monkeys and rodents, such as chinese hamster ovary (CHO) cells, NIH/3T3, COS, 293, VERO, HeLa etc (see Kriegler M. in "Gene Transfer and Expression: A Laboratory Manual", New York, Freeman & Co. 1990), and stem cells, including embryonic stem cells and hemopoietic stem cells, zygotes, fibroblasts, lymphocytes, kidney, liver, muscle, and skin cells.

Examples of insect cells include baculo lepidoptera.

Examples of plant cells include maize, rice, wheat, cotton, soybean, and sugarcane. Plant cells such as those derived from Nicotiana and Arabidopsis are preferred

Examples of fungi include penicillium, aspergillus, such as Aspergillus nidulans, podospora, neurospora, such as Neurospora crassa, saccharomyces, such as Saccharomyces cerevisiae (budding yeast), Schizosaccharomyces, such as Schizosaccharomyces pombe (fission yeast), Pichia spp, such as Pichia pastoris, and Hansenula polymorpha (methylotropic yeasts).

The choice of host will depend on a number of factors, depending on the intended use of the engineered host, including pathogenicity, substrate range, environmental hardiness, presence of key intermediates, ease of genetic manipulation, and likelihood of promiscuous transfer of genetic information to other organisms. Particularly advantageous hosts are E. coli, lactobacilli, Streptomycetes, Actinomycetes and filamentous fungi.

A preferred host cell is yeast due to the following characteristics: it is fast growing, eukaryotic, allows scalable culture capabilities, genetic tools are available, it is metabolically flexible, can have a relatively permeable cell membrane/wall and folds more heterologous eukaryotic proteins correctly than prokaryotic cells.

Thus, an illustrative and not limiting list of suitable yeast host cells comprise: baker's yeast, Kluyveromyces marxianus, K. lactis, Candida utilis, Phaffia rhodozyma, Saccharomyces boulardii, Pichia pastoris, Hansenula polymorpha, Yarrowia lipolytica, Candida paraffinica, Schwanniomyces castellii, Pichia stipitis, Candida shehatae, Rhodotorula glutinis, Lipomyces lipofer, Cryptococcos curvatus, Candida spp. (e.g. C. palmioleophila), Yarrowia lipolytica, Candida guilliermondii, Candida, Rhodotorula spp., Saccharomycopsis spp., Aureobasidium pullulans, Candida brumptii, Candida hydrocarbofumarica, Torulopsis, Candida tropicalis, Saccharomyces cerevisiae, Rhodotorula rubra, Candida flaveri, Eremothecium ashbyii, Pichia spp., Pichia pastoris, Schizosaccharomyces pompe (fission yeast), Kluyveromyces, Hansenula, Kloeckera, Pichia, Pachysolen spp., or Torulopsis bombicola.

Preferably, the host cell comprises at least one mutation in a central biosynthetic pathway. This mutation can be complemented by one or more selectable markers inserted into the vector comprising the heterologous expressible nucleotide sequences so that cells containing the vector(s) can be selected.

In any one host cell it is possible to make all sorts of combinations of expressible nucleotide sequences from all possible sources. Furthermore, it is possible to make combinations of promoters and/or spacers and/or introns and/or terminators in combination with one and the same expressible nucleotide sequence.

In a preferred embodiment the cells to be evolved are produced by inserting concatemers comprising the multitude of cassettes into a host cell, in which the concatemers can be maintained and the expressible nucleotide sequences can be expressed in a co-ordinated way. The cassettes comprised in the concatemers may be cut out from the host cell and re-assembled due to their uniform structure with - preferably - compatible restriction sites between the cassettes.

The cells as defined in the present invention are preferably collected into populations for use in the present invention. The composition of cells subjected to evolution is then produced by selecting cells from a population or from several sub-populations. A population of individual cells is a population of expression constructs prepared from randomly assembled or even concatenated expressible nucleotide sequences derived from a plurality of species of donor organisms, in which expressible nucleotide sequences are operably associated with regulatory regions that drives expression of the expressible nucleotide sequences in an appropriate host cell. The host cells used are capable of producing functional gene products of the donor organisms. Upon expression in the host cell, gene products of the donor organism(s) may interact to form novel biochemical pathways.

The population according to this embodiment of the invention may in any one cell comprise a unique and preferably random combination of a high number of expression cassettes being heterologous to the host cells. Through this random combination of expression cassettes novel and unique combinations of gene products are obtained in each cell. Such populations are especially adapted in the discovery of novel metabolic pathways created through the non-native combinations of gene products.

In a preferred embodiment a population may be defined as a population comprising a collection of individual cells, the cells being denoted
cell₁, cell₂, ...., cellᵢ, wherein i ≥ 2,
each cell comprising at least one concatemer of individual oligonucleotide cassettes, each concatemer comprising an oligonucleotide of the following formula:

   [rs₂-SP-PR-X-TR-SP-rs₁]ₙ
wherein rs₁ and rs₂ together denote a restriction site, SP denotes a spacer of at least two bases, X denotes an expressible nucleotide sequence, PR denotes a promoter capable of functioning in said cell, capable of regulating the expression of X in said cell, TR denotes a terminator, and n ≥ 2, and
wherein at least one concatemer of cell₁ is different from a concatemer of cell₂.

In the present context the nucleotide sequence of the formula [rs₂-SP-PR-X-TR-SP-rs₁]ₙ is also referred to as an expression cassette of the formula [rs₂-SP-PR-X-TR-SP-rs₁]ₙ.

Sub-populations may comprise cells as defined above for populations, but mostly the cells of a sub-population will have at least one trait in common, such as common promoter combinations, genetic material from a common species, a common phenotype or the like.

The function of the populations and sub-populations is to act as a source of diversity when obtaining the composition of cells to be evolved. Thus, in one embodiment the composition is a collection of subcompositions, wherein a subcomposition is a collection of individual cells having at least one phenotype in common. In a preferred embodiment the composition comprises at least 2 individual subcompositions, said subcompositions being different, such as at least 5 individual sub-compositions, such as at least 10 individual sub-compositions, wherein each sub-composition comprises at least 10 genetically different cells, such as at least 50 genetically different cells, such as at least 100 genetically different cells, such as at least 10³ genetically different cells, such as at least 10⁴ genetically different cells, such as at least 10⁵ genetically different cells, such as at least 10⁶ genetically different cells, such as at least 10⁷ genetically different cells, such as at least 10⁸ genetically different cells, such as at least 10⁹ genetically different cells.

The composition of cells preferably comprises at least 20 genetically different cells, such as at least 50 genetically different cells, such as at least 100 genetically different cells, such as at least 150 genetically different cells, such as at least 200 genetically different cells, such as at least 250 genetically different cells, such as at least 500 genetically different cells, such as at least 750 genetically different cells, such as at least 1000 genetically different cells, such as at least 10⁴ genetically different cells, such as at least 10⁵ genetically different cells, such as at least 10⁶ genetically different cells, such as at least 10⁷ genetically different cells, such as at least 10⁸ genetically different cells, such as at least 10⁹ genetically different cells.

In a preferred embodiment at least a majority of the individual cells have different genetic patterns or genotypes, thereby representing a great diversity.

The term "founding population" or a "founder populations" shall mean a Cell Population that has not itself been subjected to a selection round, in the present context also referred to as composition of cells. Optionally the expression constructs within the cell population are constructed such that genetic material from species that are known from prior art to produce compounds of a desired structure class, or compounds that have a desired functional effect, or are associated with a desired functional effect independent of knowledge of the compounds, predominate.

The term "daughter population" is a cell population having been subjected to at least one selection round. In the present context the daughter population is also referred to as a further modified composition.

### Controlling The Genetic Basis of the Diversity

### Sources of Genes

The natural world contains a significant amount of genetic diversity. Various authorities estimate that there are at least 10⁷ different species, and that each of these species contains on average at least 10⁴ genes. Even allowing for the fact that many of these genes are relatively conserved between species this represents a high level of genetic diversity.

One approach that can be envisaged for the purposes of the current invention is to source genetic material so as to maximise the taxonomic diversity of the genes obtained.

A second is to preferentially source genetic material from organisms that are known or reputed to produce molecules of the structural class or with the functional effects desired or are known or reputed to have a desired functional effect without the molecule being known, or are taxonomically related to any such organism.

A third approach is selection of genes of particular interest.

A fourth approach is to select genes that generally extend the host metabolic pathways.

Optionally these approaches can be combined in any suitable manner.

Genes can be sourced through the collection and processing of genetic material of various forms. The expressible nucleotide sequences that can be inserted into the vectors, concatemers, and cells according to this invention encompass any type of nucleotide such as RNA, DNA. Such a nucleotide sequence could be obtained e.g. from cDNA, which by its nature is expressible. But it is also possible to use sequences of genomic DNA, coding for specific genes. Preferably, the expressible nucleotide sequences correspond to full length genes such as substantially full length cDNA, but nucleotide sequences coding for shorter peptides than the original full length clones may also be used. Shorter peptides may still retain the catalytic activity of the native proteins. Thus, a preferred embodiment of this invention is to source and collect messenger transcripts (mRNA) for obtaining cDNA.

Another way to obtain expressible nucleotide sequences is through chemical synthesis of nucleotide sequences coding for known peptide or protein sequences. Thus the expressible DNA sequences does not have to be a naturally occurring sequence, although it may be preferable for practical purposes to primarily use naturally occurring nucleotide sequences. Whether the DNA is single or double stranded will depend on the vector system used.

By the term "Expression state" is meant a state of gene expression (i.e the mRNA transcript popuilation) in a specific cell, tissue, combination of tissues or organism or organisms of a given species as sampled at any one time. Different expression states are found in different individuals, or in the same individual at different point in time, or in the same individual at different points its life-cycle or in the same individual under differing external conditions. The expression states of given cells or tissues of a given individual will also vary with respect to other cells or tissues of the same individual. Different expression states may also be obtained in the same organ or tissue in any one species or individual by exposing the tissues or organs to different environmental conditions comprising but not limited to changes in developmental stage, age, disease, infection, drought, humidity, salinity, exposure to xenobiotics, physiological effectors, temperature, pressure, pH, light, gaseous environment, chemicals such as toxins.

In the following the invention is described in the order in which the steps of obtaining a transformed host cell containing an evolvable artificial chromosome may be performed, starting with the entry vector.

In most cases the orientation with respect to the promoter of an expressible nucleotide sequence will be such that the coding strand is transcribed into a proper mRNA. It is however conceivable that the sequence may be reversed generating an antisense transcript in order to block expression of a specific gene.

Each cell of the *cell population* is initially produced by combining genes selected from at least one expression state. It is of course also possible from the onset to combine genes from two, three, four or more expression states in one host cell or to combine genes from different organisms in one cell. In some embodiments of the invention it is preferred to combine genes from a large variety of organisms into a single host in a manner so that each cell comprises at least two expressible nucleotide sequences, said sequences being heterologous to the cell, i.e. the sequences are not found in the native cell type.

A wide variety of combinations of expressible nucleotide sequences from all possible sources may occur in the cells. Furthermore, it is possible to make combinations of promoters and/or spacers and/or introns and/or terminators in combination with one and the same expressible nucleotide sequence.

Thus in any one cell there may preferably be expressible nucleotide sequences from two different expression states. Furthermore, these two different expression states may be from one species or advantageously from two different species. Any one host cell may also comprise expressible nucleotide sequences from at least three species, such as from at least four, five, six, seven, eight, nine or ten species, or from more than 15 species such as from more than 20 species, for example from more than 30, 40 or 50 species, such as from more than 100 different species, for example from more than 300 different species, such as from more than 500 different species, for example from more than 750 different species, thereby obtaining combinations of large numbers of expressible nucleotide sequences from a large number of species. In this way potentially unlimited numbers of combinations of expressible nucleotide sequences can be combined across different expression states. These different expression states may represent at least two different tissues, such as at least two organs, such as at least two species, such as at least two genera. The different species may be from at least two different phylae, such as from at least two different classes, such as from at least two different divisions, more preferably from at least two different sub-kingdoms, such as from at least two different kingdoms. Thus expressible nucleotide sequences may be combined from a eukaryote and a prokaryote into one and the same cell.

According to another embodiment of the invention, the expressible nucleotide sequences may be from one and the same expression state. The products of these sequences may interact with the products of the genes in the host cell and with each other and form new enzyme combinations leading to novel biochemical pathways.

### Sources of genetic diversity

Examples of groups of species and individual species known to produce compounds with structural or functional utility include without limitation
- Bacteria: Streptomyces , Micromonospora, Norcadia, Actinomadura, Actinoplanes, Streptosporangium, Microbispora, Kitasatosporiam, Azobacterium, Rhizobium, Achromobacterium, Enterobacterium, Brucella, Micrococcus, Lactobacillus, Bacillus (B.t. toxins), Clostridium (toxins), Brevibacterium, Pseudomonas, Aerobacter, Vibrio, Halobacterium, Mycoplasma, Cytophaga, Myxococcus
- Fungi: Amanita muscaria (fly agaric, ibotenic acid, muscimol), Psilocybe (psilocybin) Physarium, Fuligo, Mucor, Phytophtora, Rhizopus, Aspergillus, Penicillium (penicillin), Coprinus, Phanerochaete, Acremonium (Cephalosporin), Trochoderma, Helminthosporium, Fusarium, Alternaria, Myrothecium, Saccharomyces
- Algae: Digenea simplex (kainic acid, antihelminthic), Laminaria anqustata (laminine, hypotensive)
- Lichens: Usnea fasciata (vulpinicacid, antimicrobial; usnic acid, antitumor)
- Higher Plants: Artemisia (artemisinin), Coleus (forskolin), Desmodium (K channel agonist), Catharanthus (Vinca alkaloids), Digitalis (cardiac glycosides), Podophyllum (podophyllotoxin), Taxus (taxol), Cephalotaxus (homoharringtonine), Camptotheca (Camptothecin), Camellia sinensis (Tea), Cannabis indica, Cannabis sativa (Hemp), Erythroxylum coca (Coca), Lophophora williamsii (PeyoteMyristica fragrans (Nutmeg), Nicotiana, Papaver somniferum (Opium Poppy), Phalaris arundinacea (Reed canary grass)
- Protozoa: Ptychodiscus brevis; Dinoflagellates (brevitoxin, cardiovascular)
- Sponges: Microciona prolifera (ectyonin, antimicrobial) Cryptotethya cryta (D-arabino furanosides)
- Coelenterata: Portuguese Man o War & other jellyfish and medusoid toxins.
- Corals: Pseudoterogonia species (Pseudoteracins, anti-inflammatory), Erythropodium (erythrolides, anti-inflammatory)
- Aschelminths: Nematode secretory compounds
- Molluscs: Conus toxins, sea slug toxins, cephalapod neurotransmitters, squid inks
- Annelida: Lumbriconereis heteropa (nereistoxin, insecticidal)
- Arachnids: Dolomedes ("fishing spider" venoms)
- Crustacea: Xenobalanus (skin adhesives)
- Insects: Epilachna (mexican bean beetle alkaloids)
- Spinunculida: Bonellia viridis (bonellin,neuroactive)
- Bryozoans: Bugula neritina (bryostatins,anti cancer)
- Echinoderms: Crinoid chemistry
- Tunicates: Trididemnum solidum (didemnin,anti-tumor and anti-viral; Ecteinascidia turbinata ecteinascidins, anti-tumor)
- Vertebrates: Eptatretus stoutii (eptatretin,cardioactive), Trachinus draco (proteinaceous toxins, reduce blood pressure, respiration and reduce heart rate). Dendrobatid frogs (batrachotoxins, pumiliotoxins, histrionicotoxins, and other polyamines); Snake venom toxins; Orinthorhynohus anatinus (duck-billed platypus venom), modified
carotenoids, retinoids and steroids; Avians: histrionicotoxins, modified carotenoids, retinoids and steroids

### Controlling Gene Expression - Expression Cassettes

Genes primarily give rise to selectable phenotypes through transcription of the gene to RNA and translation of the RNA to protein. Furthermore phenotypes are often the result of interactions between multiple genes and their gene products

Thus it is an element of the current invention that the heterologous genes are provided in a format whereby their individual and collective expression (transcription to RNA) can be controlled.

It is likely that through the combination of a high number of non-native genes in a host cell combinations of genes or single genes are inserted that are lethal or sub-lethal to the host cell. Through the co-ordinated expression of the genes in the host cell it is possible not only to initiate the expression of any subset of genes but also to repress such expression, e.g. of lethal or sub-lethal genes.

Through external regulation of the promoters controlling the expressible nucleotides sequences novel and non-naturally occurring combinations of expressed genes can be obtained. Since these novel and non-natural combinations of gene products are found in one and the same cell, the heterologous gene products may affect the metabolism of the host cell in novel ways and thus cause it to produce novel primary or secondary metabolites and/or known metabolites in novel amounts and/or known metabolites in novel compartments of the cell or outside the cells. The novel metabolic pathways and/or novel or modified metabolites may be obtained without substantially recombining the introduced genes with a segment in the host genome or an episome of the host cells by as well as without intra- or extra concatemeric recombination.

By having expressible nucleotide sequences under the control of a number of independently inducible or repressible promoters, a large number of different expression states can be created inside one single cell by selectively turning on and off groups of the inserted expressible nucleotide sequences. The number of independently inducible and/or repressible promoters in one cell may vary from 1 to 10, such as 2, 3, 4, 5, 6, 7, 8, or 9, or even up to 15, 20, 25 or above 50 promoters.

In the evolution steps the functionality of the controllable promoters of the cells is used, since due to the controllable promoters it is possible during the screening and selection step to switch promoters on and off, thereby creating a greater diversity of expressed genes.

The term promoter is used with its normal meaning, i.e. a DNA sequence to which RNA polymerase binds and initiates transcription. The promoter determines the polarity of the transcript by specifying which strand will be transcribed.
- Bacterial promoters normally consist of -35 and -10 (relative to the transcriptional start) consensus sequences which are bound by a specific sigma factor and RNA polymerase.
- Eukaryotic promoters are more complex. Most promoters utilized in expression vectors are transcribed by RNA polymerase II. General transcription factors (GTFs) first bind specific sequences near the transcriptional start and then recruit the binding of RNA polymerase II. In addition to these minimal promoter elements, small sequence elements are recognized specifically by modular DNA-binding / trans-activating proteins (e.g. AP-1, SP-1) which regulate the activity of a given promoter.
- Viral promoters may serve the same function as bacterial and eukaryotic promoters. Upon viral infection of their host, viral promoters direct transcription either by using host transcriptional machinery or by supplying virally encoded enzymes to substitute part of the host machinery. Viral promoters are recognised by the transcriptional machinery of a large number of host organisms and are therefore often used in cloning and expression vectors.

Promoters may furthermore comprise regulatory elements, which are DNA sequence elements which act in conjunction with promoters and bind either repressors (e.g., lacO/ LAC Iq repressor system in E. coli) or inducers (e.g., gal1 /GAL4 inducer system in yeast). In either case, transcription is virtually "shut off" until the promoter is derepressed or induced, at which point transcription is "turned-on". The choice of promoter in the cassette is primarily dependent on the host organism into which the cassette is intended to be inserted. An important requirement to this end is that the promoter should preferably be capable of functioning in the host cell, in which the expressible nucleotide sequence is to be expressed.

Preferably the promoter is an externally controllable promoter, such as an inducible promoter and/or a repressible promoter. The promoter may be either controllable (repressible/inducible) by chemicals such as the absence/presence of chemical inducers, e.g. metabolites, substrates, metals, hormones, sugars. The promoter may likewise be controllable by certain physical parameters such as temperature, pH, redox status, growth stage, developmental stage, or the promoter may be inducible/repressible by a synthetic inducer/repressor such as the gal inducer.

In order to avoid unintentional interference with the gene regulation systems of the host cell, and in order to improve controllability of the co-ordinated gene expression the promoter is preferably a synthetic promoter. Suitable promoters are described in US 5,798,227, US 5,667,986. Principles for designing suitable synthetic eukaryotic promoters are disclosed in US 5,559,027, US 5,877,018 or US 6,072,050.

Synthetic inducible eukaryotic promoters for the regulation of transcription of a gene may achieve improved levels of protein expression and lower basal levels of gene expression. Such promoters preferably contain at least two different classes of regulatory elements, usually by modification of a native promoter containing one of the inducible elements by inserting the other of the inducible elements. For example, additional metal responsive elements IR:Es) and/or glucocorticoid responsive elements (GREs) may be provided to native promoters. Additionally, one or more constitutive elements may be functionally disabled to provide the lower basal levels of gene expression.

Preferred examples of promoters include but is not limited to those promoters being induced and/or repressed by any factor selected from the group comprising carbohydrates, e.g. galactose; low inorganic phosphase levels; temperature, e.g. low or high temperature shift; metals or metal ions, e.g. copper ions; hormones, e.g. dihydrotestosterone; deoxycorticosterone; heat shock (e.g. 39°C); methanol; redox-status; growth stage, e.g. developmental stage; synthetic inducers, e.g. gal inducer.

Examples of such promoters include ADH 1, PGK 1, GAP 491, TPI, PYK, ENO, PMA 1, PHO5, GAL 1, GAL 2, GAL 10, MET25, ADH2, MEL 1, CUP 1, HSE, AOX, MOX, SV40, CaMV, Opaque-2, GRE, ARE, PGK/ARE hybrid, CYC/GRE hybrid, TPI/α2 operator, AOX 1, MOX A.

More preferably, however the promoter is selected from hybrid promoters such as PGK/ARE hybrid, CYC/GRE hybrid or from synthetic promoters. Such promoters can be controlled without interfering too much with the regulation of native genes in the expression host.

In the following, examples of known yeast promoters that may be used in conjunction with the present invention are shown. The examples are by no way limiting and only serve to indicate to the skilled practitioner how to select or design promoters that are useful according to the present invention.

Although numerous transcriptional promoters which are functional in yeasts have been described in the literature, only some of them have proved effective for the production of polypeptides by the recombinant route. There may be mentioned in particular the promoters of the PGK genes (3-phosphoglycerate kinase, TDH genes encoding GAPDH (Glyceraldehyde phosphate dehydrogenase), TEF1 genes (Elongation factor 1), MFα1 (α sex pheromone precursor) which are considered as strong constitutive promoters or alternatively the regulatable promoter CYCI which is repressed in the presence of glucose or PHO5 which can be regulated by thiamine. However, for reasons which are often unexplained, they do not always allow the effective expression of the genes which they control. In this context, it is always advantageous to be able to have new promoters in order to generate new effective host/vector systems. Furthermore, having a choice of effective promoters in a given cell also makes it possible to envisage the production of multiple proteins in this same cell (for example several enzymes of the same metabolic chain) while avoiding the problems of recombination between homologous sequences.

In general, a promoter region is situated in the 5' region of the genes and comprises all the elements allowing the transcription of a DNA fragment placed under their control, in particular:
(1) a so-called minimal promoter region comprising the TATA box and the site of initiation of transcription, which determines the position of the site of initiation as well as the basal level of transcription. In Saccharomyces cerevisiae, the length of the minimal promoter region is relatively variable. Indeed, the exact location of the TATA box varies from one gene to another and may be situated from -40 to - 120 nucleotides upstream of the site of the initiation (Chen and Struhl, 1985, EMBO J., 4, 3273-3280)
(2) sequences situated upstream of the TATA box (immediately upstream up to several hundreds of nucleotides) which make it possible to ensure an effective level of transcription either constitutively (relatively constant level of transcription all along the cell cycle, regardless of the conditions of culture) or in a regulatable manner (activation of transcription in the presence of an activator and/or repression in the presence of a repressor). These sequences, may be of several types: activator, inhibitor, enhancer, inducer, repressor and may respond to cellular factors or varied culture conditions.

Examples of such promoters are the ZZA1 and ZZA2 promoters disclosed in US 5,641,661, the EF1-α protein promoter and the ribosomal protein S7 gene promoter disclosed in WO 97/44470" the COX 4 promoter and two unknown promoters (SEQ ID No: 1 and 2 in the document) disclosed in US 5,952,195. Other useful promoters include the HSP150 promoter disclosed in WO 98/54339 and the SV40 and RSV promoters disclosed in US 4,870,013 as well as the PyK and GAPDH promoters disclosed in EP 0 329 203 A1.

More preferably the invention employs the use of synthetic promoters. Synthetic promoters are often constructed by combining the minimal promoter region of one gene with the upstream regulating sequences of another gene. Enhanced promoter control may be obtained by modifying specific sequences in the upstream regulating sequences, e.g. through substitution or deletion or through inserting multiple copies of specific regulating sequences. One advantage of using synthetic promoters is that they can be controlled without interfering too much with the native promoters of the host cell.

One such synthetic yeast promoter comprises promoters or promoter elements of two different yeast-derived genes, yeast killer toxin leader peptide, and amino terminus of IL-1β (WO 98/54339).

Another example of a yeast synthetic promoter is disclosed in US 5,436,136 (Hinnen et al), which concerns a yeast hybrid promoter including a 5' upstream promoter element comprising upstream activation site(s) of the yeast PHO5 gene and a 3' downstream promoter element of the yeast GAPDH gene starting at nucleotide -300 to -180 and ending at nucleotide -1 of the GAPDH gene.

Another example of a yeast synthetic promoter is disclosed in US 5,089,398 (Rosenberg et al). This disclosure describes a promoter with the general formula - (P.R.(2)-P.R.(1))-
wherein:
P.R.(1) is the promoter region proximal to the coding sequence and having the transcription initiation site, the RNA polymerase binding site, and including the TATA box, the CAAT sequence, as well as translational regulatory signals, e.g., capping sequence, as appropriate;
P.R.(2) is the promoter region joined to the 5'-end of P.R.(1) associated with enhancing the efficiency of transcription of the RNA polymerase binding region;

In US 4,945,046 (Horii et al) discloses a further example of how to design a synthetic yeast promoter. This specific promoter comprises promoter elements derived both from yeast and from a mammal. The hybrid promoter consists essentially of Saccharomyces cerevisiae PHO5 or GAP-DH promoter from which the upstream activation site (UAS) has been deleted and replaced by the early enhancer region derived from SV40 virus.

Co-ordinated expression of gene subsets can also be utilised to identify which heterologous genes are responsible for the production of a given phenotype.

In the following the sequence of steps to be taken when starting with the isolation of mRNA until insertion to an entry vector for providing the cells according to the invention is described. In short the sequence may include the following steps
i) isolating mRNA from an expression state,
ii) obtaining substantially full length cDNA clones corresponding to the mRNA sequences,
iii) inserting the substantially full length cDNA clones into a cloning site in a cassette in a primary vector, said cassette being of the general formula in 5'→3' direction:

   [RS1-RS2-SP-PR-CS-TR-SP-RS2'-RS1']

   wherein CS denotes a cloning site.

### Expression cassettes

The expression cassettes according to the present invention are preferably arranged as a cassette of nucleotides in a highly ordered sequence, the cassette having the general formula in 5'→3' direction:

[RS1-RS2-SP-PR-CS-TR-SP-RS2'-RS1']

wherein RS1 and RS1' denote restriction sites, RS2 and RS2' denote restriction sites different from RS1 and RS1', SP denotes a spacer sequence of at least two nucleotides, PR denotes a promoter, CS denotes a cloning site, and TR denotes a terminator, all of them being as discussed elsewhere in this specification.

It is an advantage to have two different restriction sites flanking both sides of the expression construct. By treating the primary vectors with restriction enzymes cleaving both restriction sites, the expression construct and the primary vector will be left with two non-compatible ends. This facilitates a concatenation process, since the empty vectors do not participate in the concatenation of expression constructs.

In principle, any restriction site, for which a restriction enzyme is known can be used. These include the restriction enzymes generally known and used in the field of molecular biology such as those described in Sambrook, Fritsch, Maniatis, "A laboratory Manual", 2^{nd} edition. Cold Spring Habor Laboratory Press, 1989.

The restriction site recognition sequences preferably are of a substantial length, so that the likelihood of occurrence of an identical restriction site within the cassette is minimised. Thus the first restriction site may comprise at least 6 bases, but more preferably the recognition sequence comprises at least 7 or 8 bases. Restriction sites having 7 or more non N bases in the recognition sequence are generally known as "rare restriction sites" (see example 17). However, the recognition sequence may also be at least 10 bases, such as at least 15 bases, for example at least 16 bases, such as at least 17 bases, for example at least 18 bases, such as at least 18 bases, for example at least 19 bases, for example at least 20 bases, such as at least 21 bases, for example at least 22 bases, such as at least 23 bases, for example at least 25 bases, such as at least 30 bases, for example at least 35 bases, such as at least 40 bases, for example at least 45 bases, such as at least 50 bases.

Preferably the first restriction site RS1 and RS1' is recognised by a restriction enzyme generating blunt ends of the double stranded nucleotide sequences. By generating blunt ends at this site, the risk that the vector participates in a subsequent concatenation is greatly reduced. The first restriction site may also give rise to sticky ends, but these are then preferably non-compatible to the sticky ends resulting from the second restriction site, RS2 and RS2'.

According to a preferred embodiment of the invention, the second restriction site, RS2 and RS2' comprises a rare restriction site. Thus, the longer the recognition sequence of the rare restriction site the more rare it is and the less likely is it that the restriction enzyme recognising it will cleave the nucleotide sequence at other - undesired - positions.

The rare restriction site may furthermore serve as a PCR priming site. Thereby it is possible to copy the cassettes via PCR techniques and thus indirectly "excise" the cassettes from a vector.

Single-stranded compatible ends may be created by digestion with restriction enzymes. For concatenation a preferred enzyme for excising the cassettes would be a rare cutter, i.e. an enzyme that recognises a sequence of 7 or more nucleotides. Examples of enzymes that cut very rarely are the meganucleases, many of which are intron encoded, like e.g. I-Ceu I, I-Sce I, I-Ppo I, and PI-Psp I (see example 17d for more). Other preferred enzymes recognize a sequence of 8 nucleotides like e.g. Asc I, AsiS I, CciN I, CspB I, Fse I, MchA I, Not I, Pac I, Sbf I, Sda I, Sgf I, SgrA I, Sse232 I, and Sse8387 I, all of which create single stranded, palindromic compatible ends.

Other preferred rare cutters, which may also be used to control orientation of individual cassettes in the concatemer are enzymes that recognize non-palindromic sequences like e.g. Aar I, Sap I, Sfi I, Sdi I, and Vpa (see example 17c for more).

Alternatively, cassettes can be prepared by the addition of restriction sites to the ends, e.g. by PCR or ligation to linkers (short synthetic dsDNA molecules). Restriction enzymes are continuously being isolated and characterised and it is anticipated that many of such novel enzymes can be used to generate single-stranded compatible ends according to the present invention.

It is conceivable that single stranded compatible ends can be made by cleaving the vector with synthetic cutters. Thus, a reactive chemical group that will normally be able to cleave DNA unspecffically can cut at specific positions when coupled to another molecule that recognises and binds to specific sequences. Examples of molecules that recognise specific dsDNA sequences are DNA, PNA, LNA, phosphothioates, peptides, and amides. See e.g. Armitage, B.(1998) Chem. Rev. 98: 1171-1200, who describes photocleavage using e.g. anthraquinone and UV light; Dervan P.B. & Bürli R.W. (1999) Curr. Opin. Chem. Biol. 3: 688-93 describes the specific binding of polyamides to DNA; Nielsen, P.E. (2001) Curr. Opin. Biotechnol. 12: 16-20 describes the specific binding of PNA to DNA, and Chemical Reviews special thematic issue: RNA/DNA Cleavage (1998) vol. 98 (3) Bashkin J.K. (ed.) ACS publications, describes several examples of chemical DNA cleavers.

Single-stranded compatible ends may also be created e.g. by using PCR primers including dUTP and then treating the PCR product with Uracil-DNA glycosylase (Ref: US 5,035,996) to degrade part of the primer. Alternatively, compatible ends can be created by tailing both the vector and insert with complimentary nucleotides using Terminal Transferase (Chang, LMS, Bollum TJ (1971) J Biol Chem 246:909).

The spacer sequence located between the RS2 and the PR sequence is preferably a non-transcribed spacer sequence. The purpose of the spacer sequence(s) is to minimise recombination between different concatemers present in the same cell or between cassettes present in the same concatemer, but it may also serve the purpose of making the nucleotide sequences in the cassettes more "host" like. A further purpose of the spacer sequence is to reduce the occurrence of hairpin formation between adjacent palindromic sequences, which may occur when cassettes are assembled head to head or tail to tail. Spacer sequences may also be convenient for introducing short conserved nucleotide sequences that may serve e.g. as PCR primer sites or as target for hybridization to e.g. nucleic acid or PNA or LNA probes allowing affinity purification of cassettes.

The cassette may also optionally comprise another spacer sequence of at least two nucleotides between TR and RS2. When cassettes are cut out from a vector and concatenated into concatemers of cassettes, the spacer sequences together ensure that there is a certain distance between two successive identical promoter or terminator sequences. This distance may comprise at least 50 bases, such as at least 60 bases, for example at least 75 bases, such as at least 100 bases, for example at least 150 bases, such as at least 200 bases, for example at least 250 bases, such as at least 300 bases, for example at least 400 bases, for example at least 500 bases, such as at least 750 bases, for example at least 1000 bases, such as at least 1100 bases, for example at least 1200 bases, such as at least 1300 bases, for example at least 1400 bases, such as at least 1500 bases, for example at least 1600 bases, such as at least 1700 bases, for example at least 1800 bases, such as at least 1900 bases, for example at least 2000 bases, such as at least 2100 bases, for example at least 2200 bases, such as at least 2300 bases, for example at least 2400 bases, such as at least 2500 bases, for example at least 2600 bases, such as at least 2700 bases, for example at least 2800 bases, such as at least 2900 bases, for example at least 3000 bases, such as at least 3200 bases, for example at least 3500 bases, such as at least 3800 bases, for example at least 4000 bases, such as at least 4500 bases, for example at least 5000 bases, such as at least 6000 bases.

The number of the nucleotides between the spacer located 5' to the PR sequence and the one located 3' to the TR sequence may be any. However, it may be advantageous to ensure that at least one of the spacer sequences comprises between 100 and 2500 bases, preferably between 200 and 2300 bases, more preferably between 300 and 2100 bases, such as between 400 and 1900 bases, more preferably between 500 and 1700 bases, such as between 600 and 1500 bases, more preferably between 700 and 1400 bases.

If the intended host cell is yeast, the spacers present in a concatemer should perferably comprise a combination of a few ARSes with varying lambda phage DNA fragments.

Preferred examples of spacer sequences include but are not limited to: Lamda phage DNA, prokaryotic genomic DNA such as E. coli genomic DNA, ARSes.

The cloning site in the cassette in the primary vector should be designed so that any nucleotide sequence can be cloned into it.

The cloning site in the cassette preferably allows directional cloning. Hereby is ensured that transcription in a host cell is performed from the coding strand in the intended direction and that the translated peptide is identical to the peptide for which the original nucleotide sequence codes.

However according to some embodiments it may be advantageous to insert the sequence in opposite direction. According to these embodiments, so-called antisense constructs may be inserted which prevent functional expression of specific genes involved in specific pathways. Thereby it may become possible to divert metabolic intermediates from a prevalent pathway to another less dominant pathway.

The cloning site in the cassette may comprise multiple cloning sites, generally known as MCS or polylinker sites, which is a synthetic DNA sequence encoding a series of restriction endonuclease recognition sites. These sites are engineered for convenient cloning of DNA into a vector at a specific position and for directional cloning of the insert.

Cloning of cDNA does not have to involve the use of restriction enzymes. Other alternative systems include but are not limited to:
- Creator^{™} Cre-IoxP system from Clontech, which uses recombination and IoxP sites
- use of Lambda attachment sites (att-λ), such as the Gateway^{™} system from Life Technologies.

Both of these systems are directional.

The role of the terminator sequence is to limit transcription to the length of the coding sequence. An optimal terminator sequence is thus one, which is capable of performing this act in the host cell.

In prokaryotes, sequences known as transcriptional terminators signal the RNA polymerase to release the DNA template and stop transcription of the nascent RNA.

In eukaryotes, RNA molecules are transcribed well beyond the end of the mature mRNA molecule. New transcripts are enzymatically cleaved and modified by the addition of a long sequence of adenylic acid residues known as the poly-A tail. A polyadenylation consensus sequence is located about 10 to 30 bases upstream from the actual cleavage site.

Preferred examples of yeast derived terminator sequences include, but are not limited to: ADN1, CYC1, GPD, ADH1 alcohol dehydrogenase.

Depending on the nature of the host cell, it may be advantageous that at least one cassette comprises an intron between the promoter and the expressible nucleotide sequence, more preferable that substantially all cassettes comprise an intron between the promoter and the expressible nucleotide sequence. The choice of intron sequence depends on requirements of the host cell.

Thus, optionally the cassette in the vector comprises an intron sequence, which may be located 5' or 3' to the expressible nucleotide sequence. The design and layout of introns is well known in the art. The choice of intron design largely depends on the intended host cell, in which the expressible nucleotide sequence is eventually to be expressed. The effects of having intron sequence in the expression cassettes are those generally associated with intron sequences.

Examples of yeast introns can be found in the literature and in specific databases such as Ares Lab Yeast Intron Database (Version 2.1) as updated on 15 April 2000. Earlier versions of the database as well as extracts of the database have been published in: "Genome-wide bioinformatic and molecular analysis of introns in Saccharomyces cerevisiae." by Spingola M, Grate L, Haussler D, Ares M Jr. (RNA 1999 Feb;5(2):221-34) and "Test of intron predictions reveals novel splice sites, alternatively spliced mRNAs and new introns in meiotically regulated genes of yeast." by Davis CA, Grate L, Spingola M, Ares M Jr, (Nucleic Acids Res 2000 Apr 15;28(8):1700-6).

### Primary vectors (entry vectors)

By the term entry vector is meant a vector for storing and amplifying cDNA or other expressible nucleotide sequences using the cassettes according to the present invention. The entry vectors or primary vectors are preferably able to propagate in E. coli or any other suitable standard host cell. It should preferably be amplifiable and amenable to standard normalisation and enrichment procedures.

The entry vector may be of any type of DNA that has the basic requirements of a) being able to replicate itself in at least one suitable host organism and b) allows insertion of foreign DNA which is then replicated together with the vector and c) preferably allows selection of vector molecules that contain insertions of said foreign DNA. In a preferred embodiment the vector is able to replicate in standard hosts like yeasts, bacteria and it should preferably have a high copy number per host cell. It is also preferred that the vector in addition to a host specific origin of replication, contains an origin of replication for a single stranded virus, such as e.g. the f1 origin for filamentous phages. This will allow the production of single stranded nucleic acid which may be useful for normalisation and enrichment procedures of cloned sequences. A vast number of cloning vectors have been described which are commonly used and references may be given to e.g. Sambrook,J; Fritsch, E.F; and Maniatis T. (1989) Molecular Cloning: A laboratory manual. Cold Spring Harbour Laboratory Press, USA, Netherlands Culture Collection of Bacteria (www.cbs.knaw.nl/NCCB/collection.htm) or Department of Microbial Genetics, National Institute of Genetics, Yata 1111 Mishima Shizuoka 411-8540, Japan (www.shigen.nig.ac.jp/cvector/cvector.html). A few type-examples that are the parents of many popular derivatives are M13mp10, pUC18, Lambda gt 10, and pYAC4. Examples of primary vectors include but are not limited to M13K07, pBR322, pUC18, pUC19, pUC118, pUC119, pSP64, pSP65, pGEM-3, pGEM-3Z, pGEM-3Zf(-), pGEM-4, pGEM-4Z, πAN13, pBluescript II, CHARON 4A, λ⁺, CHARON 21 A, CHARON 32, CHARON 33, CHARON 34, CHARON 35, CHARON 40, EMBL3A, λ2001, λDASH, λFIX, λgt10, λgt11, λgt18, λgt20, λgt22, λORF8, λZAP/R, pJB8, c2RB, pcos1 EMBL

Methods for cloning of cDNA or genomic DNA into a vector are well known in the art. Reference may be given to J. Sambrook, E.F. Fritsch, T. Maniatis: Molecular Cloning, A Laboratory Manual (2nd edition, Cold Spring Harbor Laboratory Press, 1989).

One example of a circular model entry vector is described in Figure 10. The vector, EVE contains the expression cassette, R1-R2-Spacer-Promoter-Multi Cloning Site-Terminator-Spacer-R2-R1. The vector furthermore contains a gene for ampicillin resistance, AmpR, and an origin of replication for E.coli, ColE1.

The entry vectors EVE4, EVE5, and EVE8 shown in Figures 11, 12, and 13. These all contain Srfl as R1 and Ascl as R2. Both of these sites are palindromic and are regarded as rare restriction sites having 8 bases in the recognition sequence. The vectors furthermore contain the AmpR ampicillin resistance gene, and the ColE1 origin or replication for E.coli as well as f1, which is an origin of replication for filamentous phages, such as M13. EVE4 (Fig. 11) contains the MET25 promoter and the ADH1 terminator. Spacer 1 and spacer 2 are short sequences deriving from the multiple cloning site, MCS. EVE5 (Fig. 12) contains the CUP1 promoter and the ADH1 terminator. EVE8 (Fig. 13) contains the CUP1 promoter and the ADH1 terminator. The spacers of EVE8 are a 550 bp lambda phage DNA (spacer 3) and an ARS sequence from yeast (spacer 4).

### Nucleotide library (entry library)

A schematic illustration of the steps leading from expression steps to a nucleotide library are illustrated in figure 8.

Methods as well as suitable vectors and host cells for constructing and maintaining a library of nucleotide sequences in a cell are well known in the art. The primary requirement for the library is that is should be possible to store and amplify in it a number of primary vectors (constructs) according to this invention, the vectors (constructs) comprising expressible nucleotide sequences from at least one expression state and wherein at least two vectors (constructs) are different.

One specific example of such a library is the well known and widely employed cDNA libraries. The advantage of the cDNA library is mainly that it contains only DNA sequences corresponding to transcribed messenger RNA in a cell. Suitable methods are also present to purify the isolated mRNA or the synthesised cDNA so that only substantially full-length cDNA is cloned into the library.

Methods for optimisation of the process to yield substantially full length cDNA may comprise size selection, e.g. electrophoresis, chromatography, precipitation or may comprise ways of increasing the likelihood of getting full length cDNAs, e.g. the SMART^{™} method (Clonetech) or the CapTrap^{™} method (Stratagene).

Preferably the method for making the nucleotide library comprises obtaining a substantially full length cDNA population comprising a normalised representation of cDNA species. More preferably a substantially full length cDNA population comprises a normalised representation of cDNA species characteristic of a given expression state.

Normalisation reduces the redundancy of clones representing abundant mRNA species and increases the relative representation of clones from rare mRNA species.

Methods for normalisation of cDNA libraries are well known in the art. Reference may be given to suitable protocols for normalisation such as those described in US 5,763,239 (DIVERSA) and WO 95/08647 and WO 95/11986. and Bonaldo, Lennon, Soares, Genome Research 1996, 6:791-806; Ali, Holloway, Taylor, Plant Mol Biol Reporter, 2000,18:123-132.

Enrichment methods are used to isolate clones representing mRNA which are characteristic of a particular expression state. A number of variations of the method broadly termed as subtractive hybrisation are known in the art. Reference may be given to Sive, John, Nucleic Acid Res, 1988, 16:10937; Diatchenko, Lau, Campbell et al, PNAS, 1996, 93:6025-6030; Carninci, Shibata, Hayatsu, Genome Res, 2000, 10:1617-30, Bonaldo, Lennon, Soares, Genome Research 1996, 6:791-806; Ali, Holloway, Taylor, Plant Mol Biol Reporter, 2000, 18:123-132. For example, enrichment may be achieved by doing additional rounds of hybridization similar to normalization procedures, using e.g. cDNA from a library of abundant clones or simply a library representing the uninduced state as a driver against a tester library from the induced state. Alternatively mRNA or PCR amplified cDNA derived from the expression state of choice can be used to subtract common sequences from a tester library. The choice of driver and tester population will depend on the nature of target expressible nucleotide sequences in each particular experiment.

Finally, enrichment may be achieved by subtractive hybridisation followed by colony picking.

In the library an expressible nucleotide sequence coding for one peptide is preferably found in different but similar vectors under the control of different promoters. Preferably the library comprises at least three primary vectors with an expressible nucleotide sequence coding for the same peptide under the control of three different promoters. More preferably the library comprises at least four primary vectors with an expressible nucleotide sequence coding for the same peptide under the control of four different promoters. More preferably the library comprises at least five primary vectors with an expressible nucleotide sequence coding for the same peptide under the control of five different promoters, such as comprises at lest six primary vectors with an expressible nucleotide sequence coding for the same peptide under the control of six different promoters, for example comprises at least seven primary vectors with an expressible nucleotide sequence coding for the same peptide under the control of seven different promoters, for example comprises at least eight primary vectors with an expressible nucleotide sequence coding for the same peptide under the control of eight different promoters, such as comprises at least nine primary vectors with an expressible nucleotide sequence coding for the same peptide under the control of nine different promoters, for example comprises at least ten primary vectors with an expressible nucleotide sequence coding for the same peptide under the control of ten different promoters.

The expressible nucleotide sequence coding for the same peptide preferably comprises essentially the same nucleotide sequence, more preferably the same nucleotide sequence.

By having a library with what may be termed one gene under the control of a number of different promoters in different vectors, it is possible to construct from the nucleotide library an array of combinations of genes and promoters. Preferably, one library comprises a complete or substantially complete combination such as a two dimensional array of genes and promoters, wherein substantially all genes are found under the control of substantially all of a selected number of promoters.

According to another embodiment of the invention the nucleotide library comprises combinations of expressible nucleotide sequences combined in different vectors with different spacer sequences and/or different intron sequences. Thus any one expressible nucleotide sequence may be combined in a two, three, four or five dimensional array with different promoters and/or different spacers and/or different introns and/or different terminators. The two, three, four or five dimensional array may be complete or incomplete, since not all combinations will have to be present.

The library may suitably be maintained in a host cell comprising prokaryotic cells or eukaryotic cells. Preferred prokaryotic host organisms may include but are not limited to Escherichia coli, Bacillus subtilis, Streptomyces lividans, Streptomyces coelicolor Pseudomonas aeruginosa, Myxococcus xanthus.

Yeast species such as Saccharomyces cerevisiae (budding yeast), Schizosaccharomyces pombe (fission yeast), Pichia pastoris, and Hansenula polymorpha (methylotropic yeasts) may also be used. Filamentous ascomycetes, such as Neurospora crassa and Aspergillus nidulans may also be used. Plant cells such as those derived from Nicotiana and Arabidopsis are preferred. Preferred mammalian host cells include but are not limited to those derived from humans, monkeys and rodents, such as chinese hamster ovary (CHO) cells, NIH/3T3, COS, 293, VERO, HeLa etc (see Kriegler M. in "Gene Transfer and Expression: A Laboratory Manual", New York, Freeman & Co. 1990).

### Concatemers

Fig. 9 shows a flow chart of the steps leading from an entry library comprising expressible nucleotide sequences to evolvable artificial chromosomes (EVAC) transformed into an appropriate host cell. Fig. 9a shows one way of producing the EVACs which includes concatenation, size selection and insertion into an artificial chromosome vector. Fig. 9b shows a one step procedure for concatenation and ligation of vector arms to obtain EVACs.

For the purposes of providing a method for assembling multiple expression cassettes ("cassettes") into a single host cell, and allowing their facile remixing between cells, the expression cassettes are assembled into concatemers.

A concatemer is a series of linked units. The concatemers according to the invention may comprise a selection of expressible nucleotide sequences from just one expression state and can thus be assembled from one library representing this expression state or it may comprise cassettes from a number of different expression states. The concatemers according to the invention are especially suitable for ligating into an artificial chromosome, which may be inserted into a host cell for coordinated expression. For this purpose, the variation among and between cassettes may be such as to minimise the chance of cross over as the host cell undergoes cell division such as through minimising the level of repeat sequences occurring in any one concatemer, since it is not an object of this embodiment of the invention to obtain recombination of concatemers with a segment in the host genome or an epitope of the host cells nor is it an object to obtain intra- or extra concatemeric recombination.

According to a preferred embodiment of the invention the concatemer comprises at least a first cassette and a second cassette, said first cassette being different from said second cassette. More preferably, the concatemer comprises cassettes, wherein substantially all cassettes are different. The difference between the cassettes may arise from differences between promoters, and/or expressible nucleotide sequences, and/or spacers, and/or introns and/or terminators.

The number of cassettes in a single concatemer is largely determined by the host species into which the concatemer is eventually to be inserted and the vector through which the insertion is carried out. The concatemer thus may comprise at least 10 cassettes, such as at least 15, for example at least 20, such as at least 25, for example at least 30, such as from 30 to 60 or more than 60, such as at least 75, for example at least 100, such as at least 200, for example at least 500, such as at least 750, for example at least 1000, such as at least 1500, for example at least 2000 cassettes.

Each of the cassettes may be laid out as described above.

Thus, in a preferred embodiment a concatemer is used to denote a number of serially linked nucleotide cassettes, wherein at least two of the serially linked nucleotide units comprises a cassette having the basic structure

[rs₂-SP-PR-X-TR-SP-rs₁]

wherein
rs₁ and rs₂ together denote a restriction site,
SP denotes a spacer of at least two nucleotide bases,
PR denotes a promoter, capable of functioning in a host cell,
X denotes an expressible nucleotide sequence,
TR denotes a terminator, and
SP denotes a spacer of at least two nucleotide bases.
   wherein the variables of the cassette have the meaning as defined elsewhere in this specification. Optionally the cassettes comprise an intron sequence between the promoter and the expressible nucleotide sequence and/or between the terminator and the expressible nucleotide sequence as discussed above.

According to one aspect of the invention, a concatemer comprises cassettes with expressible nucleotide from different expression states, so that non-naturally occurring combinations or non-native combinations of expressible nucleotide sequences are obtained.

According to a preferred embodiment of the invention the concatemer comprises at least a first cassette and a second cassette, said first cassette being different from said second cassette. More preferably, the concatemer comprises cassettes, wherein substantially all cassettes are different. The difference between the cassettes may arise from differences between promoters, and/or expressible nucleotide sequences, and/or spacers, and/or terminators, and/or introns.

The concatenation may be carried out in different ways.

Cassettes to be concatenated are normally excised from a vector or they are synthesised through PCR. After excision the cassettes may be separated from the vector through size fractionation such as gel filtration or through tagging of known sequences in the cassettes. The isolated cassettes may then be ligated together either through interaction between sticky ends or through ligation of blunt ends.

More preferably the cassettes may be concatenated without an intervening purification step through excision from a vector with two restriction enzymes, one leaving sticky ends on the cassettes and the other one leaving blunt ends in the vectors.

An alternative way of producing concatemers free of vector sequences would be to PCR amplify the cassettes from a single stranded primary vector. The PCR product must include the restriction sites RS2 and RS2' which are subsequently cleaved by its cognate enzyme(s). Concatenation can then be performed using the digested PCR product, essentially without interference from the single stranded primary vector template or the small double stranded fragments, which have been cut form the ends.

When the vectors comprising the cassettes are single stranded, the cassettes may be excised and be made double stranded through PCR techniques, which only prime the cassette sequence and not the vector sequence. Sticky ends can be made by cleaving with a restriction enzyme leaving sticky ends and the cassettes can be assembled without interaction from the single stranded vector fragments.

The concatemer may be assembled or concatenated by concatenation of at least two cassettes of nucleotide sequences each cassette comprising a first sticky end, a spacer sequence, a promoter, an expressible nucleotide sequence, a terminator, and a second sticky end.

After concatenation has been completed, concatemers of the desired size may be selected through size selection, such as selection for concatemers having at least 10 cassettes, such as at least 15, for example at least 20, such as at least 25, for example at least 30, such as from 30 to 60 or more than 60, such as at least 75, for example at least 100, such as at least 200, for example at least 500, such as at least 750, for example at least 1000, such as at least 1500, for example at least 2000 cassettes. The number of cassettes in each concatemer may be controlled by size fractionation after concatenation, since the size of the concatemers is approximately proportional to the number of cassettes.

Preferably at least one inserted concatemer in each cell comprises at least one selectable marker. Selectable markers generally provide a means to select, for growth, only those cells which contain a vector. The selectable markers are inserted into the concatemers to complement one or more mutations in a central biosynthetic pathway, these mutations being inserted into the host cells. Such markers are of two types: drug resistance and auxotrophic. A drug resistance marker enables cells to detoxify an exogenously added drug that would otherwise kill the cell. Auxotrophic markers allow cells grow in media lacking an essential component by enabling cells to synthesise the essential component (usually an amino acid).

Illustrative and non-limiting examples of common selectable markers with a brief description of their mode of action follow:

### Prokaryotic

- Ampicillin: interferes with a terminal reaction in bacterial cell wall synthesis. The resistance gene (bla) encodes beta-lactamase which cleaves the beta-Iactam ring of the antibiotic thus detoxifying it.
- Tetracycline: prevents bacterial protein synthesis by binding to the 30S ribosomal subunit. The resistance gene (tet) specifies a protein that modifies the bacterial membrane and prevents transport of the antibiotic into the cell.
- Kanamycin: binds to the 70S ribosomes and causes misreading of messenger RNA. The resistant gene (nptH) modifies the antibiotic and prevents interaction with the ribosome.
- Streptomycin: binds to the 30S ribosomal subunit, causing misreading of messenger RNA. The resistance gene (Sm) modifies the antibiotic and prevents interaction with the ribosome.
- Zeocin: this new bleomycin-family antibiotic intercalates into the DNA and cleaves it. The Zeocin resistance gene encodes a 13,665 dalton protein. This protein confers resistance to Zeocin by binding to the antibiotic and preventing it from binding DNA. Zeocin is effective on most aerobic cells and can be used for selection in mammalian cell lines, yeast, and bacteria.
- Auxotrophic markers.
**Eukaryotic**
- Hygromycin: a aminocyclitol that inhibits protein synthesis by disrupting ribosome translocation and promoting mistranslation. The resistance gene (hph) detoxifies hygromycin -B- phosphorylation.
- Histidinol: cytotoxic to mammalian cells by inhibiting histidyl-tRNA synthesis in histidine free media. The resistance gene (hisD) product inactivates histidinol toxicity by converting it to the essential amino add, histidine.
- Neomycin (G418): blocks protein synthesis by interfering with ribosomal functions. The resistance gene ADH encodes amino glycoside phosphotransferase which detoxifies G418.
- Uracil: Laboratory yeast strains carrying a mutated gene which encodes orotidine -5'- phosphate decarboxylase, an enzyme essential for uracil biosynthesis, are unable to grow in the absence of exogenous uracil. A copy of the wild-type gene (ura4+, S. pombe or URA3 S. cerevisiae) carried on the vector will complement this defect in transformed cells.
- Adenosine: Laboratory strains carrying a deficiency in adenosine synthesis maybe complemented by a vector carrying the wild type gene, ADE 2.
- Amino acids: Vectors carrying the wild-type genes for LEU2, TRP 1, HIS 3 or LYS 2 may be used to complement strains of yeast deficient in these genes.
- Zeocin: this new bleomycin-family antibiotic intercalates into the DNA and cleaves it The Zeocin resistance gene encodes a 13,665 dalton protein. This protein confers resistance to Zeocin by binding to the antibiotic and preventing it from binding DNA. Zeocin is effective on most aerobic cells and can be used for selection in mammalian cell lines, yeast, and bacteria.

The number of concatemers in one single cell may be at least one concatemer per cell, preferably at least 2 concatemers per cell, more preferably 3 per cell, such as 4 per cell, more preferably 5 per cell, such as at least 5 per cell, for example at least 6 per cell, such as 7, 8, 9 or 10 per cell, for example more than 10 per cell. As described above, each concatemer may preferably comprise up to 1000 cassettes, and it is envisages that one concatemer may comprise up to 2000 cassettes. By inserting up to 10 concatemers into one single cell, this cell may thus be enriched with up to 20,000 new expressible genes, which under suitable conditions may be turned on and off by regulation of the regulatable promoters. However it may be more preferable to provide cells having anywhere between 10 and 1000 novel genes, such as 20-900 novel genes, for example 30 to 800 novel genes, such as 40 to 700 novel genes, for example 50 to 600 novel genes, such as from 60 to 300 novel genes. The genes may advantageously be located on 1 to 10 such as from 2 to 5 different concatemers in the cells. Each concatemer may advantageously comprise from 10 to 1000 genes, such as from 10 to 750 genes, such as from 10 to 500 genes, such as from 10 to 200 genes, such as from 20 to 100 genes, for example from 30 to 60 genes.

The concatemers may be inserted into the host cells according to any known transformation technique, preferably according to such transformation techniques that ensure stable and not transient transformation of the host cell. The concatemers may thus be inserted as an artificial chromosome which is replicated by the cells as they divide or they may be inserted into the chromosomes of the host cell. The concatemer may also be inserted in the form of a plasmid such as a plasmid vector, a phage vector, a viral vector, a cosmid vector, that is replicated by the cells as they divide. Any combination of the three insertion methods is also possible. One or more concatemers may thus be integrated into the chromosome(s) of the host cell and one or more concatemers may be inserted as plasmids or artificial chromosomes. One or more concatemers may be inserted as artificial chromosomes and one or more may be inserted into the same cell via a plasmid.

The basic requirements for a functional artificial chromosome have been described in US 4,464,472. An artificial chromosome or a functional minichromosome, as it may also be termed must comprise a DNA sequence capable of replication and stable mitotic maintenance in a host cell comprising a DNA segment coding for centromere-like activity during mitosis of said host and a DNA sequence coding for a replication site recognized by said host.

Suitable artificial chromosomes include a Yeast Artificial Chromosome (YAC) (see e.g. Murray et al, Nature 305:189-193; or US 4,464,472), a mega Yeast Artificial Chromosome (mega YAC), a Bacterial Artificial Chromosome (BAC), a mouse artificial chromosome, a Mammalian Artificial Chromosome (MAC) (see e.g. US 6,133,503 or US 6,077,697), an Insect Artificial Chromosome (BUGAC), an Avian Artificial Chromosome (AVAC), a Bacteriophage Artificial Chromosome, a Baculovirus Artificial Chromosome, a plant artificial chromosome (US 5,270,201), a BIBAC vector (US 5,977,439) or a Human Artificial Chromosome (HAC).

The artificial chromosome is preferably so large that the host cell perceives it as a "real" chromosome and maintains it and transmits it as a chromosome. For yeast and other suitable host species, this will often correspond approximately to the size of the smallest native chromosome in the species. For Saccharomyces, the smallest chromosome has a size of 225 Kb.

MACs may be used to construct artificial chromosomes from other species, such as insect and fish species. The artificial chromosomes preferably are fully functional stable chromosomes. Two types of artificial chromosomes may be used. One type, referred to as SATACs [satellite artificial chromosomes] are stable heterochromatic chromosomes, and the other type are minichromosomes based on amplification of euchromatin.

Mammalian artificial chromosomes provide extra-genomic specific integration sites for introduction of genes encoding proteins of interest and permit megabase size DNA integration, such as integration of concatemers according to the invention.

According to another embodiment of the invention, the concatemer may be integrated into the host chromosomes or cloned into other types of vectors, such as a plasmid vector, a phage vector, a viral vector or a cosmid vector.

A preferable artificial chromosome vector is one that is capable of being conditionally amplified in the host cell, e.g. in yeast. The amplification preferably is at least a 10 fold amplification. Furthermore, it is advantageous that the cloning site of the artificial chromosome vector can be modified to comprise the same restriction site as the one bordering the cassettes described above, i.e. RS2 and/or RS2'.

It is also conceivable that recombination can be used to generate concatemers, e.g. through the modification of techniques like the Creator system (Clontech) which uses the Cre-loxP mechanism (ref: Sauer B 1993 Methods Enzymol 225:890-900) to directionally join DNA molecules by recombination or like the Gateway system (Life Technologies, US 5,888,732) using lambda att attachment sites for directional recombination (Landy A 1989, Ann Rev Biochem 58:913). It is envisaged that also lambda cos site dependent systems can be developed to allow concatenation.

The concatemer may be assembled or concatenated by concatenation of at least two cassettes of nucleotide sequences each cassette comprising a first sticky end, a spacer sequence, a promoter, an expressible nucleotide sequence, a terminator, and a second sticky end. A flow chart of the procedure is shown in figure 9a.

Preferably concatenation further comprises
starting from a primary vector [RS1-RS2-SP-PR-X-TR-SP-RS2'-RS1'],
wherein X denotes an expressible nucleotide sequence,
RS1 and RS1' denote restriction sites,
RS2 and RS2' denote restriction sites different from RS1 and RS1',
SP denotes a spacer sequence of at least two nucleotides,
PR denotes a promoter,
TR denotes a terminator,
   i) cutting the primary vector with the aid of at least one restriction enzyme specific for RS2 and RS2' obtaining cassettes having the general formula [rs₂-SP-PR-X-TR-SP-rs₁] wherein rs₁ and rs₂ together denote a functional restriction site RS2 or RS2',
   ii) assembling the cut out cassettes through interaction between rs₁ and rs₂.

According to an especially preferred embodiment, vector arms each having a RS2 or RS2' in one end and a non-complementary overhang or a blunt end in the other end are added to the concatenation mixture together with the cassettes described above to further simplify the procedure (see Fig. 9b). One example of a suitable vector for providing vector arms is disclosed in Fig. 15 TRP1, URA3, and HIS3 are auxotrophic marker genes, and AmpR is an antibiotic marker gene. CEN4 is a centromer and TEL are telomeres. ARS1 and PMB1 allow replication in yeast and E. coli respectively. BamH I and Asc I are restriction enzyme recognition sites. The nucleotide sequence of the vector is set forth in SEQ ID NO 4. The vector is digested with BamHI and AscI to liberate the vector arms, which are used for ligation to the concatemer.

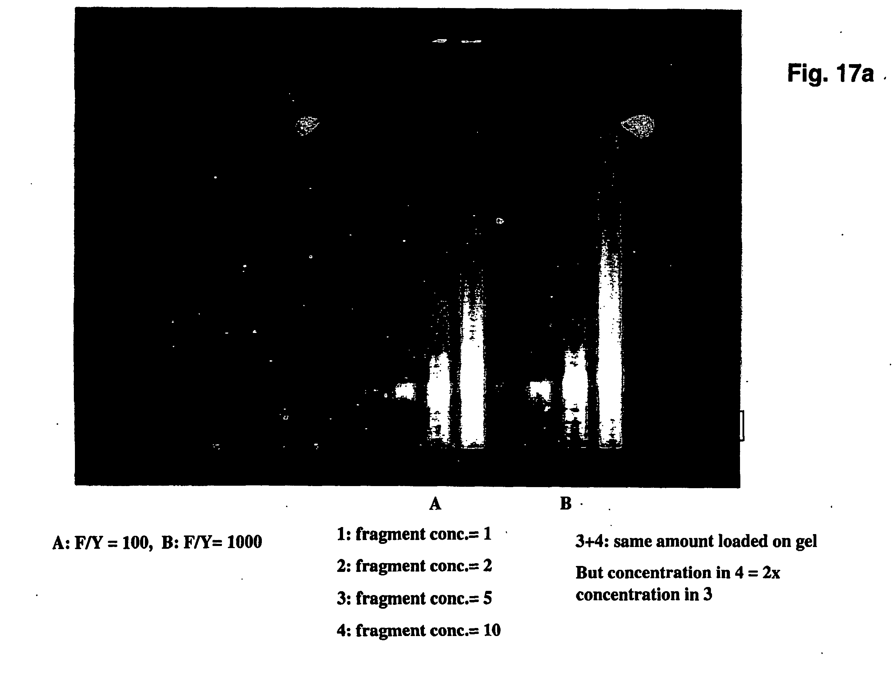

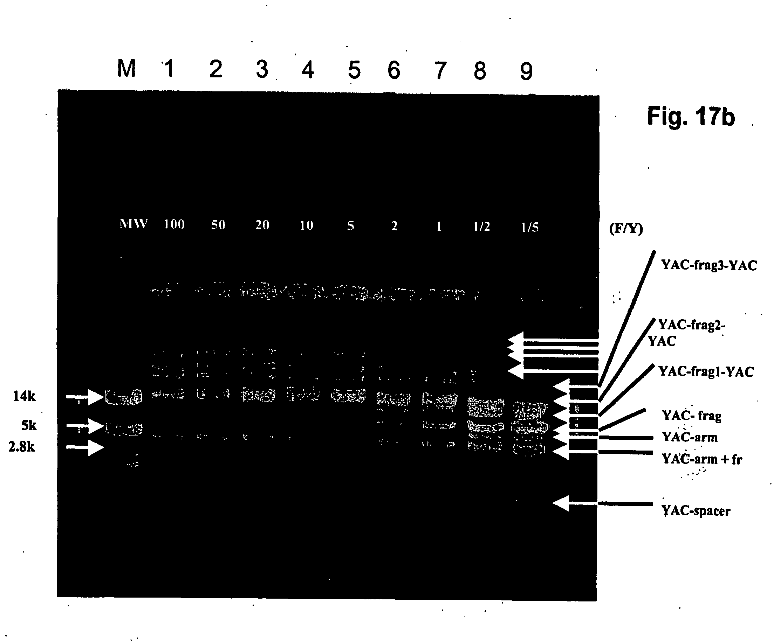

The general concatenation strategy is illustrated in Figure 16. The ratio of vector arms to cassettes determines the maximum number of cassettes in the concatemer as illustrated in figure 17. The vector arms preferably are artificial chromosome vector arms such as those described in Fig. 15.

It is of course also possible to add stopper fragments to the concatenation solution, the stopper fragments each having a RS2 or RS2' in one end and a non-complementary overhang or a blunt end in the other end. The ratio of stopper fragments to cassettes can likewise control the maximum size of the concatemer.

As an alternative to providing vector arms for the concatenation procedure is possible to ligate the concatemer into an artificial chromosome selected from the group comprising yeast artificial chromosome, mega yeast artificial chromosome, bacterial artificial chromosome, mouse artificial chromosome, human artificial chromosome.

The number of concatemers in one single cell may be at least one concatemer per cell, preferably at least 2 concatemers per cell, more preferably 3 per cell, such as 4 per cell, more preferably 5 per cell, such as at least 5 per cell, for example at least 6 per cell, such as 7, 8, 9 or 10 per cell, for example more than 10 per cell. As described above, each concatemer may preferably comprise up to 1000 cassettes, and it is envisages that one concatemer may comprise up to 2000 cassettes. By inserting up to 10 concatemers into one single cell, this cell may thus be enriched with up to 20,000 heterologous expressible genes, which under suitable conditions may be turned on and off by regulation of the regulatable promoters.

Often it is more preferable to provide cells having anywhere between 10 and 1000 heterologous genes, such as 20-900 heterologous genes, for example 30 to 800 heterologous genes, such as 40 to 700 heterologous genes, for example 50 to 600 heterologous genes, such as from 60 to 300 heterologous genes or from 100 to 400 heterologous genes which are inserted as 2 to 4 artificial chromosomes each containing one concatemer of genes. The genes may advantageously be located on 1 to 10 such as from 2 to 5 different concatemers in the cells. Each concatemer may advantageously comprise from 10 to 200 genes, such as from 20 to 100 genes, for example from 30 to 60 genes, or from 50 to 100 genes.

### EXAMPLES OF MULTIPLE PARAMETER SCREENS

### Example 1: Multiple Parameter Screen for ligand activators of RARβ and P450s metabolism

A yeast strain expressing the P450's CYP3A4α, CYP2C9, CYP2D6, RARβ and containing a retionoic acid (RA) responsive promoter driving the transcription of the HIS3 marker is mated with a producer yeast strain (comprising a population of cells each containing a set of heterologous expression cassettes located on artificial chromosomes). When the diploids are plated on HIS selective media, only cells that produce RAR activators will survive. All construct assembly and handling done according to standard procedures described in Current protocols in molecular biology 1999, John Wiley & Sons, Inc.

### Example 2: Multiple Parameter Screen for P450s metabolism and ligands that activate RARβ but not RARα

The same diploid yeast cells as in example 1 except that the reporter gene for RARβ is GFP (Green Fluorescent Protein) are co-gel-encapsulated with another yeast cell line that expresses RARα and contains a RA responsive promoter driving the transcription of YFP (Yellow Fluorescent Protein). The gel encapsulation is done as in Sahar et al., 1994., Flow cytometric analysis of entire microbial colonies. Cytometry 15: 213-221. The gel particles are then sorted for high Green Fluorescence intensity and low Yellow Fluorescence intensity.

### Example 3: Multiple Parameter Screen for P450 metabolism and ligands that activate RARβ but not RARα or RARγ

The same gel particles as in example 2 are double encapsulated with a yeast cell line that expresses RARγ and contains a RA responsive promoter driving the transcription of CFP (Cyan Fluorescent Protein). The double gel encapsulation is done in principal as in Gift et al., 1996, Nature Biotechnology, Vol. 14, 884-887. The gel particles are then sorted for high green fluorescence intensity and low yellow and cyan fluorescence intensities.

### Example 4: Multiple Parameter Screen for P450 metabolism and ligands that activate PPARγ and PPARα in a mammalian system

A producer yeast strain comprising a population of cells each containing a set of heterologous expression cassettes located on artificial chromosomes and expressing the P450s, CYP3A4α and CYP2C, is co-gel-encapsulated with a mammalian cell line expressing PPARγ, PPARα, and containing a PPARγ responsive promoter driving the transcription of YFP and a PPARα responsive promoter driving the transcription of CFP. Handling and manipulation of mammalian cells done according to standard procedures described in Current protocols in molecular biology 1999, John Wiley & Sons, Inc. The gel particles are sorted for high yellow and cyan fluorescence intensity.

### Example 5: Multiple Parameter Screen for multi-drug resistant S. aureus (MRSA) growth inhibition and DNA Polymerase III inhibition

The producer strains (comprising a population of cells each containing a set of heterologous expression cassettes located on artificial chromosomes) are gel encapsulated using conditions that give on average one strain per bead as described in US 4,399,219. The beads are placed under conditions that allow the producer strains to grow and the heterologous genes to be expressed. The beads are double encapsulated with a MRSA strain and in the presence of a substrate of DNA polymerase III that when metabolised produces fluorescent molecules. Gel droplets that have small or no MRSA colonies and which are not fluorescent are selected.

### Example 6A: Multiple Parameter Screen for DNA Topoisomerase II inhibition and growth inhibition of HeLa cells

The producer strains (comprising a population of cells each containing a set of heterologous expression cassettes located on artificial chromosomes) in a media containing a stain that binds to DNA double strand breaks are plated. HeLa cells are plated over the producer strains. Producer strains in areas where cancer cell growth was inhibited and which are coloured are selected. See figure 3B

### Example 6B: Multiple Parameter Screen for specific DNA Topoisomerase II poisons

The producer strains (comprising a population of cells each containing a set of heterologous expression cassettes located on artificial chromosomes) are gel encapsulated using conditions that give on average one strain per bead as described in US 4,399,219. The beads are placed under conditions that allow the producer strains to grow and the heterologous genes to be expressed. The beads are double gel encapsulated with yeast cells bearing the topisomerase II mutant top2-1 (like JN394t2-1), expressing GFP and yeast cells deleted for yeast topoisomerase I (like JN394t1), expressing DsRed. The beads are sorted for green and red fluorescence on a FACS. Beads with do not produce red or green fluorescence produce produced topoisomerase I specific poisons. Beads producing only green fluorescent have topoisomerase II specific poisons. The reporter strains described are based on the strains described in Methods In molecular biology, 95, 315-327. See figure 3B.

### EXAMPLES OF OPTIMISATION PROCESSES USING THE CURRENT EVOLUTIONARY APPROACHES

Examples of how the current invention can be used to evolve cells with potential pharmaceutical, industrial, agronomic, or nutritional utility are now provided.

### Evolution of specific structural classes via short to medium sized pathways

### Example 7: Evolution of Carotenoid like compounds

### UTILITY

Carotenoids are natural pigments displaying yellow, orange, pink, red and blue colours. A major role is protection against oxidative damage. Carotenoids are both pharmaceutically relevant (used to treat bronchial asthma and involved in the prevention of cancer) and of commercial value.

### SCREENING & SELECTION STRATEGY

- Production of a different colour by host cell. Screen done manually by plating and picking
- Anti-oxidant protection. Screen done using methylene blue as a producer of singlet oxygen species.

### PROCEDURE

- STEP 1: Essentially full length cDNA is made from the species in the list provided in this example.
- STEP 2: cDNA libraries are made using a pool of 4 entry vectors: pEVE4, pEVE5, pEVE8 and pEVE9 in a proportion of 30:30:1:30. See Figures 11, 12, 13, and 14.
- STEP 3: Each cDNA library is normalised essentially as method 4 described in Bonaldo, MF et al. (1996) Genome Res. 6: 791-806.
- STEP 4: Coding sequences from a non-normalised yeast (*Saccharomyces cerevisiae*) cDNA library are amplified by PCR and are used as driver for subtractive hybridization against single stranded circular DNA prepared from the normalized library produced in step 3, in order to remove household genes. Remaining single stranded circles are purified, converted to double stranded DNA and used to transform E.coli.
- STEP 5: EVAC (Evolvable Artificial Chromosome) containing cell populations are made using 10 different normalised and enriched cDNA libraries in each.
**Preparation of expression cassettes**
1. inoculate 5 ml of LB-medium (Sigma) containing 100 µg/L ampicillin with library inoculum corresponding to a 10+ fold representation of library. Grow overnight.
2. make plasmid miniprep from 1.5 ml of culture (E.g. Qiaprep spin miniprep kit)
3. digest plasmid w. Srf 1
4. dephosphorylate fragments and heat inactivate phosphatase( 20 min, 80°C)
5. digest w. Asc1
6. run 1/10 of reaction in 1 % agarose gel to estimate amount of fragment
**Preparation of pYAC4-Asc arms**
1. inoculate 150 ml of LB medium (Sigma) with a single colony of DH5α containing pYAC4-AscI
2. grow to OD₆₀₀ ∼ 1, harvest cells and make plasmid preparation
3. digest 100µg pYAC4-Ascl w. BamH1 and Asc1
4. dephosphorylate fragments and heat inactivate phosphatase( 20 min, 80°C)
5. purify fragments(e.g. Qiaquick Gel Extraction Kit)
6. run 1 % agarose gel to estimate amount of fragment
   Preparation of EVACs

1, mix expression cassette fragments with YAC-arms so that cassette/arm ration is -1000/1
2. if needed concentrate mixture (use e.g. Microcon YM30) so fragment concentration > 75 ng/µL of reaction
3. add 1 U T4 DNA ligase, incubate 16C, 1-3 h . Stop reaction by adding 1 µL of 500 mM EDTA
4. run pulsed field gel (CHEF III, 1% LMP agarose, ½ strength TBE, angle 120, temperature 12 C, voltage 5.6V/cm, switch time ramping 5 - 25 s, run time 30 h) Load sample in 2 lanes.
5. Stain part of the gel that contains molecular weight markers
6. cut sample lanes corresponding to MW. 100 - 500 kb
7. agarose gel in high NaCL agarase buffer. 1 u agarase / 100 mg gel
8. concentrate preparation to < 20 µL
9. transform suitable yeast strain w. preparation using electroporation: 100 ml of YPD is inoculated with one yeast colony and grown to OD₆₀₀ ≅ 1.3 to 1.5.
   The culture is harvested by centrifuging at 4000 x g and 4°C. The cells are re-suspended in 16 ml sterile H₂O. Add 2 ml 10 x TE buffer, pH 7.5 and swirl to mix. Add 2 ml 10 x lithium acetate solution (1 M, pH 7.5) and swirI to mix. Shake gently 45 min at 30°C. Add 1.0 ml 0.5 M DTE while swirling. Shake gently 15 min at 30°C. The yeast suspension is diluted to 100 ml with sterile water. The cells are washed and concentrated by centrifuging at 4000 x g, resuspending the pellet in 50 ml ice-cold sterile water, centrifuging at 4000 x g, resuspending the pellet in 5 ml ice-cold sterile water, centrifuging at 4000 x g and resuspending the pellet in 0.1 ml ice-cold sterile 1 M sorbitol. The electroporation was done using a *Bio-Rad Gene Pulser*. In a sterile 1.5-ml microcentrifuge tube 40 µl concentrated yeast cells were mixed with 5 µl 1:10 diluted EVAC preparation. The yeast-DNA mix is transferred to an ice-cold 0.2-cm-gap disposable electroporation cuvette and pulsed at 1.5 kV, 25 µF, 200 Ω. 1 ml ice-cold 1 M sorbitol is added to the cuvette to recover the yeast. Aliquots are spread on selective plates containing 1 M sorbitol. Incubate at 30°C until colonies appear.
- STEP 6: The EVAC containing cell libraries produced in step 5) are pooled into one screening population.
- STEP 7: The screening population is divided into two equal portions. One of the portions is screened for anti-oxidant properties (step 8) and the other for differential colour production (step 9).

- STEP 8: Anti-oxidant screen:
a. The screening population is amplified ten times and divided in 10 portions.
b. The sub populations are grown in liquid culture under selective conditions for the artificial chromosomes to an OD₆₀₀ of 0.6 - 1.0.
c. The heterologous genes are induced/de-repressed by re-suspending the cells in a medium lacking methionine and with 200 µM Cu₂SO₄ and the cells are incubated under inducing conditions for 24 hours prior to screening.
d. Each sub population is exposed to 1 out of a range of 5 concentrations of Methylene blue. Immediately after exposure to Methylene blue, the cells are irradiated with a 200 W halogen lamp for 2 hours.
e. Aliquots of each population re plated in different dilutions and remaiing population stored,
f. Survival rates are determined after 48 hours. The surviving cell population exposed to the highest concentration of Methylene blue, where cells statistically representing 10% of the parent cell lines survived, is selected.
- STEP 9: Differential colour production screen:
a. The screening population is grown on plates under selective conditions for the artificial chromosomes to an OD₆₀₀ of 0.6 - 1.0.
b. The heterologous genes are induced/de-repressed by re-suspending the cells in a medium lacking methionine and with 200 µM Cu₂SO₄ for 24 hours prior to screening.
c. Coloured cells are selected.
d. The cells representing (by statistics) the 10% strongest colour expressing cell lines are selected.
   Remixing of genetic diversity selected in steps 8 and 9 is done by excising the expression cassettes, mixing them and religating into new EVACs. This process is described in steps 10 to 13:
- STEP 10: Each of the populations selected in steps 8 and 9 is amplified and equal amounts of each amplified population are pooled.
- STEP 11: Total DNA is isolated following standard procedures.
- STEP 12: The total DNA is digested with Ascl and DNA fragments of the appropriate size (2-10kB) are isolated.
- STEP 13: New EVACs containing the purified DNA fragments and 10% of cassettes (w/w) that have not been used to assemble EVACs in any previous synthesis are synthesised essentially as described in step 5.
- STEP 14: Steps 7 to 13 are repeated 5 times always taking forward the best 10% of cell lines from each screen.
- STEP 15: The new cell population resulting from the completion of the fifth cycle is not divided and is grown in liquid culture under selective conditions for the artificial chromosomes to an OD₆₀₀ of 0.6 - 1.0. The heterologous genes are induced/de-repressed by re-suspending the cells in a medium lacking methionine and with 200 µM Cu₂SO₄
- STEP 16: The combined population is now screened for colour and anti-oxidant activity simultaneously. The concentration of Methylene blue used is the highest concentration where, after 2 hours of exposure to the methylene blue, the number of surviving coloured cells represents 5% of the cell lines in the original population. Cells that survive this concentration of MB and are coloured are selected.
- STEP 17: The selected population is amplified and new EVACs are produced as described in steps 11 to 13.
- STEP 18: Steps 15 to 17 are repeated 30 times always taking forward cells representing the best 5% of cell lines.

- STEP 19: Cell lines that can survive a 10X higher concentration of Methylene blue than the original population and have clearly visible bright yellow to red colours are taken out of the evolution process. The genes responsible for these activities are subcloned and characterised by DNA sequencing.
- STEP 20: Cells with the characteristics described in step 19 and that have significantly different genotypes are analysed using standard natural product chemistry in order to identify the compound(s) responsible for the phenotype.

### PRIORITISED SPECIES AND TAXONOMIC GROUPS

Sourced species are divided into:
- Species that produce carotenoids: plants, algae, some fungii and photosynthetic bacteria
- Species that modify carotenoids to produce other carotenoids: some animals
- Specific genes

### Species that produce carotenoids:

Plants: Actinidia deliciosa (Kiwi); Arabidopsis thaliana; *Brassica rapa, Tagetes erecta* (Marigold flowers), *Olea europaea* (olive), *Lactuca sativa var. romaine* (romaine lettuce), *Quercus robur* (oak), *Pinus pinaster* (maritime pine), *Capsicum annuum* (Pepper), *Bixa orellana, Sarcina lutea, Viola tricolor, Lonicera japonica, Delonix regia, Zea mays* (maize), *Eschscholzia californica, Carica papaya* (papaya), *Daucus carota* (carrot), *Lycopersicon esculentum* (tomato), *Crocus sativus* (saffron), *Verbascum phlomoides, Physalis alkekengi, Gentiana spp., Nicotiana tabacum, Pittosporum tobira*
Algae: *Rhizophora mangle* (red mangrove), *Haematococcus pluvialis* (Green Algae), *Enteromorpha linza* (Patagonian macroalga), *Ulva lactuca* (Sea lettuce), *Caulerpa mexicana, Gigartina sp, Polysiphonia sp, Porphyra sp, Macrocystis pyrifera* (Giant kelp), *Sargassun sp., Nanochlorum eucaryotum*, Dunaliella bardawil, Scenedesmus *obliquus, Oscillatoria rubescens, Phormidium luridum, Arthrospira spp., Astasia ocellata, Fucus vesiculosus, Bathycoccus prasinos, Micromonas pusilla, Botryococcus braunii,*
Fungi: *Xanthophyllomyces dendrorhous, Neurospora crassa, Cantharellus cibarius, Phycomyces blakesleeanus, Puccinia graminis, Epicoccum spp., Lycogola epidendron*
Bacteria: Roseiflexus castenholzii, Streptococcus faecium, *Rhodopseudomonas acidophila*, Erwinia herbicola, Agrobacterium aurantiacum, *Halorhodospira abdelmalekii Halorhodospira halochloris, Anabaena PCC 7120,* Chlorobium tepidum, Cholroflexus aurantiacus. Thermus thermophilus, flexibacter spp., Rhodobacter capsulatus, *Staphylococcus aureus, Deinococcus radiodurans, Meiothermus ruber, Chloroflexus aurantiacus*

### Species that modify carotenoids:

Birds: *Carduelis tristis, Cardinalis cardinalis,* flamingo
Fishes*: Carassius auratus* (goldfish), *Micropterus salmoides* (black bass), *Paracheirodon axelrodi* (cardinal tetra), *Amphiprion ocellaris* (common clown), *Zebrasoma flavescens* (Yellow tang), *Synchiropus splendidus* (mandarinfish), *Lactoria cornuta* (long-horned cowfish)
Invertebrates: *Cucumaria japonica* (sea cucumber), *lanthella basta* (sponge), *Clibanarius erythropus* (hermit crab), *Daphnia magna, Homarus americanus* (Lobster), *Paralithodes brevipes* (King Crab), *Fusinus perplexus* (seashell), *Halichondria okadai* (sponge), *Suberites massa* (sponge), *Pentacta australis* (sea cucumber), *Pseudocentrotus depressus,* (sea urchin), *Ophiuroidda spp*. (brittle star), *Papilio xuthus* (butterfly), *Mytilus coruscus* (japanese sea mussel), *Crassostrea gigas* (oyster), *Glossodoris spp*., (sea slug), *Fromia elegans* (star fish), *Actinia equina* (strawberry beadlet anemone), *Anemonia viridis* (anemone), *Hippolysmata graghami*, (shrimp), *Lysmata debelius* (shrimp), *Halocynthia papillosa* (sea squirt), *Crossaster papposus* (starfish)

### Specific Carotenoid genes:

ggps, psy, pds, zds, Icy-b, Icy-e, bhy, zep *(Gentiana sp.)*, idi, crtC, crtF *(Rhodobacter capsulatus)*, crtE, crtB, crtl, crtY, crtZ *(Erwinia uredovora)*, zds *(Nostoc anabaena)*, pds *(Synechococcus PCC7942)*, crtE, crtB, crtI, crtY, crtZ *(Erwinia herbicola)*, crtM, crtN *(Staphylococcus aureus)*, crtl, crtYb *(Xanthophyllomyces dendrorhous)*, ccs, crtL *(Capsicum annuum)*, crtL, bchy *(Nicotiana tabacum)*, Icy-b, Icy-e *(Prochlorococcus sp.)*, idi *(Saccharomyces cerevisiae)*, crtl, crtYe, crtYf, crtEb *(Corynebacterium sp.)*, psy-1 *(Lycopersicon esculentum)*, al1 *(Neurospora crassa)*

### Example 8: Evolution of Omega fatty acid like compounds

### UTILITY

Unsaturated fatty acids are important components for normal cellular function, are involved in cell membrane fluidity and serve as precursors to eicosanoids, including prostaglandins and leukotrines. In mammals, these eicosanoids are involved in inflammatory responses, regulation of blood pressure, and reproductive function

### SCREENING & SELECTION STRATEGY

- Cell membrane fluidity (Flow cytometry screen)
- Tolerance to cold (growth assay)

### PROCEDURE.

The same procedure as that described in Example 7 is performed, except that the following changes are made to the following numbered steps
- Step 7: The screening population is divided into 2 equal portions. One of the portions is screened for tolerance to cold (step 8) and the other for cell membrane fluidity (step 9)
- Step 8: Tolerance to cold:
a. The screening population is amplified ten times and divided in 10 portions.
b. The sub populations are grown in liquid culture under selective conditions for the artificial chromosomes to an OD₆₀₀ of 0.6 - 1.0.
c. The heterologous genes are induced/de-repressed by re-suspending the cells in a medium lacking methionine and with 200 µM Cu₂SO₄ and the cells are grown under inducting conditions for 24 hours prior to screening.
d. Each sub population is plated and grown at temperatures ranging from 10 to 30°C. Cold tolerance is determined when the first colonies appear on a given plate (at a given temperature). The cell population growing at the lowest temperature after a predetermined period of time where cells statistically representing 10% of the original cell lines grow within the same defined period of time, is selected.
- Step 9: Cell membrane fluidity screen:
a. The screening population is grown in liquid culture under selective conditions for the artificial chromosomes to an OD₆₀₀ of 0.6 - 1.0.
b. The heterologous genes are induced/de-repressed by re-suspending the cells in a medium lacking methionine and with 200 µM Cu₂SO₄ The cells are grown under inducting conditions for 20 hours with 1 tenth of the library being analysed by flow cytometry after every 2 hours.
c. For each of the sub populations flow cytometry is used to isolate the 10 % of cell lines with the most fluid cell membranes but which are still growing normally. This is done essentially as described in Benderitter M. et al, Cytometry, 2000, 39(2), 151-7
- Step 10: Each of the populations selected in steps 8) and 9) is amplified. For each of the selected populations, steps 11-13 are repeated separately.
- Step 16: The combined population is now screened for ethanol resistance and increased membrane fluidity. The concentration of ethanol used is the highest concentration where, after overnight exposure to ethanol, the number of surviving cells whose membrane fluidity exceeds the average fluidity by more than 2 x the standard deviation at 30 °C represents 5% of the cell lines in the original population.
- Step 19: Cells that can survive a 1.5 x higher concentration of Ethanol than the original population and have a cell membrane fluidity that exceeds the average fluidity of the original population by more than 5 x the standard deviation at 30 °C are taken out of the evolution process. The genes responsible for these activities are characterised by subcloning and DNA sequencing.

### PRIORITISED SPECIES AND TAXONOMIC GROUPS

- Plants (in particular seeds)
- Animals (in particular adipose tissues)
- Fish
- Random group of taxonomically diverse eukaryotic species

### Evolution of specific structural classes via longer pathways

### Example 9: Evolution of Retinoid like compounds

### UTILITY:

Retinoids are derivatives of vitamin A and are modulators of cellular proliferation as well as effectors of morphogenic changes. Activity of retinoids as antineoplastic agents has been demonstrated in several *in vivo* experimental carcinogen models (mainly for skin, respiratory tract, urinary bladder, breast, digestive tract) Cellular responses to retinoids are generally mediated by two families of nuclear receptors (RARs and RXRs) that belong to the steroid-thyroid hormone (or nuclear) receptor superfamily and behave as ligand-activated transcription factors that bind as dimers to the *cis*-acting response elements of target genes.

Different retinoic acid receptor isotypes display a characteristic pattern of tissue distribution, RARα being the most ubiquitously distributed. RARβ plays an important role in lung development and has been proposed to have a tumour suppressor function in lung.

### SCREENING & SELECTION STRATEGY

- To obtain carotenoid like compounds screens for colour production and anti-oxidant protection are used (see example 7).
- To obtain retinoid like compounds an activation assay of retinoic acid receptor, RARβ is used. This assay employs a reporter system. Reporter construct is initially placed intra-cellularly and later on extra-cellularly.

### PROCEDURE

The same procedure as that described in Example 7 is performed, except that the following changes are made to the following numbered steps.
- Step 5: EVAC containing cell populations are made using 10 different normalised and enriched cDNA libraries in each. EVACs are transformed into the cell population evolved in example 7.
1. Grow the carotenoid producing cell population to mid log, 2 x 10⁶ to 2 x 10⁷ cells/ml in liquid medium, at 30°C and with aeration, under selective conditions for the EVACs.
2. Spin to pellet cells at 400 x g for 5 minutes; discard supernatant.
3. Resuspend cells in a total of 9 ml TE, pH 7.5. Spin to pellet cells and discard supernatant.
4. Gently resuspend cells in 5 ml 0.1 M Lithium/Cesium Acetate solution, pH 7.5.
5. Incubate at 30°C for 1 hour with gentle shaking.
6. Spin at 400 x g for 5 minutes to pellet cells and discard supernatant.
7. Gently resuspend in 1 ml TE, pH 7.5. Cells are now ready for transformation.
8. In a 1.5 ml tube combine:
   - 100 µl yeast cells
   - 5 µl carrier DNA (10 mg/ml)
   - 5 µl Histamine Solution
   - 5/100 of an EVAC preparation in a 10 µl volume (max). (One EVAC preparation is made of 100 µg of entry vector library plasmid mixture)
9. Gently mix and incubate at room temperature for 30 minutes.
10. In a separate tube, combine 0.8 ml 50% (w/v) PEG 4000 and 0.1 ml TE and 0.1 ml of 1 M LiAc for each transformation reaction. Add 1 ml of this PEG/TE/LiAc mix to each transformation reaction. Mix cells into solution with gentle pipetting.
11. Incubate at 30°C for 1 hour.
12. Heat shock at 42°C for 15 minutes; cool to 30°C.
13. Pellet cells in a microcentrifuge at high speed for 5 seconds and remove supernatant.
14. Resuspend in 200 µl of rich media and plate in appropriate selective media
15. Incubate at 30°C for 48-120 hours until transformed colonies appear.
- Step 7: The screening population is not divided
- Step 8: Retinoic Acid Receptor Activation:
a. The screening population is amplified ten times.
b. The EVAC containing cell population is mated with a reporter strain containing a reporter construct in an yeast vector and a yeast expression plasmid containing the cDNA for the human retinoic acid receptor (RARβ) and cultured under selection for the haploid cells, the reporter system and the EVACs. The reporter gene used is β-galactosidase. The reporter strain is constructed essentially as described in Salerno et al. 1996, Nucleic Acids Res. 24(4), 566-72.
c. The heterologous genes are induced/de-repressed by re-suspending the cells in a medium lacking methionine and with 200 µM Cu₂SO₄. The cells are grown under induction conditions for 18 hours. Growth and β-galactosidase activity are assayed in 96 well microtiter plates essentially as described in Coldham et al., 1997, Environ. Health Perspect., 105(7), 734-42.
d. Cells with the 10% highest β-galactosidase activity are selected.
- Step 9: There is no step 9.

Remixing of genetic diversity selected in step 8 is done by physical re-isolation and re-transformation of EVACs.
- Step 10: The population selected in step 8 is grown in 5 ml of YPD to an OD₆₀₀ > 1.0
- Step 11: Two 100 µl plugs of total DNA are produced as described in BioRad's "CHEF genomic DNA plug kits" manual, Procure n.2
- Step 12: EVACs are purified and isolated:
a. Plugs are cut and loaded into 3 slots of a pulsed field gel
b. Run PFGE
   i. For EVACs < 1000 kb : Chef **III,** 1% Agarose, 1/2 strength TBE, 6V/cm, 14°C, 120° angle, 50 - 90 sec. Switch time, 22 h runtime.
   ii. For EVACs > 1000 kb. Chef III, 1% Agarose, 1/2 strength TBE, 6V/cm, 14°C, 120° angle, 60-120 sec. Switch time, 24 h runtime
c. stain one lane to identify position of EVACs
d. cut corresponding part of the two non-stained lanes and digest the agarose by agarase treatment following standard procedures e.g. Pulsed Field Gel Electrophoresis. A practical approach.(Ed. A.P. Monaco) Oxford University Press 1995.
e. Concentrate agarased preparation to 100 µL by ultrafiltration.(e.g. Microcon YM-30, Millipore)
f. add 400 TE to retentate and repeat concentration step. Repeat and concentrate to 25 µL
- Step 13: EVACs are transformed into yeast as before.
- Step 16: Step 8 is essentially repeated but the cell population is co-cultured together with a reporter strain under selection for both strains. Thus compounds that activate RARβ now have to be reasonably hydrophilic in order to cross the cell membranes. The producer strain is also transformed with P450s as described in example 1. This procedure allows the simultaneous screen for activation of RARβ, compound permeability and P450 metabolism.
- Step 19: Commencing with the tenth cycle, in step 16, cells that show a similar activation of RARβ as 0.1 µM of retinoic acid are taken out of the evolution process. The genes responsible for these activities are characterised by subcloning and DNA sequencing

### PRIORITISED SPECIES AND TAXONOMIC GROUPS:

- Species that metabolise carotenoids to retinoids: mammals (specially liver and retina tissues), fishes (liver), insects and other animals.

### Example 10: Evolution of Taxol like compounds

### UTILITY.

Taxol is a higly effective anticancer drug used widely in the treatment of various carcinomas, melanomas and sarcomas. The unique mode of action of this drug, as well as its outstanding potency makes Taxol one of the most efficacious anticancer agents in current use. Taxol promotes assembly of microtubules and prevents depolymerization. This induces bundles of stable microtubules and blocks cell cycle mitosis.

### SCREENING & SELECTION STRATEGY

- Stabilisation of microtubule assembly. The microtubule assembly is scored by FACS using gel encapsulation and measuring light scatter.
- Growth inhibition of murine fibroblast cells. The growth inhibition assay is done using gel microdroplets and flow cytometry.

### PROCEDURE

Taxol's biosynthesis pathway is thought to involve between 12 and 20 enzymatic steps starting from geranylgeranyl diphosphate. The first committed step in the formation of taxol involves the cyclisation of geranylgeranyl diphosphate. After a few more enzymatic steps, an intermediate called Baccatin-III is synthesised. This intermediate is available from renewable natural sources and can thus be used as a precursor to feed a cell population in order to evolve a population of cells able to produce Taxol like compounds. Once this has been achieved, the genes identified in the process should be locked. The number of steps from a yeast metabolite to Baccatin-III can be reduced by incorporating in the Taxol producing population the first genes of the pathway which are known: crtE (phytoene synthase) which can modify a yeast metabolite, IPP, to produce geranylgeranyl diphosphate, taxadiene synthase and taxadiene 5α-hydroxylase. After a second module of evolution where no precursor feeding is used, a population of cells able to produce Taxol like compounds will have been established.

For each modular evolution the procedure as that described in Example 9 is performed, except that the following changes are made to the following numbered steps.
- Step 7: The screening population is divided into 2 equal portions. One of the portions is screened for stabilisation of microtubule assembly (step 8) and the other growth inhibition of murin fibroblast cells (step 9)
- Step 8: Stabilisation of microtubule assembly:
a. The heterologous genes are induced/de-repressed by re-suspending the cells in a medium lacking methionine and with 200 µM Cu₂SO₄ and the cells are grown under inducting conditions for 24 hours prior to screening.
b. The EVAC containing cells are co-encapsulated with 5 mg tubulin /ml.
c. Gel capsules are suspended in polymerisation buffer and incubated at 37 C for 1 h. A fraction of the population is passed through the flow cytometer to determine the average light scatter.
d. In parallel a control population of microcapsules are incubated in the same buffer lacking GTP. The population is passed through the flow cytometer to establish light scatter of microcapsules containing depolymerised tubulin.
e. The suspension is cooled to 5 C for 30 min. and microcapsules maintaining a light scatter closest to the light scatter on the capsules containing polymerised tubulin is recovered and processed through step 10.
f. The 10% of the cell population with the highest light scatter is selected.
- Step 9: Growth inhibition of murine fibroblast cells:
a. The screening population is grown in liquid culture under selective conditions for the artificial chromosomes to an OD₆₀₀ of 0.6 - 1.0.
*b*. The heterologous genes are induced/de-repressed by re-suspending the cells in a medium lacking methionine and with 200 µM Cu₂SO_{4.} a. An EVAC containing cell population is grown under induction conditions.
c. Murine fibroblast cells are co-encapsulated with the EVAC containing cells in gel microdroplets in fibroblast growth media.
d. Incubation is conducted for 24 hours, after which the droplets are screened by flow cytometry and the level of cell proliferation of the murine fibroblasts in each droplet is measured.
e. Droplets with the lowest cell proliferation are selected such that 10% of host cell lines entering the screen are selected.
- Step 10: Each of the populations selected in steps 8) and 9) is amplified. For each of the selected populations, steps 11-13 are repeated separately.
- Step 16: The entire population is now screened simultaneously for stabilisation of microtubule assembly and for growth inhibition of murine fibroblast cells using a double gel encapsulation system.

- Step 19: Cells that have an activity similar to that produced by a 10 nM concentration of Taxol are taken out of the evolution process. The genes responsible for these activities are characterised by subcloning and DNA sequencing.

### PRIORITISED SPECIES AND TAXONOMIC GROUPS

Sourced species are divided into:
- Species that produce Taxol and other Taxol like compounds,
- Organisms phylogeneticly related to the Taxol producers
- Organisms known to have relevant pharmacological activities, i.e., anticancer
- Specific genes coding for enzymes known to be involved in the Taxol biosynthesis

Species that produce Taxol:
Plants: Taxus brevifolia, Taxus cuspidata, Taxus yunnanensis, Taxus canadensis, Taxus baccata, Taxus wallichiana, Taxus mairei, Taxus chinensis, Taxus media;
Fungii: Taxomyces andreanae, Taxomyces wallichiana, Taxomyces baccata, Taxomyces canadensis

Organisms phylogeneticaly related to the Taxol producers:
Same family: *Taxus globosa, Taxus biternata, Taxus caespitosa, Taxus recurvata, Taxus umbraculifera, Taxus concorta, Taxus sumatrana, Torreya grandis, Torreya nucifera;* Cupressaceae Family : *Callitris arborea, Chamaecyparis lawsoniana, Cupressus arizonica, Juniperus chinensis, Juniperus recurvus, Tetraclinis articulata, Thuja occidentalis, Widdringtonia cupressoides;* Podocarpaceae Family : *Podocarpus ferrugineus*; Araucariaceae Family: *Agathis alba, Araucaria imbricata, Agathis australis,* Pinaceae Family: *Abies balsamea, Abies webbiana, Cedrus deodora, Larix europaea, Picea rubens, Pinus australis, Pinus pinaster, Pinus wallichiana, Pseudotsuga taxifolia, Tsuga canadensis*, Cephalotaxaceae family: C. fortunei, C. Hauringtonic, Sciadopityaceae family: Sciadopitys verticillata

Organisms reported to have antineoplastic activity:
PLANTS:*Chelidonium majus* (Celandine-plant), *Rheum officinale* (Chinese Rhubarb, Root), *Rheum rhabarbarum* (Rhubarb, Root), *Allium cepa* (Onion, bulb), *Aloe vera* (Aloe, plant), *Arachis hypogaea* (Groundnut, Seed), *Brassica oleracea var. capitata* (Cabbage, Leaf), *Cassia tora* (Sickle Senna, Seed), *Coptis chinensis* (Chinese Goldthread, Rhizome), *Coptis japonica* (Huang-Lia, Rhizome), *Coptis spp* (Generic Goldthread, Rhizome), *Corydalis spp* (Fumewort, Plant), *Eschscholzia califomica* (California Poppy, Shoot), *Glaucium flavum* (Horned Poppy, Root), *Papaver somniferum* (Opium Poppy, Plant), *Polygonum multiflorum* (Chinese Cornbind, Root), *Rheum palmatum* (Chinese Rhubarb, Root), *Rumex hymenosepalus* (Canaigre, Root), *Sanguinaria canadensis* (Bloodroot, Root), *Senna alata* (Ringworm Bush, Plant), *Adonis vemalis* (Spring Adonis, Plant),
ANIMALS, Sponges: *Corticium sp, Zyzzya cf. fuliginosa, Chondropsis sp, Diacamus erythraenus;* jellyfish: *Carybdea rastonii, Chrysaora quinquecirrha;* anemones: *Actinia equina, Anemonia viridis*, Insects: *Papilio polyxenes, Drosophila melanogaster, Rhodnius prolixus, Apis mellifera, Lacanobia oleracea;* Spiders: *Tarantula keratouveitis, Loxosceles deserta, Loxosceles reclusa;* Crabs: Clibanarius longitarsus, Tachypleus tridentatus, Uca pugilat, Worms: *Schistosoma mansoni;* Snails: *Lippia sidoides, Lymnaea stagnalis, Stylocheilus longicauda, Biomphalaria glabrata;* Snakes: *Bothrops jararaca, Crotalus durissus, Vipera aspis, Sistrurus Malarius Barbouri;* Sea urchin: *Toxopneustes pileolus*; Starfish: *Acalycigorgia inermis, Asterina pectinifera, Fromia monilis*,

### Evolution of cells towards specific drug targets

### Example 11: Evolution of HIV protease inhibitors

### UTILITY.

Acquired immunodeficiency syndrome (AIDS) is a fatal pathogenic disease caused by the HIV virus. AIDS is prevalent in almost every country on the globe, and it is estimated that 36 million people are infected worldwide. HIV Protease is an enzyme expressed by the HIV Virus. Molecules that inhibit HIV Protease have utility in the treatment of HIV infections. Desirable attributes of such molecules include activity against HIV, specificity for HIV protease, ability to cross cell membranes in order to reach the virus and others.

### SCREENING & SELECTION STRATEGY

Assays that measure the activity of HIV Protease can be constructed by labelling the protein substrate of the protease with a fluorescent dye such that in the undigested substrate the fluorescence is quenched but in the enzymatically digested substrate the fluorescence is unquenched. Procedures for constructing such screens are given in "Activity and dimerization of human immunodeficiency virus protease as a function of solvent composition and enzyme concentration." Jordan SP, Zugay J, Darke PL, Kuo LC. J Biol Chem 267, 20028-20032 (1992).

### PROCEDURE.

The same procedure as that described in Example 7 is performed, except that the following changes are made to the following numbered steps
- Step 7: The screening population is not divided.
- Step 8: The EVAC containing cell population is grown for 12 hours under conditions that induce expression. A reporter assay constructed as above is incorporated proximal to the host cell in a microdroplet. The microdroplet is incubated in screening medium. At 2 hour intervals 10% of the microdroplets are run through a flow cytometer and screened for level of fluorescence. In each 2 hour batch those droplets with the lowest level of fluorescence are selected such that statistically 10% of host cell lines are represented in the selected cells. Selected cells are immediately placed into media that halts induction of the heterologous genes. The selected cells from each batch are pooled
- Step 9: There is no step 9
- Step 10: The population selected in step 8 is amplified
- Step 16: Cells are now screened simultaneously for P450 metabolism and HIV protease inhibition. The selection criteria are set such that 5% of the cell lines entering the screen are selected
- Step 19: Cells that inhibit HIV protease to the same extent as Indinavir does at a 25nM concentration are taken out of the evolution process. The genes responsible for these activities are characterised by subcloning and DNA sequencing

### PRIORITISED SPECIES AND TAXONOMIC GROUPS

- Random group of taxonomically diverse eukaryotic species

### Example 12: Evolution of DNA Topoisomerase II Poisons

### UTILITY.

Topoisomerase II is an essential enzyme in cell division - it regulates the topology of DNA and in particular performs a process whereby it cuts open double stranded DNA, passes another strand through the break, and then reseals the break. Compounds (such as doxorubicin and etoposide) that cause the toposiomerase II enzyme to generate but not seal these DNA double strand breaks have proven utility as anti-cancer agents. New compounds with such activity but different pharmacological properties have utility as compounds for the treatment of cancer, and indeed other proliferative diseases.

### SCREENING & SELECTION STRATEGY

Topoisomerase II poisons (such as doxorubicin and etoposide) are preferentially toxic to cells that have high levels of topoisomerase II - their toxic effect is achieved by causing the topoisomerase enzyme to generate double strand breaks in DNA.

Their effects are antagonised by compounds (such as chloroquine and dexrazoxane) that act on the enzyme without causing the double strand breaks. These properties can be used to construct a process for selecting and evolving cells that produce compounds that act as topoisomerase poisons

Yeast host cells are described in the scientific literature that conditionally express functional human topoisomerase II, Wasserman R. et al, Cancer Research, 1993, 53, 3591.

### PROCEDURE

The same procedure as that described in Example 7 is performed, except that the following changes are made to the following numbered steps
- Step 5.: EVAC containing cell populations are made using 10 different normalised and enriched cDNA libraries.
- Step 7: The screening population is not divided.
- Step 8: Topoisomerase Inhibitory activity:
a. The population is grown in liquid culture under selective conditions for the artificial chromosomes to an OD₆₀₀ of 0.6 - 1.0.
b. The heterologous genes are induced/de-repressed by re-suspending the cells in a medium lacking methionine and with 200 µM Cu₂SO₄ and at 35°C. The cells are grown under induction conditions for 12 hours. Every 3 hours 25 % of the cell population is screened for DNA damage by flow cytometry. The 25% of cells in each titre showing least DNA damage are discarded and the remainder immediately placed in non-inducing media that contains dexrazoxane at 100 micromolar concentration
c. Those cells that survive and are selected from step 8b are resuspended and grown under induction conditions for a further 12 hours, but this time with dexrazoxane at 100 micromolar concentration throughout the 12 hour period At the end of the period the cells are screened for DNA damage by flow cytometry and cells representing the 25% of cell lines showing least DNA damage are selected.
- Step 9: Inhibition of cancer cell growth:
a. The population is grown in liquid culture under selective conditions for the artificial chromosomes to an OD₆₀₀ of 0.6 - 1.0.
b. The heterologous genes are induced/de-repressed by re-suspending the cells in a medium lacking methionine and with 200 µM Cu₂SO₄ and at 35°C. The cells are plated and grown under induction conditions for 48hours. A cancer cell line is overlayed on the induced cell population. Yeast cells present in zones clear of cancer cells are selected.
- Step 10: The population selected in steps 8 and 9 are amplified
- Step 16: The cell population is now screened simultaneously for DNA topoisomerase II inhibition and for growth inhibition of cancer cells using a double gel encapsulation system.
- Step 19: Commencing with the tenth cycle cells that show DNA damage of an equivalent level to that caused by the addition of 5 micromolar etoposide and that show cancer cell growth inhibition are taken out of the evolution process. The genes responsible for these activities are characterised by subcloning and DNA sequencing

### PRIORITISED SPECIES AND TAXONOMIC GROUPS

- Plants that produce podophyllotoxins
- Species generally known to have anticancer activity

### Example 13: Evolution of p53 Activators

### UTILITY

p53 is a well known tumour suppressor gene that induces apoptosis in otherwise oncogenic cells. Dysfunctional p53 can mean that the apoptosis does not occur and the oncogenic cell proliferates. A large proportion of tumours have dysfunctional p53 protein.

p53 protein acts as a transcription factor. Many forms of dysfunctional p53 have lost the ability to act as a transcription factor. Tumour cells with such a dysfunctional form accumulate the p53 protein but transcription and hence apoptosis does not occur. In such tumour cells, compounds that restore the ability of such dysfunctional p53 to initiate transcription, and hence apoptosis would have utility as anti-cancer agents. Rastinejad F., Science, 1999,286,2507-2510.

### SCREENING & SELECTION STRATEGY

p53 is a transcription factor. A standard intracellular reporter system for p53 comprises a) a genetic construct whereby a gene encoding for GFP or a similar reporter protein is placed under the control of a heterologous p53 inducible promoter, together with b) an inducible genetic construct encoding for a dysfunctional p53. Such a construct can be used to screen for compounds that activate the transcription of the GFP.

### PROCEDURE

The same procedure as that described in Example 7 is performed, except that the following changes are made to the following numbered steps
- Step 5.: EVAC containing cell populations are made using 10 different normalised and enriched cDNA libraries in each. The cell populations are then further transformed according to standard protocols with a p53 reporter system as described above
- Step 7: The screening population is not divided
- Step 8: p53 activation screen:
a. The population is grown in liquid culture under selective conditions for the artificial chromosomes and the p53 reporter system to an OD₆₀₀ of 0.6 - 1.0.
b. The heterologous genes are induced/de-repressed by re-suspending the cells in a medium lacking methionine and with 200 µM Cu₂SO₄ and the cells are grown under induction conditions for 36 hours. Any cells producing GFP are discarded.
c. The dysfunctional p53 is induced and induction maintained for 5 days, alongside induction/de-repression of the EVAC genes. Cells are observed for production of GFP after 1 hour and then at 12 hour intervals throughout the five days. Those cells that produce GFP are selected.
- Step 9: There is no step 9
- Step 10: The populations selected in step 8) is amplified
- Step 16: Each cell is gel encapsulated and allowed to grow to form clonal sub populations. Clonal populations are double gel encapsulated with a tumour cell line and the gel droplets are screened for GFP production and induction of apoptosis. The selection criteria are set such that 5% of the cell lines entering the screen are selected.
- Step 19: Cells that produce GFP within 1 hour of induction and induce apoptosis are taken out of the evolution process. The genes responsible for these activities are characterised by subcloning and DNA sequencing.

### PRIORITISED SPECIES AND TAXONOMIC GROUPS

- Organisms reported to have anticancer properties (see list in example 10)
- Random group of taxonomically diverse eukaryotic species

### Example 14: Evolution of Fumarate Reductase inibitors

### UTILITY

Fumarate reductase reduces fumarate to succinate and is an essential step in anaerobic metabolism for many organisms including pathogens from such genera as Leishmania, Helicobacter, Staphylococcus and Streptococcus.

Compounds that inhibit the activity of fumarate reductase can prevent such parasites or pathogens from completing their life cycle and hence are of utility in controlling such diseases. Because fumarate reductase does not occur in humans, such compounds should not have significant toxicity to humans.

### SCREENING & SELECTION STRATEGY

Fumarate reductase activity is measured by the rate at which it oxidises NADH upon the addition of fumarate. The progress of the enzymatic reaction is measured spectrophotometrically at 340nm. For detailed protocols see Chen et al., Antimicrob. Agents Chemother., 2002, 2023-2029.

### PROCEDURE

The same procedure as that described in Example 7 is performed, except that the following changes are made to the following numbered steps
- Step 7: The screening population is not divided
- Step 8: Fumarate reductase screen:
a. The screening population is amplified ten times
b. The heterologous genes are induced/de-repressed by re-suspending the cells in a medium lacking methionine and with 200 µM Cu₂SO₄ and the cells are grown under inducting conditions for 24 hours prior to screening.
c. Fumarate reductase is co-encapsulated with EVAC containing cells in a gel microdroplet.
d. Microdroplets containing fumarate reductase but not EVAC containing cells were incubated in a liquid media containing 100 micromolar NADH and 1mM fumarate and incubated at 30 C. Aliquotes were analysed by flow cytometry to determine the optimal incubation time.
e. The microdroplet is placed in a liquid media that contains 100 micromolar NADH and 1 mM fumarate and incubated for the optimal period of time.
f. The gel microdroplets are then passed through a flow cytometer and absorption at 340nm measured. The activity of the enzyme is calculated from the level of absorption at 340nm. The cells with the lowest activity of enzyme are selected such that cells representing the 10% of cell lines entering the screen with the greatest inhibition of fumarate reductase are selected
- Step 9: There is no step 9
- Step 10: The populations selected in step 8 is amplified.
- Step 16: Gel droplets are double encapsulated with S. aureus and gel droplets are screened simultaneously for fumarate reductase inhibition and bacterial cell growth inhibition. The selection criteria are set such that 5% of the cell lines entering the screen are selected
- Step 19: Cells that show inhibition of fumarate reductase and S. aureus growth inhibition of a same or greater extent than is achieved by adding licochalcone to the droplets at a concentration of 1 micromolar are taken out of the evolution process. The genes responsible for these activities are characterised by subcloning and DNA sequencing.

### PRIORITISED SPECIES AND TAXONOMIC GROUPS

- Plant roots
- Random group of taxonomically diverse eukaryotic species

### Evolution of function independent of specific targets

### Example 15: Evolution of cytoprotectants

### UTILITY

One of the central problems of cancer chemotherapy is that the anti-cancer agents used kill normal cells as well as cancer cells. The side effects of killing the normal cells can be so severe as to be life-threatening, and frequently mean that treatment of the cancer has to be abandoned. Compounds that protect cells against such anti-cancer agents therefore have utility in reducing the side effects of cancer chemotherapy, hence improving both therapeutic outcome and patient life quality. Existing examples of such compounds include dexrazoxane and chloroquine, both of which protect cells agains the cytotoxic effects of cancer agents such as etoposide. However more such protectants are needed.

### SCREENING & SELECTION STRATEGY

Host cells can be induced screened and selected for survival in the presence of cytotoxic anti-cancer agents such as doxorubicin, taxol, vincristine and cisplatin. Over a series of selection rounds the concentration of cytotoxic agent that a cell must survive to be selected can be increased

### PROCEDURE

The same procedure as that described in Example 7 is performed, except that the following changes are made to the following numbered steps
- Step 7: The screening population is divided into two equal portions. One of the portions is screened against etoposide (step 8) and the other screened against vincristine (step 9)
- Step 8: Etoposide screen:
a. The screening population is amplified ten times and divided in 10 portions.
b. The sub populations are grown in liquid culture under selective conditions for the artificial chromosomes to an OD₆₀₀ of 0.6 - 1.0.
c. The heterologous genes are induced/de-repressed by re-suspending the cells in a medium lacking methionine and with 200 µM Cu₂SO₄ and the cells are grown under induction conditions for 36 hours prior to screening.
d. Each sub population is diluted so as to have on average each cell line represented 3 times.
e. Each sub population is exposed to 1 out of a range of 10 concentrations of etoposide. Survival rates are determined after 2 hours. The surviving cell population from the highest concentration of etoposide where cells statistically representing 10% of the cell lines survived is selected.
- Step 9: Vincristine screen:
a. The screening population is amplified ten times and divided in 10 portions.
b. The sub populations are grown in liquid culture under selective conditions for the artificial chromosomes to an OD₆₀₀ of 0.6 - 1.0.
c. The heterologous genes are induced/de-repressed by re-suspending the cells in a medium lacking methionine and with 200 µM Cu₂SO₄ and the cells are grown under induction conditions for 36 hours prior to screening.
d. Each sub population is exposed to 1 out of a range of 10 concentrations of vincristine. Survival rates are determined after 2 hours. The
surviving cell population from the highest concentration of vincristine where cells statistically representing 10% of the cell lines survived is selected.
- Step 10: Each of the populations selected in steps 8) and 9) is amplified and equal amounts of each amplified population are pooled
- Step 16: The entire population is now screened for both etoposide and vincristine resistance. The selection criteria are set such that 5% of the cell lines entering the screen are selected
- Step 19: Cells that, compared to the original population, show the ability to resist a 10-fold higher concentration of etoposide, or a 10-fold higher concentration of vincristine, or a combined 5-fold higher dose of both together, are taken out of the evolution process. The genes responsible for these activities are characterised by subcloning and DNA sequencing.

### PRIORITISED SPECIES AND TAXONOMIC GROUPS

- Plant species of the genera Vinca
- Organisms known to produce Taxol (see list in example 10)

### Example 16: Evolution of antibacterials

### UTILITY

The widespread emergence of resistance has significantly limited the efficacy of classical antibiotic therapy for bacterial disease. Fuelled largely by the excessive and often unnecessary use of antibiotics in humans and animals, antibiotic resistance has resulted in increased patient morbidity, mortality and overall cost of health care.

There is a strong medical need for new therapeutics to treat emerging antibiotic-resistant infections. A premium is placed upon inhibitors that function by a novel or at least different mechanism than currently approved antibiotics, as these would be expected to circumvent current bacterial resistance mechanisms.

### SCREENING & SELECTION STRATEGY

Screening can be done using gel microdroplets and flow cytometry or an overlay system. Use of a multiple drug-resistant strain in the primary screen will a *priori* select for hits that have activity against a multi-drug resistant strain.

The approach set out in this example can be applied to a range of micro-organisms other than the one described here. Mammalian cells can be used in one or more selection rounds and used to select for host cells that are not producing compounds with mammalian cell toxicity.

### PROCEDURE

The same procedure as that described in Example 7 is performed, except that the following changes are made to the following numbered steps
- Step 7: The screening population is not divided.
- Step 8: Antibacterial screen:
*a*. An EVAC containing cell population is grown under induction conditions.
b. *Staphylococcus aureus* is co-encapsulated with the EVAC containing cells in gel microdroplets.
c. Incubation is conducted for 24 hours, after which the droplets are screened by flow cytometry and the level of cell proliferation by the bacteria in each droplet is measured.
d. Droplets with the lowest cell proliferation are selected such that 10% of host cell lines entering the screen are selected.
- Step 9: The cell population is transformed with CYP 3A4, CYP 2C9 and CYP 2D6.
- Step 10: The population selected in step 8 is amplified.
- Step 16: The population is now screened for inhibition of bacterial growth and inhibition of CYP 3A4, CYP 2C9 and CYP 2D6. The selection criteria are set such that 5% of the cell lines entering the screen are selected
- Step 19: Cells that have shown an antibacterial activity equal to that achieved with vancomycin at a concentration of 1 µg/ml and that do not inhibit the P450s are taken out of the evolution process. The genes responsible for these activities are characterised by subcloning and DNA sequencing

### PRIORITISED SPECIES AND TAXONOMIC GROUPS

- Fungi
- Random group of taxonomically diverse eukaryotic species

### Other examples:

### EXAMPLE 17: RARE RESTRICTION ENZYMES WITH RECOGNITION SEQUENCE AND CLEAVAGE POINTS

In this example, rare restriction enzymes are listed together with their recognition sequence and cleavage points.
W = A or T; N = A, C, G, or T
- 17 a): Unique, palindromic overhang

- AscI: GG^CGCG_CC
- AsiSI: GCG_AT^CGC
- CciNI: GC^GGCC_GC
- CspBI: GC^GGCC_GC
- FseI: GG_CCGG^CC
- MchAI: GC^GGCC_GC
- NotI: GC^GGCC_GC
- PacI: TTA_AT^TAA
- SbfI: CC_TGCA^GG
- SdaI: CC_TGCA^GG
- SgfI: GCG_AT^CGC
- SgrAI: CR^CCGG_YG
- Sse232I: CG^CCGG_CG
- Sse8387I: CC_TGCA^GG

- 17b): No overhang

- BstRZ246I: ATTT^AAAT
- BstSWI: ATTT^AAAT
- MspSWI: ATTT^AAAT
- MssI: GTTT^AAAC
- PmeI: GTTT^AAAC
- SmiI: ATTT^AAAT
- SrfI: GCCC^GGGC
- SwaI: ATTT^AAAT

- 17c): Non-palindromic and/or variable overhang

- AarI: CACCTGCNNNN^NNNN_
- AbeI: CC^TCA_GC
- AloI: "NNNNN_NNNNNNNGAACNNNNNNTCCNNNNNNN_NNNNN^
- BaeI: "NNNNN_NNNNNNNNNNACNNNNGTAYCNNNNNNN_NNNNN^
- BbvCI: CC^TCA_GC
- CpoI: CG^GWC_CG
- CspI: CG^GWC_CG
- Pf127I: RG^GWC_CY
- PpiI: ^NNNNN_NNNNNNNGAACNNNNNCTCNNNNNNNN_NNNNN^
- PpuMI: RG^GWC_CY
- PpuXI: RG^GWC_CY
- Psp5lI: RG^GWC_CY
- PspPPI: RG^GWC_CY
- RsrlI: CG^GWC_CG
- Rsr2I: CG^GWC_CG
- SanDI: GG^GWC_CC
- SapI: GCTCTTCN^NNN_
- SdiI: GGCCN_NNN^NGGCC
- SexAI: A^CCWGG_T
- SfiI: GGCCN_NNN^NGGCC
- Sse1825I: GG^GWC_CC
- Sse8647I: AG^GWC_CT
- VpaK32I: GCTCTTCN^NNN_

- 17d): Meganucleases

- I-Sce I I: TAGGGATAA_CAGG^GTAAT
- I-Ceu I: ACGGTC_CTAA^GGTAG
- I-Cre I: AAACGTC_GTGA^GACAGTTT
- I-Sce II: GGTC_ACCC^TGAAGTA
- I-Sce III: GTTTTGG_TAAC^TATTTAT
- Endo. Sce: I GATGCTGC_AGGC^ATAGGCTTGTTTA
- PI-Sce I: GG_GTGC^GGAGAA
- PI-Psp I: TGGCAAACAGCTA_TTAT^GGGTATTATGGGT
- I-Ppo I: CTCTC_TTAA^GGTAG
- HO: TTTCCGC_AACA^GT
- I-Tev I: NN_NN^NNTCAGTAGATGTTTTTCTTGGTCTACCGTTT

More meganucleases have been identified, but their precise sequence of recognition has not been determined, see e.g. www.meganuclease.com

### Example 18: Concatemer size limitation experiments (use of stoppers)

Materials used:
pYAC4 (Sigma. Burke et al. 1987, science, vol 236, p 806) was digested w. EcoR1 and BamH1 and dephosphorylated
pSE420 (invitrogen) was linearised using EcoR1 and used as the model fragment for concatenation.
T4 DNA ligase (Amersham-pharmacia biotech) was used for ligation according to manufacturers instructions.

Method: Fragments and arms were mixed in the ratios(concentrations are arbitrary units) indicated on figures. Ligation was allowed to proceed for 1 h at 16C. Reaction was stopped by the addition of 1 µL 500 mM EDTA. Products were analysed by standard agarose GE (1 % agarose, ½ strength TBE) or by PFGE(CHEF III, 1% LMP agarose, ½ strength TBE, angle 120, temperature 12 C, voltage 5.6V/cm, switch time ramping 5 - 25 s, run time 30 h)

The results are shown in Figure 17a and 17b.

### Example 19: Expression of different Patterns "phenotypes" obtained using the same yeast clones under different expression conditions

Colonies were picked with a sterile toothpick and streaked sequentially onto plates corresponding to the four repressed and/or induced conditions (-Ura/-Trp, -Ura/-Trp/-Met, -Ura/-Trp/+200 µM Cu₂SO₄, -Ura/-Trp/-Met/+200 µM Cu₂SO₄). The result is shown in Figure 24.

## Claims

1. A method for evolving a cell producing at least one compound, said compound having two or more predetermined functionalities, said method comprising the steps of
a) providing a composition of cells, said composition of cells comprising cells each with at least two heterologous expressible nucleotide sequences, at least one of said heterologous sequences being located on an artificial chromosome in said cell, at least 2 cells of the composition containing different heterologous expressible nucleotide sequences, said cells being denoted producer cells,
b) performing one screening of said population of cells for at least 2 parameters related to the functionalities, and determining a selection criterion for each parameter,
c) selecting cells meeting at least one predetermined selection criterion for each parameter,
d) combining expressible nucleotide sequences of the selected cells with each other and/or with expressible nucleotide sequences from another composition of cells, thereby obtaining at least one new composition of cells, said new composition of cells comprising cells with at least two heterologous expressible nucleotide sequences, at least one of said heterologous sequences being located on an artificial chromosome in said cell, at least 2 cells of the composition containing different heterologous expressible nucleotide sequences,
e) repeating steps b) to d) until at least one cell has acquired a compound having the at least two predetermined functionalities.

2. The method according to claim 1, wherein the compound is contained within said producer cell or the compound is excreted from said producer cell.

3. The method according to any of the preceding claims, wherein step c) comprises selecting cells meeting a predetermined selection criterion of more than two parameters.

4. The method according to any of the preceding claims, wherein at least one parameter is a parameter related to activity against a pharmacological target,

5. The method according to any of the preceding claims, wherein at least one parameter is a parameter related to activity against a pharmacological target, and at least one further parameter is related to an absorption, distribution, metabolism and/or excretion.

6. The method according to any of the preceding claims, wherein at least one parameter is a parameter related to a desired pharmacological property and another parameter is related to an undesired pharmacological property.

7. The method according to any of the preceding claims, wherein a parameter related to activity against a pharmacological target is inhibition or stimulation of growth of a reporter cell.

8. The method of claim 7, wherein the reporter cell is selected from a group consisting of bacteria, fungi, protozoa, helminth, algae, plants, invertebrates, vertebrates, mammalian cells, pathogenic microorganisms, agricultural pests, cells infected by an intracellular pathogen, virus-infected cells and tumor cells.

9. The method of claim 7, wherein the reporter cell is a whole live organism, e.g., bacteria, fungi, protozoa, algae, plants, invertebrates, insects, tardigrada, parasites or agricultural pests.

10. The method according to any of the preceding claims, wherein the producer cell has been transformed to produce at least one drug metabolising enzyme.

11. The method according to claim 10, wherein the drug metabolising enzyme is a Cytochrome P450, such as at least one P450 being selected from the group consisting of CYP1A2, CYP2B6, CYP2C19, CYP2C9, CYP2D6, CYP2E1, CYP3A4, CYP3A5, CYP3A7, CYP2C8, CYP2C18 and their allelic variants.

12. The method according to claim10, wherein the drug metabolising enzyme is placed outside the producer cells with the heterologous expressible nucleotide sequences, or in the reporter cell.

13. The method according to claim10, wherein at least one drug metabolising enzyme is placed outside the producer cells with the heterologous expressible nucleotide sequences, and at least one is placed inside said cells.

14. The method according to claim10, wherein at least two different drug metabolising enzyme are used, such as at least 3, for example at least 4, such as at least 5, for example at least 7, or such as at least 10.

15. The method according to any of the preceding claims, wherein one functionality is the ability of the compound to be taken up by a mammalian cell, including a human cell.

16. The method according to any of the preceding claims, wherein one functionality is toxicity.

17. The method according to claim 16, wherein toxicity is screened by using a cell proliferation assay

18. The method according to any of the preceding claims, wherein one functionality is mutagenicity.

19. The method according to any of the preceding claims, wherein one functionality is teratogenicity.

20. The method according to any of the preceding claims, wherein two or more functionalities are related to the same target, such as the same enzyme.

21. The method according to any of the preceding claims, comprising performing at least one screening for at least 6 parameters related to the two or more functionalities.

22. The method according to any of the preceding claims, wherein the stringency of the selection criteria/criterion is increased for at least some of the repeats.

23. The method according to any of the preceding claims, wherein the type of selection criteria/criterion is changed for at least some of the repeats.

24. The method according to any of the preceding claims, wherein one parameter related to the two or more functionalities in an early round of screening is a generic parameter, and one parameter related to the functionality in a later round is a more specific parameter.

25. The method according to any of the preceding claims, wherein the number of parameters screened for simultaneously increases with the number of screening rounds.

26. The method according to any of the preceding claims, wherein at least one screening is a medium based screen, such as using unusual substrates, growing cells on toxin comprising medium, or growing cells on inhibitor comprising medium.

27. The method according to any of the preceding claims, wherein at least one selection criterion is selection on the basis of survival, superior growth, deviating morphology, stickiness, spectral properties, or (modulation of) enzyme activity.

28. The method according to any of the preceding claims wherein at least one selection criterion is selected from at least one physical criterion, such as temperature, osmolarity, light, and electricity, leading to cells being selected on the basis of survival, superior growth, deviating morphology, stickiness, spectral properties, (modulation of) enzyme activity, activation of a receptor, or prevention of an activating molecule binding to a receptor.

29. The method according to any of the preceding claims, wherein at least one selection criterion is the capability of survival of cells when grown in media having increasing concentrations of a substance compared to concentrations the unmodified cell can survive.

30. The method according to any of the preceding claims, wherein the two or more functionalities are related to production of non-native metabolites, preferably secondary metabolites.

31. The method according to any of the preceding claims, comprising the use of a fluorescent analyser.

32. The method according to any of the preceding claims, wherein the selection of positive cells meeting the at least one selection criterion is performed by means of fluorescence activated cell sorters (FACS).

33. The method according to claim 32, wherein the sorting is based on a signal from an intracellular reporter system, said reporter system being native or heterologous to the producer cell.

34. The method of claim32, wherein at least two selection criteria are selected on by means of FACS, such as at least 3 criteria, for example at least 4 criteria, such as at least 5 criteria, for example at least 7 criteria, such as at least 10 criteria.

35. A method according to any of the preceding claims, wherein the selection of positive cells meeting the at least one selection criterion is performed by means of survival in the presence of other organisms.

36. A method according to any of the preceding claims, wherein the selection of positive cells meeting the at least one selection criterion is performed by means of having a growth advantage in media where growth requires the expression of reporter genes that when giving a desired response produces a compound vital for cell survival, e.g. antibiotic resistance marker, cell cycle control proteins, or auxotrophic markers.

37. The method according to any of the preceding claims 26 to36, wherein selection comprises use of any of the selection methods in any order.

38. The method according to any of the preceding claims 26 to36, wherein selection comprises any combination of the selection methods.

39. The method according to any of the preceding claims, wherein the producer cells are enclosed in a screening unit comprising reporter systems.

40. The method according to claim 39, wherein the producer cells are enclosed in gel droplets comprising agarose, polysaccharide, carbohydrate, alginate, carrageenan, chitosan, cellulose, pectin, dextran, or polyacrylamide.

41. The method according to claim 39, wherein the producer cells are enclosed in a layer essentially non-penetrable by the compounds being screened.

42. The method according to claim 39, wherein the non-penetrable material is a lipid material.

43. The method according to claim 39, wherein producer cells are encapsulated into gel droplets comprising reporter system(s) prior to sorting by FACS.

44. The method according to claim 39, wherein the producer cells and the reporter system(s) are encapsulated into one layer of gel droplets.

45. The method according to claim 39, wherein one layer comprises the producer cells and one or more reporter system(s), and a second layer comprises one or more different reporter system(s), and optionally a third or fourth or further layer comprises one or more reporter system(s).

46. The method according to claim 39, wherein the producer cell is encapsulated in one layer of the gel droplet and at least one reporter system is encapsulated in another layer of the same gel droplet.

47. The method according to any of the preceding claims, wherein the selection of positive cells meeting the at least one selection criterion is performed by means of an overlay assay, said overlay assay comprising reporter system(s), and manual or automatic picking of positive cells.

48. The method according to any of the preceding claims, wherein the selection of positive cells meeting the at least one selection criterion is performed by means of placing a single clonal producer cell line in one well of a microtiterplate, said well comprising reporter system(s), and manual or automatic picking of positive cells.

49. The method according to any of the preceding claims, wherein said reporter system is a cell based reporter system, being native to the reporter cell or being heterologous to the reporter cell.

50. The method according to any of the preceding claims, wherein said reporter system is a cell free reporter system.

51. The method according to any of the preceding claims, wherein the selection of positive cells meeting the at least one selection criterion is performed by means of plating cells on a medium, and manual or automatic picking of positive cells.

52. The method according to any of the preceding claims, wherein a composition of cells comprises at least 10³, preferably at least 10⁹ genetically different cells.

53. The method according to any of the preceding claims, wherein a composition of cells comprises a collection of cells from one species.

54. The method according to claim 1, wherein at least one producer cell comprises at least 10 different heterologous expressible nucleotide sequences, such as at least 15, for example at least 20, such as at least 25, for example at least 30, such as from 30 to 60 or more than 60, such as at least 75, for example at least 100, such as at least 200, for example at least 500, such as at least 750, for example at least 1000, such as at least 1500, or for example at least 2000.

55. The method according to claim 54, wherein any one producer cell comprises expressible nucleotide sequences coming from different expression states.

56. The method according to any of the preceding claims, wherein substantially all heterologous expressible nucleotide sequences are located on one or more artificial chromosomes.

57. The method according to any of the preceding claims, wherein the composition of cells comprises at least one cell, said at least one cell comprising:
a) at least two expression cassettes of the following formula: [rs₂-SP-PR-X-TR-SP-rs₁]
wherein
rs₁ and rs₂ together denotes a restriction site,
SP individually denotes a spacer,
PR denotes a promoter, capable of functioning in said at least one cell,
X denotes an expressible nucleotide sequence, and
TR denotes a terminator.

58. The method according to any of the preceding claims, wherein the at least two expressible nucleotide sequences are individually selected from the group consisting of genes and full-length cDNA sequences.

59. The method according to any of the preceding claims, wherein at least one cell of the composition comprises at least one concatemer of individual oligonucleotide cassettes, each concatemer comprising an oligonucleotide of the following formula in 5'→3' direction
[rs₂-SP-PR-X-TR-SP-rs₁]ₙ
wherein
rs₁ and rs₂ together denote a restriction site,
SP individually denotes a spacer of at least two nucleotide bases,
PR denotes a promoter, capable of functioning in said at least one cell,
X denotes an expressible nucleotide sequence,
TR denotes a terminator, and
wherein n ≥ 2, and
wherein at least two expressible nucleotide sequences are from different expression states.

60. The method according to claim 55 or59, wherein the at least two different expression states represent at least two different tissues, such as at least two organs, such as at least two species, or such as at least two genera.

61. The method according to claim 55 or59, wherein any one producer cell comprises expressible nucleotide sequences from at least 10 species.

62. The method according to claim 60, wherein the two different species are from at least two different phylae, such as from at least two different classes, such as from at least two different divisions, more preferably from at least two different sub-kingdoms, or such as from at least two different kingdoms.

63. The method according to claim 57 or59, wherein substantially all rs₁-rs₂ sequences are recognised by the same restriction enzyme, more preferably wherein substantially all rs₁-rs₂ sequences are substantially identical.

64. The method according to claim 57 or59, wherein n is at least 10, such as at least 15, for example at least 20, such as at least 25, for example at least 30, such as from 30 to 60 or more than 60, such as at least 75, for example at least 100, such as at least 200, for example at least 500, such as at least 750, for example at least 1000, such as at least 1500, or for example at least 2000.

65. The method according to any of the preceding claims, wherein the producer cell comprises a prokaryotic cell selected from the group consisting of bacteria such as Escherichia coli, Bacillus subtilis, Streptomyces lividans, Streptomyces coelicolor Pseudomonas aeruginosa, Myxococcus xanthus.

66. The method according to any of the preceding claims, wherein the producer cell comprises a eukaryotic cell selected from the group consisting of: yeasts; filamentous ascomycetes; plant cells and mammalian host cells.

67. The method according to claim 59, wherein the nucleotide sequence of at least one concatemer, preferably the nucleotide sequence from substantially all concatemers have been designed to minimise the level of repeat sequences in any one concatemer.

68. The method according to claim 1, wherein said another composition of cells in step (d) comprises cells that contain expressible nucleotide sequences likely to confer at least one of the functionalities to the cells.

69. The method according to claim 1, wherein said another composition of cells in step (d) was previously screened for a third functionality.

70. The method according to claim 1, wherein said another composition in step (d) comprises heterologous expressible nucleotide sequences from expression states known to produce compounds with at least one of the desired functionalities.

71. The method according to claim 1, wherein said another composition in step (d) comprises heterologous expressible nucleotide sequences that code for similar enzyme activities as heterologous expressible nucleotide sequences in the selected composition of cells.

72. The method according to claim 1, wherein said another composition of cells in step (d) comprises cells capable of expressing at least one predetermined protein/enzyme or synthesising at least one predetermined compound or substance.

73. The method according to claim 1, wherein said another composition of cells in step (d) is chosen at random.

74. The method according to claim 1, wherein said another composition in step (d) is identical to the selected composition and expressible nucleotide sequences are mixed to obtain new combinations of the selected expressible nucleotide sequences.

75. The method according to any of the preceding claims 1-73, wherein the combination of expressible sequences is a combination of artificial chromosomes in the cells.

76. The method according to claim 75, wherein said combination of chromosomes is obtained by a sexual cross between cells.

77. The method according to any of the preceding claims 1 to 76, wherein substantially all heterologous expressible nucleotide sequences are located on one or more artificial chromosomes.

78. The method according to any of the preceding claims 1 to 77, wherein the combination of heterologous expressible sequences is conducted by isolating the heterologous expressible sequences from at least two different cells, combining the individual heterologous expressible sequences into novel combinations, and introducing the combined heterologous expressible sequences into cells to obtain cells with at least 2 cells with different combinations.

79. The method according to any of the preceding claims 1 to 77, wherein the combination of heterologous expressible nucleotide sequences is conducted by amplifying expression cassettes by PCR, mixing amplified expression constructs, combining the individual expressible nucleotide sequences into novel combinations and introducing the combined heterologous expressible nucleotide sequences into cells to obtain cells with at least 2 cells with different combinations.

80. The method according to any of the preceding claims 1 - 79, wherein said composition is a collection of sub-compositions.

81. The method according to claim 80, wherein a sub-composition is a collection of individual cells having at least one phenotype in common.

82. The method according to claim 80, wherein each sub-composition comprises at least 10 genetically different cells.

83. A screening system comprising a producer cell comprising (i) two or more different heterologous expressible nucleotide sequences, at least one of said heterologous sequences being located on an artificial chromosome in said cell, and (ii) at least two reporter systems, wherein each of the reporter systems is directed to a parameter related to a functionality of one compound produced by the cell.

84. The screening system according to claim 83, comprising a producer cell encapsulated in a gel droplet together with the at least two reporter systems.

85. The screening system according to claim 83, comprising a producer cell in a liquid environment comprising the at least two reporter systems.

86. The screening system according to claim 83, wherein a reporter system is located within the producer cell

87. The screening system according to claim 83, wherein a reporter system is an extracellular reporter system, which is cell based or cell free.

88. The screening system according to claim 83, wherein the screening system is adapted for being used in a method as defined by any of the claims 1- 82.

89. A method for generation of lead compounds, said method comprising screening a composition of producer cells for at least two parameters related to at least two predetermined functionalities, said composition of cells comprising cells each with at least two heterologous expressible nucleotide sequences, at least one of said heterologous sequences being located on an artificial chromosome in said cell, at least 2 cells of the composition contain different heterologous expressible nucleotide sequences.

90. The method according to claim 89, wherein the lead compounds are drug lead compounds and at least one parameter is related to absorption, distribution, metabolism, excretion or toxicity and at least one further parameter is related to activity against a pharmacological target.

91. The method according to any of the preceding claims -89-90, wherein screening is as defined by any of the claims 1- 67.

## Patentansprüche

1. Verfahren zum Entwickeln einer Zelle, welche mindestens eine Verbindung erzeugt, wobei die Verbindung zwei oder mehr bestimmte Funktionalitäten aufweist, wobei das Verfahren die Schritte umfasst von
a) Bereitstellen einer Zell-Zusammensetzung, wobei die Zell-Zusammensetzung Zellen jeweils mit mindestens zwei heterolog exprimierbaren Nukleotidsequenzen umfasst, wobei mindestens eine der heterologen Sequenzen auf einem künstlichen Chromosom in der Zelle lokalisiert ist, wobei mindestens 2 Zellen der Zusammensetzung verschiedene heterolog exprimierbare Nukleotidsequenzen enthalten, wobei die Zellen Erzeugerzellen genannt werden,
b) Durchführen einer Durchmusterung besagter Zellpopulation auf mindestens 2 Parameter, welche die Funktionalitäten betreffen, und Bestimmen eines Auswahlkriteriums für jeden Parameter,
c) Auswählen von Zellen, welche mindestens ein bestimmtes Auswahlkriterium für jeden Parameter erfüllen,
d) Kombinieren exprimierbarer Nukleotidsequenzen der ausgewählten Zellen mit jeder anderen und/oder mit exprimierbaren Nukleotidsequenzen einer anderen Zell-Zusammensetzung, wodurch mindestens eine neue Zell-Zusammensetzung erhalten wird, wobei die neue Zell-Zusammensetzung Zellen mit mindestens zwei heterolog exprimierbaren Nukleotidsequenzen umfasst, wobei mindestens eine der heterologen Sequenzen auf einem künstlichen Chromosom in der Zelle lokalisiert ist, wobei mindestens 2 Zellen der Zusammensetzung verschiedene heterolog exprimierbare Nukleotidsequenzen enthalten,
e) Wiederholen der Schritte b) bis d) bis mindestens eine Zelle sich eine Verbindung mit den mindestens zwei bestimmten Funktionalitäten angeeignet hat.

2. Verfahren nach Anspruch 1, wobei die Verbindung in der Erzeugerzelle enthalten ist oder die Verbindung von der Erzeugerzelle abgesondert wird.

3. Verfahren nach einem der vorstehenden Ansprüche, wobei Schritt c) ein Auswählen von Zellen umfasst, die ein bestimmtes Auswahlkriterium von mehr als zwei Parametern erfüllen.

4. Verfahren nach einem der vorstehenden Ansprüche, wobei mindestens ein Parameter ein Parameter ist, welcher eine Aktivität gegen ein pharmakologisches Ziel betrifft.

5. Verfahren nach einem der vorstehenden Ansprüche, wobei mindestens ein Parameter ein Parameter ist, welcher eine Aktivität gegen ein pharmakologisches Ziel betrifft, und mindestens ein weiterer Parameter Absorption, Verteilung, Stoffwechsel und/oder Absonderung betrifft.

6. Verfahren nach einem der vorstehenden Ansprüche, wobei mindestens ein Parameter ein Parameter ist, welcher eine erwünschte pharmakologische Eigenschaft betrifft und ein anderer Parameter eine nicht erwünschte pharmakologische Eigenschaft betrifft.

7. Verfahren nach einem der vorstehenden Ansprüche, wobei ein Parameter, welcher eine Aktivität gegen ein pharmakologisches Ziel betrifft, Inhibierung oder Stimulierung des Wachstums einer Reporterzelle ist.

8. Verfahren nach Anspruch 7, wobei die Reporterzelle ausgewählt ist aus einer Gruppe bestehend aus Bakterien, Pilzen, Protozoen, Darmwurm, Algen, Pflanzen, Invertebraten, Vertebraten, Säugerzellen, pathogenen Mikroorganismen, Agrarschädlingen, durch ein intrazelluläres Pathogen infizierte Zellen, Virus-Infizierte Zellen und Tumorzellen.

9. Verfahren nach Anspruch 7, wobei die Reporterzelle ein ganzer lebender Organismus, beispielsweise Bakterien, Pilze, Protozoen, Algen, Pflanzen, Invertebraten, Insekten, Bärtierchen, Parasiten oder Agrarschädlinge, ist.

10. Verfahren nach einem der vorstehenden Ansprüche, wobei die Erzeugerzelle transformiert wurde, um mindestens ein Arzneimittel verstoffwechselndes Enzym zu erzeugen.

11. Verfahren nach Anspruch 10, wobei das Arzneimittel verstoffwechselnde Enzym ein Cytochrom P450 ist, wie beispielsweise mindestens ein P450 ausgewählt aus der Gruppe bestehend aus CYP1A2, CYP2B6, CYP2C19, CYP2C9 CYP2D6, CYP2E1, CYP3A4, CYP3A5, CYP3A7, CYP2C8, CYP2C18, und deren allelischen Varianten.

12. Verfahren nach Anspruch 10, wobei das Arzneimittel verstoffwechselnde Enzym außerhalb der Erzeugerzellen mit den heterolog exprimierbaren Nukleotidsequenzen oder in der Reporterzelle platziert ist.

13. Verfahren nach Anspruch 10, wobei mindestens ein ein Arzneimittel verstoffwechselndes Enzym außerhalb der Erzeugerzellen mit den heterolog exprimierbaren Nukleotidsequenzen platziert ist, und mindestens eines in den Zellen platziert ist.

14. Verfahren nach Anspruch 10, wobei mindestens zwei verschiedene Arzneimittel verstoffwechselnde Enzyme verwendet werden, wie beispielsweise mindestens 3, wie beispielsweise mindestens 4, wie beispielsweise mindestens 5, wie beispielsweise mindestens 7, oder wie beispielsweise mindestens 10.

15. Verfahren nach einem der vorstehenden Ansprüche, wobei eine Funktionalität der Verbindung die Befähigung ist, von einer Säugerzelle, einschließlich einer menschlichen Zelle, aufgenommen zu werden.

16. Verfahren nach einem der vorstehenden Ansprüche, wobei eine Funktionalität Toxizität ist.

17. Verfahren nach Anspruch 16, wobei nach Toxizität durch Verwenden eines Zellproliferationsassays durchmustert wird.

18. Verfahren nach einem der vorstehenden Ansprüche, wobei eine Funktionalität Mutagenität ist.

19. Verfahren nach einem der vorstehenden Ansprüche, wobei eine Funktionalität Teratogenität ist.

20. Verfahren nach einem der vorstehenden Ansprüche, wobei zwei oder mehr Funktionalitäten das gleiche Ziel, wie beispielsweise das gleiche Enzym, betreffen.

21. Verfahren nach einem der vorstehenden Ansprüche, umfassend ein Durchführen von mindestens einer Durchmusterung auf mindestens 6 Parameter, welche die zwei oder mehr Funktionalitäten betreffen.

22. Verfahren nach einem der vorstehenden Ansprüche, wobei die Stringenz der Auswahlkriterien/des Auswahlkriteriums für mindestens einige der Wiederholungen erhöht wird.

23. Verfahren nach einem der vorstehenden Ansprüche, wobei die Art der Auswahlkriterien/des Auswahlkriteriums für mindestens einige der Wiederholungen geändert wird.

24. Verfahren nach einem der vorstehenden Ansprüche, wobei ein Parameter, der die zwei oder mehr Funktionalitäten in einer frühen Durchmusterungsrunde betrifft, ein generischer Parameter ist, und ein Parameter, der die Funktionalitäten in einer späteren Runde betrifft, ein spezifischerer Parameter ist.

25. Verfahren nach einem der vorstehenden Ansprüche, wobei sich die Zahl der zu durchmusternden Parameter mit der Zahl der Durchmusterungsrunden gleichzeitig erhöht.

26. Verfahren nach einem der vorstehenden Ansprüche, wobei mindestens eine Durchmusterung eine auf einem Medium beruhende Durchmusterung, wie beispielsweise ein Verwenden unüblicher Substrate, Wachsen von Zellen auf Toxin umfassendem Medium, oder Wachsen von Zellen auf Inhibitor umfassendem Medium, ist.

27. Verfahren nach einem der vorstehenden Ansprüche, wobei mindestens ein Auswahlkriterium eine Auswahl auf der Grundlage von Überleben, überlegenen Wachstums, abweichender Morphologie, Klebrigkeit, Spektraleigenschaften oder (Modulierung einer) Enzymaktivität ist.

28. Verfahren nach einem der vorstehenden Ansprüche, wobei mindestens ein Auswahlkriterium ausgewählt ist aus mindestens einem physikalischen Kriterium, wie beispielsweise Temperatur, Osmolarität, Licht und Elektrizität, was zu Zellen führt, die ausgewählt werden auf der Grundlage von Überleben, überlegenen Wachstums, abweichender Morphologie, Klebrigkeit, Spektraleigenschaften, (Modulierung einer) Enzymaktivität, Aktivierung eines Rezeptors oder Verhindern eines Bindens eines aktivierenden Moleküls an einen Rezeptor.

29. Verfahren nach einem der vorstehenden Ansprüche, wobei mindestens ein Auswahlkriterium die Befähigung von Zellen zu überleben ist, sofern sie in Medien mit erhöhten Konzentrationen einer Substanz im Vergleich zu den Konzentrationen gezüchtet werden, welche die nicht modifizierte Zelle überleben kann.

30. Verfahren nach einem der vorstehenden Ansprüche, wobei die zwei oder mehr Funktionalitäten eine Erzeugung von nicht-nativen Metaboliten, vorzugsweise Sekundärmetaboliten, betreffen.

31. Verfahren nach einem der vorstehenden Ansprüche, umfassend die Verwendung eines Fluoreszenzmessgeräts.

32. Verfahren nach einem der vorstehenden Ansprüche, wobei die Auswahl von positiven Zellen, welche das mindestens eine Auswahlkriterium erfüllen, mittels fluoreszenzbasierter Durchflusszytometrie (FACS) durchgeführt wird.

33. Verfahren nach Anspruch 32, wobei das Sortieren auf einem Signal von einem intrazellulären Reportersystem beruht, worin das Reportersystem für die Erzeugerzelle nativ oder heterolog ist.

34. Verfahren nach Anspruch 32, wobei mindestens zwei Auswahlkriterien, wie beispielsweise mindestens 3 Kriterien, wie beispielsweise mindestens 4 Kriterien, wie beispielsweise mindestens 5 Kriterien, wie beispielsweise mindestens 7 Kriterien, wie beispielsweise mindestens 10 Kriterien, mittels FACS ausgewählt werden.

35. Verfahren nach einem der vorstehenden Ansprüche, wobei die Auswahl von positiven Zellen, welche das mindestens eine Auswahlkriterium erfüllen, mittels Überleben in der Gegenwart von anderen Organismen durchgeführt wird.

36. Verfahren nach einem der vorstehenden Ansprüche, wobei die Auswahl von positiven Zellen, welche das mindestens eine Auswahlkriterium erfüllen, mittels eines Aufweisens eines Wachstumsvorteils in Medien durchgeführt wird, worin ein Wachstum die Expression von Reportergenen erfordert, welche, sofern eine erwünschte Reaktion erfolgt, eine Verbindung erzeugen, welche für das Zellüberleben unerlässlich ist, wie beispielsweise einen Antibiotika Resistenzmarker, Steuerungsproteine des Zellzyklus oder auxotrophe Marker.

37. Verfahren nach einem der vorstehenden Ansprüche 26 bis 36, wobei die Auswahl die Verwendung von irgendeinem der Auswahlverfahren in irgendeiner Reihenfolge umfasst.

38. Verfahren nach einem der vorstehenden Ansprüche 26 bis 36, wobei die Auswahl irgendeine Kombination der Auswahlverfahren umfasst.

39. Verfahren nach einem der vorstehenden Ansprüche, wobei die Erzeugerzellen in einer Durchmusterungseinheit eingeschlossen sind, welche Reportersysteme umfasst.

40. Verfahren nach Anspruch 39, wobei die Erzeugerzellen in Geltröpfchen eingeschlossen sind, welche Agarose, Polysaccharid, Kohlenhydrat, Alginat, Carrageenan, Chitosan, Zellulose, Pektin, Dextran oder Polyacrylamid umfassen.

41. Verfahren nach Anspruch 39, wobei die Erzeugerzellen in einer Schicht eingeschlossen sind, welche für die zu durchmusternden Verbindungen im Wesentlichen undurchlässig ist.

42. Verfahren nach Anspruch 39, wobei das undurchlässige Material ein Lipid-Material ist.

43. Verfahren nach Anspruch 39, wobei Erzeugerzellen vor einem Sortieren mit FACS in Geltröpfchen eingekapselt werden, welche (ein) Reportersystem(e) umfassen.

44. Verfahren nach Anspruch 39, wobei die Erzeugerzellen und das (die) Reportersystem(e) in einer Schicht von Geltröpfchen eingekapselt sind.

45. Verfahren nach Anspruch 39, wobei eine Schicht die Erzeugerzellen und ein oder mehrere Reportersystem(e) umfasst, und eine zweite Schicht ein oder mehrere verschiedene Reportersystem(e) umfasst, und wahlweise eine dritte oder vierte oder weitere Schicht, welche ein oder mehrere Reportersystem(e) umfasst.

46. Verfahren nach Anspruch 39, wobei die Erzeugerzelle in einer Schicht des Geltröpfchens eingekapselt ist, und mindestens ein Reportersystem in einer anderen Schicht des gleichen Geltröpfchens eingekapselt ist.

47. Verfahren nach einem der vorstehenden Ansprüche, wobei die Auswahl der positiven Zellen, welche das mindestens eine Auswahlkriterium erfüllen, durch einen Überschichtungsassay durchgeführt wird, worin der Überschichtungsassay (ein) Reportersystem(e) und ein manuelles oder automatisches Picken positiver Zellen umfasst.

48. Verfahren nach einem der vorstehenden Ansprüche, wobei die Auswahl der positiven Zellen, welche das mindestens eine Auswahlkriterium erfüllen, durchgeführt wird durch, Überführen einer einzelnen klonalen Erzeugerzelllinie in eine Vertiefung einer Mikrotiterplatte, worin die Vertiefung (ein) Reportersystem(e) umfasst, und manuelles oder automatisches Picken positiver Zellen.

49. Verfahren nach einem der vorstehenden Ansprüche, wobei das Reportersystem ein zellbasierendes Reportersystem ist, welches für die Reporterzelle nativ ist oder für die Reporterzelle heterolog ist.

50. Verfahren nach einem der vorstehenden Ansprüche, wobei das Reportersystem ein zellfreies Reportersystem ist.

51. Verfahren nach einem der vorstehenden Ansprüche, wobei die Auswahl von positiven Zellen, welche das mindestens eine Auswahlkriterium erfüllen, durch Plattieren von Zellen auf einem Medium und manuellem oder automatischen Picken positiver Zellen durchgeführt wird.

52. Verfahren nach einem der vorstehenden Ansprüche, wobei eine Zell-Zusammensetzung mindestens 10³, vorzugsweise mindestens 10⁹, genetisch verschiedene Zellen umfasst.

53. Verfahren nach einem der vorstehenden Ansprüche, wobei eine Zell-Zusammensetzung eine Sammlung von Zellen einer Spezies umfasst.

54. Verfahren nach Anspruch 1, wobei mindestens eine Erzeugerzelle mindestens 10 verschiedene heterolog exprimierbare Nukleotidsequenzen, wie beispielsweise mindestens 15, wie beispielsweise mindestens 20, wie beispielsweise mindestens 25, wie beispielsweise mindestens 30, wie beispielsweise 30 bis 60 oder mehr als 60, wie beispielsweise mindestens 75, wie beispielsweise mindestens 100, wie beispielsweise mindestens 200, wie beispielsweise mindestens 500, wie beispielsweise mindestens 750, wie beispielsweise mindestens 1000, wie beispielsweise mindestens 1500, oder wie beispielsweise mindestens 2000, umfasst.

55. Verfahren nach Anspruch 54, wobei irgendeine Erzeugerzelle exprimierbare Nukleotidsequenzen umfasst, die von verschiedenen Epressionszuständen herrühren.

56. Verfahren nach einem der vorstehenden Ansprüche, wobei im Wesentlichen alle heterolog exprimierbaren Nukleotidsequenzen auf einem oder mehreren künstlichen Chromosomen lokalisiert sind.

57. Verfahren nach einem der vorstehenden Ansprüche, wobei die Zell-Zusammensetzung mindestens eine Zelle umfasst, worin die mindestens eine Zelle umfasst:
a) mindestens zwei Expressionskassetten der nachstehend aufgeführten Formel:
[rs₂-SP-PR-X-TR-SP-rs₁],
worin
rs₁ und rs₂ zusammen eine Restriktionsschnittstelle bezeichnen,
SP einzeln einen Abstandshalter bezeichnet,
PR einen Promoter bezeichnet, welcher in der mindestens einen Zelle wirken kann,
X bezeichnet eine exprimierbare Nukleotidsequenz, und
TR bezeichnet einen Terminator.

58. Verfahren nach einem der vorstehenden Ansprüche, wobei die mindestens zwei exprimierbaren Nukleotidsequenzen einzeln aus der Gruppe bestehend aus Genen und Volllängen cDNA Sequenzen ausgewählt sind.

59. Verfahren nach einem der vorstehenden Ansprüche, wobei mindestens eine Zelle der Zusammensetzung mindestens ein Konkatemer einzelner Oligonukleotid Kassetten umfasst, worin jedes Konkatemer ein Oligonukleotid der nachstehend aufgeführten Formel in 5'→3' Richtung umfasst
[rs₂-SP-PR-X-TR-SP-rs₁]ₙ,
worin
rs₁ und rs₂ zusammen eine Restriktionsschnittstelle bezeichnen,
SP einzeln einen Abstandshalter von mindestens zwei Nukleotidbasen bezeichnet,
PR einen Promoter bezeichnet, welcher in der mindestens einen Zelle wirken kann,
X bezeichnet eine exprimierbare Nukleotidsequenz, und
TR bezeichnet einen Terminator, und
worin n ≥ 2 ist, und
worin mindestens zwei exprimierbare Nukleotidsequenzen von verschiedenen Expressionszuständen herrühren.

60. Verfahren nach Anspruch 55 oder 59, wobei die mindestens zwei verschiedenen Expressionszustände mindestens zwei verschiedene Gewebe abbilden, wie beispielsweise mindestens zwei Organe, wie beispielsweise mindestens zwei Spezies, oder wie beispielsweise mindestens zwei Gattungen.

61. Verfahren nach Anspruch 55 oder 59, wobei irgendeine Erzeugerzelle exprimierbare Nukleotidsequenzen von mindestens 10 Spezies umfasst.

62. Verfahren nach Anspruch 60, wobei die zwei verschiedenen Spezies von mindestens zwei verschiedenen Stämmen stammen, wie beispielsweise von mindestens zwei verschiedenen Klassen, wie beispielsweise von mindestens zwei verschiedenen Einteilungen, bevorzugter von mindestens zwei verschiedenen Unter-Reichen, oder wie beispielsweise von mindestens zwei verschiedenen Reichen.

63. Verfahren nach Anspruch 57 oder 59, wobei im Wesentlichen alle rs₁-rs₂ Sequenzen durch das gleiche Restriktionsenzym erkannt werden, bevorzugter wobei im Wesentlichen alle rs₁-rs₂ Sequenzen im Wesentlichen gleich sind.

64. Verfahren nach Anspruch 57 oder 59, wobei n mindestens 10 beträgt, wie beispielsweise mindestens 15, wie beispielsweise mindestens 20, wie beispielsweise mindestens 25, wie beispielsweise mindestens 30, wie beispielsweise 30 bis 60 oder mehr als 60, wie beispielsweise mindestens 75, wie beispielsweise mindestens 100, wie beispielsweise mindestens 200, wie beispielsweise mindestens 500, wie beispielsweise mindestens 750, wie beispielsweise mindestens 1000, wie beispielsweise mindestens 1500, oder wie beispielsweise mindestens 2000.

65. Verfahren nach einem der vorstehenden Ansprüche, wobei die Erzeugerzelle eine prokaryotische Zelle umfasst, ausgewählt aus der Gruppe bestehend aus Bakterien, wie beispielsweise Escherichia coli, Bacillus subtilis, Streptomyces lividans, Streptomyces coelicolor, Pseudomonas aeruginosa, Myxococcus xanthus.

66. Verfahren nach einem der vorstehenden Ansprüche, wobei die Erzeugerzelle eine eukaryotische Zelle umfasst, ausgewählt aus der Gruppe bestehend aus Hefen, filamentösen Ascomyceten, Pflanzenzellen und Säugerwirtszellen.

67. Verfahren nach Anspruch 59, wobei die Nukleotidsequenz von mindestens einem Konkatemer, vorzugsweise die Nukleotidsequenz von im Wesentlichen allen Konkatemeren, dazu bestimmt ist, das Niveau von wiederholten Sequenzen in irgendeinem Konkatemer zu verringern.

68. Verfahren nach Anspruch 1, wobei die andere Zell-Zusammensetzung in Schritt (d) Zellen umfasst, welche exprimierbare Nukleotidsequenzen enthalten, welche mindestens eine der Funktionalitäten den Zellen wahrscheinlich verleihen.

69. Verfahren nach Anspruch 1, wobei die andere Zell-Zusammensetzung in Schritt (d) zuvor auf eine dritte Funktionalität durchmustert wurde.

70. Verfahren nach Anspruch 1, wobei die andere Zusammensetzung in Schritt (d) heterolog exprimierbare Nukleotidsequenzen von Expressionszuständen umfasst, von denen bekannt ist, dass sie Verbindungen mit mindestens einer der erwünschten Funktionalitäten erzeugen.

71. Verfahren nach Anspruch 1, wobei die andere Zusammensetzung in Schritt (d) heterolog exprimierbare Nukleotidsequenzen umfasst, welche für ähnliche Enzymaktivitäten wie heterolog exprimierbare Nukleotidsequenzen in der ausgewählten Zell-Zusammensetzung codieren.

72. Verfahren nach Anspruch 1, wobei die andere Zell-Zusammensetzung in Schritt (d) Zellen umfasst, welche mindestens ein bestimmtes Protein/Enzym exprimieren oder mindestens eine bestimmte Verbindung oder Substanz synthetisieren können.

73. Verfahren nach Anspruch 1, wobei die andere Zell-Zusammensetzung in Schritt (d) zufällig gewählt wird.

74. Verfahren nach Anspruch 1, wobei die andere Zusammensetzung in Schritt (d) mit der ausgewählten Zusammensetzung identisch ist und exprimierbare Nukleotidsequenzen beigemischt werden, um neue Kombinationen der ausgewählten exprimierbaren Nukleotidsequenzen zu erhalten.

75. Verfahren nach einem der vorstehenden Ansprüche 1-73, wobei die Kombination von exprimierbaren Sequenzen eine Kombination von künstlichen Chromosomen in den Zellen darstellt.

76. Verfahren nach Anspruch 75, wobei die Kombination von Chromosomen durch eine geschlechtliche Kreuzung zwischen Zellen erhalten wird.

77. Verfahren nach einem der vorstehenden Ansprüche 1 bis 76, wobei im Wesentlichen alle heterolog exprimierbaren Nukleotidsequenzen auf einem oder mehreren künstlichen Chromosomen lokalisiert sind.

78. Verfahren nach einem der vorstehenden Ansprüche 1 bis 77, wobei die Kombination der heterolog exprimierbaren Sequenzen durchgeführt wird durch, Isolieren der heterolog exprimierbaren Sequenzen von mindestens zwei verschiedenen Zellen, Kombinieren der einzelnen heterolog exprimierbaren Sequenzen in neue Kombinationen, und Einbringen der kombinierten heterolog exprimierbaren Sequenzen in Zellen, um Zellen zu erhalten, wobei mindestens 2 Zellen mit verschiedenen Kombinationen erhalten werden.

79. Verfahren nach einem der vorstehenden Ansprüche 1 bis 77, wobei die Kombination von heterolog exprimierbaren Nukleotidsequenzen durchgeführt wird durch, Amplifizieren von Expressionskassetten durch PCR, Mischen der amplifizierten Expressionskonstrukte, Kombinieren der einzelnen exprimierbaren Nukleotidsequenzen in neue Kombinationen und Einbringen der kombinierten heterolog exprimierbaren Nukleotidsequenzen in Zellen, um Zellen zu erhalten, wobei mindestens 2 Zellen mit verschiedenen Kombinationen erhalten werden.

80. Verfahren nach einem der vorstehenden Ansprüche 1 bis 79, wobei die Zusammensetzung eine Sammlung von Unter-Zusammensetzungen ist.

81. Verfahren nach Anspruch 80, wobei die Unter-Zusammensetzung eine Sammlung einzelner Zellen mit mindestens einem gemeinsamen Phänotyp ist.

82. Verfahren nach Anspruch 80, wobei jede Unter-Zusammensetzung mindestens 10 genetisch verschiedene Zellen umfasst.

83. Durchmusterungssystem, umfassend eine Erzeugerzelle, welche umfasst, (i) zwei oder mehr verschiedene heterolog exprimierbare Nukleotidsequenzen, worin mindestens eine der heterologen Sequenzen auf einem künstlichen Chromosom in der Zelle lokalisiert ist, und (ii) mindestens zwei Reportersysteme, worin jedes der Reportersysteme auf einen Parameter gerichtet ist, der eine Funktionalität einer durch die Zelle erzeugten Verbindung betrifft.

84. Durchmusterungssystem nach Anspruch 83, eine Erzeugerzelle umfassend, welche in einem Geltröpfchen zusammen mit den mindestens zwei Reportersystemen eingekapselt ist.

85. Durchmusterungssystem nach Anspruch 83, eine Erzeugerzelle in einer flüssigen Umgebung umfassend, welche die mindestens zwei Reportersysteme umfasst.

86. Durchmusterungssystem nach Anspruch 83, worin ein Reportersystem in der Erzeugerzelle lokalisiert ist.

87. Durchmusterungssystem nach Anspruch 83, worin ein Reportersystem ein extrazelluläres Reportersystem ist, welches zellbasierend oder zellfrei ist.

88. Durchmusterungssystem nach Anspruch 83, worin das Durchmusterungssystem für eine Verwendung in einem nach irgendeinem der Ansprüche 1 bis 82 festgelegten Verfahren angepasst ist.

89. Verfahren zur Erzeugung von Leitverbindungen, wobei das Verfahren ein Durchmustern einer Zusammensetzung von Erzeugerzellen auf mindestens zwei Parameter umfasst, welche mindestens zwei bestimmte Funktionalitäten betreffen, wobei die Zell-Zusammensetzung Zellen jeweils mit mindestens zwei heterolog exprimierbaren Nukleotidsequenzen umfasst, wobei mindestens eine der heterologen Sequenzen auf einem künstlichen Chromosom in der Zelle lokalisiert ist, wobei mindestens 2 Zellen der Zusammensetzung verschiedene heterolog exprimierbare Nukleotidsequenzen enthalten.

90. Verfahren nach Anspruch 89, wobei die Leitverbindungen Arzneimittel-Leitverbindungen sind und mindestens ein Parameter Absorption, Verteilung, Stoffwechsel, Absonderung oder Toxizität betrifft und mindestens ein weiterer Parameter eine Aktivität gegen ein pharmakologisches Ziel betrifft.

91. Verfahren nach einem der vorstehenden Ansprüche 89 bis 90, wobei Durchmusterung wie durch irgendeinen der Ansprüche 1 bis 67 festgelegt ist.

## Revendications

1. Procédé pour faire évoluer une cellule produisant au moins un composé, ledit composé ayant deux ou plusieurs fonctionnalités prédéterminées, ledit procédé comprenant les étapes consistant à :
a) fournir une composition de cellules, ladite composition de cellules comprenant des cellules avec chacune au moins deux séquences de nucléotides hétérologues exprimables, au moins une desdites séquences hétérologues étant située sur un chromosome artificiel dans ladite cellule, au moins deux cellules de la composition contenant différentes séquences de nucléotides hétérologues exprimables, lesdites cellules étant appelées cellules productrices,
b) réaliser un criblage de ladite population de cellules pour au moins deux paramètres liés aux fonctionnalités, et à déterminer un critère de sélection pour chaque paramètre,
c) choisir des cellules satisfaisant à au moins un critère de sélection prédéterminé pour chaque paramètre,
d) combiner les séquences de nucléotides exprimables des cellules choisies les unes avec les autres et/ou avec les séquences de nucléotides exprimables d'une autre composition de cellules, obtenant ainsi au moins une nouvelle composition de cellules, ladite nouvelle composition de cellules comprenant des cellules avec au moins deux séquences de nucléotides hétérologues exprimables, au moins une desdites séquences hétérologues étant située sur un chromosome artificiel de ladite cellule, au moins deux cellules de la composition contenant différentes séquences de nucléotides hétérologues exprimables,
e) répéter les étapes b) à d) jusqu'à ce qu'au moins une cellule ait acquits un composé ayant lesdites au moins deux fonctionnalités prédéterminées.

2. Procédé selon la revendication 1, dans lequel le composé est contenu dans ladite cellule productrice ou le composé est excrété à partir de ladite cellule productrice.

3. Procédé selon l'une des revendications précédentes, dans lequel l'étape c) consiste à choisir des cellules satisfaisant un critère de sélection prédéterminé de plus de deux paramètres.

4. Procédé selon l'une des revendications précédentes, dans lequel au moins un paramètre est un paramètre lié à l'activité contre une cible pharmacologique.

5. Procédé selon l'une des revendications précédentes, dans lequel au moins un paramètre est un paramètre lié à l'activité contre une cible pharmacologique, et au moins un autre paramètre est lié à une absorption, à une distribution, à un métabolisme et/ou à une excrétion.

6. Procédé selon l'une des revendications précédentes, dans lequel au moins un paramètre est un paramètre lié à une propriété pharmacologique souhaitée et un autre paramètre est lié à une propriété pharmacologique non souhaitée.

7. Procédé selon l'une des revendications précédentes, dans lequel un paramètre lié à une activité contre une cible pharmacologique est l'inhibition ou la stimulation de la croissance d'une cellule rapporteuse.

8. Procédé selon la revendication 7, dans lequel la cellule rapporteuse est choisie parmi un groupe constitué par des bactéries, des champignons, des protozoaires, des helminthes, des algues, des plantes, des invertébrés, des vertébrés, des cellules de mammifère, des microorganismes pathogènes, des pesticides agricoles, des cellules infectées par un pathogène intracellulaire, des cellules infectées par des virus et des cellules tumorales.

9. Procédé selon la revendication 7, dans lequel la cellule rapporteuse est un organisme vivant entier, par ex. une bactérie, un champignon, un protozoaire, une algue, une plante, un invertébré, un insecte, un tardigrade, un parasite ou un pesticide agricole.

10. Procédé selon l'une des revendications précédentes, dans lequel la cellule productrice a été transformée pour produire au moins une enzyme métabolisant un médicament.

11. Procédé selon la revendication 10, dans lequel l'enzyme métabolisant un médicament est un Cytochrome P450, tel qu'au moins un P450 choisi parmi le groupe constitué par CYP1A2, CYP2B6, CYP2C19, CYP2C9, CYP2D6, CYP2E1, CYP3A4, CYP3A5, CYP3A7, CYP2C8, CYP2C18 et leurs variantes alléliques.

12. Procédé selon la revendication 10, dans lequel l'enzyme métabolisant un médicament est placée hors des cellules productrices avec les séquences de nucléotides hétérologues exprimables, ou dans la cellule rapporteuse.

13. Procédé selon la revendication 10, dans lequel au moins une enzyme métabolisant un médicament est placée hors des cellules productrices avec les séquences de nucléotides hétérologues exprimables, et au moins une est placée dans lesdites cellules.

14. Procédé selon la revendication 10, dans lequel on utilise au moins deux enzymes différentes métabolisant un médicament, telles qu'au moins 3, par exemple au moins 4, telles qu'au moins 5, par exemple au moins 7, ou telles qu'au moins 10.

15. Procédé selon l'une des revendications précédentes, dans lequel une fonctionnalité est la capacité du composé à être assimilé par une cellule de mammifère, comprenant une cellule humaine.

16. Procédé selon l'une des revendications précédentes, dans lequel une fonctionnalité est la toxicité.

17. Procédé selon la revendication 16, dans lequel la toxicité est criblée en utilisant un dosage de la prolifération cellulaire.

18. Procédé selon l'une des revendications précédentes, dans lequel une fonctionnalité est la mutagénicité.

19. Procédé selon l'une des revendications précédentes, dans lequel une fonctionnalité est la tératogénicité.

20. Procédé selon l'une des revendications précédentes, dans lequel deux ou plusieurs fonctionnalités sont liées à la même cible, telle que la même enzyme.

21. Procédé selon l'une des revendications précédentes, comprenant l'étape consistant à réaliser au moins un criblage pour au moins 6 paramètres liés aux deux ou plusieurs fonctionnalités.

22. Procédé selon l'une des revendications précédentes, dans lequel la stringence du critère de sélection/du critère est augmentée pour au moins certaines des répétitions.

23. Procédé selon l'une des revendications précédentes, dans lequel le type de critère de sélection/critère est modifié pour au moins certaines des répétitions.

24. Procédé selon l'une des revendications précédentes, dans lequel un paramètre lié aux deux ou plusieurs fonctionnalités dans un cycle précoce de criblage est un paramètre générique, et un paramètre lié à la fonctionnalité dans un cycle tardif est un paramètre plus spécifique.

25. Procédé selon l'une des revendications précédentes, dans lequel le nombre de paramètres criblés augmente simultanément au nombre de cycles de criblage.

26. Procédé selon l'une des revendications précédentes, dans lequel au moins un criblage est un criblage en fonction d'un milieu, consistant par exemple à utiliser des substrats rares, à cultiver des cellules dans un milieu comprenant des toxines, ou à cultiver des cellules dans un milieu comprenant des inhibiteurs.

27. Procédé selon l'une des revendications précédentes, dans lequel au moins un critère de sélection est la sélection sur la base de la survie, de la croissance supérieure, d'une morphologie anormale, de l'aspect poisseux, des propriétés spectrales, ou de (la modulation de) l'activité enzymatique.

28. Procédé selon l'une des revendications précédentes, dans lequel au moins un critère de sélection est choisi parmi au moins un critère physique, tel que la température, l'osmolarité, la lumière, et l'électricité, conduisant à des cellules choisies sur la base de la survie, de la croissance supérieure, d'une morphologie anormale, de l'aspect poisseux, des propriétés spectrales, de (la modulation de) l'activité enzymatique, de l'activation d'un récepteur, ou de la prévention d'une liaison entre une molécule d'activation et un récepteur.

29. Procédé selon l'une des revendications précédentes, dans lequel au moins un critère de sélection est la capacité de survie des cellules cultivées dans des milieux ayant des concentrations croissantes d'une substance par rapport aux concentrations auxquelles peut survivre la cellule non modifiée.

30. Procédé selon l'une des revendications précédentes, dans lequel deux ou plusieurs fonctionnalités sont lisées à la production de métabolites non natifs, de préférence de métabolites secondaires.

31. Procédé selon l'une des revendications précédentes, comprenant l'utilisation d'un analyseur fluorescent.

32. Procédé selon l'une des revendications précédentes, dans lequel le choix des cellules positives satisfaisant ledit au moins un critère de sélection est réalisé au moyen de trieurs de cellules activées par fluorescence (FACS).

33. Procédé selon la revendication 32, dans lequel le tri est basé sur un signal provenant d'un système rapporteur intracellulaire, ledit système rapporteur étant natif ou hétérologue par rapport à la cellule productrice.

34. Procédé selon la revendication 32, dans lequel au moins deux critères de sélection sont évalués au moyen de FACS, tels qu'au moins 3 critères, par exemple au moins 4 critères, tels qu'au moins 5 critères, par exemple au moins 7 critères, tels qu'au moins 10 critères.

35. Procédé selon l'une des revendications précédentes, dans lequel le choix des cellules positives satisfaisant ledit au moins un critère de sélection est réalisé en fonction de la survie en présence d'autres organismes.

36. Procédé selon l'une des revendications précédentes, dans lequel le choix des cellules positives satisfaisant ledit au moins un critère de sélection est réalisé selon que les cellules ont un avantage de croissance dans les milieux où la croissance nécessite l'expression de gènes rapporteurs qui, en donnant une réponse souhaitée, produisent un composé nécessaire à la survie des cellules, par exemple un marqueur de la résistance aux antibiotiques, des protéines de contrôle du cycle cellulaire, ou des marqueurs auxotrophes.

37. Procédé selon l'une des revendications 26 à 36 précédentes, dans lequel la sélection comprend l'utilisation de l'un des procédés de sélection quel que soit ordre.

38. Procédé selon l'une des revendications 26 à 36 précédentes, dans lequel la sélection comprend n'importe quelle combinaison des procédés de sélection.

39. Procédé selon l'une des revendications précédentes, dans lequel les cellules productrices sont contenues dans une unité de criblage comprenant des systèmes rapporteurs.

40. Procédé selon la revendication 39, dans lequel les cellules productrices sont contenues dans des gouttelettes de gel comprenant l'agarose, le polysaccharide, le carbohydrate, l'alginate, la carraghénine, le chitosan, la cellulose, la pectine, le dextran, ou le polyacrylamide.

41. Procédé selon la revendication 39, dans lequel les cellules productrices sont contenues dans une couche essentiellement non pénétrable par les composés criblées.

42. Procédé selon la revendication 39, dans lequel le matériau non pénétrable est un matériau lipidique.

43. Procédé selon la revendication 39, dans lequel les cellules productrices sont encapsulées dans des gouttelettes de gel comprenant un ou des système(s) rapporteur(s) avant le tri par FACS.

44. Procédé selon la revendication 39, dans lequel les cellules productrices et le(s) système(s) rapporteur(s) sont encapsulés dans une couche de gouttelettes de gel.

45. Procédé selon la revendication 39, dans lequel une couche comprend les cellules productrices et un ou plusieurs système(s) rapporteur(s), et une seconde couche comprend un ou plusieurs système(s) rapporteur(s) différent(s), et éventuellement une troisième ou quatrième couche, ou plus, comprend un ou plusieurs système(s) rapporteur(s).

46. Procédé selon la revendication 39, dans lequel la cellule productrice est encapsulée dans une couche des gouttelettes de gel et au moins un système rapporteur est encapsulé dans une autre couche des mêmes gouttelettes de gel.

47. Procédé selon l'une des revendications précédentes, dans lequel le choix des cellules positives satisfaisant ledit au moins un critère de sélection est réalisé au moyen d'un test de recouvrement, ledit test de recouvrement comprenant un ou plusieurs système(s) rapporteur(s), et d'une collecte manuelle ou automatique des cellules positives.

48. Procédé selon l'une des revendications précédentes, dans lequel le choix des cellules positives satisfaisant ledit au moins un critère de sélection est réalisé en plaçant une seule lignée de cellules productrices clonales dans une cupule de microplaque, ladite cupule comprenant un ou plusieurs système(s) rapporteur(s), et en opérant une collecte manuelle ou automatique des cellules positives.

49. Procédé selon l'une des revendications précédentes, dans lequel ledit système rapporteur est un système rapporteur à base de cellules, natif ou hétérologue par rapport à la cellule rapporteuse.

50. Procédé selon l'une des revendications précédentes, dans lequel ledit système rapporteur est un système rapporteur exempt de cellules.

51. Procédé selon l'une des revendications précédentes, dans lequel le choix des cellules positives satisfaisant ledit au moins un critère de sélection est réalisé en disposant les cellules sur un milieu, et en opérant une collecte manuelle ou automatique des cellules positives.

52. Procédé selon l'une des revendications précédentes, dans lequel une composition des cellules comprend au moins 10³, de préférence au moins 10⁹, cellules génétiquement différentes.

53. Procédé selon l'une des revendications précédentes, dans lequel une composition des cellules comprend une collection de cellules d'une même espèce.

54. Procédé selon la revendication 1, dans lequel au moins une cellule productrice comprend au moins 10 séquences différentes de nucléotides hétérologues exprimables, telles qu'au moins 15, par exemple au moins 20, telles qu'au moins 25, par exemple au moins 30, telles que de 30 à 60 ou plus de 60, telles qu'au moins 75, par exemple au moins 100, telles qu'au moins 200, par exemple au moins 500, telles qu'au moins 750, par exemple au moins 1 000, telles qu'au moins 1 500, par exemple au moins 2 000.

55. Procédé selon la revendication 54, dans lequel l'une quelconque des cellules productrices comprend des séquences de nucléotides exprimables provenant de différents états d'expression.

56. Procédé selon l'une des revendications précédentes, dans lequel sensiblement toutes les séquences de nucléotides hétérologues exprimables sont situées sur un ou plusieurs chromosome(s) artificiel(s).

57. Procédé selon l'une des revendications précédentes, dans lequel la composition de cellules comprend au moins une cellule, ladite au moins une cellule comprenant :
a) au moins deux cassettes d'expression de formule suivante :
[rs₂-SP-PR-X-TR-SP-rs₁]
où
rs₁ et rs₂ désignent ensemble un site de restriction,
SP désigne individuellement un espaceur,
PR désigne un promoteur, capable de fonctionner dans ladite au moins une cellule,
X désigne une séquence de nucléotides exprimables, et
TR désigne un terminateur.

58. Procédé selon l'une des revendications précédentes, dans lequel lesdits au moins deux séquences de nucléotides expressibles sont individuellement choisies parmi le groupe constitué par les gènes et les séquences d'ADNc de pleine longueur.

59. Procédé selon l'une des revendications précédentes, dans lequel au moins une cellule de la composition comprend au moins un concatémère de cassettes d'oligonucléotides individuelles, chaque concatémère comprenant un oligonucléotide de formule suivante dans le sens 5'→3' :
[rs₂-SP-PR-X-TR-SP-rs₁]ₙ
où
rs₁ et rs₂ désignent ensemble un site de restriction,
SP désigne individuellement un espaceur d'au moins deux bases nucléotidiques,
PR désigne un promoteur, capable de fonctionner dans ladite au moins une cellule,
X désigne une séquence de nucléotides exprimables,
TR désigne un terminateur, et
où n≥2, et
où au moins deux séquences de nucléotides exprimables proviennent de différents états d'expression.

60. Procédé selon la revendication 55 ou 59, dans lequel lesdits au moins deux états d'expression différents représentent au moins deux tissus différents, tels qu'au moins deux organes, tels qu'au moins deux espèces, ou tels qu'au moins deux genres.

61. Procédé selon la revendication 55 ou 59, dans lequel l'une quelconque des cellules productrices comprend des séquences de nucléotides expressibles provenant d'au moins 10 espèces.

62. Procédé selon la revendication 60, dans lequel les deux espèces différentes proviennent d'au moins deux embranchements différents, tels que d'au moins deux classes différentes, tels que d'au moins deux divisions différentes, de façon plus préférée d'au moins deux sous-règnes différents, ou tels que d'au moins deux règnes différents.

63. Procédé selon la revendication 57 ou 59, dans lequel sensiblement toutes les séquences rs₁-rs₂ sont reconnues par la même enzyme de restriction, de façon plus préférée dans lequel sensiblement toutes les séquences rs₁-rs₂ sont sensiblement identiques.

64. Procédé selon la revendication 57 ou 59, dans lequel n est au moins 10, tel qu'au moins 15, par exemple au moins 20, tel qu'au moins 25, par exemple au moins 30, tel que de 30 à 60 ou plus de 60, tel qu'au moins 75, par exemple au moins 100, tel qu'au moins 200, par exemple au moins 500, tel qu'au moins 750, par exemple au moins 1 000, tel qu'au moins 1 500, par exemple au moins 2 000.

65. Procédé selon l'une des revendications précédentes, dans lequel la cellule productrice comprend une cellule procaryote choisie parmi le groupe constitué par les bactéries telles qu'Escherichia coli, Bacillus subtilis, Streptomyces lividans, Streptomyces coelicolor, Pseudomonas aeruginosa, Myxococcus xanthus.

66. Procédé selon l'une des revendications précédentes, dans lequel la cellule productrice comprend une cellule eucaryote choisie parmi le groupe constitué par les levures, les ascomycètes filamenteux, les cellules végétales et les cellules des parasites de mammifère.

67. Procédé selon la revendication 59, dans lequel la séquence de nucléotides d'au moins un concatémère, de préférence la séquence de nucléotides d'essentiellement tous les concatémères, a été conçue pour minimiser le niveau de séquences de répétition dans l'un quelconque des concatémères.

68. Procédé selon la revendication 1, dans lequel ladite autre composition de cellules de l'étape d) comprend des cellules qui contiennent des séquences de nucléotides expressibles susceptibles de conférer au moins une des fonctionnalités aux cellules.

69. Procédé selon la revendication 1, dans lequel ladite autre composition de cellules de l'étape d) a été préalablement criblée pour une autre fonctionnalité.

70. Procédé selon la revendication 1, dans lequel ladite autre composition de l'étape d) comprend des séquences de nucléotides hétérologues exprimables provenant d'états d'expression dont on sait qu'ils produisent des composés avec au moins une des fonctionnalités souhaitées.

71. Procédé selon la revendication 1, dans lequel ladite autre composition de l'étape d) comprend des séquences de nucléotides hétérologues exprimables qui codent pour des activités enzymatiques similaires à celles des séquences de nucléotides hétérologues exprimables dans la composition de cellules choisie.

72. Procédé selon la revendication 1, dans lequel ladite autre composition de cellules de l'étape d) comprend des cellules capables d'exprimer au moins une protéine/enzyme prédéterminée ou de synthétiser au moins un composé ou une substance prédéterminée.

73. Procédé selon la revendication 1, dans lequel ladite autre composition de l'étape d) est choisie de façon aléatoire.

74. Procédé selon la revendication 1, dans lequel ladite autre composition de l'étape d) est identique à la composition choisie et les séquences de nucléotides exprimables sont mélangées pour obtenir de nouvelles combinaisons des séquences de nucléotides exprimables choisies.

75. Procédé selon l'une des revendications 1 à 73 précédentes, dans lequel la combinaison des séquences exprimables est une combinaison de chromosomes artificiels dans les cellules.

76. Procédé selon la revendication 75, dans lequel ladite combinaison de chromosomes est obtenue par croisement sexué entre les cellules.

77. Procédé selon l'une des revendications 1 à 76 précédentes, dans lequel essentiellement toutes les séquences de nucléotides hétérologues exprimables sont situées sur un ou plusieurs chromosome(s) artificiel(s).

78. Procédé selon l'une des revendications 1 à 77 précédentes, dans lequel la combinaison de séquences hétérologues exprimables est réalisée en insolant les séquences hétérologues exprimables à partir d'au moins deux cellules différentes, en combinant les séquences hétérologues exprimables individuelles en de nouvelles combinaisons, et en introduisant les séquences hétérologues exprimables combinées dans des cellules pour obtenir des cellules avec au moins deux cellules avec différentes combinaisons.

79. Procédé selon l'une des revendications 1 à 77 précédentes, dans lequel la combinaison de séquences hétérologues exprimables est réalisée en amplifiant les cassettes d'expression par PCR, en mélangeant les produits d'expression amplifiés, en combinant les séquences exprimables individuelles en de nouvelles combinaisons, et en introduisant les séquences de nucléotides hétérologues exprimables combinées dans des cellules pour obtenir des cellules avec au moins deux cellules avec différentes combinaisons.

80. Procédé selon l'une des revendications 1 à 79 précédentes, dans lequel ladite composition est une collection de sous-compositions.

81. Procédé selon la revendication 80, dans lequel une sous-composition est une collection de cellules individuelles ayant au moins un phénotype en commun.

82. Procédé selon la revendication 80, dans lequel chaque sous-composition comprend au moins 10 cellules génétiquement différentes.

83. Système de criblage comprenant une cellule productrice comprenant (i) deux ou plusieurs séquences de nucléotides hétérologues exprimables différentes, au moins une desdites séquences hétérologues étant située sur un chromosome artificiel de ladite cellule, et (ii) au moins deux systèmes rapporteurs, dans lequel chacun des systèmes rapporteurs est dirigé vers un paramètre lié à une fonctionnalité d'un composé produit par la cellule.

84. Système de criblage selon la revendication 83, comprenant une cellule productrice encapsulée dans une gouttelette de gel avec lesdits au moins deux systèmes rapporteurs.

85. Système de criblage selon la revendication 83, comprenant une cellule productrice dans un environnement liquide comprenant lesdits au moins deux systèmes rapporteurs.

86. Système de criblage selon la revendication 83, dans lequel un système rapporteur est situé dans la cellule productrice.

87. Système de criblage selon la revendication 83, dans lequel un système rapporteur est un système rapporteur extracellulaire, qui est à base de cellules ou exempt de cellules.

88. Système de criblage selon la revendication 83, dans lequel le système de criblage est adapté pour être utilité dans un procédé tel que défini dans l'une des revendications 1 à 82.

89. Procédé de génération de composés têtes de série, ledit procédé comprenant les étapes consistant à cribler une composition de cellules productrices pour au moins deux paramètres liés à au moins deux fonctionnalités prédéterminées, ladite composition de cellules comprenant des cellules avec chacune au moins deux séquences de nucléotides hétérologues exprimables, au moins une desdites séquences hétérologues étant située sur un chromosome artificiel dans ladite cellule, au moins deux cellules de la composition contenant différentes séquences de nucléotides hétérologues exprimables.

90. Procédé selon la revendication 89, dans lequel les composés têtes de série sont des composés têtes de série de médicament et au moins un paramètre est lié à l'absorption, la distribution, le métabolisme, l'excrétion ou la toxicité et au moins un autre paramètre est lié à l'activité contre une cible pharmacologique.

91. Procédé selon l'une des revendications 89 et 90 précédentes, dans lequel le criblage est tel que défini dans l'une des revendications 1 à 67.
